# FASCICULE DE BREVET EUROPEEN

(11) **EP 4 176 082 B1**
(45) Date de publication et mention de la délivrance du brevet: **17.06.2026**
(21) Numéro de dépôt: 21746508.7
(22) Date de dépôt: 05.07.2021
(51) Int. Cl.: C12Q 1/6883

(54) **PROCÉDÉ POUR DÉTERMINER LE RISQUE DE COMPLICATION CHEZ UN PATIENT**
VERFAHREN ZUR BESTIMMUNG DES KOMPLIKATIONSRISIKOS FÜR EINEN PATIENTEN
METHOD TO DETERMINE THE RISK OF COMPLICATIONS FOR A PATIENT

(30) Priorité: 06.07.2020 FR 2007131
(43) Date de publication de la demande: 10.05.2023
(73) Titulaire: BIOMÉRIEUX, 69280 Marcy L'Etoile (FR); Bioaster, 69007 Lyon (FR)
(72) Inventeur: VACHOT, Laurence, 38600 Fontaine (FR); MALLET, François, 69100 VILLEURBANNE (FR); MONNERET, Guillaume, 69008 Lyon (FR); MOUCADEL, Virginie, 38360 Sassenage (FR); PACHOT, Alexandre, 01400 Sulignat (FR); PERONNET, Estelle, 69009 Lyon (FR); RIMMELÉ, Thomas, 69007 Lyon (FR); TEXTORIS, Julien, 69100 VILLEURBANNE (FR); VENET, Fabienne, 69008 Lyon (FR)
(74) Mandataire: Germain Maureau
(86) Numéro de dépôt international: PCT/FR2021/051228
(87) Numéro de publication internationale: WO 2022/008826

(56) Documents cités:
- WO-A1-2013/140103
- WO-A1-2016/145426
- WO-A1-2020/002602
- WO-A1-2020/096984
- WO-A2-2014/201516
- FR-A1- 3 085 689
- US-A1- 2009 203 534
- TIMOTHY E. SWEENEY ET AL: "A comprehensive time-course-based multicohort analysis of sepsis and sterile inflammation reveals a robust diagnostic gene set", SCIENCE TRANSLATIONAL MEDICINE, vol. 7, no. 287, 13 May 2015 (2015-05-13), US, pages 287ra71 - 287ra71, XP055649321, ISSN: 1946-6234, DOI: 10.1126/scitranslmed.aaa5993

## Description

La présente invention concerne un procédé *in vitro* ou *ex vivo* pour déterminer un risque de complication chez un patient, comprenant une étape de mesure de l'expression de C3AR1, dans un échantillon biologique dudit patient.

Certains patients peuvent être confrontés à des risques de complication, qu'il importe de pouvoir identifier aussi précocément que possible, pour une meilleure prise en charge de ces patients. Outre le risque de décès, le développement d'infections associées aux soins est une complication importante liée aux soins médicaux, en particulier dans les structures de soins médicalisées comme les hôpitaux (où l'on parlera plus précisément d'infections nosocomiales). Il a été montré que les infections nosocomiales en unités de soins intensifs, qui surviennent chez 20 à 40% des patients, étaient associées à une morbidité et une mortalité accrues, une durée plus longue du besoin en soins de suppléance aux défaillances d'organe(s), une durée de séjour plus longue à l'hôpital, des coûts de santé plus importants, et une utilisation plus importante d'antibiotiques, contribuant à la résistance antimicrobienne. L'occurrence des infections associées aux soins est particulièrement exacerbée depuis quelques années, de par l'augmentation des pathogènes multirésistants. L'Organisation Mondiale de la Santé (OMS) estime à environ 5 millions le nombre d'infections nosocomiales dans les hôpitaux en Europe, conduisant à environ 50 000 morts et un surcoût annuel de 13 à 24 milliards d'euros. De nombreux facteurs influent sur la survenue et le développement des infections associées aux soins, comme l'état général de santé du patient, mais également des facteurs liés à la prise en charge du patient (e.g. l'administration d'antibiotiques et/ou l'utilisation de dispositifs médicaux invasifs), des facteurs liés à l'environnement hospitalier (e.g. le ratio du nombre d'infirmières par rapport au nombre de patients), et l'utilisation variable des techniques aseptiques par le personnel hospitalier. Des recommandations ont été publiées, et la mise en place de programmes de contrôle des infections a été encouragée, en particulier par le *U.S. Department of Health and Human Services,* le Centre européen pour la prévention et le contrôle des maladies, l'OMS et les agences nationales, pour lesquels la prévention et la réduction des infections associées aux soins sont devenues une priorité majeure. Il a été montré que les programmes de contrôle des infections associées aux soins se révèlent particulièrement efficaces pour réduire les infections sévères. Cependant, il a été estimé qu'un maximum de 65 à 70% des cas d'infections du sang et des voies urinaires, liées à la pose de cathéters, et de 55% des cas de pneumonie associées à la ventilation mécanique et d'infections au niveau du site chirurgical, pourraient être évités. Par ailleurs, l'observance et l'application des procédures selon les recommandations peuvent être compliquées dans certains hôpitaux, particulièrement dans les pays à faibles ou moyens revenus. L'identification précoce des patients à risque de développer une infection associée aux soins serait une étape-clé dans la prévention de ces infections et la prise en charge de ces patients. D'après certaines modélisations, un biomarqueur qui réduirait le temps d'identification des infections associées aux soins chez une population à haut risque, permettrait de réduire la mortalité chez ces patients, avec un bon rapport coût/efficacité. Cependant, il n'existe actuellement pas en clinique de test de diagnostic *in vitro* permettant d'identifier les patients à haut risque de contracter une infection associée aux soins.

Le gène C3AR1 (*complement component 3a receptor 1*) code pour le récepteur du complément C3a, une protéine relarguée lors de l'activation du complément. La liaison de l'anaphylatoxin C3a sur son récepteur active la chimiotaxie et l'opsonisation des bactéries. Dans la littérature, il a été montré que le transcrit du gène C3AR1 était plus fortement exprimé chez les patients septiques que chez des volontaires sains (Napier et al. (2016), J. Exp. Med. 213(11):2365-2382). Par ailleurs, une liste de onze gènes comprenant C3AR1 a permis de distinguer des patients souffrant d'une inflammation stérile de patients développant une inflammation liée à une infection (Sweeney et al. (2015), Sci. Transl. Med. 7(287):287ra71). Ces études suggèrent donc une potentielle utilité de C3AR1 comme biomarqueur diagnostic.

Or, il a à présent été découvert que, de façon tout-à-fait surprenante, la mesure de l'expression du gène C3AR1 permettait de déterminer le risque de complication chez un patient. Cela n'avait jamais été montré ou suggéré dans la littérature. Les patients ayant un risque élevé de complication, en particulier ceux présentant un risque élevé de décès et/ou de contracter une infection associée aux soins, pourraient avantageusement bénéficier d'une prise en charge individualisée.

Ainsi, la présente invention a pour objet un procédé *in vitro* ou *ex vivo* pour déterminer un risque de survenue de complication chez un patient choisi parmi un risque de décès ou un risque de survenue d'une infection associée aux soins, comprenant une étape de mesure de l'expression du gène C3AR1, dans un échantillon biologique dudit patient. Dans le cadre de la présente invention :
- le terme « patient » désigne un individu (être humain), qui est entré en contact avec un professionnel de santé, tel qu'un médecin (par exemple, un médecin généraliste) ou une structure médicale ou un établissement de santé (par exemple, un hôpital, et plus particulièrement le service des urgences, le service de réanimation, une unité de soins intensifs ou une unité de soins continus, ou une structure médicalisée pour personnes âgées, de type EHPAD). Le patient peut par exemple être une personne âgée, dans le cadre d'un protocole de vaccination (notamment en EHPAD ou encore chez un médecin généraliste) ;
- le risque de complication correspond à un risque d'évolution défavorable de l'état de santé d'un patient, et inclut notamment le risque de survenue d'une infection associée aux soins (primaire ou secondaire), de réactivation virale, d'augmentation du score SOFA d'au moins un point, d'apparition d'au moins une défaillance d'organe, de la nécessité d'entrée aux urgences ou en réanimation ou encore de décès ;
- une infection est dite « associée aux soins », si elle survient au cours ou au décours d'une prise en charge (diagnostique, thérapeutique, palliative, préventive, éducative ou opératoire) d'un patient par un professionnel de santé, et si elle n'était pas présente au début de la prise en charge. Les infections associées aux soins (IAS) comprennent les infections contractées au sein d'un établissement de santé (dites infections nosocomiales) mais aussi lors de soins délivrés hors de ce cadre. Lorsque l'état infectieux au début de la prise en charge n'est pas connu précisément, un délai d'au moins 48 heures ou un délai supérieur à la période d'incubation est couramment accepté pour définir une IAS. Pour les infections du site opératoire, on considère habituellement comme associées aux soins les infections survenant dans les 30 jours suivant l'intervention ou, s'il y a mise en place d'un implant, d'une prothèse ou d'un matériel prothétique dans l'année qui suit l'intervention. L'infection peut être d'origine bactérienne, fongique ou encore virale. Il peut s'agir également de la réactivation de virus latents potentiellement pathogènes, tels les virus d'herpes, par exemple le CMV ;
- par « échantillon biologique », on se réfère à tout échantillon provenant d'un patient, et pouvant être de différentes natures, comme le sang ou ses dérivés, les expectorations, l'urine, les selles, la peau, le liquide céphalo-rachidien, le liquide de lavage broncho-alvéolaire, le liquide de ponction de la cavité abdominale, la salive, les sécrétions gastriques, le sperme, le liquide séminal, les larmes, la moelle épinière, ganglion du nerf trijumeau, tissu adipeux, tissu lymphoïde, tissu placentaire, tissu du tractus gastro-intestinal, tissu du tractus génital, tissu du système nerveux central. En particulier, cet échantillon peut être un fluide biologique, comme un échantillon de sang ou un échantillon dérivé du sang, qui peut notamment être choisi parmi le sang total (tel que collecté de la voie veineuse, c'est-à-dire contenant les cellules blanches et rouges, les plaquettes et le plasma), le plasma, le sérum, ainsi que tout(tous) type(s) de cellules extraites à partir du sang, telles que les cellules mononuclées sanguines périphériques (ou PBMC, contenant les lymphocytes (B, T et cellules NK), les cellules dendritiques et les monocytes), des sous-populations de cellules B, des monocytes purifiés, ou des neutrophiles ;
- par « mesure de l'expression de C3AR1» on désigne la mesure de l'expression du gène C3AR1, c'est-à-dire du gène codant la protéine C3AR1.

De préférence, dans le procédé tel que décrit précédemment :
- le patient est un patient au sein d'un établissement de santé, de préférence au sein d'un hôpital, de préférence encore au sein du service des urgences, du service de réanimation, en unité de soins intensifs ou en unité de soins continus ; de manière particulièrement préférée, le patient est un patient en état septique (plus particulièrement, en choc septique), un patient atteint de brûlures (plus particulièrement, de brûlures graves), un patient atteint de traumatismes (plus particulièrement, de traumatismes graves), ou un patient opéré par chirurgie (plus particulièrement, une chirurgie lourde) ; et
- le procédé permet de déterminer le risque de complication, incluant le risque de décès et/ou le risque de survenue d'une infection associée aux soins, plus particulièrement une infection nosocomiale, chez ledit patient.

Dans le cas d'un patient en état septique (déjà atteint d'une première infection), le procédé selon l'invention permet notamment de déterminer le risque de survenue d'une infection secondaire. Par patient en état septique (ou patient atteint de sepsis), on entend un patient présentant au moins une défaillance d'organe menaçant le pronostic vital et causé par une réponse inappropriée de l'hôte à une infection. Par choc septique, on entend un sous-type de sepsis, dans lequel une hypotension persiste, malgré un remplissage vasculaire adéquat.

Selon un mode de réalisation particulier, le risque de complication chez le patient est choisi parmi le risque de décès et le risque d'une infection associée aux soins. Selon ce mode de réalisation particulier, l'infection associée aux soins peut être une maladie nosocomiale.

De préférence, le procédé selon l'invention, tel que décrit précédemment, dans tous ses modes de réalisation, permet :
de déterminer le risque de décès chez un patient dans les 30 jours à partir du jour de l'agression immuno-inflammatoire (*i.e.* le traumatisme pour les patients atteints de traumatismes, la brûlure pour les patients atteints de brûlures, la chirurgie pour les patients opérés par chirurgie ou le diagnostic de sepsis pour les patients en état septique), c'est-à-dire au cours du 1^{er}, du 2^{ème}, du 3^{ème}, du 4^{ème}, du 5^{ème}, du 6^{ème}, du 7^{ème}, du 8^{ème}, du 9^{ème},du 10^{ème},du 11^{ème}, du 12^{ème}, du 13^{ème}, du 14^{ème},du 15^{ème},du 16^{ème}, du 17^{ème}, du 18^{ème}, du 20^{ème}, du 21^{ème},du 22^{ème}, du 23^{ème}, du 24^{ème}, du 25^{ème}, du 26^{ème}, du 27^{ème}, du 28^{ème}, du 29^{ème} ou du 30^{ème} jour à partir de l'agression immuno-inflammatoire (le 1^{er} jour correspondant ici au jour de survenue de l'agression immuno-inflammatoire),
et/ou de déterminer le risque de survenue d'une infection associée aux soins chez un patient :
   - dans les 30 jours à partir du jour de l'agression immuno-inflammatoire, de préférence encore dans les 15 jours à partir du jour de l'agression immuno-inflammatoire, c'est-à-dire au cours du 1^{er}, du 2^{ème}, du 3^{ème}, du 4^{ème}, du 5^{ème}, du 6^{ème}, du 7^{ème}, du 8^{ème}, du 9^{ème}, du 10^{ème}, du 11^{ème}, du 12^{ème}, du 13^{ème}, du 14^{ème} ou du 15^{ème} jour à partir de l'agression immuno-inflammatoire (le 1^{er} jour correspondant ici au jour de survenue de l'agression immuno-inflammatoire) ;
le prélèvement de l'échantillon biologique pouvant en particulier avoir été effectué au cours du 1^{er}, du 2^{ème}, du 3^{ème}, du 4^{ème}, du 5^{ème}, du 6^{ème}, du 7^{ème}, du 8^{ème}, du 9^{ème}, du 10^{ème}, du 11^{ème}, du 12^{ème}, du 13^{ème}, du 14^{ème} ou du 15^{ème} jour à partir de l'agression immuno-inflammatoire, de préférence au cours du 1^{er}, du 2^{ème}, du 3^{ème}, du 4^{ème}, du 5^{ème}, du 6^{ème} ou du 7^{ème} jour à partir de l'agression immuno-inflammatoire, de préférence encore au cours du 3^{ème}, du 4^{ème}, du 5^{ème}, du 6^{ème} ou du 7^{ème} jour à partir de l'agression immuno-inflammatoire ; et/ou
   - dans les 7 jours, dans les 6 jours, dans les 5 jours ou dans les 4 jours suivant le jour où le prélèvement de l'échantillon biologique a été effectué (quel que soit le jour où ce prélèvement a été effectué), c'est-à-dire au cours du 1^{er}, du 2^{ème}, du 3^{ème}, du 4^{ème}, du 5^{ème}, du 6^{ème} ou du 7^{ème} jour suivant le jour où le prélèvement de l'échantillon biologique a été effectué (le 1^{er} jour correspondant ici au lendemain du jour où le prélèvement de l'échantillon biologique a été effectué).

De préférence, le procédé tel que décrit précédemment, dans tous ses modes de réalisation, comprend en outre une étape de mesure, dans l'échantillon biologique du patient, de l'expression :
- d'au moins un gène sélectionné parmi la famille des gènes codant pour des molécules impliquées dans le système immunitaire inné ; et/ou
- d'au moins un gène sélectionné parmi la famille des gènes codant pour des molécules du cycle cellulaire ; et/ou
- d'au moins un gène sélectionné parmi la famille des gènes codant pour des cytokines ; et/ou
- d'au moins un gène sélectionné parmi la famille des gènes codant pour des cytokines anti-inflammatoires ; et/ou
- d'au moins un gène sélectionné parmi la famille des gènes codant pour des récepteurs à cytokines pro-inflammatoires, localisés dans le chromosome 2, au niveau de la région 2q11-2q12 ; et/ou
- d'au moins un gène sélectionné parmi la famille des gènes codant pour des cytokines pro-inflammatoires ; et/ou
- d'au moins un gène sélectionné parmi la famille des gènes codant pour des molécules impliquées dans la formation du cytosquelette ; et/ou
- d'au moins un gène sélectionné parmi la famille des gènes codant pour des molécules impliquées dans l'expression et/ou la transcription de gènes ; et/ou
- d'au moins un gène sélectionné parmi la famille des gènes codant pour des facteurs de croissance ; et/ou
- d'au moins un gène sélectionné parmi la famille des gènes codant pour des molécules du métabolisme ; et/ou
- d'au moins un gène sélectionné parmi la famille des gènes codant pour des molécules impliquées dans le système immunitaire adaptatif ; et/ou
- d'au moins un gène sélectionné parmi la famille des gènes codant pour des molécules impliquées dans la transduction du signal ; et/ou
- d'au moins un gène sélectionné parmi la famille des gènes codant pour des molécules impliquées dans la modulation de la phase aigüe ; et/ou
- d'au moins un Rétrovirus Endogène Humain (HERV).

Les molécules impliquées dans le système immunitaire inné sont bien connues de l'homme du métier. Le système immunitaire inné comprend l'ensemble des cellules et mécanismes permettant de façon immédiate et non spécifique la défense de l'organisme contre tout type d'agents infectieux. Les molécules impliquées dans le système immunitaire innée sont notamment les peptides anti-microbiens, le système du complément, l'interféron I-alpha et I-beta, et les protéines de la phase aiguë. A titre d'exemples de gènes codant pour des molécules impliquées dans le système immunitaire inné, nous pouvons citer les gènes suivants : GNLY, S100A9, ADGRE3, CD177, CX3CR1, IFIH1, OAS2, OAS3.

Les molécules du cycle cellulaire sont également bien connues de l'homme du métier. Elles sont impliquées dans la régulation d'au moins une des phases du cycle cellulaire, à savoir la phase G1, la phase S, la phase G2 et la phase M. Ainsi, il peut s'agir des molécules assurant l'exécution et la transition de chaque phase du cycle cellulaire, telles que les kinases cycline-dépendantes, ou des molécules assurant la surveillance de la bonne exécution de chaque phase. A titre d'exemples de gènes codant pour des molécules du cycle cellulaire, nous pouvons citer les gènes CCNB1IP1, CDKN1A, CDKN1B, CDKN1C, CDKN2B, CDKN2C et CDKN2D.

Les cytokines sont également des molécules bien connues de l'homme du métier. Ces molécules solubles sont synthétisées par un grand nombre de cellules, en particulier les lymphocytes et les macrophages, et sont impliquées dans le développement et la régulation des réponses immunitaires. Il existe différent types de cytokines, notamment les chimiokines, les interférons, les interleukines (cytokine pro-inflammatoire et anti-inflammatoire), les lymphokines et le facteur de nécrose tumorale (TNF). A titre d'exemples de gènes codant pour des cytokines, nous pouvons citer les gènes suivants : IL15, IL2, MCP1(CCL2), CXCL10. A titre d'exemples de gènes codant pour des cytokines anti-inflammatoires, nous pouvons citer les gènes suivants : IL10, IL1RN. A titre d'exemples de gènes codant pour des récepteurs à cytokines pro-inflammatoires, localisés dans le chromosome 2, au niveau de la région 2q11-2q12, nous pouvons citer les gènes suivants : IL18R1, IL1R2, IL1R1 et IL18RAP. A titre d'exemples de gènes codant pour des cytokines pro-inflammatoires, nous pouvons citer les gènes suivants : IFNG, IL1B, IL17A, IL18, IL6, TNF.

Les molécules impliquées dans la formation du cytosquelette sont également bien connues de l'homme du métier. Il s'agit notamment des molécules impliquées dans l'obtention des filaments protéiques constitutifs du cytosquelette, à savoir les microfilaments d'actine, les microtubules et les filaments intermédiaires. A titre d'exemples de gènes codant pour des molécules impliquées dans la formation du cytosquelette, nous pouvons citer les gènes suivants : ARL14EP, GSN.

Les molécules impliquées dans l'expression et/ou la transcription des gènes sont bien connues de l'homme du métier. Ces molécules permettent l'activité des gènes et définissent dans quelle mesure un gène est exprimé. A titre d'exemples de gènes codant pour des molécules impliquées dans l'expression et/ou la transcription de gènes, nous pouvons citer les gènes suivants : CIITA, DYRK2, GATA3, MDC1, NFKB1, RORC, STAT4, TBX21, TDRD9.

Les facteurs de croissance sont bien connus de l'homme du métier. Ce sont les molécules favorisant ou inhibant la croissance, la prolifération ou encore la différenciation des cellules. A titre d'exemple de gène codant pour des facteurs de croissance, nous pouvons citer le gène CSF2.

Les molécules du métabolisme sont bien connues de l'homme du métier. Il s'agit des molécules impliquées dans le catabolisme ou l'anabolisme des cellules. A titre d'exemples de gènes codant pour des molécules du métabolisme, nous pouvons citer les gènes suivants : ALOX5, BPGM, TRAP1.

Les molécules impliquées dans le système immunitaire adaptatif sont également des molécules bien connues de l'homme du métier. L'immunité adaptative ( ou acquise) est un des mécanismes d'action du système immunitaire. Le système immunitaire adaptatif est composé de cellules et de processus systémiques spécialisés qui éliminent spécifiquement chaque agent pathogène ou empêchent son développement. En particulier, l'immunité adaptative crée une mémoire immunologique après une réponse initiale à un pathogène spécifique, conduisant alors à une réponse renforcée lors de futures rencontres avec ce pathogène. A titre d'exemples de gènes codant pour des molécules impliquées dans le système immunitaire adaptatif, nous pouvons citer les gènes suivants : CD40LG, CD3D, BTLA, CD274, CTLA4, ICOS, PDCD1, TNFSF4, CD74, FCGR1A, LILRB2, TAP2.

Les molécules impliquées dans la transduction du signal sont aussi des molécules bien connues de l'homme du métier. La transduction est la deuxième étape de la cascade de signalisation qui fait suite à un signal extracellulaire et précède la réponse cellulaire interne ou externe. A titre d'exemples de gènes codant pour des molécules impliquées dans la transduction du signal, nous pouvons citer les gènes suivants : FLT1, HAVCR2, IL7R, ZAP70.

Les molécules impliquées dans la modulation de la phase aigüe sont également bien connues de l'homme du métier. La phase aigüe est une défense innée de l'organisme, en réponse à une inflammation, caractérisée par la stimulation ou l'inhibition de la synthèse et/ou de la sécrétion de protéines sanguines appelées protéines de la phase aiguë. A titre d'exemple de gène codant pour des molécules impliquées dans la modulation de la phase aigüe, nous pouvons citer le gène HP.

Les Rétrovirus Endogènes Humains (HERV) sont également connus de l'homme du métier. Les HERV sont subdivisés en trois grandes classes (classe I, classe Il et classe III) et plusieurs sous-groupes. Ce sont des rétroéléments qui se transposent dans un génome uniquement par un mode de copier-coller, par le biais d'un intermédiaire à ARN et d'une transcriptase inverse. A titre d'exemples de Rétrovirus Endogènes Humains (HERV), nous pouvons citer les HERV suivants : 190665001-HERV0376, 220247002-HERV0797, 050286701-HERV0513, 130360601-HERV0808, 021456001-MALR1017, 170369402HE41, 121601801-HERV0492, 011052202-HERV1033, 011052702-MALR1044.

**Tableau 1. Localisation chromosomique du gène C3AR1 et des autres biomarqueurs (gènes ou HERV) dont l'expression peut être mesurée en combinaison avec celle de C3AR1**

| **Biomarqueur (gène ou HERV)** | **Localisation chromosomique (GRCh38/hg38)** |
|---|---|
| **C3AR1** | **chr12:8,058,302-8,066,471** |
| ADGRE3 | chr19:14,619,117-14,690,027 |
| ALOX5 | chr10:45,374,176-45,446,121 |
| ARL14EP | chr11:30,323,099-30,338,458 |
| BPGM | chr7:134,646,779-134,679,816 |
| BTLA | chr3:112,463,966-112,499,702 |
| CCNB1IP1 | chr14:20,311,368-20,333,312 |
| CD177 | chr19:43,353,659-43,363,172 |
| CD40LG | chrX:136,648,177-136,660,390 |
| CD274 | chr9:5,450,381-5,470,567 |
| CD3D | chr11:118,338,954-118,342,744 |
| CD74 | chr5:150,400,041-150,412,936 |
| CIITA | chr16:10,866,212-10,943,021 |
| CSF2 | chr5:132,073,789-132,076,170 |
| CTLA4 | chr2:203,867,771-203,873,965 |
| CX3CR1 | chr3:39,263,494-39,281,735 |
| CXCL10 | chr4:76,021,116-76,023,536 |
| DYRK2 | chr12:67,648,338-67,665,406 |
| FCGR1A | chr1:149, 781,275-149, 792,518 |
| FLT1 | chr13:28,300,346-28,495,145 |
| GATA3 | chr10:8,045,378-8,075,201 |
| GNLY | chr2:85,685,175-85,698,854 |
| GSN | chr9:121,207,794-121,332,844 |
| HAVCR2 | chr5:157,085,832-157,142,869 |
| HP | chr16:72,054,592-72,061,056 |
| ICOS | chr2:203,936,731-203,961,579 |
| IFIH1 | chr2:162,267,074-162,318,764 |
| IFNG | ch r 12:68,154, 768-68,159, 741 |
| IL10 | chr1:206,767,602-206,774,607 |
| IL1B | chr2:112,829,751-112,836,903 |
| IL15 | chr4:141,636,583-141,733,987 |
| IL1RN | chr2:113,099,365-113,134,016 |
| IL1R1 | chr2:102,064,544-102,179,874 |
| IL1R2 | ch r2: 101,991,816-102,028,544 |
| IL17A | chr6:52,186,375-52,190,638 |
| IL18 | ch r11:112,143, 251-112, 164,117 |
| IL18RAP | chr2:102,418,558-102,452,568 |
| IL18R1 | chr2:102,311,529-102,398,777 |
| IL2 | chr4:122,451,470-122,456,725 |
| IL6 | chr7:22,725,442-22,732,002 |
| IL7R | chr5:35,852,695-35,879,603 |
| LILRB2 | chr19:54,273,812-54,281,184 |
| MDC1 | chr6:30,699,807-30,717,966 |
| MCP1 (CCL2) | chr17:34,255,218-34,257,203 |
| NFKB1 | chr4:102,501,329-102,617,302 |
| OAS2 | chr12:112,970,819-113,011,723 |
| OAS3 | chr12:112,938,433-112,973,251 |
| PDCD1 | chr2:241,849,881-241,858,908 |
| RORC | chr1:151,806,071-151,832,238 |
| S100A9 | chr1:153,357,854-153,361,027 |
| STAT4 | chr2:191,029,576-191,246,172 |
| TAP2 | chr6:32,821,833-32,838,823 |
| TBX21 | chr17:4 7, 733,236-47, 746,122 |
| TDRD9 | chr14:103,928,438-104,052,667 |
| TNF | chr6:31,575,565-31,578,336 |
| TNFSF4 | chr1:173,183,731-173,208,072 |
| TRAP1 | chr16:3,651,639-3,717,597 |
| ZAP70 | chr2:97,713,560-97,744,327 |
| 190665001-HERV0376 | chr19:54,891,074-54,891,496 |
| 220247002-HERV0797 | chr22:36,153,696-36,154,283 |
| 050286701-HERV0513 | chr5:14,551,189-14,551,685 |
| 130360601-HERV0808 | chr13:42,884,951-42,886,257 |
| 021456001-MALR1017 | chr2:102,013,616-102,013,971 |
| 170369402HE41 | chr17:35,505,737-35,508,365 |
| 121601801-HERV0492 | chr12:112,971,073-112,971,451 |
| 011052202-HERV1033 | chr1:78,623,489-78,623,954 |
| 011052702-MALR1044 | chr1:78,648,318-78,648,697 |

**Tableau 2. Classification des biomarqueurs pouvant être utilisés en combinaison avec C3AR1, en familles et selon leur fonction immunitaire. Le lieu d'expression (type cellulaire sanguin) et la localisation sont également indiqués.**

| **Familles** | **Biomarqueurs** | **Lieu d'expression (type cellulaire sanguin)** | **Localisation** | **Fonction immunitaire** |
|---|---|---|---|---|
| Famille des molécules impliquées dans le système immunitaire inné | GNLY | Lymphocytes T gamma delta (γδ), Lympocytes T CD4 et T CD8 | Intracellulaire, Sécrété (différentes isoformes) | Alarmin/defensin |
| | S100A9 | Neutrophiles, Monocytes classiques | Intracellulaire, Sécrété (différentes isoformes) | Alarmin/defensin |
| | ADGRE3 | Basophiles, Monocytes | Membrane, Sécrété (differentes isoformes) | Migration/activation/dégranulation des neutrophiles |
| | CD177 | Neutrophiles | Intracellulaire | Migration/activation/dégranulation des neutrophiles |
| | CX3CR1 | Monocytes, Lymphocytes NK, Lympocytes T CD4 et T CD8 | Membrane | Migration/activation/dégranulation des neutrophiles |
| | IFIH1 | Tout type cellulaire (Neutrophiles) | Intracellulaire | Voies des interférons |
| | OAS2 | Tout type cellulaire (Monocytes, Plasmablastes) | Intracellulaire | Voies des interférons |
| | OAS3 | Neutrophiles, Cellules dendritiques myéloïdes, Monocytes | Intracellulaire | Voies des interférons |
| Famille des molécules du cycle cellulaire | CCNB1IP1 | Tout type cellulaire (Monocytes, Neutrophiles, Basophiles) | Intracellulaire | Cycle cellulaire |
| Famille des cytokines | IL15 | Monocytes, Lymphocytes B SM, Lymphocytes MAIT | Intracellulaire, Sécrété (différentes isoformes) | Activation/prolifération des cellules T |
| | IL2 | Lymphocytes T auxiliaires Th2 et Th17, Lymphocytes T CD8, Lymphocytes T centraux-mémoires (CM) | Sécrété | Activation/prolifération des cellules T |
| | MCP1 (CCL2) | Monocytes, Lymphocytes B naïfs | Sécrété | Recrutement des monocytes |
| | CXCL10 | Monocytes | Sécrété | Transduction du signal |
| Famille des cytokines anti-inflammatoires | IL10 | Cellules dendritiques myéloïdes, Monocytes, Lymphocytes T folliculaires helper, Lymphocytes T auxiliaires Th1, lymphocytes T régulateurs, Lymphocytes T gamma delta (γδ) | Membrane, Sécrété (différentes isoformes) | Cytokine anti-inflammatoire |
| | IL1RN | Neutrophiles, Monocytes | Membrane, Sécrété (differentes isoformes) | Cytokine anti-inflammatoire |
| Famille des récepteurs à cytokines pro-inflammatoires | IL1R2 | Neutrophiles | Membrane, Sécrété (differentes isoformes) | Récepteur à cytokines pro-inflammatoires |
| | IL18RAP | Neutrophiles, Lymphocytes NK, Lympocytes T CD4, Lymphocytes MAIT, Lymphocytes T gamma delta (γδ) | Intracellulaire, Sécrété (differentes isoformes) | Récepteur à cytokines pro-inflammatoires |
| | IL18R1 | Cellules dendritiques myéloïdes, Cellules | Intracellulaire, Sécrété (differentes isoformes) | Récepteur à cytokines pro-inflammatoires |
| | | dendritiques plasmacytoïdes, Basophiles, Neutophiles, Lymphocytes T, Lymphocytes NK | | |
| | IL1R1 | Neutrophile, Lymphocytes T CD4 mémoire, Lymphocytes T régulateurs mémoire | Membrane, Sécrété (differentes isoformes) | Récepteur à cytokines pro-inflammatoires |
| Famille des cytokines pro-inflammatoires | IFNG | Lymphocytes T gamma delta (γδ), Lympocytes T CD4 et T CD8, Lymphocyte NK | Sécrété | Voie des interférons |
| | IL1B | Cellules dendritiques myéloïdes, Monocytes, Neutrophiles | Intracellulaire, Sécrété (differentes isoformes) | Cytokine pro-inflammatoire |
| | IL17A | Lymphocytes T folliculaires helper, Lymphocytes T | Sécrété | Cytokine pro-inflammatoire |
| | | auxiliaires Th17, Lymphocytes T régulateurs | | |
| | IL18 | Cellules dendritiques myéloïdes, Monocytes, Progéniteurs, Cellules dendritiques plasmacytoïdes, Basophiles, Neutophiles | Intracellulaire, Sécrété (différentes isoformes) | Cytokine pro-inflammatoire |
| | IL6 | Monocytes, Lymphocytes B naïfs | Intracellulaire, Sécrété (differentes isoformes) | Cytokine pro-inflammatoire |
| | TNF | Monocytes | Intracellulaire, Sécrété (differentes isoformes) | Cytokine pro-inflammatoire |
| Famille des molécules impliquées dans la formation du cytosquelette | ARL14EP | Tout type cellulaire sauf neutrophiles | Intracellulaire | Interaction/export complexe majeur d'histocompatibilité de classe II |
| | GSN | Tout type cellulaire (Neutrophiles, Lymphocytes B) | Intracellulaire, Sécrété (differentes isoformes) | Régulation des filaments d'actine |
| Famille des molécules impliquées dans l'expression et/ou la transcription de gènes | CIITA | Cellules dendritiques myéloïdes, Monocytes, Lymphocytes B | Intracellulaire | Présentation de l'antigène |
| | DYRK2 | Tout type cellulaire (Lymphocytes T) | Intracellulaire, Sécrété (differentes isoformes) | Croissance cellulaire |
| | GATA3 | Lymphocytes T auxiliaires Th2, Lymphocytes T régulateurs | Intracellulaire | Activation/prolifération des cellules T |
| | MDC1 | Tout type cellulaire sauf neutrophiles | Intracellulaire | Réparation de l'ADN |
| | NFKB1 | Tout type cellulaire (Cellules dendritiques myéloïdes, Monocytes, Neutrophiles) | Intracellulaire | Expression/transcription des gènes |
| | RORC | Lymphocytes MAIT, Lymphocytes T auxiliaires Th17 | Intracellulaire | Activation/prolifération des cellules T |
| | STAT4 | Lymphocytes T, Lymphocytes NK | Intracellulaire | Activation/prolifération des cellules T |
| | TBX21 | Lymphocytes NK, Lymphocytes T CD4 et T CD8, Lymphocytes MAIT, Lymphocytes T gamma delta (γδ) | Intracellulaire | Activation/prolifération des cellules T |
| | TDRD9 | Basophiles, Monocytes classiques | Intracellulaire | Expression/transcription des gènes |
| Famille des facteurs de croissance | CSF2 | Lympocytes NK, Cellules myéloïdes, Cellules lymphoïdes | Sécrété | Croissance cellulaire |
| Famille des molécules du métabolisme | ALOX5 | Basophiles, Neutrophiles, Monocytes, Lymphoctes B | Intracellulaire | Métabolisme des acides gras |
| | BPGM | Tout type cellulaire | Intracellulaire | Métabolisme des carbohydrates |
| | TRAP1 | Tout type cellulaire | Intracellulaire | Respiration mitochondriale |
| Famille des molécules impliquées dans le système immunitaire adaptatif | CD40LG | Lymphocytes T ( sauf T CD8) | Membrane, Sécrété (différentes isoformes) | Activation/prolifération des cellules T |
| | CD3D | Lymphocytes T | Intracellulaire, Sécrété (différentes isoformes) | Activation/prolifération des cellules T |
| | BTLA | Lymphocytes B, Lymphocytes B naïfs | Intracellulaire, Sécrété (différentes isoformes) | Costimulation par la famille CD28/inhibiteur de check point |
| | CD274 | Basophiles, Neutrophiles, Lymphocytes T | Membrane | Costimulation par la famille CD28/inhibiteur de check point |
| | CTLA4 | Lymphocytes T régulateurs, Lymphocytes T CD4 | Intracellulaire, Sécrété (différentes isoformes) | Costimulation par la famille CD28/inhibiteur de check point |
| | ICOS | Lymphocytes T | Membrane | Costimulation par la famille CD28/inhibiteur de check point |
| | PDCD1 | Lymphocytes T CD8 EM, Lymphocytes T | Intracellulaire, Sécrété (différentes isoformes) | Costimulation par la famille CD28/inhibiteur de check point |
| | TNFSF4 | Progéniteurs | Intracellulaire | Costimulation par la famille CD28/inhibiteur de check point |
| | CD74 | Cellules dendritiques myéloïdes, Monocytes, Lymphocytes B, Cellules dendritiques plasmacytoïde | Membrane | Présentation de l'antigène |
| | FCGR1A | Neutrophiles, Monocytes | Membrane | Présentation de l'antigène |
| | LILRB2 | Neutrophiles, Monocytes | Membrane | Présentation de l'antigène |
| | TAP2 | Tout type cellulaire (neutrophiles) | Membrane | Présentation de l'antigène |
| Famille des molécules impliquées dans la transduction du signal | FLT1 | Neutrophiles, Cellules dendritiques plasmacytoïde | Intracellulaire, Membrane, Sécrété (différentes isoformes) | Récepteur tyrosine kinases |
| | HAVCR2 | Cellules dendritiques myéloïdes, Monocytes, Lymphocytes NK | Intracellulaire, Sécrété (différentes isoformes) | Récepteur |
| | IL7R | Lymphocytes T | Intracellulaire, Sécrété (différentes isoformes) | Récepteur de cytokine |
| | ZAP70 | Lymphocytes NK, Lymphocytes T | Intracellulaire | Activation/prolifération des cellules T |
| Famille des molécules impliquées dans la modulation de la phase aigüe | HP | Monocytes, Neutrophiles, Basophiles | Intracellulaire, Sécrété (différentes isoformes) | Récepteur (liaison et absorption du ligand par récepteur de piégeage / dégranulation des neutrophiles) |

Aussi, de préférence, le procédé tel que décrit précédemment, dans tous ses modes de réalisation, comprend, outre l'étape de mesure de l'expression de C3AR1, une étape de mesure, dans l'échantillon biologique du patient, de l'expression :
- d'au moins un, deux, trois, quatre, cinq, six, sept, huit gène(s) sélectionné(s) parmi les gènes suivants : GNLY, S100A9, ADGRE3, CD177, CX3CR1, IFIH1, OAS2 et OAS3 ; et/ou
- d'au moins un, deux, trois, quatre, cinq, six, sept gène(s) sélectionné(s) parmi les gènes suivants : CCNB1IP1, CDKN1A, CDKN1B, CDKN1C, CDKN2B, CDKN2C et CDKN2D ; et/ou
- d'au moins un, deux, trois, quatre gène(s) sélectionné(s) parmi les gènes suivants : IL15, IL2, MCP1(CCL2) et CXCL10 ; et/ou
- d'au moins un, deux gène(s) sélectionnés parmi les gènes suivants : IL10 et IL1RN ; et/ou
- d'au moins un, deux, trois, quatre gène (s) sélectionné(s) parmi les gènes suivants : IL18R1, IL1R2, IL1R1 et IL18RAP ; et/ou
- d'au moins un, deux, trois, quatre, cinq, six gène(s) sélectionné(s) parmi les gènes suivants : IFNG, IL1B, IL17A, IL18, IL6 et TNF ; et/ou
- d'au moins un, deux gène(s) sélectionné(s) parmi les gènes suivants : ARL14EP et GSN ; et/ou
- d'au moins un, deux, trois, quatre, cinq, six, sept, huit, neuf gène(s) sélectionné(s parmi les gènes suivants : CIITA, DYRK2, GATA3, MDC1, NFKB1, RORC, STAT4, TBX21 et TDRD9 ; et/ou
- du gène CSF2 ; et/ou
- d'au moins un, deux, trois, quatre, cinq gène(s) sélectionné(s) parmi les gènes suivants : ALOX5, BPGM et TRAP1 ; et/ou
- d'au moins un, deux, trois, quatre, cinq, six, sept, huit, neuf, dix, onze, douze gène(s) sélectionné(s) parmi les gènes suivants : CD40LG, CD3D, BTLA, CD274, CTLA4, ICOS, PDCD1, TNFSF4, CD74, FCGR1A, LILRB2 et TAP2 ; et/ou
- d'au moins un, deux, trois, quatre gène(s) sélectionné(s) parmi les gènes suivants : FLT1, HAVCR2, IL7R et ZAP70 ; et/ou
- du gène HP ; et/ou
- d'au moins un, deux, trois, quatre, cinq, six, sept, huit, neuf HERV sélectionné(s) parmi les HERV suivants : 190665001-HERV0376, 220247002-HERV0797, 050286701-HERV0513, 130360601-HERV0808, 021456001-MALR1017, 170369402HE41, 121601801-HERV0492, 011052202-HERV1033 et 011052702-MALR1044.

De préférence encore, le procédé tel que décrit précédemment, dans tous ses modes de réalisation, comprend, outre l'étape de mesure de l'expression de C3AR1, une étape de mesure, dans l'échantillon biologique du patient, de l'expression d'au moins un, deux, trois, quatre, cinq, six, sept, huit, neuf, dix, onze, douze, treize, quatorze, quinze, seize, dix-sept, dix-huit, dix-neuf, vingt, vingt-et-un, vingt-deux, vingt-trois, vingt-quatre, vingt-cinq, vingt-six gène(s) sélectionné(s) parmi les gènes suivants : GNLY, S100A9, ADGRE3, CD177, CX3CR1, OAS2, CCNB1IP1, IL10, IL1RN, IL1R2, IFNG, TNF, ARL14EP, CIITA, GATA3, MDC1, TDRD9, BPGM, CD3D, CD274, CTLA4, CD74, TAP2, IL7R, ZAP70 et HP.

De préférence encore, le procédé tel que décrit précédemment, dans tous ses modes de réalisation, comprend, outre l'étape de mesure de l'expression de C3AR1, une étape de mesure, dans l'échantillon biologique du patient, de l'expression d'au moins un, deux, trois, quatre, cinq, six, sept, huit, neuf, dix, onze gène(s) sélectionné(s) parmi les gènes suivants : S100A9, CD177, CX3CR1, IL1R2, IFNG, CIITA, CD3D, CTLA4, CD74, TAP2et HP.

Des combinaisons particulièrement préférées d'un ou plusieurs autre(s) gène(s), dont l'expression peut être mesurée en combinaison avec l'expression de C3AR1, sont présentées dans le tableau 3.

**Tableau 3. Liste de combinaisons préférées de 1 à 11 autre(s) gène(s), dont l'expression peut être mesurée en combinaison avec l'expression de C3AR1.**

| | |
|---|---|
| **Combinaisons d'un gène** | S100A9 |
| | TAP2 |
| | CD177 |
| | CX3CR1 |
| | IL1R2 |
| | IFNG |
| | CIITA |
| | CD3D |
| | CTLA4 |
| | CD74 |
| | HP |
| **Combinaisons de deux gènes** | S100A9,TAP2 |
| | S100A9,CD177 |
| | S100A9,CX3CR1 |
| | S100A9,IL1R2 |
| | S100A9,IFNG |
| | S100A9, CIITA |
| | S100A9,CD3D |
| | S100A9,CTLA4 |
| | S100A9,CD74 |
| | S100A9,HP |
| | TAP2,CD177 |
| | TAP2,CX3CR1 |
| | TAP2,IL1R2 |
| | TAP2,IFNG |
| | TAP2,CIITA |
| | TAP2,CD3D |
| | TAP2,CTLA4 |
| | TAP2,CD74 |
| | TAP2,HP |
| | CD177,CX3CR1 |
| | CD177,IL1R2 |
| | CD177,IFNG |
| | CD177,CIITA |
| | CD177,CD3D |
| | CD177,CTLA4 |
| | CD177,CD74 |
| | CD177,HP |
| | CX3CR1,IL1R2 |
| | CX3CR1,IFNG |
| | CX3CR1,CIITA |
| | CX3CR1,CD3D |
| | CX3CR1,CTLA4 |
| | CX3CR1,CD74 |
| | CX3CR1,HP |
| | IL1R2,IFNG |
| | IL1R2,CIITA |
| | IL1R2,CD3D |
| | IL1R2,CTLA4 |
| | IL1R2,CD74 |
| | IL1R2,HP |
| | IFNG,CIITA |
| | IFNG,CD3D |
| | IFNG,CTLA4 |
| | IFNG,CD74 |
| | IFNG,HP |
| | CIITA,CD3D |
| | CIITA,CTLA4 |
| | CIITA,CD74 |
| | CIITA,HP |
| | CD3D,CTLA4 |
| | CD3D,CD74 |
| | CD3D,HP |
| | CTLA4,CD74 |
| | CTLA4,HP |
| | CD74,HP |
| **Combinaisons de trois gènes** | S100A9,TAP2,CD177 |
| | S100A9,TAP2,CX3CR1 |
| | S100A9,TAP2,IL1R2 |
| | S100A9,TAP2,IFNG |
| | S100A9,TAP2,CIITA |
| | S100A9,TAP2,CD3D |
| | S100A9,TAP2,CTLA4 |
| | S100A9,TAP2,CD74 |
| | S100A9,TAP2,HP |
| | S100A9,CD177,CX3CR1 |
| | S100A9,CD177,IL1R2 |
| | S100A9,CD177,IFNG |
| | S100A9,CD177,CIITA |
| | S100A9,CD177,CD3D |
| | S100A9,CD177,CTLA4 |
| | S100A9,CD177,CD74 |
| | S100A9,CD177,HP |
| | S100A9,CX3CR1,IL1R2 |
| | S100A9,CX3CR1,IFNG |
| | S100A9,CX3CR1,CIITA |
| | S100A9,CX3CR1,CD3D |
| | S100A9,CX3CR1,CTLA4 |
| | S100A9,CX3CR1,CD74 |
| | S100A9,CX3CR1,HP |
| | S100A9,IL1R2,IFNG |
| | S100A9,IL1R2,CIITA |
| | S100A9,IL1R2,CD3D |
| | S100A9,IL1R2,CTLA4 |
| | S100A9,IL1R2,CD74 |
| | S100A9,IL1R2,HP |
| | S100A9,IFNG,CIITA |
| | S100A9,IFNG,CD3D |
| | S100A9,IFNG,CTLA4 |
| | S100A9,IFNG,CD74 |
| | S100A9,IFNG,HP |
| | S100A9,CIITA,CD3D |
| | S100A9,CIITA,CTLA4 |
| | S100A9,CIITA,CD74 |
| | S100A9,CIITA,HP |
| | S100A9,CD3D,CTLA4 |
| | S100A9,CD3D,CD74 |
| | S100A9,CD3D,HP |
| | S100A9,CTLA4,CD74 |
| | S100A9,CTLA4,HP |
| | S100A9,CD74,HP |
| | TAP2,CD177,CX3CR1 |
| | TAP2,CD177,IL1R2 |
| | TAP2,CD177,IFNG |
| | TAP2,CD177,CIITA |
| | TAP2,CD177,CD3D |
| | TAP2,CD177,CTLA4 |
| | TAP2,CD177,CD74 |
| | TAP2,CD177,HP |
| | TAP2,CX3CR1,IL1R2 |
| | TAP2,CX3CR1,IFNG |
| | TAP2,CX3CR1,CIITA |
| | TAP2,CX3CR1,CD3D |
| | TAP2,CX3CR1,CTLA4 |
| | TAP2,CX3CR1,CD74 |
| | TAP2,CX3CR1,HP |
| | TAP2,IL1R2,IFNG |
| | TAP2,IL1R2,CIITA |
| | TAP2,IL1R2,CD3D |
| | TAP2,IL1R2,CTLA4 |
| | TAP2,IL1R2,CD74 |
| | TAP2,IL1R2,HP |
| | TAP2,IFNG,CIITA |
| | TAP2,IFNG,CD3D |
| | TAP2,IFNG,CTLA4 |
| | TAP2,IFNG,CD74 |
| | TAP2,IFNG,HP |
| | TAP2,CIITA,CD3D |
| | TAP2,CIITA,CTLA4 |
| | TAP2,CIITA,CD74 |
| | TAP2,CIITA,HP |
| | TAP2,CD3D,CTLA4 |
| | TAP2,CD3D,CD74 |
| | TAP2,CD3D,HP |
| | TAP2,CTLA4,CD74 |
| | TAP2,CTLA4,HP |
| | TAP2,CD74,HP |
| | CD177,CX3CR1,IL1R2 |
| | CD177,CX3CR1,IFNG |
| | CD177,CX3CR1,CIITA |
| | CD177,CX3CR1,CD3D |
| | CD177,CX3CR1,CTLA4 |
| | CD177,CX3CR1,CD74 |
| | CD177,CX3CR1,HP |
| | CD177,IL1R2,IFNG |
| | CD177,IL1R2,CIITA |
| | CD177,IL1R2,CD3D |
| | CD177,IL1R2,CTLA4 |
| | CD177,IL1R2,CD74 |
| | CD177,IL1R2,HP |
| | CD177,IFNG,CIITA |
| | CD177,IFNG,CD3D |
| | CD177,IFNG,CTLA4 |
| | CD177,IFNG,CD74 |
| | CD177,IFNG,HP |
| | CD177,CIITA,CD3D |
| | CD177,CIITA,CTLA4 |
| | CD177,CIITA,CD74 |
| | CD177,CIITA,HP |
| | CD177,CD3D,CTLA4 |
| | CD177,CD3D,CD74 |
| | CD177,CD3D,HP |
| | CD177,CTLA4,CD74 |
| | CD177,CTLA4,HP |
| | CD177,CD74,HP |
| | CX3CR1,IL1R2,IFNG |
| | CX3CR1,IL1R2,CIITA |
| | CX3CR1,IL1R2,CD3D |
| | CX3CR1,IL1R2,CTLA4 |
| | CX3CR1,IL1R2,CD74 |
| | CX3CR1,IL1R2,HP |
| | CX3CR1,IFNG,CIITA |
| | CX3CR1,IFNG,CD3D |
| | CX3CR1,IFNG,CTLA4 |
| | CX3CR1,IFNG,CD74 |
| | CX3CR1,IFNG,HP |
| | CX3CR1,CIITA,CD3D |
| | CX3CR1,CIITA,CTLA4 |
| | CX3CR1,CIITA,CD74 |
| | CX3CR1,CIITA,HP |
| | CX3CR1,CD3D,CTLA4 |
| | CX3CR1,CD3D,CD74 |
| | CX3CR1,CD3D,HP |
| | CX3CR1,CTLA4,CD74 |
| | CX3CR1,CTLA4,HP |
| | CX3CR1,CD74,HP |
| | IL1R2,IFNG,CIITA |
| | IL1R2,IFNG,CD3D |
| | IL1R2,IFNG,CTLA4 |
| | IL1R2,IFNG,CD74 |
| | IL1R2,IFNG,HP |
| | IL1R2,CIITA,CD3D |
| | IL1R2,CIITA,CTLA4 |
| | IL1R2,CIITA,CD74 |
| | IL1R2,CIITA,HP |
| | IL1R2,CD3D,CTLA4 |
| | IL1R2,CD3D,CD74 |
| | IL1R2,CD3D,HP |
| | IL1R2,CTLA4,CD74 |
| | IL1R2,CTLA4,HP |
| | IL1R2,CD74,HP |
| | IFNG,CIITA,CD3D |
| | IFNG,CIITA,CTLA4 |
| | IFNG,CIITA,CD74 |
| | IFNG,CIITA,HP |
| | IFNG,CD3D,CTLA4 |
| | IFNG,CD3D,CD74 |
| | IFNG,CD3D,HP |
| | IFNG,CTLA4,CD74 |
| | IFNG,CTLA4,HP |
| | IFNG,CD74,HP |
| | CIITA,CD3D,CTLA4 |
| | CIITA,CD3D,CD74 |
| | CIITA,CD3D,HP |
| | CIITA,CTLA4,CD74 |
| | CIITA,CTLA4,HP |
| | CIITA,CD74,HP |
| | CD3D,CTLA4,CD74 |
| | CD3D,CTLA4,HP |
| | CD3D,CD74,HP |
| | CTLA4,CD74,HP |
| **Combinaisons de quatre gènes** | S100A9,TAP2,CD177,CX3CR1 |
| | S100A9,TAP2,CD177,IL1R2 |
| | S100A9,TAP2,CD177,IFNG |
| | S100A9,TAP2,CD177,CIITA |
| | S100A9,TAP2,CD177,CD3D |
| | S100A9,TAP2,CD177,CTLA4 |
| | S100A9,TAP2,CD177,CD74 |
| | S100A9,TAP2,CD177,HP |
| | S100A9,TAP2,CX3CR1,IL1R2 |
| | S100A9,TAP2,CX3CR1,IFNG |
| | S100A9,TAP2,CX3CR1,CIITA |
| | S100A9,TAP2,CX3CR1,CD3D |
| | S100A9,TAP2,CX3CR1,CTLA4 |
| | S100A9,TAP2,CX3CR1,CD74 |
| | S100A9,TAP2,CX3CR1,HP |
| | S100A9,TAP2,IL1R2,IFNG |
| | S100A9,TAP2,IL1R2,CIITA |
| | S100A9,TAP2,IL1R2,CD3D |
| | S100A9,TAP2,IL1R2,CTLA4 |
| | S100A9,TAP2,IL1R2,CD74 |
| | S100A9,TAP2,IL1R2,H P |
| | S100A9,TAP2,IFNG,CIITA |
| | S100A9,TAP2,IFNG,CD3D |
| | S100A9,TAP2,IFNG,CTLA4 |
| | S100A9,TAP2,IFNG,CD74 |
| | S100A9,TAP2,IFNG,HP |
| | S100A9,TAP2,CIITA,CD3D |
| | S100A9,TAP2,CIITA,CTLA4 |
| | S100A9,TAP2,CIITA,CD74 |
| | S100A9,TAP2,CIITA,HP |
| | S100A9,TAP2,CD3D,CTLA4 |
| | S100A9,TAP2,CD3D,CD74 |
| | S100A9,TAP2,CD3D,HP |
| | S100A9,TAP2,CTLA4,CD74 |
| | S100A9,TAP2,CTLA4,HP |
| | S100A9,TAP2,CD74,HP |
| | S100A9,CD177,CX3CR1,IL1R2 |
| | S100A9,CD177,CX3CR1,IFNG |
| | S100A9,CD177,CX3CR1,CIITA |
| | S100A9,CD177,CX3CR1,CD3D |
| | S100A9,CD177,CX3CR1,CTLA4 |
| | S100A9,CD177,CX3CR1,CD74 |
| | S100A9,CD177,CX3CR1,HP |
| | S100A9,CD177,IL1R2,IFNG |
| | S100A9,CD177,IL1R2,CIITA |
| | S100A9,CD177,IL1R2,CD3D |
| | S100A9,CD177,IL1R2,CTLA4 |
| | S100A9,CD177,IL1R2,CD74 |
| | S100A9,CD177,IL1R2,HP |
| | S100A9,CD177,IFNG,CIITA |
| | S100A9,CD177,IFNG,CD3D |
| | S100A9,CD177,IFNG,CTLA4 |
| | S100A9,CD177,IFNG,CD74 |
| | S100A9,CD177,IFNG,HP |
| | S100A9,CD177,CIITA,CD3D |
| | S100A9,CD177,CIITA,CTLA4 |
| | S100A9,CD177,CIITA,CD74 |
| | S100A9,CD177,CIITA,HP |
| | S100A9,CD177,CD3D,CTLA4 |
| | S100A9,CD177,CD3D,CD74 |
| | S100A9,CD177,CD3D,HP |
| | S100A9,CD177,CTLA4,CD74 |
| | S100A9,CD177,CTLA4,HP |
| | S100A9,CD177,CD74,HP |
| | S100A9,CX3CR1,IL1R2,IFNG |
| | S100A9,CX3CR1,IL1R2,CIITA |
| | S100A9,CX3CR1,IL1R2,CD3D |
| | S100A9,CX3CR1,IL1R2,CTLA4 |
| | S100A9,CX3CR1,IL1R2,CD74 |
| | S100A9,CX3CR1,IL1R2,HP |
| | S100A9,CX3CR1,IFNG,CIITA |
| | S100A9,CX3CR1,IFNG,CD3D |
| | S100A9,CX3CR1,IFNG,CTLA4 |
| | S100A9,CX3CR1,IFNG,CD74 |
| | S100A9,CX3CR1,IFNG,HP |
| | S100A9,CX3CR1,ClIT A,CD3D |
| | S100A9,CX3CR1,CIITA,CTLA4 |
| | S100A9,CX3CR1,CIITA,CD74 |
| | S100A9,CX3CR1,CIITA,HP |
| | S100A9,CX3CR1,CD3D,CTLA4 |
| | S100A9,CX3CR1,CD3D,CD74 |
| | S100A9,CX3CR1,CD3D,HP |
| | S100A9,CX3CR1,CTLA4,CD74 |
| | S100A9,CX3CR1,CTLA4,HP |
| | S100A9,CX3CR1,CD74,HP |
| | S100A9,IL1R2,IFNG,CIITA |
| | S100A9,IL1R2,IFNG,CD3D |
| | S100A9,IL1R2,IFNG,CTLA4 |
| | S100A9,IL1R2,IFNG,CD74 |
| | S100A9,IL1R2,IFNG,HP |
| | S100A9,IL1R2,CIITA,CD3D |
| | S100A9,IL1R2,CIITA,CTLA4 |
| | S100A9,IL1R2,CIITA,CD74 |
| | S100A9,IL1R2,CIITA,HP |
| | S100A9,IL1R2,CD3D,CTLA4 |
| | S100A9,IL1R2,CD3D,CD74 |
| | S100A9,IL1R2,CD3D,HP |
| | S100A9,IL1R2,CTLA4,CD74 |
| | S100A9,IL1R2,CTLA4,HP |
| | S100A9,IL1R2,CD74,HP |
| | S100A9,IFNG,CIITA,CD3D |
| | S100A9,IFNG,CIITA,CTLA4 |
| | S100A9,IFNG,CIITA,CD74 |
| | S100A9,IFNG,CIITA,HP |
| | S100A9,IFNG,CD3D,CTLA4 |
| | S100A9,IFNG,CD3D,CD74 |
| | S100A9,IFNG,CD3D,HP |
| | S100A9,IFNG,CTLA4,CD74 |
| | S100A9,IFNG,CTLA4,HP |
| | S100A9,IFNG,CD74,HP |
| | S100A9,CIITA,CD3D,CTLA4 |
| | S100A9,CIITA,CD3D,CD74 |
| | S100A9,CIITA,CD3D,HP |
| | S100A9,CIITA,CTLA4,CD74 |
| | S100A9,CIITA,CTLA4,HP |
| | S100A9,CIITA,CD74,HP |
| | S100A9,CD3D,CTLA4,CD74 |
| | S100A9,CD3D,CTLA4, HP |
| | S100A9,CD3D,CD74,HP |
| | S100A9,CTLA4,CD74,HP |
| | TAP2,CD177,CX3CR1,IL1R2 |
| | TAP2,CD177,CX3CR1,IFNG |
| | TAP2,CD177,CX3CR1,CIITA |
| | TAP2,CD177,CX3CR1,CD3D |
| | TAP2,CD177,CX3CR1,CTLA4 |
| | TAP2,CD177,CX3CR1,CD74 |
| | TAP2,CD177,CX3CR1,HP |
| | TAP2,CD177,IL1R2,IFNG |
| | TAP2,CD177,IL1R2,CIITA |
| | TAP2,CD177,IL1R2,CD3D |
| | TAP2,CD177,IL1R2,CTLA4 |
| | TAP2,CD177,IL1R2,CD74 |
| | TAP2,CD177,IL1R2,HP |
| | TAP2,CD177,IFNG,CIITA |
| | TAP2,CD177,IFNG,CD3D |
| | TAP2,CD177,IFNG,CTLA4 |
| | TAP2,CD177,IFNG,CD74 |
| | TAP2,CD177,IFNG,HP |
| | TAP2,CD177,CIITA,CD3D |
| | TAP2,CD177,CIITA,CTLA4 |
| | TAP2,CD177,CIITA,CD74 |
| | TAP2,CD177,CIITA,HP |
| | TAP2,CD177,CD3D,CTLA4 |
| | TAP2,CD177,CD3D,CD74 |
| | TAP2,CD177,CD3D,HP |
| | TAP2,CD177,CTLA4,CD74 |
| | TAP2,CD177,CTLA4,HP |
| | TAP2,CD177,CD74,HP |
| | TAP2,CX3CR1,IL1R2,IFNG |
| | TAP2,CX3CR1,IL1R2,CIITA |
| | TAP2,CX3CR1,IL1R2,CD3D |
| | TAP2,CX3CR1,IL1R2,CTLA4 |
| | TAP2,CX3CR1,IL1R2,CD74 |
| | TAP2,CX3CR1,IL1R2,HP |
| | TAP2,CX3CR1,IFNG,CIITA |
| | TAP2,CX3CR1,IFNG,CD3D |
| | TAP2,CX3CR1,IFNG,CTLA4 |
| | TAP2,CX3CR1,IFNG,CD74 |
| | TAP2,CX3CR1,IFNG,HP |
| | TAP2,CX3CR1,CIITA,CD3D |
| | TAP2,CX3CR1,CIITA,CTLA4 |
| | TAP2,CX3CR1,CIITA,CD74 |
| | TAP2,CX3CR1,CIITA,HP |
| | TAP2,CX3CR1,CD3D,CTLA4 |
| | TAP2,CX3CR1,CD3D,CD74 |
| | TAP2,CX3CR1,CD3D,HP |
| | TAP2,CX3CR1,CTLA4,CD74 |
| | TAP2,CX3CR1,CTLA4,HP |
| | TAP2,CX3CR1,CD74,HP |
| | TAP2,IL1R2,IFNG,CIITA |
| | TAP2,IL1R2,IFNG,CD3D |
| | TAP2,IL1R2,IFNG,CTLA4 |
| | TAP2,IL1R2,IFNG,CD74 |
| | TAP2,IL1R2,IFNG,HP |
| | TAP2,IL1R2,CIITA,CD3D |
| | TAP2,IL1R2,CIITA,CTLA4 |
| | TAP2,IL1R2,CIITA,CD74 |
| | TAP2,IL1R2,CIITA,HP |
| | TAP2,IL1R2,CD3D,CTLA4 |
| | TAP2,IL1R2,CD3D,CD74 |
| | TAP2,IL1R2,CD3D,HP |
| | TAP2,IL1R2,CTLA4,CD74 |
| | TAP2,IL1R2,CTLA4,HP |
| | TAP2,IL1R2,CD74,HP |
| | TAP2,IFNG,CIITA,CD3D |
| | TAP2,IFNG,CIITA,CTLA4 |
| | TAP2,IFNG,CIITA,CD74 |
| | TAP2,IFNG,CIITA,HP |
| | TAP2,IFNG,CD3D,CTLA4 |
| | TAP2,IFNG,CD3D,CD74 |
| | TAP2,IFNG,CD3D,HP |
| | TAP2,IFNG,CTLA4,CD74 |
| | TAP2,IFNG,CTLA4,HP |
| | TAP2,IFNG,CD74,HP |
| | TAP2,CIITA,CD3D,CTLA4 |
| | TAP2,CIITA,CD3D,CD74 |
| | TAP2,CIITA,CD3D,HP |
| | TAP2,CIITA,CTLA4,CD74 |
| | TAP2,CIITA,CTLA4,HP |
| | TAP2,CIITA,CD74,HP |
| | TAP2,CD3D,CTLA4,CD74 |
| | TAP2,CD3D,CTLA4,HP |
| | TAP2,CD3D,CD74,HP |
| | TAP2,CTLA4,CD74,HP |
| | CD177,CX3CR1,IL1R2,IFNG |
| | CD177,CX3CR1,IL1R2,CIITA |
| | CD177,CX3CR1,IL1R2,CD3D |
| | CD177,CX3CR1,IL1R2,CTLA4 |
| | CD177,CX3CR1,IL1R2,CD74 |
| | CD177,CX3CR1,IL1R2,HP |
| | CD177,CX3CR1,IFNG,CIITA |
| | CD177,CX3CR1,IFNG,CD3D |
| | CD177,CX3CR1,IFNG,CTLA4 |
| | CD177,CX3CR1,IFNG,CD74 |
| | CD177,CX3CR1,IFNG,HP |
| | CD177,CX3CR1,CIITA,CD3D |
| | CD177,CX3CR1,CIITA,CTLA4 |
| | CD177,CX3CR1,CIITA,CD74 |
| | CD177,CX3CR1,CIITA,HP |
| | CD177,CX3CR1,CD3D,CTLA4 |
| | CD177,CX3CR1,CD3D,CD74 |
| | CD177,CX3CR1,CD3D,HP |
| | CD177,CX3CR1,CTLA4,CD74 |
| | CD177,CX3CR1,CTLA4,HP |
| | CD177,CX3CR1,CD74,HP |
| | CD177,IL1R2,IFNG,CIITA |
| | CD177,IL1R2,IFNG,CD3D |
| | CD177,IL1R2,IFNG,CTLA4 |
| | CD177,IL1R2,IFNG,CD74 |
| | CD177,IL1R2,IFNG,HP |
| | CD177,IL1R2,CIITA,CD3D |
| | CD177,IL1R2,CIITA,CTLA4 |
| | CD177,IL1R2,CIITA,CD74 |
| | CD177,IL1R2,CIITA,HP |
| | CD177,IL1R2,CD3D,CTLA4 |
| | CD177,IL1R2,CD3D,CD74 |
| | CD177,IL1R2,CD3D,HP |
| | CD177,IL1R2,CTLA4,CD74 |
| | CD177,IL1R2,CTLA4,HP |
| | CD177,IL1R2,CD74,HP |
| | CD177,IFNG,CIITA,CD3D |
| | CD177,IFNG,CIITA,CTLA4 |
| | CD177,IFNG,CIITA,CD74 |
| | CD177,IFNG,CIITA,HP |
| | CD177,IFNG,CD3D,CTLA4 |
| | CD177,IFNG,CD3D,CD74 |
| | CD177,IFNG,CD3D,HP |
| | CD177,IFNG,CTLA4,CD74 |
| | CD177,IFNG,CTLA4,HP |
| | CD177,IFNG,CD74,HP |
| | CD177,CIITA,CD3D,CTLA4 |
| | CD177,CIITA,CD3D,CD74 |
| | CD177,CIITA,CD3D,HP |
| | CD177,CIITA,CTLA4,CD74 |
| | CD177,CIITA,CTLA4,HP |
| | CD177,CIITA,CD74,HP |
| | CD177,CD3D,CTLA4,CD74 |
| | CD177,CD3D,CTLA4,HP |
| | CD177,CD3D,CD74,HP |
| | CD177,CTLA4,CD74,HP |
| | CX3CR1,IL1R2,IFNG,CIITA |
| | CX3CR1,IL1R2,IFNG,CD3D |
| | CX3CR1,IL1R2,IFNG,CTLA4 |
| | CX3CR1,IL1R2,IFNG,CD74 |
| | CX3CR1,IL1R2,IFNG,HP |
| | CX3CR1,IL1R2,CIITA,CD3D |
| | CX3CR1,IL1R2,CIITA,CTLA4 |
| | CX3CR1,IL1R2,CIITA,CD74 |
| | CX3CR1,IL1R2,CIITA,HP |
| | CX3CR1,IL1R2,CD3D,CTLA4 |
| | CX3CR1,IL1R2,CD3D,CD74 |
| | CX3CR1,IL1R2,CD3D,HP |
| | CX3CR1,IL1R2,CTLA4,CD74 |
| | CX3CR1,IL1R2,CTLA4,HP |
| | CX3CR1,IL1R2,CD74,HP |
| | CX3CR1,IFNG,CIITA,CD3D |
| | CX3CR1,IFNG,CIITA,CTLA4 |
| | CX3CR1,IFNG,CIITA,CD74 |
| | CX3CR1,IFNG,CIITA,HP |
| | CX3CR1,IFNG,CD3D,CTLA4 |
| | CX3CR1,IFNG,CD3D,CD74 |
| | CX3CR1,IFNG,CD3D,HP |
| | CX3CR1,IFNG,CTLA4,CD74 |
| | CX3CR1,IFNG,CTLA4,HP |
| | CX3CR1,IFNG,CD74,HP |
| | CX3CR1,CIITA,CD3D,CTLA4 |
| | CX3CR1,CIITA,CD3D,CD74 |
| | CX3CR1,CIITA,CD3D,HP |
| | CX3CR1,CIITA,CTLA4,CD74 |
| | CX3CR1,CIITA,CTLA4,HP |
| | CX3CR1,CIITA,CD74,HP |
| | CX3CR1,CD3D,CTLA4,CD74 |
| | CX3CR1,CD3D,CTLA4, H P |
| | CX3CR1,CD3D,CD74,HP |
| | CX3CR1,CTLA4,CD74,HP |
| | IL1R2,IFNG,CIITA,CD3D |
| | IL1R2,IFNG,CIITA,CTLA4 |
| | IL1R2,IFNG,CIITA,CD74 |
| | IL1R2,IFNG,CIITA,HP |
| | IL1R2,IFNG,CD3D,CTLA4 |
| | IL1R2,IFNG,CD3D,CD74 |
| | IL1R2,IFNG,CD3D,HP |
| | IL1R2,IFNG,CTLA4,CD74 |
| | IL1R2,IFNG,CTLA4,HP |
| | IL1R2,IFNG,CD74,HP |
| | IL1R2,CIITA,CD3D,CTLA4 |
| | IL1R2,CIITA,CD3D,CD74 |
| | IL1R2,CIITA,CD3D,HP |
| | IL1R2,CIITA,CTLA4,CD74 |
| | IL1R2,CIITA,CTLA4,HP |
| | IL1R2,CIITA,CD74,HP |
| | IL1R2,CD3D,CTLA4,CD74 |
| | IL1R2,CD3D,CTLA4,HP |
| | IL1R2,CD3D,CD74,HP |
| | IL1R2,CTLA4,CD74,HP |
| | IFNG,CIITA,CD3D,CTLA4 |
| | IFNG,CIITA,CD3D,CD74 |
| | IFNG,CIITA,CD3D,HP |
| | IFNG,CIITA,CTLA4,CD74 |
| | IFNG,CIITA,CTLA4,HP |
| | IFNG,CIITA,CD74,HP |
| | IFNG,CD3D,CTLA4,CD74 |
| | IFNG,CD3D,CTLA4,HP |
| | IFNG,CD3D,CD74,HP |
| | IFNG,CTLA4,CD74,HP |
| | CIITA,CD3D,CTLA4,CD74 |
| | CIITA,CD3D,CTLA4,HP |
| | CIITA,CD3D,CD74,HP |
| | CIITA,CTLA4,CD74,HP |
| | CD3D,CTLA4,CD74,HP |
| **Combinaisons de cinq gènes** | S100A9,TAP2,CD177,CX3CR1,IL1R2 |
| | S100A9,TAP2,CD177,CX3CR1,IFNG |
| | S100A9,TAP2,CD177,CX3CR1,CIITA |
| | S100A9,TAP2,CD177,CX3CR1,CD3D |
| | S100A9,TAP2,CD177,CX3CR1,CTLA4 |
| | S100A9,TAP2,CD177,CX3CR1,CD74 |
| | S100A9,TAP2,CD177,CX3CR1,HP |
| | S100A9,TAP2,CD177,IL1R2,IFNG |
| | S100A9,TAP2,CD177,IL1R2,CIITA |
| | S100A9,TAP2,CD177,IL1R2,CD3D |
| | S100A9,TAP2,CD177,IL1R2,CTLA4 |
| | S100A9,TAP2,CD177,IL1R2,CD74 |
| | S100A9,TAP2,CD177,IL1R2,HP |
| | S100A9,TAP2,CD177,IFNG,CIITA |
| | S100A9,TAP2,CD177,IFNG,CD3D |
| | S100A9,TAP2,CD177,IFNG,CTLA4 |
| | S100A9,TAP2,CD177,IFNG,CD74 |
| | S100A9,TAP2,CD177,IFNG,HP |
| | S100A9,TAP2,CD177,CIITA,CD3D |
| | S100A9,TAP2,CD177,CIITA,CTLA4 |
| | S100A9,TAP2,CD177,CIITA,CD74 |
| | S100A9,TAP2,CD177,CIITA,HP |
| | S100A9,TAP2,CD177,CD3D,CTLA4 |
| | S100A9,TAP2,CD177,CD3D,CD74 |
| | S100A9,TAP2,CD177,CD3D,HP |
| | S100A9,TAP2,CD177,CTLA4,CD74 |
| | S100A9,TAP2,CD177,CTLA4,HP |
| | S100A9,TAP2,CD177,CD74,HP |
| | S100A9,TAP2,CX3CR1,IL1R2,IFNG |
| | S100A9,TAP2,CX3CR1,IL1R2,CIITA |
| | S100A9,TAP2,CX3CR1,IL1R2,CD3D |
| | S100A9,TAP2,CX3CR1,IL1R2,CTLA4 |
| | S100A9,TAP2,CX3CR1,IL1R2,CD74 |
| | S100A9,TAP2,CX3CR1,IL1R2,HP |
| | S100A9,TAP2,CX3CR1,IFNG,CIITA |
| | S100A9,TAP2,CX3CR1,IFNG,CD3D |
| | S100A9,TAP2,CX3CR1,IFNG,CTLA4 |
| | S100A9,TAP2,CX3CR1,IFNG,CD74 |
| | S100A9,TAP2,CX3CR1,IFNG,HP |
| | S100A9,TAP2,CX3CR1,CIITA,CD3D |
| | S100A9,TAP2,CX3CR1,CIITA,CTLA4 |
| | S100A9,TAP2,CX3CR1,CIITA,CD74 |
| | S100A9,TAP2,CX3CR1,CIITA,HP |
| | S100A9,TAP2,CX3CR1,CD3D,CTLA4 |
| | S100A9,TAP2,CX3CR1,CD3D,CD74 |
| | S100A9,TAP2,CX3CR1,CD3D,HP |
| | S100A9,TAP2,CX3CR1,CTLA4,CD74 |
| | S100A9,TAP2,CX3CR1,CTLA4,HP |
| | S100A9,TAP2,CX3CR1,CD74,HP |
| | S100A9,TAP2,IL1R2,IFNG,CIITA |
| | S100A9,TAP2,IL1R2,IFNG,CD3D |
| | S100A9,TAP2,IL1R2,IFNG,CTLA4 |
| | S100A9,TAP2,IL1R2,IFNG,CD74 |
| | S100A9,TAP2,IL1R2,IFNG,HP |
| | S100A9,TAP2,IL1R2,CIITA,CD3D |
| | S100A9,TAP2,IL1R2,CIITA,CTLA4 |
| | S100A9,TAP2,IL1R2,CIITA,CD74 |
| | S100A9,TAP2,IL1R2,CIITA,HP |
| | S100A9,TAP2,IL1R2,CD3D,CTLA4 |
| | S100A9,TAP2,IL1R2,CD3D,CD74 |
| | S100A9,TAP2,IL1R2,CD3D,HP |
| | S100A9,TAP2,IL1R2,CTLA4,CD74 |
| | S100A9,TAP2,IL1R2,CTLA4,HP |
| | S100A9,TAP2,IL1R2,CD74,HP |
| | S100A9,TAP2,IFNG,CIITA,CD3D |
| | S100A9,TAP2,IFNG,CIITA,CTLA4 |
| | S100A9,TAP2,IFNG,CIITA,CD74 |
| | S100A9,TAP2,IFNG,CIITA,HP |
| | S100A9,TAP2,IFNG,CD3D,CTLA4 |
| | S100A9,TAP2,IFNG,CD3D,CD74 |
| | S100A9,TAP2,IFNG,CD3D,HP |
| | S100A9,TAP2,IFNG,CTLA4,CD74 |
| | S100A9,TAP2,IFNG,CTLA4,HP |
| | S100A9,TAP2,IFNG,CD74,HP |
| | S100A9,TAP2,CIITA,CD3D,CTLA4 |
| | S100A9,TAP2,CIITA,CD3D,CD74 |
| | S100A9,TAP2,CIITA,CD3D,HP |
| | S100A9,TAP2,CIITA,CTLA4,CD74 |
| | S100A9,TAP2,CIITA,CTLA4,HP |
| | S100A9,TAP2,CIITA,CD74,HP |
| | S100A9,TAP2,CD3D,CTLA4,CD74 |
| | S100A9,TAP2,CD3D,CTLA4,HP |
| | S100A9,TAP2,CD3D,CD74,HP |
| | S100A9,TAP2,CTLA4,CD74,HP |
| | S100A9,CD177,CX3CR1,IL1R2,IFNG |
| | S100A9,CD177,CX3CR1,IL1R2,CIITA |
| | S100A9,CD177,CX3CR1,IL1R2,CD3D |
| | S100A9,CD177,CX3CR1,IL1R2,CTLA4 |
| | S100A9,CD177,CX3CR1,IL1R2,CD74 |
| | S100A9,CD177,CX3CR1,IL1R2,HP |
| | S100A9,CD177,CX3CR1,IFNG,CIITA |
| | S100A9,CD177,CX3CR1,IFNG,CD3D |
| | S100A9,CD177,CX3CR1,IFNG,CTLA4 |
| | S100A9,CD177,CX3CR1,IFNG,CD74 |
| | S100A9,CD177,CX3CR1,IFNG,HP |
| | S100A9,CD177,CX3CR1,CIITA,CD3D |
| | S100A9,CD177,CX3CR1,CIITA,CTLA4 |
| | S100A9,CD177,CX3CR1,CIITA,CD74 |
| | S100A9,CD177,CX3CR1,ClIT A, H P |
| | S100A9,CD177,CX3CR1,CD3D,CTLA4 |
| | S100A9,CD177,CX3CR1,CD3D,CD74 |
| | S100A9,CD177,CX3CR1,CD3D,HP |
| | S100A9,CD177,CX3CR1,CTLA4,CD74 |
| | S100A9,CD177,CX3CR1,CTLA4,HP |
| | S100A9,CD177,CX3CR1,CD74,HP |
| | S100A9,CD177,IL1R2,IFNG,CIITA |
| | S100A9,CD177,IL1R2,IFNG,CD3D |
| | S100A9,CD177,IL1R2,IFNG,CTLA4 |
| | S100A9,CD177,IL1R2,IFNG,CD74 |
| | S100A9,CD177,IL1R2,IFNG,HP |
| | S100A9,CD177,IL1R2,CIITA,CD3D |
| | S100A9,CD177,IL1R2,CIITA,CTLA4 |
| | S100A9,CD177,IL1R2,CIITA,CD74 |
| | S100A9,CD177,IL1R2,CIITA,HP |
| | S100A9,CD177,IL1R2,CD3D,CTLA4 |
| | S100A9,CD177,IL1R2,CD3D,CD74 |
| | S100A9,CD177,IL1R2,CD3D,HP |
| | S100A9,CD177,IL1R2,CTLA4,CD74 |
| | S100A9,CD177,IL1R2,CTLA4,HP |
| | S100A9,CD177,IL1R2,CD74,HP |
| | S100A9,CD177,IFNG,CIITA,CD3D |
| | S100A9,CD177,IFNG,CIITA,CTLA4 |
| | S100A9,CD177,IFNG,CIITA,CD74 |
| | S100A9,CD177,IFNG,CIITA,HP |
| | S100A9,CD177,IFNG,CD3D,CTLA4 |
| | S100A9,CD177,IFNG,CD3D,CD74 |
| | S100A9,CD177,IFNG,CD3D,HP |
| | S100A9,CD177,IFNG,CTLA4,CD74 |
| | S100A9,CD177,IFNG,CTLA4,HP |
| | S100A9,CD177,IFNG,CD74,HP |
| | S100A9,CD177,ClIT A,CD3D,CTLA4 |
| | S100A9,CD177,CIITA,CD3D,CD74 |
| | S100A9,CD177,ClIT A,CD3D, HP |
| | S100A9,CD177,CIITA,CTLA4,CD74 |
| | S100A9,CD177,CIITA,CTLA4,HP |
| | S100A9,CD177,CIITA,CD74,HP |
| | S100A9,CD177,CD3D,CTLA4,CD74 |
| | S100A9,CD177,CD3D,CTLA4,HP |
| | S100A9,CD177,CD3D,CD74,HP |
| | S100A9,CD177,CTLA4,CD74, HP |
| | S100A9,CX3CR1,IL1R2,IFNG,CIITA |
| | S100A9,CX3CR1,IL1R2,IFNG,CD3D |
| | S100A9,CX3CR1,IL1R2,IFNG,CTLA4 |
| | S100A9,CX3CR1,IL1R2,IFNG,CD74 |
| | S100A9,CX3CR1,IL1R2,IFNG,HP |
| | S100A9,CX3CR1,IL1R2,CIITA,CD3D |
| | S100A9,CX3CR1,IL1R2,CIITA,CTLA4 |
| | S100A9,CX3CR1,IL1R2,CIITA,CD74 |
| | S100A9,CX3CR1,IL1R2,CIITA,HP |
| | S100A9,CX3CR1,IL1R2,CD3D,CTLA4 |
| | S100A9,CX3CR1,IL1R2,CD3D,CD74 |
| | S100A9,CX3CR1,IL1R2,CD3D,HP |
| | S100A9,CX3CR1,IL1R2,CTLA4,CD74 |
| | S100A9,CX3CR1,IL1R2,CTLA4,HP |
| | S100A9,CX3CR1,IL1R2,CD74,HP |
| | S100A9,CX3CR1,IFNG,CIITA,CD3D |
| | S100A9,CX3CR1,IFNG,CIITA,CTLA4 |
| | S100A9,CX3CR1,IFNG,CIITA,CD74 |
| | S100A9,CX3CR1,IFNG,CIITA,HP |
| | S100A9,CX3CR1,IFNG,CD3D,CTLA4 |
| | S100A9,CX3CR1,IFNG,CD3D,CD74 |
| | S100A9,CX3CR1,IFNG,CD3D,HP |
| | S100A9,CX3CR1,IFNG,CTLA4,CD74 |
| | S100A9,CX3CR1,IFNG,CTLA4,HP |
| | S100A9,CX3CR1,IFNG,CD74,HP |
| | S100A9,CX3CR1,CIITA,CD3D,CTLA4 |
| | S100A9,CX3CR1,CIITA,CD3D,CD74 |
| | S100A9,CX3CR1,ClIT A,CD3D, H P |
| | S100A9,CX3CR1,CIITA,CTLA4,CD74 |
| | S100A9,CX3CR1,CIITA,CTLA4,HP |
| | S100A9,CX3CR1,CIITA,CD74,HP |
| | S100A9,CX3CR1,CD3D,CTLA4,CD74 |
| | S100A9,CX3CR1,CD3D,CTLA4,HP |
| | S100A9,CX3CR1,CD3D,CD74,HP |
| | S100A9,CX3CR1,CTLA4,CD74,HP |
| | S100A9,IL1R2,IFNG,CIITA,CD3D |
| | S100A9,IL1R2,IFNG,CIITA,CTLA4 |
| | S100A9,IL1R2,IFNG,CIITA,CD74 |
| | S100A9,IL1R2,IFNG,CIITA,HP |
| | S100A9,IL1R2,IFNG,CD3D,CTLA4 |
| | S100A9,IL1R2,IFNG,CD3D,CD74 |
| | S100A9,IL1R2,IFNG,CD3D,HP |
| | S100A9,IL1R2,IFNG,CTLA4,CD74 |
| | S100A9,IL1R2,IFNG,CTLA4,HP |
| | S100A9,IL1R2,IFNG,CD74,HP |
| | S100A9,IL1R2,CIITA,CD3D,CTLA4 |
| | S100A9,IL1R2,CIITA,CD3D,CD74 |
| | S100A9,IL1R2,CIITA,CD3D,HP |
| | S100A9,IL1R2,CIITA,CTLA4,CD74 |
| | S100A9,IL1R2,CIITA,CTLA4,HP |
| | S100A9,IL1R2,CIITA,CD74,HP |
| | S100A9,IL1R2,CD3D,CTLA4,CD74 |
| | S100A9,IL1R2,CD3D,CTLA4,HP |
| | S100A9,IL1R2,CD3D,CD74,HP |
| | S100A9,IL1R2,CTLA4,CD74,HP |
| | S100A9,IFNG,CIITA,CD3D,CTLA4 |
| | S100A9,IFNG,CIITA,CD3D,CD74 |
| | S100A9,IFNG,CIITA,CD3D,HP |
| | S100A9,IFNG,CIITA,CTLA4,CD74 |
| | S100A9,IFNG,CIITA,CTLA4,HP |
| | S100A9,IFNG,CIITA,CD74,HP |
| | S100A9,IFNG,CD3D,CTLA4,CD74 |
| | S100A9,IFNG,CD3D,CTLA4,HP |
| | S100A9,IFNG,CD3D,CD74,HP |
| | S100A9,IFNG,CTLA4,CD74,HP |
| | S100A9,CIITA,CD3D,CTLA4,CD74 |
| | S100A9,ClIT A,CD3D,CTLA4, H P |
| | S100A9,CIITA,CD3D,CD74,HP |
| | S100A9,CIITA,CTLA4,CD74,HP |
| | S100A9,CD3D,CTLA4,CD74, H P |
| | TAP2,CD177,CX3CR1,IL1R2,IFNG |
| | TAP2,CD177,CX3CR1,IL1R2,CIITA |
| | TAP2,CD177,CX3CR1,IL1R2,CD3D |
| | TAP2,CD177,CX3CR1,IL1R2,CTLA4 |
| | TAP2,CD177,CX3CR1,IL1R2,CD74 |
| | TAP2,CD177,CX3CR1,IL1R2,HP |
| | TAP2,CD177,CX3CR1,IFNG,CIITA |
| | TAP2,CD177,CX3CR1,IFNG,CD3D |
| | TAP2,CD177,CX3CR1,IFNG,CTLA4 |
| | TAP2,CD177,CX3CR1,IFNG,CD74 |
| | TAP2,CD177,CX3CR1,IFNG,HP |
| | TAP2,CD177,CX3CR1,CIITA,CD3D |
| | TAP2,CD177,CX3CR1,CIITA,CTLA4 |
| | TAP2,CD177,CX3CR1,CIITA,CD74 |
| | TAP2,CD177,CX3CR1,CIITA,HP |
| | TAP2,CD177,CX3CR1,CD3D,CTLA4 |
| | TAP2,CD177,CX3CR1,CD3D,CD74 |
| | TAP2,CD 177,CX3CR1,CD3D, HP |
| | TAP2,CD177,CX3CR1,CTLA4,CD74 |
| | TAP2,CD177,CX3CR1,CTLA4,HP |
| | TAP2,CD 177,CX3CR1,CD74, HP |
| | TAP2,CD177,IL1R2,IFNG,CIITA |
| | TAP2,CD177,IL1R2,IFNG,CD3D |
| | TAP2,CD177,IL1R2,IFNG,CTLA4 |
| | TAP2,CD177,IL1R2,IFNG,CD74 |
| | TAP2,CD177,IL1R2,IFNG,HP |
| | TAP2,CD177,IL1R2,CIITA,CD3D |
| | TAP2,CD177,IL1R2,CIITA,CTLA4 |
| | TAP2,CD177,IL1R2,CIITA,CD74 |
| | TAP2,CD177,IL1R2,CIITA,HP |
| | TAP2,CD177,IL1R2,CD3D,CTLA4 |
| | TAP2,CD177,IL1R2,CD3D,CD74 |
| | TAP2,CD177,IL1R2,CD3D,HP |
| | TAP2,CD177,IL1R2,CTLA4,CD74 |
| | TAP2,CD177,IL1R2,CTLA4,HP |
| | TAP2,CD177,IL1R2,CD74,HP |
| | TAP2,CD177,IFNG,CIITA,CD3D |
| | TAP2,CD177,IFNG,CIITA,CTLA4 |
| | TAP2,CD177,IFNG,CIITA,CD74 |
| | TAP2,CD177,IFNG,CIITA,HP |
| | TAP2,CD177,IFNG,CD3D,CTLA4 |
| | TAP2,CD177,IFNG,CD3D,CD74 |
| | TAP2,CD177,IFNG,CD3D,HP |
| | TAP2,CD177,IFNG,CTLA4,CD74 |
| | TAP2,CD177,IFNG,CTLA4,HP |
| | TAP2,CD177,IFNG,CD74,HP |
| | TAP2,CD177,CIITA,CD3D,CTLA4 |
| | TAP2,CD177,CIITA,CD3D,CD74 |
| | TAP2,CD177,CIITA,CD3D,HP |
| | TAP2,CD177,CIITA,CTLA4,CD74 |
| | TAP2,CD177,CIITA,CTLA4,HP |
| | TAP2,CD177,CIITA,CD74,HP |
| | TAP2,CD177,CD3D,CTLA4,CD74 |
| | TAP2,CD177,CD3D,CTLA4,HP |
| | TAP2,CD177,CD3D,CD74,HP |
| | TAP2,CD177,CTLA4,CD74,HP |
| | TAP2,CX3CR1,IL1R2,IFNG,CIITA |
| | TAP2,CX3CR1,IL1R2,IFNG,CD3D |
| | TAP2,CX3CR1,IL1R2,IFNG,CTLA4 |
| | TAP2,CX3CR1,IL1R2,IFNG,CD74 |
| | TAP2,CX3CR1,IL1R2,IFNG,HP |
| | TAP2,CX3CR1,IL1R2,CIITA,CD3D |
| | TAP2,CX3CR1,IL1R2,CIITA,CTLA4 |
| | TAP2,CX3CR1,IL1R2,CIITA,CD74 |
| | TAP2,CX3CR1,IL1R2,CIITA,HP |
| | TAP2,CX3CR1,IL1R2,CD3D,CTLA4 |
| | TAP2,CX3CR1,IL1R2,CD3D,CD74 |
| | TAP2,CX3CR1,IL1R2,CD3D,HP |
| | TAP2,CX3CR1,IL1R2,CTLA4,CD74 |
| | TAP2,CX3CR1,IL1R2,CTLA4,HP |
| | TAP2,CX3CR1,IL1R2,CD74,HP |
| | TAP2,CX3CR1,IFNG,CIITA,CD3D |
| | TAP2,CX3CR1,IFNG,CIITA,CTLA4 |
| | TAP2,CX3CR1,IFNG,CIITA,CD74 |
| | TAP2,CX3CR1,IFNG,CIITA,HP |
| | TAP2,CX3CR1,IFNG,CD3D,CTLA4 |
| | TAP2,CX3CR1,IFNG,CD3D,CD74 |
| | TAP2,CX3CR1,IFNG,CD3D,HP |
| | TAP2,CX3CR1,IFNG,CTLA4,CD74 |
| | TAP2,CX3CR1,IFNG,CTLA4,HP |
| | TAP2,CX3CR1,IFNG,CD74,HP |
| | TAP2,CX3CR1,CIITA,CD3D,CTLA4 |
| | TAP2,CX3CR1,CIITA,CD3D,CD74 |
| | TAP2,CX3CR1,CIITA,CD3D,HP |
| | TAP2,CX3CR1,CIITA,CTLA4,CD74 |
| | TAP2,CX3CR1,CIITA,CTLA4,HP |
| | TAP2,CX3CR1,CIITA,CD74,HP |
| | TAP2,CX3CR1,CD3D,CTLA4,CD74 |
| | TAP2,CX3CR1,CD3D,CTLA4,HP |
| | TAP2,CX3CR1,CD3D,CD74,HP |
| | TAP2,CX3CR1,CTLA4,CD74,HP |
| | TAP2,IL1R2,IFNG,CIITA,CD3D |
| | TAP2,IL1R2,IFNG,CIITA,CTLA4 |
| | TAP2,IL1R2,IFNG,CIITA,CD74 |
| | TAP2,IL1R2,IFNG,CIITA,HP |
| | TAP2,IL1R2,IFNG,CD3D,CTLA4 |
| | TAP2,IL1R2,IFNG,CD3D,CD74 |
| | TAP2,IL1R2,IFNG,CD3D,HP |
| | TAP2,IL1R2,IFNG,CTLA4,CD74 |
| | TAP2,IL1R2,IFNG,CTLA4,HP |
| | TAP2,IL1R2,IFNG,CD74,HP |
| | TAP2,IL1R2,CIITA,CD3D,CTLA4 |
| | TAP2,IL1R2,CIITA,CD3D,CD74 |
| | TAP2,IL1R2,CIITA,CD3D,HP |
| | TAP2,IL1R2,CIITA,CTLA4,CD74 |
| | TAP2,IL1R2,CIITA,CTLA4,HP |
| | TAP2,IL1R2,CIITA,CD74,HP |
| | TAP2,IL1R2,CD3D,CTLA4,CD74 |
| | TAP2,IL1R2,CD3D,CTLA4,HP |
| | TAP2,IL1R2,CD3D,CD74,HP |
| | TAP2,IL1R2,CTLA4,CD74,HP |
| | TAP2,IFNG,CIITA,CD3D,CTLA4 |
| | TAP2,IFNG,CIITA,CD3D,CD74 |
| | TAP2,IFNG,CIITA,CD3D,HP |
| | TAP2,IFNG,CIITA,CTLA4,CD74 |
| | TAP2,IFNG,CIITA,CTLA4,HP |
| | TAP2,IFNG,CIITA,CD74,HP |
| | TAP2,IFNG,CD3D,CTLA4,CD74 |
| | TAP2,IFNG,CD3D,CTLA4,HP |
| | TAP2,IFNG,CD3D,CD74,HP |
| | TAP2,IFNG,CTLA4,CD74,HP |
| | TAP2,CIITA,CD3D,CTLA4,CD74 |
| | TAP2,CIITA,CD3D,CTLA4,HP |
| | TAP2,CIITA,CD3D,CD74,HP |
| | TAP2,CIITA,CTLA4,CD74,HP |
| | TAP2,CD3D,CTLA4,CD74,HP |
| | CD177,CX3CR1,IL1R2,IFNG,CIITA |
| | CD177,CX3CR1,IL1R2,IFNG,CD3D |
| | CD177,CX3CR1,IL1R2,IFNG,CTLA4 |
| | CD177,CX3CR1,IL1R2,IFNG,CD74 |
| | CD177,CX3CR1,IL1R2,IFNG,HP |
| | CD177,CX3CR1,IL1R2,CIITA,CD3D |
| | CD177,CX3CR1,IL1R2,CIITA,CTLA4 |
| | CD177,CX3CR1,IL1R2,CIITA,CD74 |
| | CD177,CX3CR1,IL1R2,CIITA,HP |
| | CD177,CX3CR1,IL1R2,CD3D,CTLA4 |
| | CD177,CX3CR1,IL1R2,CD3D,CD74 |
| | CD177,CX3CR1,IL1R2,CD3D,HP |
| | CD177,CX3CR1,IL1R2,CTLA4,CD74 |
| | CD177,CX3CR1,IL1R2,CTLA4,HP |
| | CD177,CX3CR1,IL1R2,CD74,HP |
| | CD177,CX3CR1,IFNG,CIITA,CD3D |
| | CD177,CX3CR1,IFNG,CIITA,CTLA4 |
| | CD177,CX3CR1,IFNG,CIITA,CD74 |
| | CD177,CX3CR1,IFNG,CIITA,HP |
| | CD177,CX3CR1,lFNG,CD3D,CTLA4 |
| | CD177,CX3CR1,IFNG,CD3D,CD74 |
| | CD177,CX3CR1,IFNG,CD3D,HP |
| | CD177,CX3CR1,IFNG,CTLA4,CD74 |
| | CD177,CX3CR1,IFNG,CTLA4,HP |
| | CD177,CX3CR1,IFNG,CD74,HP |
| | CD177,CX3CR1,CIITA,CD3D,CTLA4 |
| | CD177,CX3CR1,CIITA,CD3D,CD74 |
| | CD177,CX3CR1,CIITA,CD3D,HP |
| | CD177,CX3CR1,CIITA,CTLA4,CD74 |
| | CD177,CX3CR1,CIITA,CTLA4,HP |
| | CD177,CX3CR1,CIITA,CD74,HP |
| | CD177,CX3CR1,CD3D,CTLA4,CD74 |
| | CD177,CX3CR1,CD3D,CTLA4,HP |
| | CD177,CX3CR1,CD3D,CD74,HP |
| | CD177,CX3CR1,CTLA4,CD74,HP |
| | CD177,IL1R2,IFNG,CIITA,CD3D |
| | CD177,IL1R2,IFNG,CIITA,CTLA4 |
| | CD177,IL1R2,IFNG,CIITA,CD74 |
| | CD177,IL1R2,IFNG,CIITA,HP |
| | CD177,IL1R2,IFNG,CD3D,CTLA4 |
| | CD177,IL1R2,IFNG,CD3D,CD74 |
| | CD177,IL1R2,IFNG,CD3D,HP |
| | CD177,IL1R2,IFNG,CTLA4,CD74 |
| | CD177,IL1R2,IFNG,CTLA4,HP |
| | CD177,IL1R2,IFNG,CD74,HP |
| | CD177,IL1R2,CIITA,CD3D,CTLA4 |
| | CD177,IL1R2,CIITA,CD3D,CD74 |
| | CD177,IL1R2,CIITA,CD3D,HP |
| | CD177,IL1R2,CIITA,CTLA4,CD74 |
| | CD177,IL1R2,CIITA,CTLA4,HP |
| | CD177,IL1R2,CIITA,CD74,HP |
| | CD177,IL1R2,CD3D,CTLA4,CD74 |
| | CD177,IL1R2,CD3D,CTLA4,HP |
| | CD177,IL1R2,CD3D,CD74,HP |
| | CD177,IL1R2,CTLA4,CD74,HP |
| | CD177,IFNG,CIITA,CD3D,CTLA4 |
| | CD177,IFNG,CIITA,CD3D,CD74 |
| | CD177,IFNG,CIITA,CD3D,HP |
| | CD177,IFNG,CIITA,CTLA4,CD74 |
| | CD177,IFNG,CIITA,CTLA4,HP |
| | CD177,IFNG,CIITA,CD74,HP |
| | CD177,IFNG,CD3D,CTLA4,CD74 |
| | CD177,IFNG,CD3D,CTLA4,HP |
| | CD177,IFNG,CD3D,CD74,HP |
| | CD177,IFNG,CTLA4,CD74,HP |
| | CD177,CIITA,CD3D,CTLA4,CD74 |
| | CD177,CIITA,CD3D,CTLA4,HP |
| | CD177,CIITA,CD3D,CD74,HP |
| | CD177,CIITA,CTLA4,CD74,HP |
| | CD177,CD3D,CTLA4,CD74,HP |
| | CX3CR1,IL1R2,IFNG,CIITA,CD3D |
| | CX3CR1,IL1R2,IFNG,CIITA,CTLA4 |
| | CX3CR1,IL1R2,IFNG,CIITA,CD74 |
| | CX3CR1,IL1R2,IFNG,CIITA,HP |
| | CX3CR1,IL1R2,IFNG,CD3D,CTLA4 |
| | CX3CR1,IL1R2,IFNG,CD3D,CD74 |
| | CX3CR1,IL1R2,IFNG,CD3D,HP |
| | CX3CR1,IL1R2,IFNG,CTLA4,CD74 |
| | CX3CR1,IL1R2,IFNG,CTLA4,HP |
| | CX3CR1,IL1R2,IFNG,CD74,HP |
| | CX3CR1,IL1R2,CIITA,CD3D,CTLA4 |
| | CX3CR1,IL1R2,CIITA,CD3D,CD74 |
| | CX3CR1,IL1R2,CIITA,CD3D,HP |
| | CX3CR1,IL1R2,CIITA,CTLA4,CD74 |
| | CX3CR1,IL1R2,CIITA,CTLA4,HP |
| | CX3CR1,IL1R2,CIITA,CD74,HP |
| | CX3CR1,IL1R2,CD3D,CTLA4,CD74 |
| | CX3CR1,IL1R2,CD3D,CTLA4,HP |
| | CX3CR1,IL1R2,CD3D,CD74,HP |
| | CX3CR1,IL1R2,CTLA4,CD74,HP |
| | CX3CR1,IFNG,CIITA,CD3D,CTLA4 |
| | CX3CR1,IFNG,CIITA,CD3D,CD74 |
| | CX3CR1,IFNG,CIITA,CD3D,HP |
| | CX3CR1,IFNG,CIITA,CTLA4,CD74 |
| | CX3CR1,IFNG,CIITA,CTLA4,HP |
| | CX3CR1,IFNG,CIITA,CD74,HP |
| | CX3CR1,IFNG,CD3D,CTLA4,CD74 |
| | CX3CR1,IFNG,CD3D,CTLA4,HP |
| | CX3CR1,IFNG,CD3D,CD74,HP |
| | CX3CR1,IFNG,CTLA4,CD74,HP |
| | CX3CR1,CIITA,CD3D,CTLA4,CD74 |
| | CX3CR1,CIITA,CD3D,CTLA4,HP |
| | CX3CR1,CIITA,CD3D,CD74,HP |
| | CX3CR1,CIITA,CTLA4,CD74,HP |
| | CX3CR1,CD3D,CTLA4,CD74, H P |
| | IL1R2,IFNG,CIITA,CD3D,CTLA4 |
| | IL1R2,IFNG,CIITA,CD3D,CD74 |
| | IL1R2,IFNG,CIITA,CD3D,HP |
| | IL1R2,IFNG,CIITA,CTLA4,CD74 |
| | IL1R2,IFNG,CIITA,CTLA4,HP |
| | IL1R2,IFNG,CIITA,CD74,HP |
| | IL1R2,IFNG,CD3D,CTLA4,CD74 |
| | IL1R2,IFNG,CD3D,CTLA4,HP |
| | IL1R2,IFNG,CD3D,CD74,HP |
| | IL1R2,IFNG,CTLA4,CD74,HP |
| | IL1R2,CIITA,CD3D,CTLA4,CD74 |
| | IL1R2,CIITA,CD3D,CTLA4,HP |
| | IL1R2,CIITA,CD3D,CD74,HP |
| | IL1R2,CIITA,CTLA4,CD74,HP |
| | IL1R2,CD3D,CTLA4,CD74,HP |
| | IFNG,CIITA,CD3D,CTLA4,CD74 |
| | IFNG,CIITA,CD3D,CTLA4,HP |
| | IFNG,CIITA,CD3D,CD74,HP |
| | IFNG,CIITA,CTLA4,CD74,HP |
| | IFNG,CD3D,CTLA4,CD74,HP |
| | CIITA,CD3D,CTLA4,CD74,HP |
| **Combinaisons de six gènes** | S100A9,TAP2,CD177,CX3CR1,IL1R2,IFNG |
| | S100A9,TAP2,CD177,CX3CR1,IL1R2,CIITA |
| | S100A9,TAP2,CD177,CX3CR1,IL1R2,CD3D |
| | S100A9,TAP2,CD177,CX3CR1,IL1R2,CTLA4 |
| | S100A9,TAP2,CD177,CX3CR1,IL1R2,CD74 |
| | S100A9,TAP2,CD177,CX3CR1,IL1R2,HP |
| | S100A9,TAP2,CD177,CX3CR1,IFNG,CIITA |
| | S100A9,TAP2,CD177,CX3CR1,IFNG,CD3D |
| | S100A9,TAP2,CD177,CX3CR1,IFNG,CTLA4 |
| | S100A9,TAP2,CD177,CX3CR1,IFNG,CD74 |
| | S100A9,TAP2,CD177,CX3CR1,IFNG,HP |
| | S100A9,TAP2,CD177,CX3CR1,CIITA,CD3D |
| | S100A9,TAP2,CD177,CX3CR1,CIITA,CTLA4 |
| | S100A9,TAP2,CD177,CX3CR1,CIITA,CD74 |
| | S100A9,TAP2,CD177,CX3CR1,CIITA,HP |
| | S100A9,TAP2,CD177,CX3CR1,CD3D,CTLA4 |
| | S100A9,TAP2,CD177,CX3CR1,CD3D,CD74 |
| | S100A9,TAP2,CD177,CX3CR1,CD3D,HP |
| | S100A9,TAP2,CD177,CX3CR1,CTLA4,CD74 |
| | S100A9,TAP2,CD177,CX3CR1,CTLA4,HP |
| | S100A9,TAP2,CD177,CX3CR1,CD74,HP |
| | S100A9,TAP2,CD177,IL1R2,IFNG,CIITA |
| | S100A9,TAP2,CD177,IL1R2,IFNG,CD3D |
| | S100A9,TAP2,CD177,IL1R2,IFNG,CTLA4 |
| | S100A9,TAP2,CD177,IL1R2,IFNG,CD74 |
| | S100A9,TAP2,CD177,IL1R2,IFNG,HP |
| | S100A9,TAP2,CD177,IL1R2,CIITA,CD3D |
| | S100A9,TAP2,CD177,IL1R2,CIITA,CTLA4 |
| | S100A9,TAP2,CD177,IL1R2,CIITA,CD74 |
| | S100A9,TAP2,CD177,IL1R2,CIITA,HP |
| | S100A9,TAP2,CD177,IL1R2,CD3D,CTLA4 |
| | S100A9,TAP2,CD177,IL1R2,CD3D,CD74 |
| | S100A9,TAP2,CD177,IL1R2,CD3D,HP |
| | S100A9,TAP2,CD177,IL1R2,CTLA4,CD74 |
| | S100A9,TAP2,CD177,IL1R2,CTLA4,HP |
| | S100A9,TAP2,CD177,IL1R2,CD74,HP |
| | S100A9,TAP2,CD177,IFNG,CIITA,CD3D |
| | S100A9,TAP2,CD177,IFNG,CIITA,CTLA4 |
| | S100A9,TAP2,CD177,IFNG,CIITA,CD74 |
| | S100A9,TAP2,CD177,IFNG,CIITA,HP |
| | S100A9,TAP2,CD177,IFNG,CD3D,CTLA4 |
| | S100A9,TAP2,CD177,IFNG,CD3D,CD74 |
| | S100A9,TAP2,CD177,IFNG,CD3D,HP |
| | S100A9,TAP2,CD177, IFNG,CTLA4,CD74 |
| | S100A9,TAP2,CD177,IFNG,CTLA4,HP |
| | S100A9,TAP2,CD177,IFNG,CD74,HP |
| | S100A9,TAP2,CD177,CIITA,CD3D,CTLA4 |
| | S100A9,TAP2,CD177,CIITA,CD3D,CD74 |
| | S100A9,TAP2,CD177,CIITA,CD3D,HP |
| | S100A9,TAP2,CD177,CIITA,CTLA4,CD74 |
| | S100A9,TAP2,CD177,CIITA,CTLA4,HP |
| | S100A9,TAP2,CD177,CIITA,CD74,HP |
| | S100A9,TAP2,CD177,CD3D,CTLA4,CD74 |
| | S100A9,TAP2,CD177,CD3D,CTLA4,HP |
| | S100A9,TAP2,CD177,CD3D,CD74,HP |
| | S100A9,TAP2,CD177,CTLA4,CD74,HP |
| | S100A9,TAP2,CX3CR1,IL1R2,IFNG,CIITA |
| | S100A9,TAP2,CX3CR1,IL1R2,IFNG,CD3D |
| | S100A9,TAP2,CX3CR1,IL1R2,IFNG,CTLA4 |
| | S100A9,TAP2,CX3CR1,IL1R2,IFNG,CD74 |
| | S100A9,TAP2,CX3CR1,IL1R2,IFNG,HP |
| | S100A9,TAP2,CX3CR1,IL1R2,CIITA,CD3D |
| | S100A9,TAP2,CX3CR1,IL1R2,CIITA,CTLA4 |
| | S100A9,TAP2,CX3CR1,IL1R2,CIITA,CD74 |
| | S100A9,TAP2,CX3CR1,IL1R2,CIITA,HP |
| | S100A9,TAP2,CX3CR1,IL1R2,CD3D,CTLA4 |
| | S100A9,TAP2,CX3CR1,IL1R2,CD3D,CD74 |
| | S100A9,TAP2,CX3CR1,IL1R2,CD3D,HP |
| | S100A9,TAP2,CX3CR1,IL1R2,CTLA4,CD74 |
| | S100A9,TAP2,CX3CR1,IL1R2,CTLA4,HP |
| | S100A9,TAP2,CX3CR1,IL1R2,CD74,HP |
| | S100A9,TAP2,CX3CR1,IFNG,CIITA,CD3D |
| | S100A9,TAP2,CX3CR1,IFNG,CIITA,CTLA4 |
| | S100A9,TAP2,CX3CR1,IFNG,CIITA,CD74 |
| | S100A9,TAP2,CX3CR1,IFNG,CIITA,HP |
| | S100A9,TAP2,CX3CR1,IFNG,CD3D,CTLA4 |
| | S100A9,TAP2,CX3CR1,IFNG,CD3D,CD74 |
| | S100A9,TAP2,CX3CR1,IFNG,CD3D,HP |
| | S100A9,TAP2,CX3CR1,IFNG,CTLA4,CD74 |
| | S100A9,TAP2,CX3CR1,IFNG,CTLA4,HP |
| | S100A9,TAP2,CX3CR1,IFNG,CD74,HP |
| | S100A9,TAP2,CX3CR1,CIITA,CD3D,CTLA4 |
| | S100A9,TAP2,CX3CR1,CIITA,CD3D,CD74 |
| | S100A9,TAP2,CX3CR1,CIITA,CD3D,HP |
| | S100A9,TAP2,CX3CR1,CIITA,CTLA4,CD74 |
| | S100A9,TAP2,CX3CR1,CIITA,CTLA4,HP |
| | S100A9,TAP2,CX3CR1,CIITA,CD74,HP |
| | S100A9,TAP2,CX3CR1,CD3D,CTLA4,CD74 |
| | S100A9,TAP2,CX3CR1,CD3D,CTLA4,HP |
| | S100A9,TAP2,CX3CR1,CD3D,CD74,HP |
| | S100A9,TAP2,CX3CR1,CTLA4,CD74,HP |
| | S100A9,TAP2,IL1R2,IFNG,CIITA,CD3D |
| | S100A9,TAP2,IL1R2,IFNG,CIITA,CTLA4 |
| | S100A9,TAP2,IL1R2,IFNG,CIITA,CD74 |
| | S100A9,TAP2,IL1R2,IFNG,CIITA,HP |
| | S100A9,TAP2,IL1R2,IFNG,CD3D,CTLA4 |
| | S100A9,TAP2,IL1R2,IFNG,CD3D,CD74 |
| | S100A9,TAP2,IL1R2,IFNG,CD3D,HP |
| | S100A9,TAP2,IL1R2,IFNG,CTLA4,CD74 |
| | S100A9,TAP2,IL1R2,IFNG,CTLA4,HP |
| | S100A9,TAP2,IL1R2,IFNG,CD74,HP |
| | S100A9,TAP2,IL1R2,CIITA,CD3D,CTLA4 |
| | S100A9,TAP2,IL1R2,CIITA,CD3D,CD74 |
| | S100A9,TAP2,IL1R2,CIITA,CD3D,HP |
| | S100A9,TAP2,IL1R2,CIITA,CTLA4,CD74 |
| | S100A9,TAP2,IL1R2,CIITA,CTLA4,HP |
| | S100A9,TAP2,IL1R2,CIITA,CD74,HP |
| | S100A9,TAP2,IL1R2,CD3D,CTLA4,CD74 |
| | S100A9,TAP2,IL1R2,CD3D,CTLA4,HP |
| | S100A9,TAP2,IL1R2,CD3D,CD74,HP |
| | S100A9,TAP2,IL1R2,CTLA4,CD74,HP |
| | S100A9,TAP2,IFNG,CIITA,CD3D,CTLA4 |
| | S100A9,TAP2,IFNG,CIITA,CD3D,CD74 |
| | S100A9,TAP2,IFNG,CIITA,CD3D,HP |
| | S100A9,TAP2,IFNG,CIITA,CTLA4,CD74 |
| | S100A9,TAP2,IFNG,CIITA,CTLA4,HP |
| | S100A9,TAP2,IFNG,CIITA,CD74,HP |
| | S100A9,TAP2,IFNG,CD3D,CTLA4,CD74 |
| | S100A9,TAP2,IFNG,CD3D,CTLA4,HP |
| | S100A9,TAP2,IFNG,CD3D,CD74,HP |
| | S100A9,TAP2,IFNG,CTLA4,CD74,HP |
| | S100A9,TAP2,CIITA,CD3D,CTLA4,CD74 |
| | S100A9,TAP2,CIITA,CD3D,CTLA4,HP |
| | S100A9,TAP2,CIITA,CD3D,CD74,HP |
| | S100A9,TAP2,CIITA,CTLA4,CD74,HP |
| | S100A9,TAP2,CD3D,CTLA4,CD74,HP |
| | S100A9,CD177,CX3CR1,IL1R2,IFNG,CIITA |
| | S100A9,CD177,CX3CR1,IL1R2,IFNG,CD3D |
| | S100A9,CD177,CX3CR1,IL1R2,IFNG,CTLA4 |
| | S100A9,CD177,CX3CR1,IL1R2,IFNG,CD74 |
| | S100A9,CD177,CX3CR1,IL1R2,IFNG,HP |
| | S100A9,CD177,CX3CR1,IL1R2,CIITA,CD3D |
| | S100A9,CD177,CX3CR1,IL1R2,CIITA,CTLA4 |
| | S100A9,CD177,CX3CR1,IL1R2,CIITA,CD74 |
| | S100A9,CD177,CX3CR1,IL1R2,CIITA,HP |
| | S100A9,CD177,CX3CR1,IL1R2,CD3D,CTLA4 |
| | S100A9,CD177,CX3CR1,IL1R2,CD3D,CD74 |
| | S100A9,CD177,CX3CR1,IL1R2,CD3D,HP |
| | S100A9,CD177,CX3CR1,IL1R2,CTLA4,CD74 |
| | S100A9,CD177,CX3CR1,IL1R2,CTLA4,HP |
| | S100A9,CD177,CX3CR1,IL1R2,CD74,HP |
| | S100A9,CD177,CX3CR1,IFNG,CIITA,CD3D |
| | S100A9,CD177,CX3CR1,IFNG,CIITA,CTLA4 |
| | S100A9,CD177,CX3CR1,IFNG,CIITA,CD74 |
| | S100A9,CD177,CX3CR1,IFNG,CIITA,HP |
| | S100A9,CD177,CX3CR1,IFNG,CD3D,CTLA4 |
| | S100A9,CD177,CX3CR1,IFNG,CD3D,CD74 |
| | S100A9,CD177,CX3CR1,IFNG,CD3D,HP |
| | S100A9,CD177,CX3CR1,IFNG,CTLA4,CD74 |
| | S100A9,CD177,CX3CR1,IFNG,CTLA4,HP |
| | S100A9,CD177,CX3CR1,IFNG,CD74,HP |
| | S100A9,CD177,CX3CR1,CIITA,CD3D,CTLA4 |
| | S100A9,CD177,CX3CR1,CIITA,CD3D,CD74 |
| | S100A9,CD177,CX3CR1,CIITA,CD3D,HP |
| | S100A9,CD177,CX3CR1,CIITA,CTLA4,CD74 |
| | S100A9,CD177,CX3CR1,CIITA,CTLA4,HP |
| | S100A9,CD177,CX3CR1,CIITA,CD74,HP |
| | S100A9,CD177,CX3CR1,CD3D,CTLA4,CD74 |
| | S100A9,CD177,CX3CR1,CD3D,CTLA4,HP |
| | S100A9,CD177,CX3CR1,CD3D,CD74,HP |
| | S100A9,CD177,CX3CR1,CTLA4,CD74,HP |
| | S100A9,CD177,IL1R2,IFNG,CIITA,CD3D |
| | S100A9,CD177,IL1R2,IFNG,CIITA,CTLA4 |
| | S100A9,CD177,IL1R2,IFNG,CIITA,CD74 |
| | S100A9,CD177,IL1R2,IFNG,CIITA,HP |
| | S100A9,CD177,IL1R2,IFNG,CD3D,CTLA4 |
| | S100A9,CD177,IL1R2,IFNG,CD3D,CD74 |
| | S100A9,CD177,IL1R2,IFNG,CD3D,HP |
| | S100A9,CD177,IL1R2,IFNG,CTLA4,CD74 |
| | S100A9,CD177,IL1R2,IFNG,CTLA4,HP |
| | S100A9,CD177,IL1R2,IFNG,CD74,HP |
| | S100A9,CD177,IL1R2,CIITA,CD3D,CTLA4 |
| | S100A9,CD177,IL1R2,CIITA,CD3D,CD74 |
| | S100A9,CD177,IL1R2,CIITA,CD3D,HP |
| | S100A9,CD177,IL1R2,CIITA,CTLA4,CD74 |
| | S100A9,CD177,IL1R2,CIITA,CTLA4,HP |
| | S100A9,CD177,IL1R2,CIITA,CD74,HP |
| | S100A9,CD177,I1L1R2,CD3D,CTLA4,CD74 |
| | S100A9,CD177,IL1R2,CD3D,CTLA4,HP |
| | S100A9,CD177,IL1R2,CD3D,CD74,HP |
| | S100A9,CD177,IL1R2,CTLA4,CD74,HP |
| | S100A9,CD177,IFNG,CIITA,CD3D,CTLA4 |
| | S100A9,CD177,IFNG,CIITA,CD3D,CD74 |
| | S100A9,CD177,IFNG,CIITA,CD3D,HP |
| | S100A9,CD177,IFNG,CIITA,CTLA4,CD74 |
| | S100A9,CD177,IFNG,CIITA,CTLA4,HP |
| | S100A9,CD177,IFNG,CIITA,CD74,HP |
| | S100A9,CD177,IFNG,CD3D,CTLA4,CD74 |
| | S100A9,CD177,IFNG,CD3D,CTLA4,HP |
| | S100A9,CD177,IFNG,CD3D,CD74,HP |
| | S100A9,CD177,IFNG,CTLA4,CD74,HP |
| | S100A9,CD177,CIITA,CD3D,CTLA4,CD74 |
| | S100A9,CD177,CIITA,CD3D,CTLA4,HP |
| | S100A9,CD177,CIITA,CD3D,CD74,HP |
| | S100A9,CD177,CIITA,CTLA4,CD74,HP |
| | S100A9,CD177,CD3D,CTLA4,CD74,HP |
| | S100A9,CX3CR1,IL1R2,IFNG,CIITA,CD3D |
| | S100A9,CX3CR1,IL1R2,IFNG,CIITA,CTLA4 |
| | S100A9,CX3CR1,IL1R2,IFNG,CIITA,CD74 |
| | S100A9,CX3CR1,IL1R2,IFNG,CIITA,HP |
| | S100A9,CX3CR1,IL1R2,IFNG,CD3D,CTLA4 |
| | S100A9,CX3CR1,IL1R2,IFNG,CD3D,CD74 |
| | S100A9,CX3CR1,IL1R2,IFNG,CD3D,HP |
| | S100A9,CX3CR1,IL1R2,IFNG,CTLA4,CD74 |
| | S100A9,CX3CR1,IL1R2,IFNG,CTLA4,HP |
| | S100A9,CX3CR1,IL1R2,IFNG,CD74,HP |
| | S100A9,CX3CR1,IL1R2,CIITA,CD3D,CTLA4 |
| | S100A9,CX3CR1,IL1R2,CIITA,CD3D,CD74 |
| | S100A9,CX3CR1,IL1R2,CIITA,CD3D,HP |
| | S100A9,CX3CR1,IL1R2,CIITA,CTLA4,CD74 |
| | S100A9,CX3CR1,IL1R2,CIITA,CTLA4,HP |
| | S100A9,CX3CR1,IL1R2,CIITA,CD74,HP |
| | S100A9,CX3CR1,IL1R2,CD3D,CTLA4,CD74 |
| | S100A9,CX3CR1,IL1R2,CD3D,CTLA4,HP |
| | S100A9,CX3CR1,IL1R2,CD3D,CD74,HP |
| | S100A9,CX3CR1,IL1R2,CTLA4,CD74,HP |
| | S100A9,CX3CR1,IFNG,CIITA,CD3D,CTLA4 |
| | S100A9,CX3CR1,IFNG,CIITA,CD3D,CD74 |
| | S100A9,CX3CR1,IFNG,CIITA,CD3D,HP |
| | S100A9,CX3CR1,IFNG,CIITA,CTLA4,CD74 |
| | S100A9,CX3CR1,IFNG,CIITA,CTLA4,HP |
| | S100A9,CX3CR1,IFNG,CIITA,CD74,HP |
| | S100A9,CX3CR1,IFNG,CD3D,CTLA4,CD74 |
| | S100A9,CX3CR1,IFNG,CD3D,CTLA4,HP |
| | S100A9,CX3CR1,IFNG,CD3D,CD74,HP |
| | S100A9,CX3CR1,IFNG,CTLA4,CD74,HP |
| | S100A9,CX3CR1,CIITA,CD3D,CTLA4,CD74 |
| | S100A9,CX3CR1,CIITA,CD3D,CTLA4,HP |
| | S100A9,CX3CR1,CIITA,CD3D,CD74,HP |
| | S100A9,CX3CR1,CIITA,CTLA4,CD74,HP |
| | S100A9,CX3CR1,CD3D,CTLA4,CD74,HP |
| | S100A9,IL1R2,IFNG,CIITA,CD3D,CTLA4 |
| | S100A9,IL1R2,IFNG,CIITA,CD3D,CD74 |
| | S100A9,IL1R2,IFNG,CIITA,CD3D,HP |
| | S100A9,IL1R2,IFNG,CIITA,CTLA4,CD74 |
| | S100A9,IL1R2,IFNG,CIITA,CTLA4,HP |
| | S100A9,IL1R2,IFNG,CIITA,CD74,HP |
| | S100A9,IL1R2,IFNG,CD3D,CTLA4,CD74 |
| | S100A9,IL1R2,IFNG,CD3D,CTLA4,HP |
| | S100A9,IL1R2,IFNG,CD3D,CD74,HP |
| | S100A9,IL1R2,IFNG,CTLA4,CD74,HP |
| | S100A9,IL1R2,CIITA,CD3D,CTLA4,CD74 |
| | S100A9,IL1R2,CIITA,CD3D,CTLA4,HP |
| | S100A9,IL1R2,CIITA,CD3D,CD74,HP |
| | S100A9,IL1R2,CIITA,CTLA4,CD74,HP |
| | S100A9,IL1R2,CD3D,CTLA4,CD74,HP |
| | S100A9,IFNG,CIITA,CD3D,CTLA4,CD74 |
| | S100A9,IFNG,CIITA,CD3D,CTLA4,HP |
| | S100A9,IFNG,CIITA,CD3D,CD74,HP |
| | S100A9,IFNG,CIITA,CTLA4,CD74,HP |
| | S100A9,IFNG,CD3D,CTLA4,CD74,HP |
| | S100A9,CIITA,CD3D,CTLA4,CD74,HP |
| | TAP2,CD177,CX3CR1,IL1R2,IFNG,CIITA |
| | TAP2,CD177,CX3CR1,IL1R2,IFNG,CD3D |
| | TAP2,CD177,CX3CR1,IL1R2,IFNG,CTLA4 |
| | TAP2,CD177,CX3CR1,IL1R2,IFNG,CD74 |
| | TAP2,CD177,CX3CR1,IL1R2,IFNG,HP |
| | TAP2,CD177,CX3CR1,IL1R2,CIITA,CD3D |
| | TAP2,CD177,CX3CR1,IL1R2,CIITA,CTLA4 |
| | TAP2,CD177,CX3CR1,IL1R2,CIITA,CD74 |
| | TAP2,CD177,CX3CR1,IL1R2,CIITA,HP |
| | TAP2,CD177,CX3CR1,IL1R2,CD3D,CTLA4 |
| | TAP2,CD177,CX3CR1,IL1R2,CD3D,CD74 |
| | TAP2,CD177,CX3CR1,IL1R2,CD3D,HP |
| | TAP2,CD177,CX3CR1,IL1R2,CTLA4,CD74 |
| | TAP2,CD177,CX3CR1,IL1R2,CTLA4,HP |
| | TAP2,CD177,CX3CR1,IL1R2,CD74,HP |
| | TAP2,CD177,CX3CR1,IFNG,CIITA,CD3D |
| | TAP2,CD177,CX3CR1,IFNG,CIITA,CTLA4 |
| | TAP2,CD177,CX3CR1,IFNG,CIITA,CD74 |
| | TAP2,CD177,CX3CR1,IFNG,CIITA,HP |
| | TAP2,CD177,CX3CR1,IFNG,CD3D,CTLA4 |
| | TAP2,CD177,CX3CR1,IFNG,CD3D,CD74 |
| | TAP2,CD177,CX3CR1,IFNG,CD3D,HP |
| | TAP2,CD177,CX3CR1,IFNG,CTLA4,CD74 |
| | TAP2,CD177,CX3CR1,IFNG,CTLA4,HP |
| | TAP2,CD177,CX3CR1,IFNG,CD74,HP |
| | TAP2,CD177,CX3CR1,CIITA,CD3D,CTLA4 |
| | TAP2,CD177,CX3CR1,CIITA,CD3D,CD74 |
| | TAP2,CD177,CX3CR1,CIITA,CD3D,HP |
| | TAP2,CD177,CX3CR1, CIITA,CTLA4,CD74 |
| | TAP2,CD177,CX3CR1,CIITA,CTLAA,HP |
| | TAP2,CD177,CX3CR1,CIITA,CD74,HP |
| | TAP2,CD177,CX3CR1,CD3D,CTLA4,CD74 |
| | TAP2,CD177,CX3CR1,CD3D,CTLA4,HP |
| | TAP2,CD177,CX3CR1,CD3D,CD74,HP |
| | TAP2,CD177,CX3CR1,CTLA4,CD74,HP |
| | TAP2,CD177,IL1R2,IFNG,CIITA,CD3D |
| | TAP2,CD177,IL1R2,IFNG,CIITA,CTLA4 |
| | TAP2,CD177,IL1R2,IFNG,CIITA,CD74 |
| | TAP2,CD177,IL1R2,IFNG,CIITA,HP |
| | TAP2,CD177,IL1R2,IFNG,CD3D,CTLA4 |
| | TAP2,CD177,IL1R2,IFNG,CD3D,CD74 |
| | TAP2,CD177,IL1R2,IFNG,CD3D,HP |
| | TAP2,CD177,IL1R2,IFNG,CTLA4,CD74 |
| | TAP2,CD177,IL1R2,IFNG,CTLA4,HP |
| | TAP2,CD177,IL1R2,IFNG,CD74,HP |
| | TAP2,CD177,IL1R2,CIITA,CD3D,CTLA4 |
| | TAP2,CD177,IL1R2,CIITA,CD3D,CD74 |
| | TAP2,CD177,IL1R2,CIITA,CD3D,HP |
| | TAP2,CD177,IL1R2,CIITA,CTLA4,CD74 |
| | TAP2,CD177,IL1R2,CIITA,CTLA4,HP |
| | TAP2,CD177,IL1R2,CIITA,CD74,HP |
| | TAP2,CD177,IL1R2,CD3D,CTLA4,CD74 |
| | TAP2,CD177,IL1R2,CD3D,CTLA4,HP |
| | TAP2,CD177,IL1R2,CD3D,CD74,HP |
| | TAP2,CD177,IL1R2,CTLA4,CD74,HP |
| | TAP2,CD177,IFNG,CIITA,CD3D,CTLA4 |
| | TAP2,CD177,IFNG,CIITA,CD3D,CD74 |
| | TAP2,CD177,IFNG,CIITA,CD3D,HP |
| | TAP2,CD177,IFNG,CIITA,CTLA4,CD74 |
| | TAP2,CD177,IFNG,CIITA,CTLA4,HP |
| | TAP2,CD177,IFNG,CIITA,CD74,HP |
| | TAP2,CD177,IFNG,CD3D,CTLA4,CD74 |
| | TAP2,CD177,IFNG,CD3D,CTLA4,HP |
| | TAP2,CD177,IFNG,CD3D,CD74,HP |
| | TAP2,CD177,IFNG,CTLA4,CD74,HP |
| | TAP2,CD177,CIITA,CD3D,CTLA4,CD74 |
| | TAP2,CD177,CIITA,CD3D,CTLA4, HP |
| | TAP2,CD177,CIITA,CD3D,CD74,HP |
| | TAP2,CD177,CIITA,CTLA4,CD74,HP |
| | TAP2,CD177,CD3D,CTLA4,CD74,HP |
| | TAP2,CX3CR1,IL1R2,IFNG,CIITA,CD3D |
| | TAP2,CX3CR1,IL1R2,IFNG,CIITA,CTLA4 |
| | TAP2,CX3CR1,IL1R2,IFNG,CIITA,CD74 |
| | TAP2,CX3CR1,IL1R2,IFNG,CIITA,HP |
| | TAP2,CX3CR1,IL1R2,IFNG,CD3D,CTLA4 |
| | TAP2,CX3CR1,IL1R2,IFNG,CD3D,CD74 |
| | TAP2,CX3CR1,IL1R2,IFNG,CD3D,HP |
| | TAP2,CX3CR1,IL1R2,IFNG,CTLA4,CD74 |
| | TAP2,CX3CR1,IL1R2,IFNG,CTLA4,HP |
| | TAP2,CX3CR1,IL1R2,IFNG,CD74,HP |
| | TAP2,CX3CR1,IL1R2,CIITA,CD3D,CTLA4 |
| | TAP2,CX3CR1,IL1R2,CIITA,CD3D,CD74 |
| | TAP2,CX3CR1,IL1R2,CIITA,CD3D,HP |
| | TAP2,CX3CR1,IL1R2,CIITA,CTLA4,CD74 |
| | TAP2,CX3CR1,IL1R2,CIITA,CTLA4,HP |
| | TAP2,CX3CR1,IL1R2,CIITA,CD74,HP |
| | TAP2,CX3CR1,IL1R2,CD3D,CTLA4,CD74 |
| | TAP2,CX3CR1,IL1R2,CD3D,CTLA4,HP |
| | TAP2,CX3CR1,IL1R2,CD3D,CD74,HP |
| | TAP2,CX3CR1,IL1R2,CTLA4,CD74,HP |
| | TAP2,CX3CR1,IFNG,CIITA,CD3D,CTLA4 |
| | TAP2,CX3CR1,IFNG,CIITA,CD3D,CD74 |
| | TAP2,CX3CR1,IFNG,CIITA,CD3D,HP |
| | TAP2,CX3CR1,IFNG,CIITA,CTLA4,CD74 |
| | TAP2,CX3CR1,IFNG,CIITA,CTLA4,HP |
| | TAP2,CX3CR1,IFNG,CIITA,CD74,HP |
| | TAP2,CX3CR1,IFNG,CD3D,CTLA4,CD74 |
| | TAP2,CX3CR1,IFNG,CD3D,CTLA4,HP |
| | TAP2,CX3CR1,IFNG,CD3D,CD74,HP |
| | TAP2,CX3CR1,IFNG,CTLA4,CD74,HP |
| | TAP2,CX3CR1,CIITA,CD3D,CTLA4,CD74 |
| | TAP2,CX3CR1,CIITA,CD3D,CTLA4,HP |
| | TAP2,CX3CR1,CIITA,CD3D,CD74,HP |
| | TAP2,CX3CR1,CIITA,CTLA4,CD74,HP |
| | TAP2,CX3CR1,CD3D,CTLA4,CD74,HP |
| | TAP2,IL1R2,IFNG,CIITA,CD3D,CTLA4 |
| | TAP2,IL1R2,IFNG,CIITA,CD3D,CD74 |
| | TAP2,IL1R2,IFNG,CIITA,CD3D,HP |
| | TAP2,IL1R2,IFNG,CIITA,CTLA4,CD74 |
| | TAP2,IL1R2,IFNG,CIITA,CTLA4,HP |
| | TAP2,IL1R2,IFNG,CIITA,CD74,HP |
| | TAP2,IL1R2,IFNG,CD3D,CTLA4,CD74 |
| | TAP2,IL1R2,IFNG,CD3D,CTLA4,HP |
| | TAP2,IL1R2,IFNG,CD3D,CD74,HP |
| | TAP2,IL1R2,IFNG,CTLA4,CD74,HP |
| | TAP2,IL1R2,CIITA,CD3D,CTLA4,CD74 |
| | TAP2,IL1R2,CIITA,CD3D,CTLA4,HP |
| | TAP2,IL1R2,CIITA,CD3D,CD74,HP |
| | TAP2,IL1R2,CIITA,CTLA4,CD74,HP |
| | TAP2,IL1R2,CD3D,CTLA4,CD74,HP |
| | TAP2,IFNG,CIITA,CD3D,CTLA4,CD74 |
| | TAP2,IFNG,CIITA,CD3D,CTLA4,HP |
| | TAP2,IFNG,CIITA,CD3D,CD74,HP |
| | TAP2,IFNG,CIITA,CTLA4,CD74,HP |
| | TAP2,IFNG,CD3D,CTLA4,CD74,HP |
| | TAP2,CIITA,CD3D,CTLA4,CD74,HP |
| | CD177,CX3CR1,IL1R2,IFNG,CIITA,CD3D |
| | CD177,CX3CR1,IL1R2,IFNG,CIITA,CTLA4 |
| | CD177,CX3CR1,IL1R2,IFNG,CIITA,CD74 |
| | CD177,CX3CR1,IL1R2,IFNG,CIITA,HP |
| | CD177,CX3CR1,IL1R2,IFNG,CD3D,CTLA4 |
| | CD177,CX3CR1,IL1R2,IFNG,CD3D,CD74 |
| | CD177,CX3CR1,IL1R2,IFNG,CD3D,HP |
| | CD177,CX3CR1,IL1R2,IFNG,CTLA4,CD74 |
| | CD177,CX3CR1,IL1R2,IFNG,CTLA4,HP |
| | CD177,CX3CR1,IL1R2,IFNG,CD74,HP |
| | CD177,CX3CR1,IL1R2,CIITA,CD3D,CTLA4 |
| | CD177,CX3CR1,IL1R2,CIITA,CD3D,CD74 |
| | CD177,CX3CR1,IL1R2,CIITA,CD3D,HP |
| | CD177,CX3CR1,IL1R2,CIITA,CTLA4,CD74 |
| | CD177,CX3CR1,IL1R2,CIITA,CTLA4,HP |
| | CD177,CX3CR1,IL1R2,CIITA,CD74,HP |
| | CD177,CX3CR1,IL1R2,CD3D,CTLA4,CD74 |
| | CD177,CX3CR1,IL1R2,CD3D,CTLA4,HP |
| | CD177,CX3CR1,IL1R2,CD3D,CD74,HP |
| | CD177,CX3CR1,IL1R2,CTLA4,CD74,HP |
| | CD177,CX3CR1,IFNG,CIITA,CD3D,CTLA4 |
| | CD177,CX3CR1,IFNG,CIITA,CD3D,CD74 |
| | CD177,CX3CR1,IFNG,CIITA,CD3D,HP |
| | CD177,CX3CR1,IFNG,CIITA,CTLA4,CD74 |
| | CD177,CX3CR1,IFNG,CIITA,CTLA4,HP |
| | CD177,CX3CR1,IFNG,CIITA,CD74,HP |
| | CD177,CX3CR1,IFNG,CD3D,CTLA4,CD74 |
| | CD177,CX3CR1,IFNG,CD3D,CTLA4,HP |
| | CD177,CX3CR1,IFNG,CD3D,CD74,HP |
| | CD177,CX3CR1,IFNG,CTLA4,CD74,HP |
| | CD177,CX3CR1,CIITA,CD3D,CTLA4,CD74 |
| | CD177,CX3CR1,CIITA,CD3D,CTLA4,HP |
| | CD177,CX3CR1,CIITA,CD3D,CD74,HP |
| | CD177,CX3CR1,CIITA,CTLA4,CD74,HP |
| | CD177,CX3CR1,CD3D,CTLA4,CD74,HP |
| | CD177,IL1R2,IFNG,CIITA,CD3D,CTLA4 |
| | CD177,IL1R2,IFNG,CIITA,CD3D,CD74 |
| | CD177,IL1R2,IFNG,CIITA,CD3D,HP |
| | CD177,IL1R2,IFNG,CIITA,CTLA4,CD74 |
| | CD177,IL1R2,IFNG,CIITA,CTLA4,HP |
| | CD177,IL1R2,IFNG,CIITA,CD74,HP |
| | CD177,IL1R2,IFNG,CD3D,CTLA4,CD74 |
| | CD177,IL1R2,IFNG,CD3D,CTLA4,HP |
| | CD177,IL1R2,IFNG,CD3D,CD74,HP |
| | CD177,IL1R2,IFNG,CTLA4,CD74,HP |
| | CD177,IL1R2,CIITA,CD3D,CTLA4,CD74 |
| | CD177,IL1R2,CIITA,CD3D,CTLA4,HP |
| | CD177,IL1R2,CIITA,CD3D,CD74,HP |
| | CD177,IL1R2,CIITA,CTLA4,CD74,HP |
| | CD177,IL1R2,CD3D,CTLA4,CD74,HP |
| | CD177,IFNG,CIITA,CD3D,CTLA4,CD74 |
| | CD177,IFNG,CIITA,CD3D,CTLA4,HP |
| | CD177,IFNG,CIITA,CD3D,CD74,HP |
| | CD177,IFNG,CIITA,CTLA4,CD74,HP |
| | CD177,IFNG,CD3D,CTLA4,CD74,HP |
| | CD177,CIITA,CD3D,CTLA4,CD74,HP |
| | CX3CR1,IL1R2,IFNG,CIITA,CD3D,CTLA4 |
| | CX3CR1,IL1R2,IFNG,CIITA,CD3D,CD74 |
| | CX3CR1,IL1R2,IFNG,CIITA,CD3D,HP |
| | CX3CR1,IL1R2,IFNG,CIITA,CTLA4,CD74 |
| | CX3CR1,IL1R2,IFNG,CIITA,CTLA4,HP |
| | CX3CR1,IL1R2,IFNG,CIITA,CD74,HP |
| | CX3CR1,IL1R2,IFNG,CD3D,CTLA4,CD74 |
| | CX3CR1,IL1R2,IFNG,CD3D,CTLA4,HP |
| | CX3CR1,IL1R2,IFNG,CD3D,CD74,HP |
| | CX3CR1,IL1R2,IFNG,CTLA4,CD74,HP |
| | CX3CR1,IL1R2,CIITA,CD3D,CTLA4,CD74 |
| | CX3CR1,IL1R2,CIITA,CD3D,CTLA4,HP |
| | CX3CR1,IL1R2,CIITA,CD3D,CD74,HP |
| | CX3CR1,IL1R2,CIITA,CTLA4,CD74,HP |
| | CX3CR1,IL1R2,CD3D,CTLA4,CD74,HP |
| | CX3CR1,IFNG,CIITA,CD3D,CTLA4,CD74 |
| | CX3CR1,IFNG,CIITA,CD3D,CTLA4,HP |
| | CX3CR1,IFNG,CIITA,CD3D,CD74,HP |
| | CX3CR1,IFNG,CIITA,CTLA4,CD74,HP |
| | CX3CR1,IFNG,CD3D,CTLA4,CD74,HP |
| | CX3CR1,CIITA,CD3D,CTLA4,CD74,HP |
| | IL1R2,IFNG,CIITA,CD3D,CTLA4,CD74 |
| | IL1R2,lFNG,CllTA,CD3D,CTLA4,HP |
| | IL1R2,IFNG,CIITA,CD3D,CD74,HP |
| | IL1R2,IFNG,CIITA,CTLA4,CD74,HP |
| | IL1R2,IFNG,CD3D,CTLA4,CD74,HP |
| | IL1R2,CIITA,CD3D,CTLA4,CD74,HP |
| | IFNG,CIITA,CD3D,CTLA4,CD74,HP |
| **Combinaisons de sept gènes** | S100A9,TAP2,CD177,CX3CR1,IL1R2,IFNG,CIITA |
| | S100A9,TAP2,CD177,CX3CR1,IL1R2,IFNG,CD3D |
| | S100A9,TAP2,CD177,CX3CR1,IL1R2,IFNG,CTLA4 |
| | S100A9,TAP2,CD177,CX3CR1,IL1R2,IFNG,CD74 |
| | S100A9,TAP2,CD177,CX3CR1,IL1R2,IFNG,HP |
| | S100A9,TAP2,CD177,CX3CR1,IL1R2,CIITA,CD3D |
| | S100A9,TAP2,CD177,CX3CR1,IL1R2,CIITA,CTLA4 |
| | S100A9,TAP2,CD177,CX3CR1,IL1R2,CIITA,CD74 |
| | S100A9,TAP2,CD177,CX3CR1,IL1R2,CIITA,HP |
| | S100A9,TAP2,CD177,CX3CR1,IL1R2,CD3D,CTLA4 |
| | S100A9,TAP2,CD177,CX3CR1,IL1R2,CD3D,CD74 |
| | S100A9,TAP2,CD177,CX3CR1,IL1R2,CD3D,HP |
| | S100A9,TAP2,CD177,CX3CR1,IL1R2,CTLA4,CD74 |
| | S100A9,TAP2,CD177,CX3CR1,IL1R2,CTLA4,HP |
| | S100A9,TAP2,CD177,CX3CR1,IL1R2,CD74,HP |
| | S100A9,TAP2,CD177,CX3CR1,IFNG,CIITA,CD3D |
| | S100A9,TAP2,CD177,CX3CR1,lFNG,CllTA,CTLA4 |
| | S100A9,TAP2,CD177,CX3CR1,IFNG,CIITA,CD74 |
| | S100A9,TAP2,CD177,CX3CR1,IFNG,CIITA,HP |
| | S100A9,TAP2,CD177,CX3CR1,IFNG,CD3D,CTLA4 |
| | S100A9,TAP2,CD177,CX3CR1,IFNG,CD3D,CD74 |
| | S100A9,TAP2,CD177,CX3CR1,IFNG,CD3D,HP |
| | S100A9,TAP2,CD177,CX3CR1,IFNG,CTLA4,CD74 |
| | S100A9,TAP2,CD177,CX3CR1,IFNG,CTLA4,HP |
| | S100A9,TAP2,CD177,CX3CR1,IFNG,CD74,HP |
| | S100A9,TAP2,CD177,CX3CR1,CIITA,CD3D,CTLA4 |
| | S100A9,TAP2,CD177,CX3CR1,CIITA,CD3D,CD74 |
| | S100A9,TAP2,CD177,CX3CR1,CIITA,CD3D, HP |
| | S100A9,TAP2,CD177,CX3CR1,CIITA,CTLA4,CD74 |
| | S100A9,TAP2,CD177, CX3CR1,CIITA,CTLA4,HP |
| | S100A9,TAP2,CD177,CX3CR1,CIITA,CD74,HP |
| | S100A9,TAP2,CD177,CX3CR1,CD3D,CTLA4,CD74 |
| | S100A9,TAP2,CD177,CX3CR1,CD3D,CTLA4,HP |
| | S100A9,TAP2,CD177,CX3CR1,CD3D,CD74,HP |
| | S100A9,TAP2,CD177,CX3CR1,CTLA4,CD74,HP |
| | S100A9,TAP2,CD177,IL1R2,IFNG,CIITA,CD3D |
| | S100A9,TAP2,CD177,IL1R2,IFNG,CIITA,CTLA4 |
| | S100A9,TAP2,CD177,IL1R2,IFNG,CIITA,CD74 |
| | S100A9,TAP2,CD177,IL1R2,IFNG,CIITA,HP |
| | S100A9,TAP2,CD177,IL1R2,IFNG,CD3D,CTLA4 |
| | S100A9,TAP2,CD177,IL1R2,IFNG,CD3D,CD74 |
| | S100A9,TAP2,CD177,IL1R2,IFNG,CD3D,HP |
| | S100A9,TAP2,CD177,IL1R2,IFNG,CTLA4,CD74 |
| | S100A9,TAP2,CD177,IL1R2,IFNG,CTLA4,HP |
| | S100A9,TAP2,CD177,IL1R2,IFNG,CD74,HP |
| | S100A9,TAP2,CD177,IL1R2,CIITA,CD3D,CTLA4 |
| | S100A9,TAP2,CD177,IL1R2,CIITA,CD3D,CD74 |
| | S100A9,TAP2,CD177,IL1R2,CIITA,CD3D,HP |
| | S100A9,TAP2,CD177,IL1R2,CIITA,CTLA4,CD74 |
| | S100A9,TAP2,CD177,IL1R2,CIITA,CTLA4,HP |
| | S100A9,TAP2,CD177,IL1R2,CIITA,CD74,HP |
| | S100A9,TAP2,CD177,IL1R2,CD3D,CTLA4,CD74 |
| | S100A9,TAP2,CD177,IL1R2,CD3D,CTLA4,HP |
| | S100A9,TAP2,CD177,IL1R2,CD3D,CD74,HP |
| | S100A9,TAP2,CD177, IL1R2,CTLA4,CD74,H P |
| | S100A9,TAP2,CD177,IFNG,CIITA,CD3D,CTLA4 |
| | S100A9,TAP2,CD177,IFNG,CIITA,CD3D,CD74 |
| | S100A9,TAP2,CD177,IFNG,CIITA,CD3D,HP |
| | S100A9,TAP2,CD177,IFNG,CIITA,CTLA4,CD74 |
| | S100A9,TAP2,CD177,IFNG,CIITA,CTLA4,HP |
| | S100A9,TAP2,CD177,IFNG,CIITA,CD74,HP |
| | S100A9,TAP2,CD177,IFNG,CD3D,CTLA4,CD74 |
| | S100A9,TAP2,CD177, IFNG,CD3D,CTLA4,H P |
| | S100A9,TAP2,CD177,IFNG,CD3D,CD74,HP |
| | S100A9,TAP2,CD177,IFNG,CTLA4,CD74,HP |
| | S100A9,TAP2,CD177,CIITA,CD3D,CTLA4,CD74 |
| | S100A9,TAP2,CD177,CIITA,CD3D,CTLA4,HP |
| | S100A9,TAP2,CD177,CIITA,CD3D,CD74,HP |
| | S100A9,TAP2,CD177,CIITA,CTLA4,CD74,HP |
| | S100A9,TAP2,CD177,CD3D,CTLA4,CD74,HP |
| | S100A9,TAP2,CX3CR1,IL1R2,IFNG,CIITA,CD3D |
| | S100A9,TAP2,CX3CR1,IL1R2,IFNG,CIITA,CTLA4 |
| | S100A9,TAP2,CX3CR1,IL1R2,IFNG,CIITA,CD74 |
| | S100A9,TAP2,CX3CR1,IL1R2,IFNG,CIITA,HP |
| | S100A9,TAP2,CX3CR1,IL1R2,IFNG,CD3D,CTLA4 |
| | S100A9,TAP2,CX3CR1,IL1R2,IFNG,CD3D,CD74 |
| | S100A9,TAP2,CX3CR1,IL1R2,IFNG,CD3D,HP |
| | S100A9,TAP2,CX3CR1,IL1R2,IFNG,CTLA4,CD74 |
| | S100A9,TAP2,CX3CR1,IL1R2,IFNG,CTLA4,HP |
| | S100A9,TAP2,CX3CR1,IL1R2,IFNG,CD74,HP |
| | S100A9,TAP2,CX3CR1,IL1R2,CIITA,CD3D,CTLA4 |
| | S100A9,TAP2,CX3CR1,IL1R2,CIITA,CD3D,CD74 |
| | S100A9,TAP2,CX3CR1,IL1R2,CIITA,CD3D,HP |
| | S100A9,TAP2,CX3CR1,IL1R2,CIITA,CTLA4,CD74 |
| | S100A9,TAP2,CX3CR1,IL1R2,CIITA,CTLA4,HP |
| | S100A9,TAP2,CX3CR1,IL1R2,CIITA,CD74,HP |
| | S100A9,TAP2,CX3CR1,IL1R2,CD3D,CTLA4,CD74 |
| | S100A9,TAP2,CX3CR1,IL1R2,CD3D,CTLA4,HP |
| | S100A9,TAP2,CX3CR1,IL1R2,CD3D,CD74,HP |
| | S100A9,TAP2,CX3CR1,IL1R2,CTLA4,CD74,HP |
| | S100A9,TAP2,CX3CR1,IFNG,CIITA,CD3D,CTLA4 |
| | S100A9,TAP2,CX3CR1,IFNG,CIITA,CD3D,CD74 |
| | S100A9,TAP2,CX3CR1,IFNG,CIITA,CD3D,HP |
| | S100A9,TAP2,CX3CR1,IFNG,CIITA,CTLA4,CD74 |
| | S100A9,TAP2,CX3CR1,IFNG,CIITA,CTLA4,HP |
| | S100A9,TAP2,CX3CR1,IFNG,CIITA,CD74,HP |
| | S100A9,TAP2,CX3CR1,IFNG,CD3D,CTLA4,CD74 |
| | S100A9,TAP2,CX3CR1,IFNG,CD3D,CTLA4,HP |
| | S100A9,TAP2,CX3CR1,IFNG,CD3D,CD74,HP |
| | S100A9,TAP2,CX3CR1,IFNG,CTLA4,CD74,HP |
| | S100A9,TAP2,CX3CR1,CIITA,CD3D,CTLA4,CD74 |
| | S100A9,TAP2,CX3CR1,CIITA,CD3D,CTLA4,HP |
| | S100A9,TAP2,CX3CR1,CIITA,CD3D,CD74,HP |
| | S100A9,TAP2,CX3CR1,CIITA,CTLA4,CD74,HP |
| | S100A9,TAP2,CX3CR1,CD3D,CTLA4,CD74,HP |
| | S100A9,TAP2,IL1R2,IFNG,CIITA,CD3D,CTLA4 |
| | S100A9,TAP2,IL1R2,IFNG,CIITA,CD3D,CD74 |
| | S100A9,TAP2,IL1R2,IFNG,CIITA,CD3D,HP |
| | S100A9,TAP2,IL1R2,IFNG,CIITA,CTLA4,CD74 |
| | S100A9,TAP2,IL1R2,IFNG,CIITA,CTLA4,HP |
| | S100A9,TAP2,IL1R2,IFNG,CIITA,CD74,HP |
| | S100A9,TAP2,IL1R2,IFNG,CD3D,CTLA4,CD74 |
| | S100A9,TAP2,IL1R2,IFNG,CD3D,CTLA4,HP |
| | S100A9,TAP2,IL1R2,IFNG,CD3D,CD74,HP |
| | S100A9,TAP2,IL1R2,IFNG,CTLA4,CD74,HP |
| | S100A9,TAP2,IL1R2,CIITA,CD3D,CTLA4,CD74 |
| | S100A9,TAP2,IL1R2,CIITA,CD3D,CTLA4,HP |
| | S100A9,TAP2,IL1R2,CIITA,CD3D,CD74,HP |
| | S100A9,TAP2,IL1R2,CIITA,CTLA4,CD74,HP |
| | S100A9,TAP2,IL1R2,CD3D,CTLA4,CD74,HP |
| | S100A9,TAP2,IFNG,CIITA,CD3D,CTLA4,CD74 |
| | S100A9,TAP2,IFNG,CIITA,CD3D,CTLA4,HP |
| | S100A9,TAP2,IFNG,CIITA,CD3D,CD74,HP |
| | S100A9,TAP2,IFNG,CIITA,CTLA4,CD74,HP |
| | S100A9,TAP2,IFNG,CD3D,CTLA4,CD74,HP |
| | S100A9,TAP2,CIITA,CD3D,CTLA4,CD74,HP |
| | S100A9,CD177,CX3CR1,IL1R2,IFNG,CIITA,CD3D |
| | S100A9,CD177,CX3CR1,IL1R2,IFNG,CIITA,CTLA4 |
| | S100A9,CD177,CX3CR1,IL1R2,IFNG,CIITA,CD74 |
| | S100A9,CD177,CX3CR1,IL1R2,IFNG,CIITA,HP |
| | S100A9,CD177,CX3CR1,IL1R2,IFNG,CD3D,CTLA4 |
| | S100A9,CD177,CX3CR1,IL1R2,IFNG,CD3D,CD74 |
| | S100A9,CD177,CX3CR1,IL1R2,IFNG,CD3D,HP |
| | S100A9,CD177,CX3CR1,IL1R2,IFNG,CTLA4,CD74 |
| | S100A9,CD177,CX3CR1,IL1R2,IFNG,CTLA4,HP |
| | S100A9,CD177,CX3CR1,IL1R2,IFNG,CD74,HP |
| | S100A9,CD177,CX3CR1,IL1R2,CIITA,CD3D,CTLA4 |
| | S100A9,CD177,CX3CR1,IL1R2,CIITA,CD3D,CD74 |
| | S100A9,CD177,CX3CR1,IL1R2,CIITA,CD3D,HP |
| | S100A9,CD177,CX3CR1,IL1R2,CIITA,CTLA4,CD74 |
| | S100A9,CD177,CX3CR1,IL1R2,CIITA,CTLA4,HP |
| | S100A9,CD177,CX3CR1,IL1R2,CIITA,CD74,HP |
| | S100A9,CD177,CX3CR1,IL1R2,CD3D,CTLA4,CD74 |
| | S100A9,CD177,CX3CR1,IL1R2,CD3D,CTLA4,HP |
| | S100A9,CD177,CX3CR1,IL1R2,CD3D,CD74,HP |
| | S100A9,CD177,CX3CR1,IL1R2,CTLA4,CD74,HP |
| | S100A9,CD177,CX3CR1,IFNG,CIITA,CD3D,CTLA4 |
| | S100A9,CD177,CX3CR1,IFNG,CIITA,CD3D,CD74 |
| | S100A9,CD177,CX3CR1,IFNG,CIITA,CD3D,HP |
| | S100A9,CD177,CX3CR1,IFNG,CIITA,CTLA4,CD74 |
| | S100A9,CD177,CX3CR1,IFNG,CIITA,CTLA4,HP |
| | S100A9,CD177,CX3CR1,IFNG,CIITA,CD74,HP |
| | S100A9,CD177,CX3CR1,IFNG,CD3D,CTLA4,CD74 |
| | S100A9,CD177,CX3CR1,IFNG,CD3D,CTLA4,HP |
| | S100A9,CD177,CX3CR1,IFNG,CD3D,CD74,HP |
| | S100A9,CD177,CX3CR1,IFNG,CTLA4,CD74,HP |
| | S100A9,CD177,CX3CR1,CIITA,CD3D,CTLA4,CD74 |
| | S100A9,CD177,CX3CR1,CIITA,CD3D,CTLA4,HP |
| | S100A9,CD177,CX3CR1,CIITA,CD3D,CD74,HP |
| | S100A9,CD177,CX3CR1,CIITA,CTLA4,CD74,HP |
| | S100A9,CD177,CX3CR1,CD3D,CTLA4,CD74,HP |
| | S100A9,CD177,IL1R2,IFNG,CIITA,CD3D,CTLA4 |
| | S100A9,CD177,IL1R2,IFNG,CIITA,CD3D,CD74 |
| | S100A9,CD177,IL1R2,IFNG,CIITA,CD3D,HP |
| | S100A9,CD177,IL1R2,IFNG,CIITA,CTLA4,CD74 |
| | S100A9,CD177,IL1R2,IFNG,CIITA,CTLA4, HP |
| | S100A9,CD177,IL1R2,IFNG,CIITA,CD74,HP |
| | S100A9,CD177,IL1R2,IFNG,CD3D,CTLA4,CD74 |
| | S100A9,CD177,IL1R2,IFNG,CD3D,CTLA4,HP |
| | S100A9,CD177,IL1R2,IFNG,CD3D,CD74,HP |
| | S100A9,CD177,IL1R2,IFNG,CTLA4,CD74,HP |
| | S100A9,CD177,IL1R2,CIITA,CD3D,CTLA4,CD74 |
| | S100A9,CD177,IL1R2,CIITA,CD3D,CTLA4,HP |
| | S100A9,CD177,IL1R2,CIITA,CD3D,CD74,HP |
| | S100A9,CD177,IL1R2,CIITA,CTLA4,CD74,HP |
| | S100A9,CD177,IL1R2,CD3D,CTLA4,CD74,HP |
| | S100A9,CD177,IFNG,CIITA,CD3D,CTLA4,CD74 |
| | S100A9,CD177,IFNG,CIITA,CD3D,CTLA4,HP |
| | S100A9,CD177,IFNG,CIITA,CD3D,CD74,HP |
| | S100A9,CD177,IFNG,CIITA,CTLA4,CD74,HP |
| | S100A9,CD177,IFNG,CD3D,CTLA4,CD74,HP |
| | S100A9,CD177,CIITA,CD3D,CTLA4,CD74,HP |
| | S100A9,CX3CR1,IL1R2,IFNG,CIITA,CD3D,CTLA4 |
| | S100A9,CX3CR1,IL1R2,IFNG,CIITA,CD3D,CD74 |
| | S100A9,CX3CR1,IL1R2,IFNG,CIITA,CD3D,HP |
| | S100A9,CX3CR1,IL1R2,IFNG,CIITA,CTLA4,CD74 |
| | S100A9,CX3CR1,IL1R2,IFNG,CIITA,CTLA4,HP |
| | S100A9,CX3CR1,IL1R2,IFNG,CIITA,CD74,HP |
| | S100A9,CX3CR1,IL1R2,IFNG,CD3D,CTLA4,CD74 |
| | S100A9,CX3CR1,IL1R2,IFNG,CD3D,CTLA4,HP |
| | S100A9,CX3CR1,IL1R2,IFNG,CD3D,CD74,HP |
| | S100A9,CX3CR1,IL1R2,IFNG,CTLA4,CD74,HP |
| | S100A9,CX3CR1,IL1R2,CIITA,CD3D,CTLA4,CD74 |
| | S100A9,CX3CR1,IL1R2,CIITA,CD3D,CTLA4,HP |
| | S100A9,CX3CR1,IL1R2,CIITA,CD3D,CD74,HP |
| | S100A9,CX3CR1,IL1R2,CIITA,CTLA4,CD74,HP |
| | S100A9,CX3CR1,IL1R2,CD3D,CTLA4,CD74,HP |
| | S100A9,CX3CR1,IFNG,CIITA,CD3D,CTLA4,CD74 |
| | S100A9,CX3CR1,IFNG,CIITA,CD3D,CTLA4,HP |
| | S100A9,CX3CR1,IFNG,CIITA,CD3D,CD74,HP |
| | S100A9,CX3CR1,IFNG,CIITA,CTLA4,CD74,HP |
| | S100A9,CX3CR1,IFNG,CD3D,CTLA4,CD74,HP |
| | S100A9,CX3CR1,CIITA,CD3D,CTLA4,CD74,HP |
| | S100A9,IL1R2,IFNG,CIITA,CD3D,CTLA4,CD74 |
| | S100A9,IL1R2,IFNG,CIITA,CD3D,CTLA4,HP |
| | S100A9,IL1R2,IFNG,CIITA,CD3D,CD74,HP |
| | S100A9,IL1R2,IFNG,CIITA,CTLA4,CD74,HP |
| | S100A9,IL1R2,IFNG,CD3D,CTLA4,CD74,HP |
| | S100A9,IL1R2,CIITA,CD3D,CTLA4,CD74,HP |
| | S100A9,IFNG,CIITA,CD3D,CTLA4,CD74,HP |
| | TAP2,CD177,CX3CR1,IL1R2,IFNG,CIITA,CD3D |
| | TAP2,CD177,CX3CR1,IL1R2,IFNG,CIITA,CTLA4 |
| | TAP2,CD177,CX3CR1,IL1R2,IFNG,CIITA,CD74 |
| | TAP2,CD177,CX3CR1,IL1R2,IFNG,CIITA,HP |
| | TAP2,CD177,CX3CR1,IL1R2,IFNG,CD3D,CTLA4 |
| | TAP2,CD177,CX3CR1,IL1R2,IFNG,CD3D,CD74 |
| | TAP2,CD177,CX3CR1,IL1R2,IFNG,CD3D,HP |
| | TAP2,CD177,CX3CR1,IL1R2,IFNG,CTLA4,CD74 |
| | TAP2,CD177,CX3CR1,IL1R2,IFNG,CTLA4,HP |
| | TAP2,CD177,CX3CR1,IL1R2,IFNG,CD74,HP |
| | TAP2,CD177,CX3CR1,IL1R2,CIITA,CD3D,CTLA4 |
| | TAP2,CD177,CX3CR1,IL1R2,CIITA,CD3D,CD74 |
| | TAP2,CD177,CX3CR1,IL1R2,CIITA,CD3D,HP |
| | TAP2,CD177,CX3CR1,IL1R2,CIITA,CTLA4,CD74 |
| | TAP2,CD177,CX3CR1,IL1R2,CIITA,CTLA4, HP |
| | TAP2,CD177,CX3CR1,IL1R2,CIITA,CD74,HP |
| | TAP2,CD177,CX3CR1,IL1R2,CD3D,CTLA4,CD74 |
| | TAP2,CD177,CX3CR1,IL1R2,CD3D,CTLA4,HP |
| | TAP2,CD177,CX3CR1,IL1R2,CD3D,CD74,HP |
| | TAP2,CD177,CX3CR1,IL1R2,CTLA4,CD74,HP |
| | TAP2,CD177,CX3CR1,IFNG,CIITA,CD3D,CTLA4 |
| | TAP2,CD177,CX3CR1,IFNG,CIITA,CD3D,CD74 |
| | TAP2,CD177,CX3CR1,IFNG,CIITA,CD3D,HP |
| | TAP2,CD177,CX3CR1,IFNG,CIITA,CTLA4,CD74 |
| | TAP2,CD177,CX3CR1,IFNG,CIITA,CTLA4,HP |
| | TAP2,CD177,CX3CR1,IFNG,CIITA,CD74,HP |
| | TAP2,CD177,CX3CR1,IFNG,CD3D,CTLA4,CD74 |
| | TAP2,CD177,CX3CR1,IFNG,CD3D,CTLA4,HP |
| | TAP2,CD177,CX3CR1,IFNG,CD3D,CD74,HP |
| | TAP2,CD177,CX3CR1,IFNG,CTLA4,CD74,HP |
| | TAP2,CD177,CX3CR1,CIITA,CD3D,CTLA4,CD74 |
| | TAP2,CD177,CX3CR1,CIITA,CD3D,CTLA4,HP |
| | TAP2,CD177,CX3CR1,CIITA,CD3D,CD74,HP |
| | TAP2,CD177,CX3CR1,CIITA,CTLA4,CD74,H P |
| | TAP2,CD177,CX3CR1,CD3D,CTLA4,CD74,HP |
| | TAP2,CD177,IL1R2,IFNG,CIITA,CD3D,CTLA4 |
| | TAP2,CD177,IL1R2,IFNG,CIITA,CD3D,CD74 |
| | TAP2,CD177,IL1R2,IFNG,CIITA,CD3D,HP |
| | TAP2,CD177,IL1R2,IFNG,CIITA,CTLA4,CD74 |
| | TAP2,CD177,IL1R2,IFNG,CIITA,CTLA4,HP |
| | TAP2,CD177,IL1R2,IFNG,CIITA,CD74,HP |
| | TAP2,CD177,IL1R2,IFNG,CD3D,CTLA4,CD74 |
| | TAP2,CD177,IL1R2,IFNG,CD3D,CTLA4,HP |
| | TAP2,CD177,IL1R2,IFNG,CD3D,CD74,HP |
| | TAP2,CD177,IL1R2,IFNG,CTLA4,CD74,HP |
| | TAP2,CD177,IL1R2,CIITA,CD3D,CTLA4,CD74 |
| | TAP2,CD177,IL1R2,CIITA,CD3D,CTLA4,HP |
| | TAP2,CD177,IL1R2,CIITA,CD3D,CD74,HP |
| | TAP2,CD177,IL1R2,CIITA,CTLA4,CD74,HP |
| | TAP2,CD177,IL1R2,CD3D,CTLA4,CD74,HP |
| | TAP2,CD177,IFNG,CIITA,CD3D,CTLA4,CD74 |
| | TAP2,CD177,IFNG,CIITA,CD3D,CTLA4,HP |
| | TAP2,CD177,IFNG,CIITA,CD3D,CD74,HP |
| | TAP2,CD177,IFNG,CIITA,CTLA4,CD74,HP |
| | TAP2,CD177,IFNG,CD3D,CTLA4,CD74,HP |
| | TAP2,CD177,CIITA,CD3D,CTLA4,CD74,HP |
| | TAP2,CX3CR1,IL1R2,IFNG,CIITA,CD3D,CTLA4 |
| | TAP2,CX3CR1,IL1R2,IFNG,CIITA,CD3D,CD74 |
| | TAP2,CX3CR1,IL1R2,IFNG,CIITA,CD3D,HP |
| | TAP2,CX3CR1,IL1R2,IFNG,CIITA,CTLA4,CD74 |
| | TAP2,CX3CR1,IL1R2,IFNG,CIITA,CTLA4,HP |
| | TAP2,CX3CR1,IL1R2,IFNG,CIITA,CD74,HP |
| | TAP2,CX3CR1,IL1R2,IFNG,CD3D,CTLA4,CD74 |
| | TAP2,CX3CR1,IL1R2,IFNG,CD3D,CTLA4,HP |
| | TAP2,CX3CR1,IL1R2,IFNG,CD3D,CD74,HP |
| | TAP2,CX3CR1,IL1R2,IFNG,CTLA4,CD74,HP |
| | TAP2,CX3CR1,IL1R2,CIITA,CD3D,CTLA4,CD74 |
| | TAP2,CX3CR1,IL1R2,CIITA,CD3D,CTLA4,HP |
| | TAP2,CX3CR1,IL1R2,CIITA,CD3D,CD74,HP |
| | TAP2,CX3CR1,IL1R2,CIITA,CTLA4,CD74,HP |
| | TAP2,CX3CR1,IL1R2,CD3D,CTLA4,CD74,HP |
| | TAP2,CX3CR1,IFNG,CIITA,CD3D,CTLA4,CD74 |
| | TAP2,CX3CR1,IFNG,CIITA,CD3D,CTLA4,HP |
| | TAP2,CX3CR1,IFNG,CIITA,CD3D,CD74,HP |
| | TAP2,CX3CR1,IFNG,CIITA,CTLA4,CD74,HP |
| | TAP2,CX3CR1,IFNG,CD3D,CTLA4,CD74,HP |
| | TAP2,CX3CR1,CIITA,CD3D,CTLA4,CD74,HP |
| | TAP2,IL1R2,IFNG,CIITA,CD3D,CTLA4,CD74 |
| | TAP2,IL1R2,IFNG,CIITA,CD3D,CTLA4,HP |
| | TAP2,IL1R2,IFNG,CIITA,CD3D,CD74,HP |
| | TAP2,IL1R2,IFNG,CIITA,CTLA4,CD74,HP |
| | TAP2,IL1R2,IFNG,CD3D,CTLA4,CD74,HP |
| | TAP2,IL1R2,CIITA,CD3D,CTLA4,CD74,HP |
| | TAP2,IFNG,CIITA,CD3D,CTLA4,CD74,HP |
| | CD177,CX3CR1,IL1R2,IFNG,CIITA,CD3D,CTLA4 |
| | CD177,CX3CR1,IL1R2,IFNG,CIITA,CD3D,CD74 |
| | CDl77,CX3CR1, IL1R2, IFNG,CIIT A,CD3D, HP |
| | CDl77,CX3CR1, IL1R2, IFNG,ClIT A,CTLA4,CD74 |
| | CD177,CX3CR1,IL1R2,IFNG,CIITA,CTLA4, HP |
| | CD177,CX3CR1,IL1R2,IFNG,CIITA,CD74,HP |
| | CD177,CX3CR1,IL1R2,IFNG,CD3D,CTLA4,CD74 |
| | CDl77,CX3CR1,IL1R2,IFNG,CD3D,CTLA4,HP |
| | CD177,CX3CR1,IL1R2,IFNG,CD3D,CD74,HP |
| | CD177,CX3CR1,IL1R2,IFNG,CTLA4,CD74,HP |
| | CD177,CX3CR1,IL1R2,CIITA,CD3D,CTLA4,CD74 |
| | CD177,CX3CR1,IL1R2,CIITA,CD3D,CTLA4,HP |
| | CD177,CX3CR1,IL1R2,CIITA,CD3D,CD74,HP |
| | CD177,CX3CR1,IL1R2,CIITA,CTLA4,CD74,HP |
| | CD177,CX3CR1,IL1R2,CD3D,CTLA4,CD74,HP |
| | CD177,CX3CR1,IFNG,CIITA,CD3D,CTLA4,CD74 |
| | CD177,CX3CR1,IFNG,CIITA,CD3D,CTLA4,HP |
| | CD177,CX3CR1,IFNG,CIITA,CD3D,CD74,HP |
| | CD177,CX3CR1,IFNG,CIITA,CTLA4,CD74,HP |
| | CD177,CX3CR1, IFNG,CD3D,CTLA4,CD74,H P |
| | CD177,CX3CR1,CIITA,CD3D,CTLA4,CD74,HP |
| | CD177,IL1R2,IFNG,CIITA,CD3D,CTLA4,CD74 |
| | CD177,IL1R2,IFNG,CIITA,CD3D,CTLA4,HP |
| | CD177,IL1R2,IFNG,CIITA,CD3D,CD74,HP |
| | CD177,IL1R2,IFNG,CIITA,CTLA4,CD74,HP |
| | CD177,IL1R2,IFNG,CD3D,CTLA4,CD74,HP |
| | CD177,IL1R2,CIITA,CD3D,CTLA4,CD74,HP |
| | CD177,IFNG,CIITA,CD3D,CTLA4,CD74,HP |
| | CX3CR1,IL1R2,IFNG,CIITA,CD3D,CTLA4,CD74 |
| | CX3CR1,IL1R2,IFNG,CIITA,CD3D,CTLA4,HP |
| | CX3CR1,IL1R2,IFNG,CIITA,CD3D,CD74,HP |
| | CX3CR1,IL1R2,IFNG,CIITA,CTLA4,CD74,HP |
| | CX3CR1,IL1R2,IFNG,CD3D,CTLA4,CD74,HP |
| | CX3CR1,IL1R2,CIITA,CD3D,CTLA4,CD74,HP |
| | CX3CR1,IFNG,CIITA,CD3D,CTLA4,CD74,HP |
| | IL1R2,IFNG,CIITA,CD3D,CTLA4,CD74,HP |
| **Combinaisons de huit gènes** | S100A9,TAP2,CD177,CX3CR1,IL1R2,IFNG,CIITA,CD3D |
| | S100A9,TAP2,CD177,CX3CR1,IL1R2,IFNG,CIITA,CTLA4 |
| | S100A9,TAP2,CD177,CX3CR1,IL1R2,IFNG,CIITA,CD74 |
| | S100A9,TAP2,CD177,CX3CR1,IL1R2,IFNG,CIITA,HP |
| | S100A9,TAP2,CD177,CX3CR1,IL1R2,IFNG,CD3D,CTLA4 |
| | S100A9,TAP2,CD177,CX3CR1,IL1R2,IFNG,CD3D,CD74 |
| | S100A9,TAP2,CD177,CX3CR1,IL1R2,IFNG,CD3D,HP |
| | S100A9,TAP2,CD177,CX3CR1,IL1R2,IFNG,CTLA4,CD74 |
| | S100A9,TAP2,CD177,CX3CR1,IL1R2,IFNG,CTLA4,HP |
| | S100A9,TAP2,CD177,CX3CR1,IL1R2,IFNG,CD74,HP |
| | S100A9,TAP2,CD177,CX3CR1,IL1R2,CIITA,CD3D,CTLA4 |
| | S100A9,TAP2,CD177,CX3CR1,IL1R2,CIITA,CD3D,CD74 |
| | S100A9,TAP2,CD177,CX3CR1,IL1R2,CIITA,CD3D,HP |
| | S100A9,TAP2,CD177,CX3CR1,IL1R2,CIITA,CTLA4,CD74 |
| | S100A9,TAP2,CD177,CX3CR1,IL1R2,CIITA,CTLA4,HP |
| | S100A9,TAP2,CD177,CX3CR1,IL1R2,CIITA,CD74,HP |
| | S100A9,TAP2,CD177,CX3CR1,IL1R2,CD3D,CTLA4,CD74 |
| | S100A9,TAP2,CD177,CX3CR1,IL1R2,CD3D,CTLA4,HP |
| | S100A9,TAP2,CD177,CX3CR1,IL1R2,CD3D,CD74,HP |
| | S100A9,TAP2,CD177,CX3CR1,IL1R2,CTLA4,CD74,HP |
| | S100A9,TAP2,CD177,CX3CR1,IFNG,CIITA,CD3D,CTLA4 |
| | S100A9,TAP2,CD177,CX3CR1,IFNG,CIITA,CD3D,CD74 |
| | S100A9,TAP2,CD177,CX3CR1,IFNG,CIITA,CD3D,HP |
| | S100A9,TAP2,CD177,CX3CR1,IFNG,CIITA,CTLA4,CD74 |
| | S100A9,TAP2,CD177,CX3CR1,IFNG,CIITA,CTLA4,HP |
| | S100A9,TAP2,CD177,CX3CR1,IFNG,CIITA,CD74,HP |
| | S100A9,TAP2,CD177,CX3CR1,IFNG,CD3D,CTLA4,CD74 |
| | S100A9,TAP2,CD177,CX3CR1,IFNG,CD3D,CTLA4,HP |
| | S100A9,TAP2,CD177,CX3CR1,IFNG,CD3D,CD74,HP |
| | S100A9,TAP2,CD177,CX3CR1,IFNG,CTLA4,CD74,HP |
| | S100A9,TAP2,CD177,CX3CR1,CIITA,CD3D,CTLA4,CD74 |
| | S100A9,TAP2,CD177,CX3CR1,CIITA,CD3D,CTLA4,HP |
| | S100A9,TAP2,CD177,CX3CR1,CIITA,CD3D,CD74,HP |
| | S100A9,TAP2,CD177,CX3CR1,CIITA,CTLA4,CD74,HP |
| | S100A9,TAP2,CD177,CX3CR1,CD3D,CTLA4,CD74,HP |
| | S100A9,TAP2,CD177,IL1R2,IFNG,CIITA,CD3D,CTLA4 |
| | S100A9,TAP2,CD177,IL1R2,IFNG,CIITA,CD3D,CD74 |
| | S100A9,TAP2,CD177,IL1R2,IFNG,CIITA,CD3D,HP |
| | S100A9,TAP2,CD177,IL1R2,IFNG,CIITA,CTLA4,CD74 |
| | S100A9,TAP2,CD177,IL1R2,IFNG,CIITA,CTLA4,HP |
| | S100A9,TAP2,CD177,IL1R2,IFNG,CIITA,CD74,HP |
| | S100A9,TAP2,CD177,IL1R2,IFNG,CD3D,CTLA4,CD74 |
| | S100A9,TAP2,CD177,IL1R2,IFNG,CD3D,CTLA4,HP |
| | S100A9,TAP2,CD177,IL1R2,IFNG,CD3D,CD74,HP |
| | S100A9,TAP2,CD177,IL1R2,IFNG,CTLA4,CD74,HP |
| | S100A9,TAP2,CD177,IL1R2,CIITA,CD3D,CTLA4,CD74 |
| | S100A9,TAP2,CD177,IL1R2,CIITA,CD3D,CTLA4,HP |
| | S100A9,TAP2,CD177,IL1R2,CIITA,CD3D,CD74,HP |
| | S100A9,TAP2,CD177,IL1R2,CIITA,CTLA4,CD74,HP |
| | S100A9,TAP2,CD177,IL1R2,CD3D,CTLA4,CD74,HP |
| | S100A9,TAP2,CD177,IFNG,CIITA,CD3D,CTLA4,CD74 |
| | S100A9,TAP2,CD177,IFNG,CIITA,CD3D,CTLA4,HP |
| | S100A9,TAP2,CD177,IFNG,CIITA,CD3D,CD74,HP |
| | S100A9,TAP2,CD177,IFNG,CIITA,CTLA4,CD74,HP |
| | S100A9,TAP2,CD177,IFNG,CD3D,CTLA4,CD74,HP |
| | S100A9,TAP2,CD177,CIITA,CD3D,CTLA4,CD74,HP |
| | S100A9,TAP2,CX3CR1,IL1R2,IFNG,CIITA,CD3D,CTLA4 |
| | S100A9,TAP2,CX3CR1,IL1R2,IFNG,CIITA,CD3D,CD74 |
| | S100A9,TAP2,CX3CR1,IL1R2,IFNG,CIITA,CD3D,HP |
| | S100A9,TAP2,CX3CR1,IL1R2,IFNG,CIITA,CTLA4,CD74 |
| | S100A9,TAP2,CX3CR1,IL1R2,IFNG,CIITA,CTLA4,HP |
| | S100A9,TAP2,CX3CR1,IL1R2,IFNG,CIITA,CD74,HP |
| | S100A9,TAP2,CX3CR1,IL1R2,IFNG,CD3D,CTLA4,CD74 |
| | S100A9,TAP2,CX3CR1,IL1R2,IFNG,CD3D,CTLA4,HP |
| | S100A9,TAP2,CX3CR1,IL1R2,IFNG,CD3D,CD74,HP |
| | S100A9,TAP2,CX3CR1,IL1R2,IFNG,CTLA4,CD74,HP |
| | S100A9,TAP2,CX3CR1,IL1R2,CIITA,CD3D,CTLA4,CD74 |
| | S100A9,TAP2,CX3CR1,IL1R2,CIITA,CD3D,CTLA4,HP |
| | S100A9,TAP2,CX3CR1,IL1R2,CIITA,CD3D,CD74,HP |
| | S100A9,TAP2,CX3CR1,IL1R2,CIITA,CTLA4,CD74,HP |
| | S100A9,TAP2,CX3CR1,IL1R2,CD3D,CTLA4,CD74,HP |
| | S100A9,TAP2,CX3CR1,IFNG,CIITA,CD3D,CTLA4,CD74 |
| | S100A9,TAP2,CX3CR1,IFNG,CIITA,CD3D,CTLA4,HP |
| | S100A9,TAP2,CX3CR1,IFNG,CIITA,CD3D,CD74,HP |
| | S100A9,TAP2,CX3CR1,IFNG,CIITA,CTLA4,CD74,HP |
| | S100A9,TAP2,CX3CR1,IFNG,CD3D,CTLA4,CD74,HP |
| | S100A9,TAP2,CX3CR1,CIITA,CD3D,CTLA4,CD74,HP |
| | S100A9,TAP2,IL1R2,IFNG,CIITA,CD3D,CTLA4,CD74 |
| | S100A9,TAP2,IL1R2,IFNG,CIITA,CD3D,CTLA4,HP |
| | S100A9,TAP2,IL1R2,IFNG,CIITA,CD3D,CD74,HP |
| | S100A9,TAP2,IL1R2,IFNG,CIITA,CTLA4,CD74,HP |
| | S100A9,TAP2,IL1R2,IFNG,CD3D,CTLA4,CD74,HP |
| | S100A9,TAP2,IL1R2,CIITA,CD3D,CTLA4,CD74,HP |
| | S100A9,TAP2,IFNG,CIITA,CD3D,CTLA4,CD74,HP |
| | S100A9,CD177,CX3CR1,IL1R2,IFNG,CIITA,CD3D,CTLA4 |
| | S100A9,CD177,CX3CR1,IL1R2,IFNG,CIITA,CD3D,CD74 |
| | S100A9,CD177,CX3CR1,IL1R2,IFNG,CIITA,CD3D,HP |
| | S100A9,CD177,CX3CR1,IL1R2,IFNG,CIITA,CTLA4,CD74 |
| | S100A9,CD177,CX3CR1,IL1R2,IFNG,CllTA,CTLA4,HP |
| | S100A9,CD177,CX3CR1,IL1R2,IFNG,CIITA,CD74,HP |
| | S100A9,CD177,CX3CR1,IL1R2,IFNG,CD3D,CTLA4,CD74 |
| | S100A9,CD177,CX3CR1,IL1R2,IFNG,CD3D,CTLA4,HP |
| | S100A9,CD177,CX3CR1,IL1R2,IFNG,CD3D,CD74,HP |
| | S100A9,CD177,CX3CR1,IL1R2,IFNG,CTLA4,CD74,HP |
| | S100A9,CD177,CX3CR1,IL1R2,CIITA,CD3D,CTLA4,CD74 |
| | S100A9,CD177,CX3CR1,IL1R2,CIITA,CD3D,CTLA4,HP |
| | S100A9,CD177,CX3CR1,IL1R2,CIITA,CD3D,CD74,HP |
| | S100A9,CD177,CX3CR1,IL1R2,CIITA,CTLA4,CD74,HP |
| | S100A9,CD177,CX3CR1,IL1R2,CD3D,CTLA4,CD74,HP |
| | S100A9,CD177,CX3CR1,IFNG,CIITA,CD3D,CTLA4,CD74 |
| | S100A9,CD177,CX3CR1,IFNG,CIITA,CD3D,CTLA4,HP |
| | S100A9,CD177,CX3CR1,IFNG,CIITA,CD3D,CD74,HP |
| | S100A9,CD177,CX3CR1,IFNG,CIITA,CTLA4,CD74,HP |
| | S100A9,CD177,CX3CR1,IFNG,CD3D,CTLA4,CD74,HP |
| | S100A9,CD177,CX3CR1,CIITA,CD3D,CTLA4,CD74,HP |
| | S100A9,CD177,IL1R2,IFNG,CIITA,CD3D,CTLA4,CD74 |
| | S100A9,CD177,IL1R2,IFNG,CIITA,CD3D,CTLA4,HP |
| | S100A9,CD177,IL1R2,IFNG,CIITA,CD3D,CD74,HP |
| | S100A9,CD177,IL1R2,IFNG,CIITA,CTLA4,CD74,HP |
| | S100A9,CD177,IL1R2,IFNG,CD3D,CTLA4,CD74,HP |
| | S100A9,CD177,IL1R2,CIITA,CD3D,CTLA4,CD74,HP |
| | S100A9,CD177,IFNG,CIITA,CD3D,CTLA4,CD74,HP |
| | S100A9,CX3CR1,IL1R2,IFNG,CIITA,CD3D,CTLA4,CD74 |
| | S100A9,CX3CR1,IL1R2,IFNG,CIITA,CD3D,CTLA4,HP |
| | S100A9,CX3CR1,IL1R2,IFNG,CIITA,CD3D,CD74,HP |
| | S100A9,CX3CR1,IL1R2,IFNG,CIITA,CTLA4,CD74,HP |
| | S100A9,CX3CR1,IL1R2,IFNG,CD3D,CTLA4,CD74,HP |
| | S100A9,CX3CR1,IL1R2,CIITA,CD3D,CTLA4,CD74,HP |
| | S100A9,CX3CR1,IFNG,CIITA,CD3D,CTLA4,CD74,HP |
| | S100A9,IL1R2,IFNG,CIITA,CD3D,CTLA4,CD74,HP |
| | TAP2,CD177,CX3CR1,IL1R2,IFNG,CIITA,CD3D,CTLA4 |
| | TAP2,CD177,CX3CR1,IL1R2,IFNG,CIITA,CD3D,CD74 |
| | TAP2,CD177,CX3CR1,IL1R2,IFNG,CIITA,CD3D,HP |
| | TAP2,CD177,CX3CR1,IL1R2,IFNG,CIITA,CTLA4,CD74 |
| | TAP2,CD177,CX3CR1,IL1R2,IFNG,CIITA,CTLA4,HP |
| | TAP2,CD177,CX3CR1,IL1R2,IFNG,CIITA,CD74,HP |
| | TAP2,CD177,CX3CR1,IL1R2,IFNG,CD3D,CTLA4,CD74 |
| | TAP2,CD177,CX3CR1,IL1R2,IFNG,CD3D,CTLA4,HP |
| | TAP2,CD177,CX3CR1,IL1R2,IFNG,CD3D,CD74,HP |
| | TAP2,CD177,CX3CR1,IL1R2,IFNG,CTLA4,CD74,HP |
| | TAP2,CD177,CX3CR1,IL1R2,CIITA,CD3D,CTLA4,CD74 |
| | TAP2,CD177,CX3CR1,IL1R2,CIITA,CD3D,CTLA4,HP |
| | TAP2,CD177,CX3CR1,IL1R2,CIITA,CD3D,CD74,HP |
| | TAP2,CD177,CX3CR1,IL1R2,CIITA,CTLA4,CD74,HP |
| | TAP2,CD177,CX3CR1,IL1R2,CD3D,CTLA4,CD74,HP |
| | TAP2,CD177,CX3CR1,IFNG,CIITA,CD3D,CTLA4,CD74 |
| | TAP2,CD177,CX3CR1,IFNG,CIITA,CD3D,CTLA4,HP |
| | TAP2,CD177,CX3CR1,IFNG,CIITA,CD3D,CD74,HP |
| | TAP2,CD177,CX3CR1,IFNG,CIITA,CTLA4,CD74,HP |
| | TAP2,CD177,CX3CR1,IFNG,CD3D,CTLA4,CD74,HP |
| | TAP2,CD177,CX3CR1,CIITA,CD3D,CTLA4,CD74,HP |
| | TAP2,CD177,IL1R2,IFNG,CIITA,CD3D,CTLA4,CD74 |
| | TAP2,CD177,IL1R2,IFNG,CIITA,CD3D,CTLA4,HP |
| | TAP2,CD177,IL1R2,IFNG,CIITA,CD3D,CD74,HP |
| | TAP2,CD177,IL1R2,IFNG,CIITA,CTLA4,CD74,HP |
| | TAP2,CD177,IL1R2,IFNG,CD3D,CTLA4,CD74,HP |
| | TAP2,CD177,IL1R2,CIITA,CD3D,CTLA4,CD74,HP |
| | TAP2,CD177,IFNG,CIITA,CD3D,CTLA4,CD74,HP |
| | TAP2,CX3CR1,IL1R2,IFNG,CIITA,CD3D,CTLA4,CD74 |
| | TAP2,CX3CR1,IL1R2,IFNG,CIITA,CD3D,CTLA4,HP |
| | TAP2,CX3CR1,IL1R2,IFNG,CIITA,CD3D,CD74,HP |
| | TAP2,CX3CR1,IL1R2,IFNG,CIITA,CTLA4,CD74,HP |
| | TAP2,CX3CR1,IL1R2,IFNG,CD3D,CTLA4,CD74,HP |
| | TAP2,CX3CR1,IL1R2,CIITA,CD3D,CTLA4,CD74,HP |
| | TAP2,CX3CR1,IFNG,CIITA,CD3D,CTLA4,CD74,HP |
| | TAP2,IL1R2,IFNG,CIITA,CD3D,CTLA4,CD74,HP |
| | CD177,CX3CR1,IL1R2,IFNG,CIITA,CD3D,CTLA4,CD74 |
| | CDl77,CX3CR1,1 L1R2, IFNG,CIIT A,CD3D,CTLA4,H P |
| | CD177,CX3CR1,IL1R2,IFNG,CIITA,CD3D,CD74,HP |
| | CD177,CX3CR1,IL1R2,IFNG,CIITA,CTLA4,CD74,HP |
| | CD177,CX3CR1,IL1R2,IFNG,CD3D,CTLA4,CD74,HP |
| | CD177,CX3CR1,IL1R2,CIITA,CD3D,CTLA4,CD74,HP |
| | CD177,CX3CR1,IFNG,CIITA,CD3D,CTLA4,CD74,HP |
| | CD177,IL1R2,IFNG,CIITA,CD3D,CTLA4,CD74,HP |
| | CX3CR1,IL1R2,IFNG,CIITA,CD3D,CTLA4,CD74,HP |
| **Combinaisons de neuf gènes** | S100A9,TAP2,CD177,CX3CR1,IL1R2,IFNG,CIITA,CD3D,CTLA4 |
| | S100A9,TAP2,CD177,CX3CR1,IL1R2,IFNG,CIITA,CD3D,CD74 |
| | S100A9,TAP2,CD177,CX3CR1,IL1R2,IFNG,CIITA,CD3D,HP |
| | S100A9,TAP2,CD177,CX3CR1,IL1R2,IFNG,CIITA,CTLA4,CD74 |
| | S100A9,TAP2,CD177,CX3CR1,IL1R2,IFNG,CIITA,CTLA4,HP |
| | S100A9,TAP2,CD177,CX3CR1,IL1R2,IFNG,CIITA,CD74,HP |
| | S100A9,TAP2,CD177,CX3CR1,IL1R2,IFNG,CD3D,CTLA4,CD74 |
| | S100A9,TAP2,CD177,CX3CR1,IL1R2,IFNG,CD3D,CTLA4,HP |
| | S100A9,TAP2,CD177,CX3CR1,IL1R2,IFNG,CD3D,CD74,HP |
| | S100A9,TAP2,CD177,CX3CR1,IL1R2,IFNG,CTLA4,CD74,HP |
| | S100A9,TAP2,CD177,CX3CR1,IL1R2,CIITA,CD3D,CTLA4,CD74 |
| | S100A9,TAP2,CD177,CX3CR1,IL1R2,CIITA,CD3D,CTLA4,HP |
| | S100A9,TAP2,CD177,CX3CR1,IL1R2,CIITA,CD3D,CD74,HP |
| | S100A9,TAP2,CD177,CX3CR1,IL1R2,CIITA,CTLA4,CD74,HP |
| | S100A9,TAP2,CD177,CX3CR1,IL1R2,CD3D,CTLA4,CD74,HP |
| | S100A9,TAP2,CD177,CX3CR1,IFNG,CIITA,CD3D,CTLA4,CD74 |
| | S100A9,TAP2,CD177,CX3CR1,IFNG,CIITA,CD3D,CTLA4,HP |
| | S100A9,TAP2,CD177,CX3CR1,IFNG,CIITA,CD3D,CD74,HP |
| | S100A9,TAP2,CD177,CX3CR1,IFNG,CIITA,CTLA4,CD74,HP |
| | S100A9,TAP2,CD177,CX3CR1,IFNG,CD3D,CTLA4,CD74,HP |
| | S100A9,TAP2,CD177,CX3CR1,CIITA,CD3D,CTLA4,CD74,HP |
| | S100A9,TAP2,CD177,IL1R2,IFNG,CIITA,CD3D,CTLA4,CD74 |
| | S100A9,TAP2,CD177,IL1R2,IFNG,CIITA,CD3D,CTLA4,HP |
| | S100A9,TAP2,CD177,IL1R2,IFNG,CIITA,CD3D,CD74,HP |
| | S100A9,TAP2,CD177,IL1R2,IFNG,CIITA,CTLA4,CD74,HP |
| | S100A9,TAP2,CD177,IL1R2,IFNG,CD3D,CTLA4,CD74,HP |
| | S100A9,TAP2,CD177,IL1R2,CIITA,CD3D,CTLA4,CD74,HP |
| | S100A9,TAP2,CD177,IFNG,CIITA,CD3D,CTLA4,CD74,HP |
| | S100A9,TAP2,CX3CR1,IL1R2,IFNG,CIITA,CD3D,CTLA4,CD74 |
| | S100A9,TAP2,CX3CR1,IL1R2,IFNG,CIITA,CD3D,CTLA4,HP |
| | S100A9,TAP2,CX3CR1,IL1R2,IFNG,CIITA,CD3D,CD74,HP |
| | S100A9,TAP2,CX3CR1,IL1R2,IFNG,CIITA,CTLA4,CD74,HP |
| | S100A9,TAP2,CX3CR1,IL1R2,IFNG,CD3D,CTLA4,CD74,HP |
| | S100A9,TAP2,CX3CR1,IL1R2,CIITA,CD3D,CTLA4,CD74,HP |
| | S100A9,TAP2,CX3CR1,IFNG,CIITA,CD3D,CTLA4,CD74,HP |
| | S100A9,TAP2,IL1R2,IFNG,CIITA,CD3D,CTLA4,CD74,HP |
| | S100A9,CD177,CX3CR1,IL1R2,IFNG,CIITA,CD3D,CTLA4,CD74 |
| | S100A9,CD177,CX3CR1,IL1R2,IFNG,CIITA,CD3D,CTLA4,HP |
| | S100A9,CD177,CX3CR1,IL1R2,IFNG,CIITA,CD3D,CD74,HP |
| | S100A9,CD177,CX3CR1,IL1R2,IFNG,CIITA,CTLA4,CD74,HP |
| | S100A9,CD177,CX3CR1,IL1R2,IFNG,CD3D,CTLA4,CD74,HP |
| | S100A9,CD177,CX3CR1,IL1R2,CIITA,CD3D,CTLA4,CD74,HP |
| | S100A9,CD177,CX3CR1,IFNG,CIITA,CD3D,CTLA4,CD74,HP |
| | S100A9,CD177,IL1R2,IFNG,CIITA,CD3D,CTLA4,CD74,HP |
| | S100A9,CX3CR1,IL1R2,IFNG,CIITA,CD3D,CTLA4,CD74,HP |
| | TAP2,CD177,CX3CR1,IL1R2,IFNG,CIITA,CD3D,CTLA4,CD74 |
| | TAP2,CD177,CX3CR1,IL1R2,IFNG,CIITA,CD3D,CTLA4,HP |
| | TAP2,CD177,CX3CR1,IL1R2,IFNG,CIITA,CD3D,CD74,HP |
| | TAP2,CD177,CX3CR1,IL1R2,IFNG,CIITA,CTLA4,CD74,HP |
| | TAP2,CD177,CX3CR1,IL1R2,IFNG,CD3D,CTLA4,CD74,HP |
| | TAP2,CD177,CX3CR1,IL1R2,CIITA,CD3D,CTLA4,CD74,HP |
| | TAP2,CD177,CX3CR1,IFNG,CIITA,CD3D,CTLA4,CD74,HP |
| | TAP2,CD177,IL1R2,IFNG,CIITA,CD3D,CTLA4,CD74,HP |
| | TAP2,CX3CR1,IL1R2,IFNG,CIITA,CD3D,CTLA4,CD74,HP |
| | CD177,CX3CR1,IL1R2,IFNG,CIITA,CD3D,CTLA4,CD74,HP |
| **Combinaisons de dix gènes** | S100A9,TAP2,CD177,CX3CR1,IL1R2,IFNG,CIITA,CD3D,CTLA4,CD74 |
| | S100A9,TAP2,CD177,CX3CR1,IL1R2,IFNG,CIITA,CD3D,CTLA4,HP |
| | S100A9,TAP2,CD177,CX3CR1,IL1R2,IFNG,CIITA,CD3D,CD74,HP |
| | S100A9,TAP2,CD177,CX3CR1,IL1R2,IFNG,CIITA,CTLA4,CD74,HP |
| | S100A9,TAP2,CD177,CX3CR1,IL1R2,IFNG,CD3D,CTLA4,CD74,HP |
| | S100A9,TAP2,CD177,CX3CR1,IL1R2,CIITA,CD3D,CTLA4,CD74,HP |
| | S100A9,TAP2,CD177,CX3CR1,IFNG,CIITA,CD3D,CTLA4,CD74,HP |
| | S100A9,TAP2,CD177,IL1R2,IFNG,CIITA,CD3D,CTLA4,CD74,HP |
| | S100A9,TAP2,CX3CR1,IL1R2,IFNG,CIITA,CD3D,CTLA4,CD74,HP |
| | S100A9,CD177,CX3CR1,IL1R2,IFNG,CIITA,CD3D,CTLA4,CD74,HP |
| | TAP2,CD177,CX3CR1,IL1R2,IFNG,CIITA,CD3D,CTLA4,CD74,HP |
| **Combinaison de onze gènes** | S100A9, TAP2, CD177, CX3CR1, IL1R2, IFNG, CIITA, CD3D, CTLA4, CD74, HP |

Conjointement à la mesure de l'expression de C3AR1, et comme démontré dans les exemples de réalisation, la mesure de l'expression d'au moins un autre gène ou d'une combinaison d'autres gènes telle que définie précédemment permet d'améliorer la spécificité et la sensibilité du procédé d'évaluation du risque de complication chez le patient, notamment du risque de décès et/ou du risque de survenue d'une infection associée aux soins.

De préférence, dans le procédé tel que décrit précédemment, dans tous ses modes de réalisation, l'échantillon biologique est un échantillon de sang, de préférence un échantillon de sang total ou un échantillon dérivé du sang (e.g. des PBMC, qui peuvent être obtenues par la méthode Ficoll, bien connue de l'homme du métier, ou des monocytes purifiés).

La mesure de l'expression (ou du niveau d'expression) d'un gène consiste à quantifier au moins un produit d'expression du gène. Le produit d'expression d'un gène, au sens de la présente invention, est toute molécule biologique issue de l'expression dudit gène. Plus particulièrement, le produit d'expression du gène peut être un transcrit ARN.

Par « transcrit », on entend les ARN, et en particulier les ARN messagers (ARNm), issus de la transcription du gène. Plus précisément, les transcrits sont les ARN produits par la transcription d'un gène suivi des modifications post-transcriptionnelles des formes pré-ARN. Dans le cadre de la présente invention, la mesure du niveau d'expression d'un ou plusieurs transcrits ARN du même gène peut être effectuée. Ainsi, de préférence, dans le procédé tel que décrit précédemment, dans tous ses modes de réalisation, l'expression du(des) gène(s) *(i.e.* l'expression de C3AR1, et éventuellement d'un ou plusieurs autres biomarqueurs d'intérêt, tels que décrits précédemment) est mesurée au niveau transcrit ARN ou ARNm. Dans le cas d'un transcrit ARNm, la détection peut être réalisée par une méthode directe, par tout procédé connu de l'homme du métier permettant de déterminer la présence dudit transcrit dans l'échantillon, ou par détection indirecte du transcrit après transformation de ce dernier en ADN, ou après amplification dudit transcrit ou après amplification de l'ADN obtenu après transformation dudit transcrit en ADN. De nombreuses méthodes existent pour la détection des acides nucléiques (voir par exemple Kricka et al., Clinical Chemistry, 1999, n° 45(4), p.453-458 ; Relier G. H. et al., DNA Probes, 2nd Ed., Stockton Press, 1993, sections 5 et 6, p.173-249). L'expression des gènes peut notamment être mesurée par Reverse Transcrption-Po|ymerase Chain Reaction ou RT-PCR, de préférence par RT-PCR quantitative ou RT-qPCR (par exemple en utilisant la technologie FilmArray^{®} ou la plateforme Biomark^{™} de Fluidigm), par séquençage (de préférence par séquençage haut débit) ou par des techniques d'hybridation (par exemple avec des micropuces d'hybridation ou par des techniques du type Nanostring^{®} nCounter^{®}).

La mesure du niveau d'expression d'un gène permet en particulier de déterminer la quantité d'un ou plusieurs transcrits présents dans l'échantillon biologique ou d'en donner une valeur dérivée. Une valeur dérivée de la quantité peut par exemple être la concentration absolue, calculée grâce à une courbe de calibration obtenue à partir de dilutions successives d'une solution d'amplicons de concentration connue. Elle peut également correspondre à la valeur de la quantité normalisée et calibrée, comme le CNRQ *(*Calibrated Normalized Relative Quantity, (Hellemans et al (2007), Genome biology 8(2):R19), qui intègre les valeurs d'un échantillon de référence (ou d'un calibrateur) et d'un ou plusieurs gènes de ménage (appelés également gènes de référence). A titre d'exemples de gènes de ménage, on peut citer les gènes DECR1, HPRT1, PPIB, RPLP0, PPIA, GLYR1, RANBP3, 18S, GAPDH et ACTB.

Ainsi, de préférence, dans le procédé tel que décrit précédemment, dans tous ses modes de réalisation, l'expression du(des) gène(s) d'intérêt est normalisée par rapport à l'expression d'un ou plusieurs gènes de ménage (ou gènes de référence), comme cela est connu de l'homme du métier ; de préférence encore en utilisant un ou plusieurs des gènes de ménage suivants : DECR1 (localisation chromosomique du gène selon le GRCh38/hg38 : chr8:90,001,352-90,053,633), HPRT1 (localisation chromosomique du gène selon le GRCh38/hg38 : chrX:134,452,842-134,520,513) et PPIB (localisation chromosomique du gène selon le GRCh38/hg38 : chr15:64,155,812-64,163,205).

De préférence, dans le procédé tel que décrit précédemment, dans tous ses modes de réalisation, l'expression du(des) gènes d'intérêt (de préférence, l'expression normalisée) dans l'échantillon biologique du patient est comparée à une valeur de référence ou à l'expression du(des) mêmes gènes d'intérêt (de préférence, l'expression normalisée) dans un échantillon biologique de référence (ces données étant utilisées pour le calcul du CNRQ, comme mentionné plus haut). L'échantillon de référence peut être par exemple un échantillon provenant d'un volontaire (individu sain), d'un patient, ou un mélange d'échantillons de plusieurs volontaires (d'une part) ou de plusieurs patients (d'autre part). L'échantillon de référence peut aussi être un échantillon prélevé d'un volontaire (ou un mélange d'échantillons prélevés sur plusieurs volontaires) puis traité *ex vivo* par un agent stimulant du système immunitaire (tel que le LPS ou lipopolysaccharide). L'échantillon de référence peut également être un mélange d'échantillon(s) non traités et d'échantillons traité(s) *ex vivo* par un agent stimulant du système immunitaire.

Ainsi, le procédé pour déterminer le risque de complication, en particulier un risque de décès et/ou un risque de survenue d'une infection associée aux soins, tel que décrit précédemment et comprenant une étape de mesure de l'expression de C3AR1 dans l'échantillon biologique du patient, peut en outre comprendre les étapes consistant à :
- comparer l'expression de C3AR1 ou une valeur dérivée de cette expression, à une valeur de référence prédéterminée ; et
- établir une conclusion quant au risque de complication, de préférence, quant au risque de décès ou au risque de surveue d'une infection associée aux soins, et de préférence encore, quant au risque de survenue d'une infection associée aux soins.

La détermination d'une valeur de référence est largement connue de l'homme du métier en ce qu'il s'agit d'une expérimentation de routine. Cette détermination consiste notamment à mettre en œuvre une méthode de dosage identique, ou pour le moins comparable, à celle mise en œuvre dans le procédé de l'invention, dans un échantillon biologique issu d'un sujet ou d'un groupe de sujets considéré(s) comme n'étant pas susceptible de souffrir d'une infection associée aux soins. Aussi, ladite détermination d'une valeur de référence peut consister en la mise en œuvre de ladite méthode de dosage dans des échantillons biologiques des deux populations étudiées, et en la détermination de la valeur du test (quantité) permettant de faire une discrimination entre ces deux populations, dans le cas présent entre celle qui va se compliquer (risque de décès ou de survenue d'ne infection associée aux soins) et celle qui ne va pas se compliquer(peu ou pas de risque de décès ou de survenue d'une infection associée aux soins).

La valeur de référence prédéterminée, utilisée pour comparer la quantité mesurée dans le cadre de l'invention, sera déterminée à partir du ou des mêmes produits d'expression de C3AR1 que ceux ou celui quantifié(s) dans l'échantillon biologique à tester.

Les échantillons à partir desquels sont déterminées les valeurs de référence, encore nommés « échantillons de référence», sont avantageusement de même nature que celle de l'échantillon biologiques à tester ou tout du moins, d'une nature compatible pour constituer une référence quand à la quantification du ou des produits d'expression de C3AR1.

Le ou les échantillons de référence utilisés sont, de préférence, issus de personnes ayant les mêmes caractéristiques ou une majorité de caractéristiques communes, notamment du même sexe et/ou d'un âge similaire ou identique et/ou de même origine ethnique, avec celles du patient pour lequel on souhaite évaluer le risque de complication.

Le ou les échantillons de référence utilisés sont de préférence issus de patients au sein du service des urgences, du service de réanimation, en unité de soins intensifs ou en unité de soins continus ; de préférence encore issus de patients en état septique (plus particulièrement, en choc septique), de patients atteints de brûlures (plus particulièrement, de brûlures graves), de patients atteints de traumatismes (plus particulièrement, de traumatismes graves), ou de patients opérés par chirurgie (plus particulièrement, une chirurgie lourde).

La détermination de la valeur de référence, ou valeur du test (quantité), permettant de faire une discrimination entre ces deux populations peut notamment être calculée grâce à la courbe ROC (Receiver Operating Characteristic Curve ), comme mis en œuvre par exemple dans les exemples ci-dessous.

Selon un mode de réalisation particulier, lorsque la valeur de la quantité du produit du produit d'expression de C3AR1 est supérieure à la valeur de référence, alors le patient est un patient à risque accru de complication, en particulier un risque accru de décès ou un risque accru de survenue d'une infection associée aux soins, de préférence une infection nosocomiale.

De préférence, le procédé pour déterminer le risque de complication, en particulier un risque de décès et/ou un risque de survenue d'une infection associée aux soins, tel que décrit précédemment, dans tous ses modes de réalisation, comprend également une étape de gestion des soins de santé pour réduire le risque de complication. Un patient identifié comme étant à risque accru de complication peut avoir une gestion des soins de santé adaptée dans le but de réduire le risque de complication et, par exemple, afin de réduire le risque de développer un sepsis, un choc septique ou encore le risque de décès. A titre d'exemples de gestion des soins, on peut citer un traitement immunomodulateur adapté au patient ou encore un traitement antibiotique prophylactique, les deux traitements pouvant être associés et/ou orienter vers un service de soins continus ou de réanimation afin de réduire le risque de complication, par exemple réduire le risque de développer un sepsis, un choc septique ou même le risque de décès dans les jours qui suivent la mesure de l'expression du ou des biomarqueur(s). De manière préférée, le traitement immunomodulateur est un traitement immunostimulant, s'il est déterminé que l'individu a un statut d'immunosuppression, ou un traitement anti-inflammatoire, s'il est déterminé que l'individu a un statut d'inflammation. Parmi les traitements immunostimulants qui peuvent être sélectionnés, on peut citer à titre d'exemples le groupe des interleukines, en particulier IL-7, IL-15 ou IL-3, des facteurs de croissance, en particulier le GM-CSF, des interférons, en particulier IFNγ, des Toll agonistes, des anticorps, en particulier des anticorps anti-PD1, anti-PDL1, anti-LAG3, anti-TIM3, anti-IL-10 ou anti-CTLA4, des transferrines et des molécules inhibitrices de l'apoptose, FLT3L, Thymosin α1, des antagonistes adrénergiques. Parmi les traitements anti-inflammatoires, on peut citer notamment le groupe des glucocorticoïdes, des agents cytostatiques, des molécules agissant sur les immunophilines et les cytokines, des molécules bloquant le récepteur à l'IL-1 et des traitements anti-TNF. Des exemples de traitements antibiotiques prophylactiques appropriés pour prévenir la pneumonie sont décrits en particulier dans les Annales Françaises d'Anesthésie et de Réanimation (30 ; 2011 ; 168-190). Inversement, un patient ne présentant pas de risque de complication pourra être orienté rapidement vers un service d'hospitalisation de jour, par exemple un service d'infectiologie, plutôt que de rester dans un service avec un monitoring rapproché dont il n'aura pas besoin.

L'invention a également pour objet un kit comprenant des moyens d'amplification (e.g. amorces) et/ou des moyens de détection (e.g. sondes) de l'expression de C3AR1 et d'un ou plusieurs autre(s) biomarqueur(s), comme indiqué précédemment, dans tous les modes de réalisation (et, de manière particulièrement préférée, un, deux, trois, quatre, cinq, six, sept, huit, neuf, dix ou onze gènes, sélectionnés dans le groupe constitué par : CD74, CIITA, IFNG, IL1R2, TAP2, CD177, HP, CX3R1, S100A9, CTLA4 et CD3D) ; ledit kit étant caractérisé en ce que l'ensemble des moyens d'amplification et/ou de détection dudit kit permettent la détection et/ou l'amplification d'au plus 100, 90, 80, 70, 60, 50, 40, 30, 29, 28, 27, 26, 25, 24, 23, 22, 21, 20, 19, 18, 17, 16, 15, 14, 13, 12, 11, 10, 9, 8, 7, 6, 5, 4, 3, 2 biomarqueurs, au total. Par « biomarqueur » (ou « marqueur »), on entend une caractéristique biologique mesurable objectivement qui représente un indicateur des processus biologiques normaux ou pathologiques ou de réponse pharmacologique à une intervention thérapeutique. En particulier, ce biomarqueur peut être détectable au niveau ARNm. Plus particulièrement, le biomarqueur peut être un biomarqueur endogène ou loci (tel qu'un gène ou un HERV / *Human Endogenous RetroVirus,* qui se retrouvent dans le matériel chromosomique d'un individu) ou un biomarqueur exogène (tel qu'un virus).

Tous les éléments et modes de réalisation décrits précédemment pour le procédé *in vitro* ou *ex vivo* selon l'invention s'appliquent également au kit objet de l'invention.

Ainsi, ledit kit peut par exemple comprendre également des moyens d'amplification et/ou de détection d'un ou plusieurs gènes de ménage (de préférence sélectionnés dans la liste constituée par : DECR1, HPRT1 et PPIB). Le kit peut également comprendre des moyens de contrôle positif permettant de qualifier la qualité de l'extraction de l'ARN, la qualité de tout procédé d'amplification et/ou d'hybridation.

Selon un mode de réalisation particulier, le kit selon l'invention comprend des moyens d'amplification (e.g. amorces) et/ou des moyens de détection (e.g. sondes) de l'expression de C3AR1 et d'au moins un ou plusieurs autres gènes sélectionnés parmi les gènes suivants : GNLY, S100A9, ADGRE3, CD177, CX3CR1, IFIH1, OAS2, OAS3, CCNB1IP1, CDKN1A, CDKN1B, CDKN1C, CDKN2B, CDKN2C, CDKN2D IL15, IL2, MCP1(CCL2), CXCL10, IL10, IL1RN, IL18R1, IL1R2, IL1R1, IL18RAP, IFNG, IL1B, IL17A, IL18, IL6, TNF, ARL14EP, GSN, CIITA, DYRK2, GATA3, MDC1, NFKB1, RORC, STAT4, TBX21, TDRD9, CSF2, ALOX5, BPGM, TRAP1, CD40LG, CD3D, BTLA, CD274, CTLA4, ICOS, PDCD1, TNFSF4, CD74, FCGR1A, LILRB2, TAP2, FLT1, HAVCR2, IL7R, ZAP70, HP, et leurs combinaisons, et parmi les HERV suivants : 190665001-HERV0376, 220247002-HERV0797, 050286701-HERV0513, 130360601-HERV0808, 021456001-MALR1017, 170369402HE41, 121601801-HERV0492, 011052202-HERV1033, 011052702-MALR1044 et leurs combinaisons ;
de préference au moins un ou plusieurs autres gènes sélectionnés parmi les gènes suivants : GNLY, S100A9, ADGRE3, CD177, CX3CR1, OAS2, CCNB1IP1, IL10, IL1RN, IL1R2, IFNG, TNF, ARL14EP, CIITA, GATA3, MDC1, TDRD9, BPGM, CD3D, CD274, CTLA4, CD74, TAP2, IL7R, ZAP70, HP, et leurs combinaisons ;
de préference encore, au moins un ou plusieurs autres gènes sélectionnés parmi les gènes suivants : S100A9, CD177, CX3CR1, IL1R2, IFNG, CIITA, CD3D, CTLA4, CD74, TAP2, HP, et leur combinaison.

Selon ce mode de réalisation, l'ensemble des moyens d'amplification et/ou de détection dudit kit permettent la détection et/ou l'amplification d'au plus 100, 90, 80, 70, 60, 50, 40, 30, 29, 28, 27, 26, 25, 24, 23, 22, 21, 20, 19, 18, 17, 16, 15, 14, 13, 12, 11, 10, 9, 8, 7, 6, 5, 4, 3, 2 biomarqueurs, au total.

On entend par « amorce » ou « amorce d'amplification », un fragment nucléotidique pouvant être constitué de 5 à 100 nucléotides, de préférence de 15 à 30 nucléotides, et possédant une spécificité d'hybridation avec une séquence nucléotidique cible, dans des conditions déterminées pour l'initiation d'une polymérisation enzymatique, par exemple dans une réaction d'amplification enzymatique de la séquence nucléotidique cible. Généralement, on utilise des « couples d'amorces », constitués de deux amorces. Lorsque l'on souhaite réaliser l'amplification de plusieurs biomarqueurs (e.g des gènes ou des HERV) différents, plusieurs couples d'amorces différents sont de préférence utilisés, ayant préférentiellement chacun une capacité à s'hybrider spécifiquement avec un biomarqueur différent.

On entend par « sonde » ou « sonde d'hybridation », un fragment nucléotidique constitué typiquement de 5 à 100 nucléotides, de préférence de 15 à 90 nucléotides, de manière encore plus préférée de 15 à 35 nucléotides, possédant une spécificité d'hybridation dans des conditions déterminées pour former un complexe d'hybridation avec une séquence nucléotidique cible. La sonde comporte également un rapporteur (tel qu'un fluorophore, une enzyme ou tout autre système de détection), qui va permettre la détection de la séquence nucléotidique cible. Dans la présente invention, la séquence nucléotidique cible peut être une séquence nucléotidique comprise dans un ARN messager (ARNm) ou une séquence nucléotidique comprise dans un ADN complémentaire (ADNc) obtenu par transcription inverse dudit ARNm. Lorsque l'on souhaite cibler plusieurs biomarqueurs (e.g. des gènes ou des HERV) différents, plusieurs sondes différentes sont de préférence utilisées, ayant préférentiellement chacune une capacité à s'hybrider spécifiquement avec un biomarqueur différent.

Par « hybridation », on entend le processus au cours duquel, dans des conditions appropriées, deux fragments nucléotidiques, tels que par exemple une sonde d'hybridation et un fragment nucléotidique cible, ayant des séquences suffisamment complémentaires, sont susceptibles de former un double brin avec des liaisons hydrogènes stables et spécifiques. Un fragment nucléotidique "capable de s'hybrider" avec un polynucléotide est un fragment pouvant s'hybrider avec ledit polynucléotide dans des conditions d'hybridation, qui peuvent être déterminées dans chaque cas de façon connue. Les conditions d'hybridation sont déterminées par la stringence, c'est-à-dire la rigueur des conditions opératoires. L'hybridation est d'autant plus spécifique qu'elle est effectuée à plus forte stringence. La stringence est définie notamment en fonction de la composition en bases d'un duplex sonde/cible, ainsi que par le degré de mésappariement entre deux acides nucléiques. La stringence peut également être fonction des paramètres de la réaction, tels que la concentration et le type d'espèces ioniques présentes dans la solution d'hybridation, la nature et la concentration d'agents dénaturants et/ou la température d'hybridation. La stringence des conditions dans lesquelles une réaction d'hybridation doit être réalisée dépendra principalement des sondes d'hybridation utilisées. Toutes ces données sont bien connues et les conditions appropriées peuvent être déterminées par l'homme du métier. En général, selon la longueur des sondes d'hybridation utilisées, la température pour la réaction d'hybridation est comprise entre environ 20 et 70°C, en particulier entre 35 et 65°C dans une solution saline à une concentration d'environ 0,5 à 1 M. On réalise ensuite une étape de détection de la réaction d'hybridation.

Par « réaction d'amplification enzymatique », on entend un processus générant de multiples copies d'un fragment nucléotidique cible, par l'action d'au moins une enzyme. De telles réactions d'amplification sont bien connues de l'homme du métier et on peut citer notamment les techniques suivantes : PCR *(Polymerase Chain Reaction),* LCR *(Ligase Chain Reaction),* RCR (*Repair Chain Reaction),* 3SR *(Self Sustained Séquence Replication*) avec la demande de brevet WO-A-90/06995, NASBA *(Nucleic Acid Sequence-Based Amplification),* TMA *(Transcription Mediated Amplification)* avec le brevet US-A-5,399,491, et LAMP (*Loop mediated isothermal amplification)* avec le brevet US6410278. Lorsque la réaction d'amplification enzymatique est une PCR, on parlera plus particulièrement de RT-PCR (RT pour *« reverse transcription »),* lorsque l'étape d'amplification est précédée d'une étape de réverse-transcription d'ARN messager (ARNm) en ADN complémentaire (ADNc), et de qPCR ou RT-qPCR lorsque la PCR est quantitative.

L'invention a également pour objet l'utilisation :
- de moyens d'amplification (e.g. amorces) et/ou de moyens de détection (e.g. sondes) de l'expression de C3AR1, et optionnellement également de l'expression d'un ou plusieurs autre(s) gène(s), comme indiqué précédemment, dans tous les modes de réalisation (et, de manière particulièrement préférée, un, deux, trois, quatre, cinq, six, sept, huit, neuf, dix ou onze gènes, sélectionnés dans le groupe constitué par : CD74, CIITA, IFNG, IL1R2, TAP2, CD177, HP, CX3R1, S100A9, CTLA4 et CD3D) ; ou
   - d'un kit comprenant de tels moyens d'amplification et/ou de détection, et de préférence l'ensemble des moyens d'amplification et/ou de détection dudit kit permettent la détection et/ou l'amplification d'au plus 100, 90, 80, 70, 60, 50, 40, 30, 29, 28, 27, 26, 25, 24, 23, 22, 21, 20, 19, 18, 17, 16, 15, 14, 13, 12, 11, 10, 9, 8, 7, 6, 5, 4, 3, 2 biomarqueurs, au total, et optionnellement ledit kit comprend également des moyens d'amplification et/ou de détection de l'expression d'un ou plusieurs gènes de ménage (de préférence sélectionnés dans la liste constituée par : DECR1, HPRT1 et PPIB),
   pour déterminer un risque de survenue de complication chez un patient choisi parmi un risque de décès ou un risque de survenue d'une infection associée aux soins. de préférence d'une infection nosocomiale, chez un patient, de préférence un patient au sein d'un établissement de santé, de préférence encore au sein d'un hôpital, de préférence encore au sein du service des urgences, du service de réanimation, en unité de soins intensifs ou en unité de soins continus ; de manière particulièrement préférée, le patient est un patient en état septique (plus particulièrement en choc septique), un patient atteint de brûlures (plus particulièrement, de brûlures graves), un patient atteint de traumatismes (plus particulièrement, de traumatismes graves), ou un patient opéré par chirurgie (plus particulièrement, une chirurgie lourde).

La présente invention est illustrée de manière non limitative par les exemples suivants.

### Exemple 1 : La mesure de l'expression de C3AR1 permet de prédire le risque de complication chez un patient

### Matériels et Méthodes

Une étude clinique observationnelle prospective, longitudinale et monocentrique a été réalisée à l'Hôpital Edouard Herriot (Lyon, France). Le design de cette étude clinique a été publié dans Rol et al (2017), BMJ Open 7(6) : e015734. L'étude clinique a été approuvée par l'Agence Nationale de Sécurité du Médicament et des produits de santé (ANSM) en novembre 2015 et le Comité de Protection des Personnes Sud-Est Il en décembre 2015. Des amendements au protocole ont été effectués en juillet 2016, puis en janvier 2017. Brièvement, un total de 377 patients, en état septique (n=35) ou en choc septique (n=72), atteints de brûlures graves (n=24), de traumatismes graves (n=137) ou hospitalisés dans un service de réanimation ou une unité de soins intensifs après une chirurgie majeure (n=109), et 175 volontaires sains ont été inclus entre décembre 2015 et mars 2018.
- Patients en état septique/en choc septique : selon le premier protocole clinique, seuls les patients en choc septique étaient inclus, sur la base d'une suspicion d'un foyer infectieux, un début de traitement par catécholamines dans les 48h suivant l'admission en réanimation et d'un traitement au catécholamines (noradrénaline) > 0.25 µg/kg/min pendant au moins 2 heures. Ensuite, les critères d'éligibilité ont été modifiés en août 2016, suite à la publication d'une nouvelle définition du choc septique, Sepsis 3 (Singer et al (2016), JAMA 810-801:(8)315). Les patients en choc septique étaient donc inclus sur la base d'une suspicion d'un foyer infectieux, un début de traitement par catécholamines dans les 48h suivant l'admission en réanimation et d'une thérapie vasopressive nécessaire pour maintenir une pression artérielle ≥ 65 mm Hg et concentration en lactate > 2 mmol/L (18 mg/dL), malgré la correction d'une hypovolémie. En 2017, a été ajoutée la possibilité d'inclure des patients en état septique (selon la définition Sepsis 3), à savoir la suspicion d'un foyer infectieux et l'augmentation du score SOFA ≥ 2 points par rapport au SOFA de base dans les 48h qui suivent l'admission en réanimation. Pour cette population, le jour 1 correspond au jour du diagnostic du sepsis ou du choc septique ;
- Traumatismes graves : dans le premier protocole, seuls les patients avec un traumatisme grave ont été inclus (Injury Severity Score (ISS) ≥ 25). En août 2016, a été ajoutée la possibilité d'inclure également des traumatismes moins graves (16 < ISS < 24). Pour cette population, le jour 1 correspond au jour d'admission en en service de réanimation ou unité de soins intensifs (^{~}jour du traumatisme) ;
- Chirurgie majeure : dans le premier protocole, seules une œsogastrectomie, une résection de la vessie de type Bricker, une duodénopancréatectomie céphalique et chirurgie de l'aorte abdominale par laparotomie ont été considérées. D'autres types de chirurgie à haut risque de complication ont été ajoutés en janvier 2017 : une pancréatectomie (totale ou caudale), des tumeurs neuroendocrines, une hépatectomie (sur le côté droit), une colectomie étendue (laparotomie), une résection abdopérinéale, une néphrectomie (laparotomie, PKD), un pontage ilio-fémoral (Scarpa). Pour cette population, le jour 1 correspond au jour de la chirurgie ;
- Brûlures graves : les patients ont été sélectionnés sur la base d'une surface totale de brûlures supérieure à 30%. Pour cette population, le jour 1 correspond au jour d'admission en service de réanimation ou unité de soins intensifs (^{~} jour de la brûlure).

Les critères d'exclusion portaient essentiellement sur des facteurs qui auraient pu impacter le statut immunitaire et biaiser les résultats (par exemple : une neutropénie sévère, des traitements corticostéroïdes, une pathologie onco-hématologique...). Chaque événement conduisant à une suspection d'infection associée aux soins, survenant au sein de l'hôpital avant le J30 a été revu indépendamment par trois médecins non impliqués dans le recrutement des patients. Vingt-six pourcents des patients ont développé au moins une infection associée aux soins avant le J30, ou avant la sortie de l'hôpital.

Des échantillons de sang ont été collectés dans des tubes PAXgene^{®} (ref. 762165, PreAnalytiX GmbH Hombrechtikon Switzerland), une fois pour les volontaires sains, et plusieurs fois pour les patients, i.e. 3-4 fois la première semaine (aux jours 1 ou 2 : J1/2, aux jours 3 ou 4 : J3/4 et aux jours 5, 6 ou 7 : J5/7), puis 3 fois à des temps plus tardifs (autour du J14, du J28 et du J60).

Le niveau d'expression de C3AR1 a été mesuré dans ces échantillons par RT-qPCR. Brièvement, l'ARN a été extrait des échantillons de sang total en utilisant le *Maxwell HT Simply RNA kit* (ref. AX2420, Promega) et la plateforme automatisée EVO (TECAN), suivant les instructions du fournisseur du kit. Ensuite, 10 ng d'ARN total ont été rétro-transcrits en ADN complémentaire (ADNc), en utilisant le master mix *Fluidigm Reverse transcription* (ref. PN100-6472 A1, Fluidigm), suivant les instructions du fournisseur. L'expression de C3AR1 a alors été quantifiée par qPCR, en utilisant le système de PCR en temps réel Biomark HD de Fluidigm. D'abord, une étape de préamplification du cDNA a été réalisée selon les recommandations du fournisseur en utilisant le master mix PreAmp (Ref. PN100-5875 C1, Fluidigm). Ensuite, une étape de traitement par exonuclease I (ref. PN100-5875 C1, Fluidigm) a été faite et les qPCR ont été réalisées sur des circuits intégrés de fluidique 192.24 (Ref. PN100-7222 C1) pour une détection par EvaGreen, comme recommandé par le fournisseur. Les cycles seuils (ou Ct) ont ensuite été déterminés. La normalisation de l'expression de C3AR1 en CNRQ (Quantité Relative Normalisée Calibrée ou *Calibrated Normalized Relative Quantity)* a été réalisée en utilisant les Ct de 3 gènes de référence (DECR1, HPRT1, PPIB) et un calibrateur (correspondant à un mélange d'échantillons traités *ex vivo* par du LPS (agent stimulant du système immunitaire) (50%) et d'échantillons non traités ex vivo (50%), provenant de volontaires/individus sains) dans chaque circuit intégré de fluidique 192.24, tel que décrit dans Hellemans et al (2007), Genome biology 8(2):R19.

En ce qui concerne l'analyse des données, les associations entre l'expression de C3AR1, mesurée à différents temps durant la première semaine, et la survenue d'un décès ou d'une infection associée aux soins avant le J30 à partir de l'inclusion dans l'étude ont été évaluées. Les résultats ont été calculés sous la forme *d'Hazard Ratios* exprimés en distance inter-quartile avec l'intervalle de confiance à 95% associé (HR IQR). Ensuite, des régressions logistiques univariées ont été implémentées pour prédire le risque d'occurrence d'une infection associée aux soins avant le J15. La puissance des valeurs prédites par la régression logistique pour discriminer entre infection associée aux soins et absence d'infection associée aux soins a été quantifiée par l'aire sous la courbe (AUC) de la courbe ROC *(Receiver Operating Characteristic),* et des intervalles de confiance à 95% ont été estimés.

Ensuite, l'association entre l'expression de C3AR1 et l'occurrence d'une infection associée aux soins a été évaluée pour différents intervalles de temps de survenue de l'infection (i.e. délais entre le prélèvement d'échantillon et la 1^{ère} survenue d'une infection). Les différents délais considérés étaient : une infection associée aux soins dans les 4 jours et dans les 7 jours après le prélèvement d'échantillon, quel que soit le moment où l'échantillon a été prélevé. Pour chaque patient ayant développé une infection associée aux soins, l'échantillon considéré correspond au plus proche prélèvement avant la survenue du premier épisode d'infection associée aux soins. Pour les patients n'ayant pas développé d'infection associée aux soins (i.e. patients contrôles), une méthode d'appariement a été utilisée pour sélectionner pour chaque cas, un patient contrôle ayant le même jour de prélèvement de l'échantillon, et des scores SOFA et Charlson proches. Finalement, un unique contrôle a été sélectionné pour chaque cas unique. Des régressions logistiques univariées ont été implémentées. La puissance des valeurs prédites par la régression logistique pour discriminer entre infection associée aux soins et absence d'infection associée aux soins a été quantifiée par l'aire sous la courbe (AUC) de la courbe ROC, et des intervalles de confiance à 95% ont été estimés.

### Résultats

Une augmentation de l'expression de C3AR1 au niveau ARNm, mesurée à J3/4 ou à J5/7 à partir de l'inclusion dans la cohorte, était associée avec un risque plus élevé d'occurrence d'une infection associée aux soins avant le J30 dans la population globale des patients (analyse univariée : J3/4 : HR IQR= 1,17 [1,01-1,35], p= 0,0316; J5/7 : HR IQR= 1,70 [1,38-2,08], p= 0,0000004). Cette association était toujours significative, pour le temps de mesure à J5/7, après ajustement avec les scores SOFA et Charlson (analyse multivariée : HR IQR= 1,68 [1,26-2,25], p= 0,0005). Une augmentation de l'expression de C3AR1 au niveau ARNm, mesurée à J1/2, J3/4 ou J5/7 à partir de l'inclusion dans la cohorte, était également associée avec un risque plus élevé de décès avant le J30 dans la population globale des patients (analyse univariée : J1/2 : HR IQR= 1,42 [1,10-1,83], p = 0,0072; J3/4 : HR IQR= 1,54 [1,27-1,85], p= 0,000007; J5/7 : HR IQR= 2,09 [1,37-3,20], p= 0,0007).

Par ailleurs, les modèles de prédiction ont montré que l'expression de C3AR1 au niveau ARNm, mesurée à J3/4 ou à J5/7 à partir de l'inclusion dans la cohorte, permettait de prédire l'occurrence d'une infection associée aux soins avant le J15 à partir de l'inclusion dans la cohorte (Tableau 4).

**Tableau 4. Performance (AUC et intervalles de confiance à 95%, avec les bornes AUC.2.5 et AUC.97.5) de la mesure de l'expression de C3AR1, mesurée à J3/4 ou à J5/7 à partir de l'inclusion dans la cohorte, pour la prédiction de survenue d'une infection associée aux soins avant le J15 à partir de l'inclusion dans la cohorte, chez des patients en état septique/en choc septique, atteints de traumatismes graves, ou hospitalisés après une chirurgie majeure.**

| **Jour de prélèvement de l'échantillon** | **AUC** | **AUC.2.5** | **AUC.97.5** |
|---|---|---|---|
| J3/4 | 0,70 | 0,62 | 0,78 |
| J5/7 | 0,75 | 0,66 | 0,84 |

Les modèles de prédiction ont également montré que l'expression de C3AR1 au niveau ARNm, permettait de prédire l'occurrence d'une infection associée aux soins dans les 4 jours ou dans les 7 jours suivant le prélèvement de l'échantillon (Tableau 5).

**Tableau 5. Performance (AUC et intervalles de confiance à 95%, avec les bornes AUC.2.5 et AUC.97.5) de la mesure de l'expression de C3AR1, pour la prédiction de survenue d'une infection associée aux soins dans les 4 jours ou dans les 7 jours suivant le prélèvement d'échantillon.**

| **Intervalle maximum de temps entre le prélèvement de l'échantillon et l'éventuelle survenue de première infection associée aux soins** | **AUC** | **AUC.2.5** | **AUC.97.5** |
|---|---|---|---|
| 4 jours | 0,66 | 0,56 | 0,76 |
| 7 jours | 0,63 | 0,54 | 0,72 |

Ainsi, les résultats obtenus montrent que la mesure de l'expression de C3AR1 seul permet de prédire le risque de complication, et en particulier la survenue d'infection(s) associée(s) aux soins dans les 15 jours à partir de l'agression immuno-inflammatoire, dans les 4 jours suivant le prélèvement de l'échantillon ou dans les 7 jours suivant le prélèvement de l'échantillon.

### Exemple 2 : La mesure de l'expression d'un ou plusieurs autre(s) biomarqueur(s), en combinaison avec la mesure de l'expression de C3AR1, permet d'améliorer la performance prédictive du risque de complication

Dans cet exemple, le niveau d'expression de C3AR1, mais également d'autres biomarqueurs (gènes ou HERV), a été mesuré par RT-qPCR. Des régressions logistiques multivariées (combinaison de la mesure de l'expression de C3AR1 et d'un ou plusieurs autre(s) biomarqueur(s) (gène(s) ou HERV)) ont ensuite été effectuées. La mesure de l'expression d'un ou plusieurs de ces autresbiomarqueurs, en plus de la mesure de l'expression de C3AR1, permet d'améliorer la performance prédictive (par rapport à la mesure de l'expression de C3AR1 seul) du risque de complication, et en particulier du risque de survenue d'une infection associée aux soins, que ce soit avant le J15 à partir de l'inclusion dans la cohorte (Tableau 6) ou dans les 4 jours ou dans les 7 jours suivant le prélèvement de l'échantillon (Tableau 7).

**Tableau 6. Performance (AUC et intervalle de confiance à 95%, avec les bornes AUC.2.5 et AUC.97.5) de la mesure de l'expression de C3AR1, en combinaison avec un ou plusieurs autre(s) biomarqueur(s) (analyse multivariée), mesurée à J3/4 ou à J5/7 à partir de l'inclusion dans la cohorte, pour la prédiction de survenue d'une infection associée aux soins avant le J15 à partir de l'inclusion dans la cohorte, chez des patients en état septique/en choc septique, atteints de traumatismes graves, ou hospitalisés après une chirurgie majeure.**

| **Jour de prélèvement de l'échantillon** | **Biomarqueurs** | **AUC** | **AUC.2.5** | **AUC.97.5** |
|---|---|---|---|---|
| J3/4 | C3AR1 + 190665001-HERV0376 | 0,7 | 0,62 | 0,78 |
| J3/4 | C3AR1 + 220247002-HERV0797 | 0,7 | 0,62 | 0,78 |
| J3/4 | C3AR1 + IL10 | 0,7 | 0,62 | 0,78 |
| J3/4 | C3AR1 + 050286701-HERV0513 | 0,7 | 0,62 | 0,78 |
| J3/4 | C3AR1 + 130360601-HERV0808 | 0,7 | 0,61 | 0,79 |
| J3/4 | C3AR1 + IL18R1 | 0,7 | 0,62 | 0,78 |
| J3/4 | C3AR1 + IL1R2 | 0,71 | 0,63 | 0,78 |
| J3/4 | C3AR1 + CD74 | 0,71 | 0,62 | 0,79 |
| J3/4 | C3AR1 + 021456001-MALR1017 | 0,71 | 0,63 | 0,79 |
| J3/4 | C3AR1 + PDCD1 | 0,71 | 0,62 | 0,8 |
| J3/4 | C3AR1 + IL1RN | 0,71 | 0,63 | 0,79 |
| J3/4 | C3AR1 + CX3CR1 | 0,71 | 0,63 | 0,79 |
| J3/4 | C3AR1 + IL15 | 0,73 | 0,65 | 0,8 |
| J3/4 | C3AR1 + IL1R2 | 0,705 | 0,628 | 0,782 |
| J3/4 | C3AR1 + IL1R2 + CD74 | 0,707 | 0,621 | 0,793 |
| J5/7 | C3AR1 + CTLA4 | 0,751 | 0,662 | 0,839 |
| J5/7 | C3AR1 + IL18 | 0,753 | 0,668 | 0,837 |
| J5/7 | C3AR1 + OAS3 | 0,753 | 0,667 | 0,839 |
| J5/7 | C3AR1 + IFIH1 | 0,758 | 0,646 | 0,87 |
| J5/7 | C3AR1 + IL1RN | 0,762 | 0,677 | 0,847 |
| J5/7 | C3AR1 + 050286701-HERV0513 | 0,764 | 0,686 | 0,842 |
| J5/7 | C3AR1 + 170369402HE41 | 0,767 | 0,688 | 0,846 |
| J5/7 | C3AR1 + 190665001-HERV0376 | 0,767 | 0,688 | 0,845 |
| J5/7 | C3AR1 + HP | 0,768 | 0,683 | 0,853 |
| J5/7 | C3AR1 + IL7R | 0,772 | 0,701 | 0,843 |
| J5/7 | C3AR1 + IL18RAP | 0,777 | 0,699 | 0,855 |
| J5/7 | C3AR1 + IL15 | 0,782 | 0,682 | 0,881 |
| J5/7 | C3AR1 + ICOS | 0,782 | 0,713 | 0,85 |
| J5/7 | C3AR1 + CD40LG | 0,784 | 0,717 | 0,852 |
| J5/7 | C3AR1 + CD3D | 0,784 | 0,715 | 0,854 |
| J5/7 | C3AR1 + STAT4 | 0,785 | 0,708 | 0,861 |
| J5/7 | C3AR1 + BTLA | 0,787 | 0,716 | 0,857 |
| J5/7 | C3AR1 + IL18R1 | 0,787 | 0,709 | 0,865 |
| J5/7 | C3AR1 + PDCD1 | 0,787 | 0,711 | 0,864 |
| J5/7 | C3AR1 + S100A9 | 0,793 | 0,707 | 0,88 |
| J5/7 | C3AR1 + CD74 | 0,799 | 0,719 | 0,879 |
| J5/7 | C3AR1 + IFNG | 0,799 | 0,729 | 0,87 |
| J5/7 | C3AR1 + CX3CR1 | 0,802 | 0,722 | 0,882 |
| J5/7 | C3AR1 + IL1R2 | 0,817 | 0,756 | 0,879 |
| J5/7 | C3AR1 + 021456001-MALR1017 | 0,839 | 0,784 | 0,893 |
| J5/7 | C3AR1 + CD177 + CIITA | 0,756 | 0,662 | 0,851 |
| J5/7 | C3AR1 + CD177 + CIITA + TAP2 | 0,775 | 0,683 | 0,867 |
| J5/7 | C3AR1 + CD177 + CIITA + TAP2 + CD3D | 0,775 | 0,686 | 0,864 |
| J5/7 | C3AR1 + CD177 + CIITA + TAP2 + CD3D + CTLA4 | 0,772 | 0,682 | 0,862 |
| J5/7 | C3AR1 + CD177 + CIITA + TAP2 + CTLA4 | 0,773 | 0,679 | 0,866 |
| J5/7 | C3AR1 + CD177 + CX3CR1 | 0,76 | 0,659 | 0,862 |
| J5/7 | C3AR1 + CD177 + CX3CR1 + CIITA | 0,767 | 0,674 | 0,859 |
| J5/7 | C3AR1 + CD177 + CX3CR1 + CIITA + CD3D | 0,763 | 0,666 | 0,86 |
| J5/7 | C3AR1 + CD177 + CX3CR1 + CIITA + CD3D + CTLA4 | 0,754 | 0,647 | 0,86 |
| J5/7 | C3AR1 + CD177 + CX3CR1 + CIITA + CTLA4 | 0,756 | 0,65 | 0,862 |
| J5/7 | C3AR1 + CD177 + CX3CR1 + CIITA + TAP2 | 0,778 | 0,688 | 0,868 |
| J5/7 | C3AR1 + CD177 + CX3CR1 + CIITA + TAP2 + CD3D | 0,777 | 0,689 | 0,866 |
| J5/7 | C3AR1 + CD177 + CX3CR1 + CIITA + TAP2 + CD3D + CTLA4 | 0,774 | 0,683 | 0,864 |
| J5/7 | C3AR1 + CD177 + CX3CR1 + CIITA + TAP2 + CTLA4 | 0,774 | 0,682 | 0,865 |
| J5/7 | C3AR1 + CD177 + CX3CR1 + TAP2 | 0,772 | 0,68 | 0,863 |
| J5/7 | C3AR1 + CD177 + CX3CR1 + TAP2 + CD3D | 0,772 | 0,68 | 0,863 |
| J5/7 | C3AR1 + CD177 + CX3CR1 + TAP2 + CD3D + CTLA4 | 0,769 | 0,676 | 0,862 |
| J5/7 | C3AR1 + CD177 + CX3CR1 + TAP2 + CTLA4 | 0,768 | 0,674 | 0,863 |
| J5/7 | C3AR1 + CD177 + S100A9 | 0,756 | 0,661 | 0,851 |
| J5/7 | C3AR1 + CD177 + S100A9 + CD3D | 0,763 | 0,656 | 0,871 |
| J5/7 | C3AR1 + CD177 + S100A9 + CD3D + CTLA4 | 0,76 | 0,648 | 0,871 |
| J5/7 | C3AR1 + CD177 + S100A9 + CIITA | 0,762 | 0,671 | 0,854 |
| J5/7 | C3AR1 + CD177 + S100A9 + CIITA + CD3D | 0,769 | 0,667 | 0,872 |
| J5/7 | C3AR1 + CD177 + S100A9 + CIITA + CD3D + CTLA4 | 0,762 | 0,655 | 0,868 |
| J5/7 | C3AR1 + CD177 + S100A9 + CIITA + TAP2 | 0,789 | 0,704 | 0,873 |
| J5/7 | C3AR1 + CD177 + S100A9 + CIITA + TAP2 + CD3D | 0,779 | 0,691 | 0,867 |
| J5/7 | C3AR1 + CD177 + S100A9 + CIITA + TAP2 + CD3D + CTLA4 | 0,781 | 0,695 | 0,868 |
| J5/7 | C3AR1 + CD177 + S100A9 + CIITA + TAP2 + CTLA4 | 0,785 | 0,699 | 0,871 |
| J5/7 | C3AR1 + CD177 + S100A9 + CX3CR1 | 0,787 | 0,69 | 0,885 |
| J5/7 | C3AR1 + CD177 + S100A9 + CX3CR1 + CD3D | 0,78 | 0,675 | 0,886 |
| J5/7 | C3AR1 + CD177 + S100A9 + CX3CR1 + CD3D + CTLA4 | 0,775 | 0,664 | 0,887 |
| J5/7 | C3AR1 + CD177 + S100A9 + CX3CR1 + CIITA | 0,786 | 0,691 | 0,882 |
| J5/7 | C3AR1 + CD177 + S100A9 + CX3CR1 + CIITA + CD3D | 0,782 | 0,68 | 0,884 |
| J5/7 | C3AR1 + CD177 + S100A9 + CX3CR1 + CIITA + CD3D + CTLA4 | 0,774 | 0,665 | 0,883 |
| J5/7 | C3AR1 + CD177 + S100A9 + CX3CR1 + CIITA + CTLA4 | 0,778 | 0,668 | 0,889 |
| J5/7 | C3AR1 + CD177 + S100A9 + CX3CR1 + CIITA + TAP2 | 0,79 | 0,704 | 0,875 |
| J5/7 | C3AR1 + CD177 + S100A9 + CX3CR1 + CIITA + TAP2 + CD3D | 0,776 | 0,684 | 0,868 |
| J5/7 | C3AR1 + CD177 + S100A9 + CX3CR1 + CIITA + TAP2 + CD3D + CTLA4 | 0,775 | 0,684 | 0,867 |
| J5/7 | C3AR1 + CD177 + S100A9 + CX3CR1 + CIITA + TAP2 + CTLA4 | 0,789 | 0,7 | 0,878 |
| J5/7 | C3AR1 + CD177 + S100A9 + CX3CR1 + CTLA4 | 0,778 | 0,666 | 0,89 |
| J5/7 | C3AR1 + CD177 + S100A9 + CX3CR1 + TAP2 | 0,792 | 0,707 | 0,876 |
| J5/7 | C3AR1 + CD177 + S100A9 + CX3CR1 + TAP2 + CD3D | 0,777 | 0,688 | 0,865 |
| J5/7 | C3AR1 + CD177 + S100A9 + CX3CR1 + TAP2 + CD3D + CTLA4 | 0,777 | 0,688 | 0,866 |
| J5/7 | C3AR1 + CD177 + S100A9 + CX3CR1 + TAP2 + CTLA4 | 0,79 | 0,704 | 0,875 |
| J5/7 | C3AR1 + CD177 + S100A9 + TAP2 | 0,786 | 0,703 | 0,869 |
| J5/7 | C3AR1 + CD177 + S100A9 + TAP2 + CD3D | 0,778 | 0,69 | 0,865 |
| J5/7 | C3AR1 + CD177 + S100A9 + TAP2 + CD3D + CTLA4 | 0,778 | 0,691 | 0,864 |
| J5/7 | C3AR1 + CD177 + S100A9 + TAP2 + CTLA4 | 0,788 | 0,703 | 0,873 |
| J5/7 | C3AR1 + CD177 + TAP2 | 0,769 | 0,676 | 0,862 |
| J5/7 | C3AR1 + CD177 + TAP2 + CD3D | 0,771 | 0,68 | 0,862 |
| J5/7 | C3AR1 + CD177 + TAP2 + CD3D + CTLA4 | 0,769 | 0,676 | 0,863 |
| J5/7 | C3AR1 + CD177 + TAP2 + CTLA4 | 0,769 | 0,676 | 0,863 |
| J5/7 | C3AR1 + CD74 + CD177 | 0,759 | 0,646 | 0,872 |
| J5/7 | C3AR1 + CD74 + CD177 + CD3D | 0,758 | 0,646 | 0,869 |
| J5/7 | C3AR1 + CD74 + CD177 + CD3D + CTLA4 | 0,752 | 0,641 | 0,863 |
| J5/7 | C3AR1 + CD74 + CD177 + CIITA + CD3D | 0,75 | 0,636 | 0,865 |
| J5/7 | C3AR1 + CD74 + CD177 + CIITA + CD3D + CTLA4 | 0,75 | 0,634 | 0,866 |
| J5/7 | C3AR1 + CD74 + CD177 + CIITA + TAP2 | 0,768 | 0,661 | 0,875 |
| J5/7 | C3AR1 + CD74 + CD177 + CIITA + TAP2 + CD3D | 0,763 | 0,658 | 0,868 |
| J5/7 | C3AR1 + CD74 + CD177 + CIITA + TAP2 + CD3D + CTLA4 | 0,767 | 0,66 | 0,873 |
| J5/7 | C3AR1 + CD74 + CD177 + CIITA + TAP2 + CTLA4 | 0,769 | 0,664 | 0,875 |
| J5/7 | C3AR1 + CD74 + CD177 + CTLA4 | 0,753 | 0,636 | 0,87 |
| J5/7 | C3AR1 + CD74 + CD177 + CX3CR1 | 0,767 | 0,658 | 0,876 |
| J5/7 | C3AR1 + CD74 + CD177 + CX3CR1 + CD3D | 0,761 | 0,649 | 0,873 |
| J5/7 | C3AR1 + CD74 + CD177 + CX3CR1 + CD3D + CTLA4 | 0,757 | 0,643 | 0,872 |
| J5/7 | C3AR1 + CD74 + CD177 + CX3CR1 + CIITA | 0,754 | 0,636 | 0,872 |
| J5/7 | C3AR1 + CD74 + CD177 + CX3CR1 + CIITA + CD3D | 0,755 | 0,638 | 0,872 |
| J5/7 | C3AR1 + CD74 + CD177 + CX3CR1 + CIITA + CD3D + CTLA4 | 0,754 | 0,634 | 0,873 |
| J5/7 | C3AR1 + CD74 + CD177 + CX3CR1 + CIITA + CTLA4 | 0,75 | 0,627 | 0,874 |
| J5/7 | C3AR1 + CD74 + CD177 + CX3CR1 + CIITA + TAP2 | 0,769 | 0,661 | 0,876 |
| J5/7 | C3AR1 + CD74 + CD177 + CX3CR1 + CIITA + TAP2 + CD3D | 0,763 | 0,658 | 0,869 |
| J5/7 | C3AR1 + CD74 + CD177 + CX3CR1 + CIITA + TAP2 + CD3D + CTLA4 | 0,765 | 0,659 | 0,872 |
| J5/7 | C3AR1 + CD74 + CD177 + CX3CR1 + CIITA + TAP2 + CTLA4 | 0,767 | 0,658 | 0,876 |
| J5/7 | C3AR1 + CD74 + CD177 + CX3CR1 + CTLA4 | 0,764 | 0,649 | 0,879 |
| J5/7 | C3AR1 + CD74 + CD177 + CX3CR1 + TAP2 | 0,778 | 0,678 | 0,879 |
| J5/7 | C3AR1 + CD74 + CD177 + CX3CR1 + TAP2 + CD3D | 0,773 | 0,673 | 0,874 |
| J5/7 | C3AR1 + CD74 + CD177 + CX3CR1 + TAP2 + CD3D + CTLA4 | 0,769 | 0,667 | 0,872 |
| J5/7 | C3AR1 + CD74 + CD177 + CX3CR1 + TAP2 + CTLA4 | 0,771 | 0,668 | 0,874 |
| J5/7 | C3AR1 + CD74 + CD177 + S100A9 | 0,768 | 0,665 | 0,87 |
| J5/7 | C3AR1 + CD74 + CD177 + S100A9 + CD3D | 0,774 | 0,68 | 0,868 |
| J5/7 | C3AR1 + CD74 + CD177 + S100A9 + CD3D + CTLA4 | 0,771 | 0,677 | 0,865 |
| J5/7 | C3AR1 + CD74 + CD177 + S100A9 + CIITA | 0,762 | 0,654 | 0,87 |
| J5/7 | C3AR1 + CD74 + CD177 + S100A9 + CIITA + CD3D | 0,772 | 0,673 | 0,871 |
| J5/7 | C3AR1 + CD74 + CD177 + S100A9 + CIITA + CD3D + CTLA4 | 0,772 | 0,669 | 0,874 |
| J5/7 | C3AR1 + CD74 + CD177 + S100A9 + CIITA + CTLA4 | 0,753 | 0,637 | 0,869 |
| J5/7 | C3AR1 + CD74 + CD177 + S100A9 + CIITA + TAP2 | 0,785 | 0,688 | 0,882 |
| J5/7 | C3AR1 + CD74 + CD177 + S100A9 + CIITA + TAP2 + CD3D | 0,783 | 0,689 | 0,876 |
| J5/7 | C3AR1 + CD74 + CD177 + S100A9 + CIITA + TAP2 + CD3D + CTLA4 | 0,785 | 0,692 | 0,878 |
| J5/7 | C3AR1 + CD74 + CD177 + S100A9 + CIITA + TAP2 + CTLA4 | 0,782 | 0,683 | 0,881 |
| J5/7 | C3AR1 + CD74 + CD177 + S100A9 + CTLA4 | 0,763 | 0,659 | 0,868 |
| J5/7 | C3AR1 + CD74 + CD177 + S100A9 + CX3CR1 | 0,788 | 0,698 | 0,878 |
| J5/7 | C3AR1 + CD74 + CD177 + S100A9 + CX3CR1 + CD3D | 0,784 | 0,688 | 0,881 |
| J5/7 | C3AR1 + CD74 + CD177 + S100A9 + CX3CR1 + CD3D + CTLA4 | 0,782 | 0,684 | 0,88 |
| J5/7 | C3AR1 + CD74 + CD177 + S100A9 + CX3CR1 + CIITA | 0,782 | 0,68 | 0,884 |
| J5/7 | C3AR1 + CD74 + CD177 + S100A9 + CX3CR1 + CIITA + CD3D | 0,783 | 0,682 | 0,883 |
| J5/7 | C3AR1 + CD74 + CD177 + S100A9 + CX3CR1 + CIITA + CD3D + CTLA4 | 0,775 | 0,671 | 0,879 |
| J5/7 | C3AR1 + CD74 + CD177 + S100A9 + CX3CR1 + CIITA + CTLA4 | 0,771 | 0,657 | 0,885 |
| J5/7 | C3AR1 + CD74 + CD177 + S100A9 + CX3CR1 + CIITA + TAP2 | 0,793 | 0,698 | 0,888 |
| J5/7 | C3AR1 + CD74 + CD177 + S100A9 + CX3CR1 + CIITA + TAP2 + CD3D | 0,79 | 0,696 | 0,884 |
| J5/7 | C3AR1 + CD74 + CD177 + S100A9 + CX3CR1 + CIITA + TAP2 + CD3D + CTLA4 | 0,791 | 0,698 | 0,884 |
| J5/7 | C3AR1 + CD74 + CD177 + S100A9 + CX3CR1 + CIITA + TAP2 + CTLA4 | 0,791 | 0,694 | 0,889 |
| J5/7 | C3AR1 + CD74 + CD177 + S100A9 + CX3CR1 + CTLA4 | 0,781 | 0,684 | 0,879 |
| J5/7 | C3AR1 + CD74 + CD177 + S100A9 + CX3CR1 + TAP2 | 0,792 | 0,704 | 0,879 |
| J5/7 | C3AR1 + CD74 + CD177 + S100A9 + CX3CR1 + TAP2 + CD3D | 0,789 | 0,7 | 0,877 |
| J5/7 | C3AR1 + CD74 + CD177 + S100A9 + CX3CR1 + TAP2 + CD3D + CTLA4 | 0,792 | 0,706 | 0,879 |
| J5/7 | C3AR1 + CD74 + CD177 + S100A9 + CX3CR1 + TAP2 + CTLA4 | 0,791 | 0,7 | 0,881 |
| J5/7 | C3AR1 + CD74 + CD177 + S100A9 + TAP2 | 0,792 | 0,703 | 0,882 |
| J5/7 | C3AR1 + CD74 + CD177 + S100A9 + TAP2 + CD3D | 0,785 | 0,697 | 0,872 |
| J5/7 | C3AR1 + CD74 + CD177 + S100A9 + TAP2 + CD3D + CTLA4 | 0,785 | 0,698 | 0,872 |
| J5/7 | C3AR1 + CD74 + CD177 + S100A9 + TAP2 + CTLA4 | 0,789 | 0,696 | 0,881 |
| J5/7 | C3AR1 + CD74 + CD177 + TAP2 | 0,773 | 0,671 | 0,875 |
| J5/7 | C3AR1 + CD74 + CD177 + TAP2 + CD3D | 0,773 | 0,672 | 0,873 |
| J5/7 | C3AR1 + CD74 + CD177 + TAP2 + CD3D + CTLA4 | 0,77 | 0,668 | 0,871 |
| J5/7 | C3AR1 + CD74 + CD177 + TAP2 + CTLA4 | 0,769 | 0,665 | 0,873 |
| J5/7 | C3AR1 + CD74 + CD3D | 0,767 | 0,663 | 0,87 |
| J5/7 | C3AR1 + CD74 + CD3D + CTLA4 | 0,769 | 0,66 | 0,877 |
| J5/7 | C3AR1 + CD74 + CIITA | 0,754 | 0,636 | 0,872 |
| J5/7 | C3AR1 + CD74 + CIITA + CD3D | 0,755 | 0,64 | 0,87 |
| J5/7 | C3AR1 + CD74 + CIITA + CD3D + CTLA4 | 0,752 | 0,633 | 0,87 |
| J5/7 | C3AR1 + CD74 + CIITA + CTLA4 | 0,749 | 0,629 | 0,87 |
| J5/7 | C3AR1 + CD74 + CIITA + TAP2 | 0,773 | 0,665 | 0,88 |
| J5/7 | C3AR1 + CD74 + CIITA + TAP2 + CD3D | 0,771 | 0,664 | 0,879 |
| J5/7 | C3AR1 + CD74 + CIITA + TAP2 + CD3D + CTLA4 | 0,769 | 0,655 | 0,883 |
| J5/7 | C3AR1 + CD74 + CIITA + TAP2 + CTLA4 | 0,766 | 0,654 | 0,878 |
| J5/7 | C3AR1 + CD74 + CTLA4 | 0,767 | 0,656 | 0,877 |
| J5/7 | C3AR1 + CD74 + CX3CR1 | 0,766 | 0,658 | 0,874 |
| J5/7 | C3AR1 + CD74 + CX3CR1 + CD3D | 0,767 | 0,664 | 0,871 |
| J5/7 | C3AR1 + CD74 + CX3CR1 + CD3D + CTLA4 | 0,769 | 0,661 | 0,878 |
| J5/7 | C3AR1 + CD74 + CX3CR1 + CIITA | 0,756 | 0,637 | 0,875 |
| J5/7 | C3AR1 + CD74 + CX3CR1 + CIITA + CD3D | 0,755 | 0,639 | 0,87 |
| J5/7 | C3AR1 + CD74 + CX3CR1 + CIITA + CD3D + CTLA4 | 0,754 | 0,633 | 0,874 |
| J5/7 | C3AR1 + CD74 + CX3CR1 + CIITA + CTLA4 | 0,753 | 0,633 | 0,873 |
| J5/7 | C3AR1 + CD74 + CX3CR1 + CIITA + TAP2 | 0,773 | 0,664 | 0,883 |
| J5/7 | C3AR1 + CD74 + CX3CR1 + CIITA + TAP2 + CD3D | 0,767 | 0,659 | 0,875 |
| J5/7 | C3AR1 + CD74 + CX3CR1 + CIITA + TAP2 + CD3D + CTLA4 | 0,768 | 0,654 | 0,882 |
| J5/7 | C3AR1 + CD74 + CX3CR1 + CIITA + TAP2 + CTLA4 | 0,767 | 0,653 | 0,881 |
| J5/7 | C3AR1 + CD74 + CX3CR1 + CTLA4 | 0,768 | 0,659 | 0,878 |
| J5/7 | C3AR1 + CD74 + CX3CR1 + TAP2 | 0,776 | 0,674 | 0,878 |
| J5/7 | C3AR1 + CD74 + CX3CR1 + TAP2 + CD3D | 0,776 | 0,678 | 0,873 |
| J5/7 | C3AR1 + CD74 + CX3CR1 + TAP2 + CD3D + CTLA4 | 0,777 | 0,675 | 0,88 |
| J5/7 | C3AR1 + CD74 + CX3CR1 + TAP2 + CTLA4 | 0,777 | 0,674 | 0,88 |
| J5/7 | C3AR1 + CD74 + IFNG | 0,77 | 0,653 | 0,886 |
| J5/7 | C3AR1 + CD74 + IFNG + CD177 | 0,755 | 0,634 | 0,877 |
| J5/7 | C3AR1 + CD74 + IFNG + CD177 + CD3D | 0,754 | 0,625 | 0,882 |
| J5/7 | C3AR1 + CD74 + IFNG + CD177 + CD3D + CTLA4 | 0,75 | 0,62 | 0,881 |
| J5/7 | C3AR1 + CD74 + IFNG + CD177 + CIITA | 0,756 | 0,633 | 0,879 |
| J5/7 | C3AR1 + CD74 + IFNG + CD177 + CIITA + CD3D | 0,754 | 0,628 | 0,881 |
| J5/7 | C3AR1 + CD74 + IFNG + CD177 + CIITA + CD3D + CTLA4 | 0,751 | 0,622 | 0,879 |
| J5/7 | C3AR1 + CD74 + IFNG + CD177 + CIITA + TAP2 | 0,778 | 0,67 | 0,886 |
| J5/7 | C3AR1 + CD74 + IFNG + CD177 + CIITA + TAP2 + CD3D | 0,769 | 0,653 | 0,885 |
| J5/7 | C3AR1 + CD74 + IFNG + CD177 + CIITA + TAP2 + CD3D + CTLA4 | 0,77 | 0,655 | 0,885 |
| J5/7 | C3AR1 + CD74 + IFNG + CD177 + CIITA + TAP2 + CTLA4 | 0,776 | 0,665 | 0,887 |
| J5/7 | C3AR1 + CD74 + IFNG + CD177 + CX3CR1 | 0,754 | 0,628 | 0,881 |
| J5/7 | C3AR1 + CD74 + IFNG + CD177 + CX3CR1 + CD3D | 0,759 | 0,63 | 0,888 |
| J5/7 | C3AR1 + CD74 + IFNG + CD177 + CX3CR1 + CD3D + CTLA4 | 0,751 | 0,616 | 0,885 |
| J5/7 | C3AR1 + CD74 + IFNG + CD177 + CX3CR1 + CIITA | 0,754 | 0,629 | 0,88 |
| J5/7 | C3AR1 + CD74 + IFNG + CD177 + CX3CR1 + CIITA + CD3D | 0,758 | 0,629 | 0,886 |
| J5/7 | C3AR1 + CD74 + IFNG + CD177 + CX3CR1 + CIITA + CD3D + CTLA4 | 0,754 | 0,621 | 0,886 |
| J5/7 | C3AR1 + CD74 + IFNG + CD177 + CX3CR1 + CIITA + CTLA4 | 0,752 | 0,621 | 0,882 |
| J5/7 | C3AR1 + CD74 + IFNG + CD177 + CX3CR1 + CIITA + TAP2 | 0,78 | 0,671 | 0,889 |
| J5/7 | C3AR1 + CD74 + IFNG + CD177 + CX3CR1 + CIITA + TAP2 + CD3D | 0,774 | 0,659 | 0,889 |
| J5/7 | C3AR1 + CD74 + IFNG + CD177 + CX3CR1 + CIITA + TAP2 + CD3D + CTLA4 | 0,772 | 0,656 | 0,888 |
| J5/7 | C3AR1 + CD74 + IFNG + CD177 + CX3CR1 + CIITA + TAP2 + CTLA4 | 0,778 | 0,665 | 0,891 |
| J5/7 | C3AR1 + CD74 + IFNG + CD177 + CX3CR1 + CTLA4 | 0,751 | 0,62 | 0,883 |
| J5/7 | C3AR1 + CD74 + IFNG + CD177 + CX3CR1 + TAP2 | 0,778 | 0,667 | 0,888 |
| J5/7 | C3AR1 + CD74 + IFNG + CD177 + CX3CR1 + TAP2 + CD3D | 0,772 | 0,656 | 0,887 |
| J5/7 | C3AR1 + CD74 + IFNG + CD177 + CX3CR1 + TAP2 + CD3D + CTLA4 | 0,77 | 0,656 | 0,885 |
| J5/7 | C3AR1 + CD74 + IFNG + CD177 + CX3CR1 + TAP2 + CTLA4 | 0,778 | 0,664 | 0,891 |
| J5/7 | C3AR1 + CD74 + IFNG + CD177 + S100A9 | 0,773 | 0,668 | 0,879 |
| J5/7 | C3AR1 + CD74 + IFNG + CD177 + S100A9 + CD3D | 0,789 | 0,683 | 0,894 |
| J5/7 | C3AR1 + CD74 + IFNG + CD177 + S100A9 + CD3D + CTLA4 | 0,791 | 0,687 | 0,896 |
| J5/7 | C3AR1 + CD74 + IFNG + CD177 + S100A9 + CIITA | 0,767 | 0,657 | 0,877 |
| J5/7 | C3AR1 + CD74 + IFNG + CD177 + S100A9 + CIITA + CD3D | 0,79 | 0,682 | 0,898 |
| J5/7 | C3AR1 + CD74 + IFNG + CD177 + S100A9 + CIITA + CD3D + CTLA4 | 0,788 | 0,68 | 0,896 |
| J5/7 | C3AR1 + CD74 + IFNG + CD177 + S100A9 + CIITA + CTLA4 | 0,763 | 0,644 | 0,882 |
| J5/7 | C3AR1 + CD74 + IFNG + CD177 + S100A9 + CIITA + TAP2 | 0,801 | 0,707 | 0,894 |
| J5/7 | C3AR1 + CD74 + IFNG + CD177 + S100A9 + CIITA + TAP2 + CD3D | 0,8 | 0,704 | 0,896 |
| J5/7 | C3AR1 + CD74 + IFNG + CD177 + S100A9 + CIITA + TAP2 + CD3D + CTLA4 | 0,806 | 0,711 | 0,901 |
| J5/7 | C3AR1 + CD74 + IFNG + CD177 + S100A9 + CIITA + TAP2 + CTLA4 | 0,797 | 0,701 | 0,892 |
| J5/7 | C3AR1 + CD74 + IFNG + CD177 + S100A9 + CTLA4 | 0,777 | 0,668 | 0,885 |
| J5/7 | C3AR1 + CD74 + IFNG + CD177 + S100A9 + CX3CR1 | 0,807 | 0,709 | 0,904 |
| J5/7 | C3AR1 + CD74 + IFNG + CD177 + S100A9 + CX3CR1 + CD3D | 0,802 | 0,695 | 0,909 |
| J5/7 | C3AR1 + CD74 + IFNG + CD177 + S100A9 + CX3CR1 + CD3D + CTLA4 | 0,802 | 0,695 | 0,909 |
| J5/7 | C3AR1 + CD74 + IFNG + CD177 + S100A9 + CX3CR1 + CIITA | 0,802 | 0,7 | 0,903 |
| J5/7 | C3AR1 + CD74 + IFNG + CD177 + S100A9 + CX3CR1 + CIITA + CD3D | 0,8 | 0,691 | 0,909 |
| J5/7 | C3AR1 + CD74 + IFNG + CD177 + S100A9 + CX3CR1 + CIITA + CD3D + CTLA4 | 0,8 | 0,691 | 0,909 |
| J5/7 | C3AR1 + CD74 + IFNG + CD177 + S100A9 + CX3CR1 + CIITA + CTLA4 | 0,795 | 0,681 | 0,908 |
| J5/7 | C3AR1 + CD74 + IFNG + CD177 + S100A9 + CX3CR1 + CIITA + TAP2 | 0,814 | 0,719 | 0,909 |
| J5/7 | C3AR1 + CD74 + IFNG + CD177 + S100A9 + CX3CR1 + CIITA + TAP2 + CD3D | 0,808 | 0,709 | 0,906 |
| J5/7 | C3AR1 + CD74 + IFNG + CD177 + S100A9 + CX3CR1 + CIITA + TAP2 + CD3D + CTLA4 | 0,81 | 0,713 | 0,907 |
| J5/7 | C3AR1 + CD74 + IFNG + CD177 + S100A9 + CX3CR1 + CIITA + TAP2 + CTLA4 | 0,813 | 0,715 | 0,912 |
| J5/7 | C3AR1 + CD74 + IFNG + CD177 + S100A9 + CX3CR1 + CTLA4 | 0,799 | 0,693 | 0,906 |
| J5/7 | C3AR1 + CD74 + IFNG + CD177 + S100A9 + CX3CR1 + TAP2 | 0,82 | 0,729 | 0,912 |
| J5/7 | C3AR1 + CD74 + IFNG + CD177 + S100A9 + CX3CR1 + TAP2 + CD3D | 0,812 | 0,715 | 0,909 |
| J5/7 | C3AR1 + CD74 + IFNG + CD177 + S100A9 + CX3CR1 + TAP2 + CD3D + CTLA4 | 0,81 | 0,713 | 0,906 |
| J5/7 | C3AR1 + CD74 + IFNG + CD177 + S100A9 + CX3CR1 + TAP2 + CTLA4 | 0,819 | 0,725 | 0,913 |
| J5/7 | C3AR1 + CD74 + IFNG + CD177 + S100A9 + TAP2 | 0,801 | 0,709 | 0,893 |
| J5/7 | C3AR1 + CD74 + IFNG + CD177 + S100A9 + TAP2 + CD3D | 0,799 | 0,703 | 0,896 |
| J5/7 | C3AR1 + CD74 + IFNG + CD177 + S100A9 + TAP2 + CD3D + CTLA4 | 0,809 | 0,715 | 0,903 |
| J5/7 | C3AR1 + CD74 + IFNG + CD177 + S100A9 + TAP2 + CTLA4 | 0,801 | 0,709 | 0,894 |
| J5/7 | C3AR1 + CD74 + IFNG + CD177 + TAP2 | 0,778 | 0,67 | 0,886 |
| J5/7 | C3AR1 + CD74 + IFNG + CD177 + TAP2 + CD3D | 0,769 | 0,654 | 0,884 |
| J5/7 | C3AR1 + CD74 + IFNG + CD177 + TAP2 + CD3D + CTLA4 | 0,769 | 0,654 | 0,884 |
| J5/7 | C3AR1 + CD74 + IFNG + CD177 + TAP2 + CTLA4 | 0,775 | 0,664 | 0,887 |
| J5/7 | C3AR1 + CD74 + IFNG + CD3D | 0,761 | 0,641 | 0,881 |
| J5/7 | C3AR1 + CD74 + IFNG + CD3D + CTLA4 | 0,758 | 0,636 | 0,881 |
| J5/7 | C3AR1 + CD74 + IFNG + CIITA | 0,766 | 0,646 | 0,885 |
| J5/7 | C3AR1 + CD74 + IFNG + CIITA + CD3D | 0,761 | 0,64 | 0,882 |
| J5/7 | C3AR1 + CD74 + IFNG + CIITA + CD3D + CTLA4 | 0,757 | 0,633 | 0,882 |
| J5/7 | C3AR1 + CD74 + IFNG + CIITA + CTLA4 | 0,757 | 0,63 | 0,884 |
| J5/7 | C3AR1 + CD74 + IFNG + CIITA + TAP2 | 0,783 | 0,677 | 0,889 |
| J5/7 | C3AR1 + CD74 + IFNG + CIITA + TAP2 + CD3D | 0,778 | 0,668 | 0,888 |
| J5/7 | C3AR1 + CD74 + IFNG + CIITA + TAP2 + CD3D + CTLA4 | 0,772 | 0,659 | 0,886 |
| J5/7 | C3AR1 + CD74 + IFNG + CIITA + TAP2 + CTLA4 | 0,772 | 0,657 | 0,887 |
| J5/7 | C3AR1 + CD74 + IFNG + CTLA4 | 0,762 | 0,641 | 0,884 |
| J5/7 | C3AR1 + CD74 + IFNG + CX3CR1 | 0,765 | 0,649 | 0,881 |
| J5/7 | C3AR1 + CD74 + IFNG + CX3CR1 + CD3D | 0,762 | 0,642 | 0,882 |
| J5/7 | C3AR1 + CD74 + IFNG + CX3CR1 + CD3D + CTLA4 | 0,761 | 0,636 | 0,885 |
| J5/7 | C3AR1 + CD74 + IFNG + CX3CR1 + CIITA | 0,764 | 0,646 | 0,883 |
| J5/7 | C3AR1 + CD74 + IFNG + CX3CR1 + CIITA + CD3D | 0,758 | 0,637 | 0,879 |
| J5/7 | C3AR1 + CD74 + IFNG + CX3CR1 + CIITA + CD3D + CTLA4 | 0,759 | 0,632 | 0,885 |
| J5/7 | C3AR1 + CD74 + IFNG + CX3CR1 + CIITA + CTLA4 | 0,76 | 0,633 | 0,887 |
| J5/7 | C3AR1 + CD74 + IFNG + CX3CR1 + CIITA + TAP2 | 0,785 | 0,679 | 0,89 |
| J5/7 | C3AR1 + CD74 + IFNG + CX3CR1 + CIITA + TAP2 + CD3D | 0,776 | 0,665 | 0,886 |
| J5/7 | C3AR1 + CD74 + IFNG + CX3CR1 + CIITA + TAP2 + CD3D + CTLA4 | 0,77 | 0,656 | 0,885 |
| J5/7 | C3AR1 + CD74 + IFNG + CX3CR1 + CIITA + TAP2 + CTLA4 | 0,774 | 0,66 | 0,887 |
| J5/7 | C3AR1 + CD74 + IFNG + CX3CR1 + CTLA4 | 0,762 | 0,638 | 0,885 |
| J5/7 | C3AR1 + CD74 + IFNG + CX3CR1 + TAP2 | 0,79 | 0,687 | 0,892 |
| J5/7 | C3AR1 + CD74 + IFNG + CX3CR1 + TAP2 + CD3D | 0,778 | 0,669 | 0,887 |
| J5/7 | C3AR1 + CD74 + IFNG + CX3CR1 + TAP2 + CD3D + CTLA4 | 0,776 | 0,665 | 0,888 |
| J5/7 | C3AR1 + CD74 + IFNG + CX3CR1 + TAP2 + CTLA4 | 0,778 | 0,667 | 0,89 |
| J5/7 | C3AR1 + CD74 + IFNG + S100A9 | 0,782 | 0,681 | 0,883 |
| J5/7 | C3AR1 + CD74 + IFNG + S100A9 + CD3D | 0,797 | 0,699 | 0,895 |
| J5/7 | C3AR1 + CD74 + IFNG + S100A9 + CD3D + CTLA4 | 0,795 | 0,695 | 0,894 |
| J5/7 | C3AR1 + CD74 + IFNG + S100A9 + CIITA | 0,78 | 0,672 | 0,888 |
| J5/7 | C3AR1 + CD74 + IFNG + S100A9 + CIITA + CD3D | 0,788 | 0,681 | 0,895 |
| J5/7 | C3AR1 + CD74 + IFNG + S100A9 + CIITA + CD3D + CTLA4 | 0,784 | 0,675 | 0,893 |
| J5/7 | C3AR1 + CD74 + IFNG + S100A9 + CIITA + CTLA4 | 0,773 | 0,655 | 0,891 |
| J5/7 | C3AR1 + CD74 + IFNG + S100A9 + CIITA + TAP2 | 0,801 | 0,706 | 0,897 |
| J5/7 | C3AR1 + CD74 + IFNG + S100A9 + CIITA + TAP2 + CD3D | 0,796 | 0,696 | 0,895 |
| J5/7 | C3AR1 + CD74 + IFNG + S100A9 + CIITA + TAP2 + CD3D + CTLA4 | 0,796 | 0,697 | 0,895 |
| J5/7 | C3AR1 + CD74 + IFNG + S100A9 + CIITA + TAP2 + CTLA4 | 0,795 | 0,695 | 0,895 |
| J5/7 | C3AR1 + CD74 + IFNG + S100A9 + CTLA4 | 0,785 | 0,68 | 0,89 |
| J5/7 | C3AR1 + CD74 + IFNG + S100A9 + CX3CR1 | 0,804 | 0,71 | 0,898 |
| J5/7 | C3AR1 + CD74 + IFNG + S100A9 + CX3CR1 + CD3D | 0,803 | 0,705 | 0,901 |
| J5/7 | C3AR1 + CD74 + IFNG + S100A9 + CX3CR1 + CD3D + CTLA4 | 0,799 | 0,696 | 0,902 |
| J5/7 | C3AR1 + CD74 + IFNG + S100A9 + CX3CR1 + CIITA | 0,796 | 0,692 | 0,9 |
| J5/7 | C3AR1 + CD74 + IFNG + S100A9 + CX3CR1 + CIITA + CD3D | 0,798 | 0,692 | 0,905 |
| J5/7 | C3AR1 + CD74 + IFNG + S100A9 + CX3CR1 + CIITA + CD3D + CTLA4 | 0,802 | 0,694 | 0,91 |
| J5/7 | C3AR1 + CD74 + IFNG + S100A9 + CX3CR1 + CIITA + CTLA4 | 0,796 | 0,685 | 0,907 |
| J5/7 | C3AR1 + CD74 + IFNG + S100A9 + CX3CR1 + CIITA + TAP2 | 0,804 | 0,708 | 0,9 |
| J5/7 | C3AR1 + CD74 + IFNG + S100A9 + CX3CR1 + CIITA + TAP2 + CD3D | 0,797 | 0,696 | 0,897 |
| J5/7 | C3AR1 + CD74 + IFNG + S100A9 + CX3CR1 + CIITA + TAP2 + CD3D + CTLA4 | 0,795 | 0,693 | 0,896 |
| J5/7 | C3AR1 + CD74 + IFNG + S100A9 + CX3CR1 + CIITA + TAP2 + CTLA4 | 0,795 | 0,692 | 0,899 |
| J5/7 | C3AR1 + CD74 + IFNG + S100A9 + CX3CR1 + CTLA4 | 0,801 | 0,699 | 0,904 |
| J5/7 | C3AR1 + CD74 + IFNG + S100A9 + CX3CR1 + TAP2 | 0,811 | 0,722 | 0,9 |
| J5/7 | C3AR1 + CD74 + IFNG + S100A9 + CX3CR1 + TAP2 + CD3D | 0,808 | 0,716 | 0,9 |
| J5/7 | C3AR1 + CD74 + IFNG + S100A9 + CX3CR1 + TAP2 + CD3D + CTLA4 | 0,809 | 0,717 | 0,901 |
| J5/7 | C3AR1 + CD74 + IFNG + S100A9 + CX3CR1 + TAP2 + CTLA4 | 0,807 | 0,713 | 0,901 |
| J5/7 | C3AR1 + CD74 + IFNG + S100A9 + TAP2 | 0,808 | 0,719 | 0,898 |
| J5/7 | C3AR1 + CD74 + IFNG + S100A9 + TAP2 + CD3D | 0,805 | 0,713 | 0,897 |
| J5/7 | C3AR1 + CD74 + IFNG + S100A9 + TAP2 + CD3D + CTLA4 | 0,806 | 0,714 | 0,897 |
| J5/7 | C3AR1 + CD74 + IFNG + S100A9 + TAP2 + CTLA4 | 0,809 | 0,717 | 0,901 |
| J5/7 | C3AR1 + CD74 + IFNG + TAP2 | 0,789 | 0,685 | 0,892 |
| J5/7 | C3AR1 + CD74 + IFNG + TAP2 + CD3D | 0,778 | 0,669 | 0,888 |
| J5/7 | C3AR1 + CD74 + IFNG + TAP2 + CD3D + CTLA4 | 0,777 | 0,666 | 0,888 |
| J5/7 | C3AR1 + CD74 + IFNG + TAP2 + CTLA4 | 0,777 | 0,665 | 0,889 |
| J5/7 | C3AR1 + CD74 + S100A9 | 0,783 | 0,686 | 0,88 |
| J5/7 | C3AR1 + CD74 + S100A9 + CD3D | 0,791 | 0,703 | 0,878 |
| J5/7 | C3AR1 + CD74 + S100A9 + CD3D + CTLA4 | 0,783 | 0,69 | 0,876 |
| J5/7 | C3AR1 + CD74 + S100A9 + CIITA | 0,772 | 0,662 | 0,882 |
| J5/7 | C3AR1 + CD74 + S100A9 + CIITA + CD3D | 0,771 | 0,666 | 0,877 |
| J5/7 | C3AR1 + CD74 + S100A9 + CIITA + CD3D + CTLA4 | 0,767 | 0,655 | 0,879 |
| J5/7 | C3AR1 + CD74 + S100A9 + CIITA + CTLA4 | 0,758 | 0,637 | 0,879 |
| J5/7 | C3AR1 + CD74 + S100A9 + CIITA + TAP2 | 0,786 | 0,686 | 0,885 |
| J5/7 | C3AR1 + CD74 + S100A9 + CIITA + TAP2 + CD3D | 0,784 | 0,686 | 0,882 |
| J5/7 | C3AR1 + CD74 + S100A9 + CIITA + TAP2 + CD3D + CTLA4 | 0,783 | 0,678 | 0,887 |
| J5/7 | C3AR1 + CD74 + S100A9 + CIITA + TAP2 + CTLA4 | 0,782 | 0,677 | 0,887 |
| J5/7 | C3AR1 + CD74 + S100A9 + CTLA4 | 0,778 | 0,677 | 0,879 |
| J5/7 | C3AR1 + CD74 + S100A9 + CX3CR1 | 0,788 | 0,696 | 0,88 |
| J5/7 | C3AR1 + CD74 + S100A9 + CX3CR1 + CD3D | 0,79 | 0,701 | 0,878 |
| J5/7 | C3AR1 + CD74 + S100A9 + CX3CR1 + CD3D + CTLA4 | 0,786 | 0,692 | 0,88 |
| J5/7 | C3AR1 + CD74 + S100A9 + CX3CR1 + CIITA | 0,78 | 0,671 | 0,888 |
| J5/7 | C3AR1 + CD74 + S100A9 + CX3CR1 + CIITA + CD3D | 0,772 | 0,667 | 0,878 |
| J5/7 | C3AR1 + CD74 + S100A9 + CX3CR1 + CIITA + CD3D + CTLA4 | 0,767 | 0,655 | 0,88 |
| J5/7 | C3AR1 + CD74 + S100A9 + CX3CR1 + CIITA + CTLA4 | 0,766 | 0,649 | 0,883 |
| J5/7 | C3AR1 + CD74 + S100A9 + CX3CR1 + CIITA + TAP2 | 0,785 | 0,685 | 0,884 |
| J5/7 | C3AR1 + CD74 + S100A9 + CX3CR1 + CIITA + TAP2 + CD3D | 0,785 | 0,688 | 0,881 |
| J5/7 | C3AR1 + CD74 + S100A9 + CX3CR1 + CIITA + TAP2 + CD3D + CTLA4 | 0,781 | 0,676 | 0,886 |
| J5/7 | C3AR1 + CD74 + S100A9 + CX3CR1 + CIITA + TAP2 + CTLA4 | 0,782 | 0,677 | 0,887 |
| J5/7 | C3AR1 + CD74 + S100A9 + CX3CR1 + CTLA4 | 0,786 | 0,687 | 0,884 |
| J5/7 | C3AR1 + CD74 + S100A9 + CX3CR1 + TAP2 | 0,79 | 0,704 | 0,875 |
| J5/7 | C3AR1 + CD74 + S100A9 + CX3CR1 + TAP2 + CD3D | 0,788 | 0,703 | 0,873 |
| J5/7 | C3AR1 + CD74 + S100A9 + CX3CR1 + TAP2 + CD3D + CTLA4 | 0,785 | 0,697 | 0,872 |
| J5/7 | C3AR1 + CD74 + S100A9 + CX3CR1 + TAP2 + CTLA4 | 0,785 | 0,696 | 0,875 |
| J5/7 | C3AR1 + CD74 + S100A9 + TAP2 | 0,792 | 0,706 | 0,878 |
| J5/7 | C3AR1 + CD74 + S100A9 + TAP2 + CD3D | 0,788 | 0,702 | 0,874 |
| J5/7 | C3AR1 + CD74 + S100A9 + TAP2 + CD3D + CTLA4 | 0,787 | 0,698 | 0,875 |
| J5/7 | C3AR1 + CD74 + S100A9 + TAP2 + CTLA4 | 0,786 | 0,696 | 0,875 |
| J5/7 | C3AR1 + CD74 + TAP2 | 0,779 | 0,677 | 0,881 |
| J5/7 | C3AR1 + CD74 + TAP2 + CD3D | 0,78 | 0,683 | 0,878 |
| J5/7 | C3AR1 + CD74 + TAP2 + CD3D + CTLA4 | 0,778 | 0,673 | 0,882 |
| J5/7 | C3AR1 + CD74 + TAP2 + CTLA4 | 0,778 | 0,676 | 0,881 |
| J5/7 | C3AR1 + CIITA | 0,778 | 0,686 | 0,87 |
| J5/7 | C3AR1 + CIITA + CD3D | 0,77 | 0,682 | 0,859 |
| J5/7 | C3AR1 + CIITA + CD3D + CTLA4 | 0,76 | 0,659 | 0,86 |
| J5/7 | C3AR1 + CIITA + CTLA4 | 0,756 | 0,653 | 0,859 |
| J5/7 | C3AR1 + CIITA + TAP2 | 0,786 | 0,697 | 0,875 |
| J5/7 | C3AR1 + CIITA + TAP2 + CD3D | 0,789 | 0,701 | 0,876 |
| J5/7 | C3AR1 + CIITA + TAP2 + CD3D + CTLA4 | 0,784 | 0,689 | 0,878 |
| J5/7 | C3AR1 + CIITA + TAP2 + CTLA4 | 0,784 | 0,693 | 0,876 |
| J5/7 | C3AR1 + CX3CR1 | 0,774 | 0,673 | 0,874 |
| J5/7 | C3AR1 + CX3CR1 + CD3D | 0,76 | 0,652 | 0,867 |
| J5/7 | C3AR1 + CX3CR1 + CD3D + CTLA4 | 0,755 | 0,638 | 0,871 |
| J5/7 | C3AR1 + CX3CR1 + CIITA | 0,794 | 0,709 | 0,878 |
| J5/7 | C3AR1 + CX3CR1 + CIITA + CD3D | 0,778 | 0,689 | 0,867 |
| J5/7 | C3AR1 + CX3CR1 + CIITA + CD3D + CTLA4 | 0,77 | 0,665 | 0,874 |
| J5/7 | C3AR1 + CX3CR1 + CIITA + CTLA4 | 0,768 | 0,665 | 0,872 |
| J5/7 | C3AR1 + CX3CR1 + CIITA + TAP2 | 0,783 | 0,692 | 0,874 |
| J5/7 | C3AR1 + CX3CR1 + CIITA + TAP2 + CD3D | 0,786 | 0,698 | 0,873 |
| J5/7 | C3AR1 + CX3CR1 + CIITA + TAP2 + CD3D + CTLA4 | 0,782 | 0,687 | 0,877 |
| J5/7 | C3AR1 + CX3CR1 + CIITA + TAP2 + CTLA4 | 0,783 | 0,691 | 0,874 |
| J5/7 | C3AR1 + CX3CR1 + CTLA4 | 0,753 | 0,637 | 0,869 |
| J5/7 | C3AR1 + CX3CR1 + TAP2 | 0,783 | 0,69 | 0,876 |
| J5/7 | C3AR1 + CX3CR1 + TAP2 + CD3D | 0,784 | 0,694 | 0,874 |
| J5/7 | C3AR1 + CX3CR1 + TAP2 + CD3D + CTLA4 | 0,775 | 0,678 | 0,873 |
| J5/7 | C3AR1 + CX3CR1 + TAP2 + CTLA4 | 0,776 | 0,681 | 0,871 |
| J5/7 | C3AR1 + IFNG + CD177 + CIITA + CD3D | 0,751 | 0,637 | 0,865 |
| J5/7 | C3AR1 + IFNG + CD177 + CIITA + TAP2 | 0,773 | 0,672 | 0,874 |
| J5/7 | C3AR1 + IFNG + CD177 + CIITA + TAP2 + CD3D | 0,769 | 0,667 | 0,872 |
| J5/7 | C3AR1 + IFNG + CD177 + CIITA + TAP2 + CD3D + CTLA4 | 0,77 | 0,668 | 0,872 |
| J5/7 | C3AR1 + IFNG + CD177 + CIITA + TAP2 + CTLA4 | 0,775 | 0,675 | 0,875 |
| J5/7 | C3AR1 + IFNG + CD177 + CX3CR1 + CIITA | 0,766 | 0,664 | 0,867 |
| J5/7 | C3AR1 + IFNG + CD177 + CX3CR1 + CIITA + CD3D | 0,759 | 0,644 | 0,874 |
| J5/7 | C3AR1 + IFNG + CD177 + CX3CR1 + CIITA + CD3D + CTLA4 | 0,758 | 0,642 | 0,875 |
| J5/7 | C3AR1 + IFNG + CD177 + CX3CR1 + CIITA + CTLA4 | 0,755 | 0,641 | 0,868 |
| J5/7 | C3AR1 + IFNG + CD177 + CX3CR1 + CIITA + TAP2 | 0,784 | 0,686 | 0,881 |
| J5/7 | C3AR1 + IFNG + CD177 + CX3CR1 + CIITA + TAP2 + CD3D | 0,776 | 0,674 | 0,878 |
| J5/7 | C3AR1 + IFNG + CD177 + CX3CR1 + CIITA + TAP2 + CD3D + CTLA4 | 0,776 | 0,674 | 0,878 |
| J5/7 | C3AR1 + IFNG + CD177 + CX3CR1 + CIITA + TAP2 + CTLA4 | 0,78 | 0,678 | 0,881 |
| J5/7 | C3AR1 + IFNG + CD177 + CX3CR1 + TAP2 | 0,76 | 0,653 | 0,866 |
| J5/7 | C3AR1 + IFNG + CD177 + CX3CR1 + TAP2 + CD3D | 0,757 | 0,649 | 0,864 |
| J5/7 | C3AR1 + IFNG + CD177 + CX3CR1 + TAP2 + CD3D + CTLA4 | 0,756 | 0,646 | 0,866 |
| J5/7 | C3AR1 + IFNG + CD177 + CX3CR1 + TAP2 + CTLA4 | 0,757 | 0,647 | 0,868 |
| J5/7 | C3AR1 + IFNG + CD177 + S100A9 + CD3D | 0,763 | 0,645 | 0,881 |
| J5/7 | C3AR1 + IFNG + CD177 + S100A9 + CD3D + CTLA4 | 0,762 | 0,644 | 0,88 |
| J5/7 | C3AR1 + IFNG + CD177 + S100A9 + CIITA | 0,758 | 0,663 | 0,853 |
| J5/7 | C3AR1 + IFNG + CD177 + S100A9 + CIITA + CD3D | 0,77 | 0,658 | 0,882 |
| J5/7 | C3AR1 + IFNG + CD177 + S100A9 + CIITA + CD3D + CTLA4 | 0,767 | 0,654 | 0,88 |
| J5/7 | C3AR1 + IFNG + CD177 + S100A9 + CIITA + TAP2 | 0,796 | 0,705 | 0,886 |
| J5/7 | C3AR1 + IFNG + CD177 + S100A9 + CIITA + TAP2 + CD3D | 0,795 | 0,696 | 0,893 |
| J5/7 | C3AR1 + IFNG + CD177 + S100A9 + CIITA + TAP2 + CD3D + CTLA4 | 0,795 | 0,698 | 0,891 |
| J5/7 | C3AR1 + IFNG + CD177 + S100A9 + CIITA + TAP2 + CTLA4 | 0,795 | 0,702 | 0,887 |
| J5/7 | C3AR1 + IFNG + CD177 + S100A9 + CX3CR1 | 0,791 | 0,683 | 0,899 |
| J5/7 | C3AR1 + IFNG + CD177 + S100A9 + CX3CR1 + CD3D | 0,781 | 0,658 | 0,904 |
| J5/7 | C3AR1 + IFNG + CD177 + S100A9 + CX3CR1 + CD3D + CTLA4 | 0,783 | 0,659 | 0,907 |
| J5/7 | C3AR1 + IFNG + CD177 + S100A9 + CX3CR1 + CIITA | 0,803 | 0,705 | 0,9 |
| J5/7 | C3AR1 + IFNG + CD177 + S100A9 + CX3CR1 + CIITA + CD3D | 0,791 | 0,678 | 0,903 |
| J5/7 | C3AR1 + IFNG + CD177 + S100A9 + CX3CR1 + CIITA + CD3D + CTLA4 | 0,789 | 0,676 | 0,902 |
| J5/7 | C3AR1 + IFNG + CD177 + S100A9 + CX3CR1 + CIITA + CTLA4 | 0,792 | 0,681 | 0,903 |
| J5/7 | C3AR1 + IFNG + CD177 + S100A9 + CX3CR1 + CIITA + TAP2 | 0,808 | 0,714 | 0,902 |
| J5/7 | C3AR1 + IFNG + CD177 + S100A9 + CX3CR1 + CIITA + TAP2 + CD3D | 0,808 | 0,708 | 0,907 |
| J5/7 | C3AR1 + IFNG + CD177 + S100A9 + CX3CR1 + CIITA + TAP2 + CD3D + CTLA4 | 0,806 | 0,709 | 0,903 |
| J5/7 | C3AR1 + IFNG + CD177 + S100A9 + CX3CR1 + CIITA + TAP2 + CTLA4 | 0,809 | 0,716 | 0,903 |
| J5/7 | C3AR1 + IFNG + CD177 + S100A9 + CX3CR1 + CTLA4 | 0,783 | 0,663 | 0,902 |
| J5/7 | C3AR1 + IFNG + CD177 + S100A9 + CX3CR1 + TAP2 | 0,808 | 0,713 | 0,902 |
| J5/7 | C3AR1 + IFNG + CD177 + S100A9 + CX3CR1 + TAP2 + CD3D | 0,804 | 0,701 | 0,908 |
| J5/7 | C3AR1 + IFNG + CD177 + S100A9 + CX3CR1 + TAP2 + CD3D + CTLA4 | 0,802 | 0,703 | 0,902 |
| J5/7 | C3AR1 + IFNG + CD177 + S100A9 + CX3CR1 + TAP2 + CTLA4 | 0,805 | 0,71 | 0,901 |
| J5/7 | C3AR1 + IFNG + CD177 + S100A9 + TAP2 | 0,795 | 0,704 | 0,886 |
| J5/7 | C3AR1 + IFNG + CD177 + S100A9 + TAP2 + CD3D | 0,798 | 0,7 | 0,896 |
| J5/7 | C3AR1 + IFNG + CD177 + S100A9 + TAP2 + CD3D + CTLA4 | 0,798 | 0,702 | 0,894 |
| J5/7 | C3AR1 + IFNG + CD177 + S100A9 + TAP2 + CTLA4 | 0,794 | 0,702 | 0,886 |
| J5/7 | C3AR1 + IFNG + CD177 + TAP2 | 0,758 | 0,653 | 0,863 |
| J5/7 | C3AR1 + IFNG + CD177 + TAP2 + CD3D | 0,753 | 0,646 | 0,86 |
| J5/7 | C3AR1 + IFNG + CD177 + TAP2 + CD3D + CTLA4 | 0,755 | 0,649 | 0,862 |
| J5/7 | C3AR1 + IFNG + CD177 + TAP2 + CTLA4 | 0,76 | 0,653 | 0,868 |
| J5/7 | C3AR1 + IFNG + CIITA | 0,759 | 0,66 | 0,857 |
| J5/7 | C3AR1 + IFNG + CIITA + CD3D | 0,762 | 0,656 | 0,869 |
| J5/7 | C3AR1 + IFNG + CIITA + CD3D + CTLA4 | 0,755 | 0,644 | 0,866 |
| J5/7 | C3AR1 + IFNG + CIITA + CTLA4 | 0,752 | 0,641 | 0,862 |
| J5/7 | C3AR1 + IFNG + CIITA + TAP2 | 0,788 | 0,694 | 0,881 |
| J5/7 | C3AR1 + IFNG + CIITA + TAP2 + CD3D | 0,785 | 0,69 | 0,88 |
| J5/7 | C3AR1 + IFNG + CIITA + TAP2 + CD3D + CTLA4 | 0,777 | 0,678 | 0,877 |
| J5/7 | C3AR1 + IFNG + CIITA + TAP2 + CTLA4 | 0,784 | 0,687 | 0,881 |
| J5/7 | C3AR1 + IFNG + CX3CR1 + CIITA | 0,776 | 0,682 | 0,87 |
| J5/7 | C3AR1 + IFNG + CX3CR1 + CIITA + CD3D | 0,765 | 0,659 | 0,87 |
| J5/7 | C3AR1 + IFNG + CX3CR1 + CIITA + CD3D + CTLA4 | 0,762 | 0,652 | 0,872 |
| J5/7 | C3AR1 + IFNG + CX3CR1 + CIITA + CTLA4 | 0,762 | 0,653 | 0,871 |
| J5/7 | C3AR1 + IFNG + CX3CR1 + CIITA + TAP2 | 0,792 | 0,699 | 0,885 |
| J5/7 | C3AR1 + IFNG + CX3CR1 + CIITA + TAP2 + CD3D | 0,785 | 0,69 | 0,88 |
| J5/7 | C3AR1 + IFNG + CX3CR1 + CIITA + TAP2 + CD3D + CTLA4 | 0,779 | 0,681 | 0,878 |
| J5/7 | C3AR1 + IFNG + CX3CR1 + CIITA + TAP2 + CTLA4 | 0,784 | 0,686 | 0,882 |
| J5/7 | C3AR1 + IFNG + CX3CR1 + TAP2 | 0,77 | 0,671 | 0,868 |
| J5/7 | C3AR1 + IFNG + CX3CR1 + TAP2 + CD3D | 0,765 | 0,664 | 0,866 |
| J5/7 | C3AR1 + IFNG + CX3CR1 + TAP2 + CD3D + CTLA4 | 0,769 | 0,666 | 0,872 |
| J5/7 | C3AR1 + IFNG + CX3CR1 + TAP2 + CTLA4 | 0,769 | 0,665 | 0,872 |
| J5/7 | C3AR1 + IFNG + S100A9 | 0,757 | 0,656 | 0,859 |
| J5/7 | C3AR1 + IFNG + S100A9 + CD3D | 0,774 | 0,665 | 0,883 |
| J5/7 | C3AR1 + IFNG + S100A9 + CD3D + CTLA4 | 0,775 | 0,666 | 0,885 |
| J5/7 | C3AR1 + IFNG + S100A9 + CIITA | 0,769 | 0,675 | 0,864 |
| J5/7 | C3AR1 + IFNG + S100A9 + CIITA + CD3D | 0,778 | 0,674 | 0,882 |
| J5/7 | C3AR1 + IFNG + S100A9 + CIITA + CD3D + CTLA4 | 0,78 | 0,674 | 0,886 |
| J5/7 | C3AR1 + IFNG + S100A9 + CIITA + CTLA4 | 0,762 | 0,652 | 0,872 |
| J5/7 | C3AR1 + IFNG + S100A9 + CIITA + TAP2 | 0,802 | 0,713 | 0,891 |
| J5/7 | C3AR1 + IFNG + S100A9 + CIITA + TAP2 + CD3D | 0,801 | 0,705 | 0,897 |
| J5/7 | C3AR1 + IFNG + S100A9 + CIITA + TAP2 + CD3D + CTLA4 | 0,801 | 0,706 | 0,896 |
| J5/7 | C3AR1 + IFNG + S100A9 + CIITA + TAP2 + CTLA4 | 0,802 | 0,71 | 0,893 |
| J5/7 | C3AR1 + IFNG + S100A9 + CTLA4 | 0,757 | 0,643 | 0,871 |
| J5/7 | C3AR1 + IFNG + S100A9 + CX3CR1 | 0,8 | 0,702 | 0,899 |
| J5/7 | C3AR1 + IFNG + S100A9 + CX3CR1 + CD3D | 0,79 | 0,681 | 0,899 |
| J5/7 | C3AR1 + IFNG + S100A9 + CX3CR1 + CD3D + CTLA4 | 0,792 | 0,682 | 0,902 |
| J5/7 | C3AR1 + IFNG + S100A9 + CX3CR1 + CIITA | 0,805 | 0,71 | 0,901 |
| J5/7 | C3AR1 + IFNG + S100A9 + CX3CR1 + CIITA + CD3D | 0,797 | 0,694 | 0,899 |
| J5/7 | C3AR1 + IFNG + S100A9 + CX3CR1 + CIITA + CD3D + CTLA4 | 0,795 | 0,686 | 0,903 |
| J5/7 | C3AR1 + IFNG + S100A9 + CX3CR1 + CIITA + CTLA4 | 0,792 | 0,684 | 0,899 |
| J5/7 | C3AR1 + IFNG + S100A9 + CX3CR1 + CIITA + TAP2 | 0,807 | 0,716 | 0,898 |
| J5/7 | C3AR1 + IFNG + S100A9 + CX3CR1 + CIITA + TAP2 + CD3D | 0,805 | 0,709 | 0,902 |
| J5/7 | C3AR1 + IFNG + S100A9 + CX3CR1 + CIITA + TAP2 + CD3D + CTLA4 | 0,803 | 0,705 | 0,901 |
| J5/7 | C3AR1 + IFNG + S100A9 + CX3CR1 + CIITA + TAP2 + CTLA4 | 0,804 | 0,71 | 0,899 |
| J5/7 | C3AR1 + IFNG + S100A9 + CX3CR1 + CTLA4 | 0,788 | 0,676 | 0,899 |
| J5/7 | C3AR1 + IFNG + S100A9 + CX3CR1 + TAP2 | 0,812 | 0,723 | 0,902 |
| J5/7 | C3AR1 + IFNG + S100A9 + CX3CR1 + TAP2 + CD3D | 0,807 | 0,714 | 0,901 |
| J5/7 | C3AR1 + IFNG + S100A9 + CX3CR1 + TAP2 + CD3D + CTLA4 | 0,807 | 0,714 | 0,901 |
| J5/7 | C3AR1 + IFNG + S100A9 + CX3CR1 + TAP2 + CTLA4 | 0,804 | 0,711 | 0,898 |
| J5/7 | C3AR1 + IFNG + S100A9 + TAP2 | 0,806 | 0,719 | 0,893 |
| J5/7 | C3AR1 + IFNG + S100A9 + TAP2 + CD3D | 0,803 | 0,709 | 0,897 |
| J5/7 | C3AR1 + IFNG + S100A9 + TAP2 + CD3D + CTLA4 | 0,804 | 0,71 | 0,898 |
| J5/7 | C3AR1 + IFNG + S100A9 + TAP2 + CTLA4 | 0,805 | 0,715 | 0,895 |
| J5/7 | C3AR1 + IFNG + TAP2 | 0,769 | 0,669 | 0,869 |
| J5/7 | C3AR1 + IFNG + TAP2 + CD3D | 0,765 | 0,664 | 0,866 |
| J5/7 | C3AR1 + IFNG + TAP2 + CD3D + CTLA4 | 0,768 | 0,666 | 0,871 |
| J5/7 | C3AR1 + IFNG + TAP2 + CTLA4 | 0,768 | 0,666 | 0,871 |
| J5/7 | C3AR1 + IL1R2 + CD177 | 0,773 | 0,673 | 0,872 |
| J5/7 | C3AR1 + IL1R2 + CD177 + CD3D | 0,755 | 0,637 | 0,873 |
| J5/7 | C3AR1 + IL1R2 + CD177 + CD3D + CTLA4 | 0,75 | 0,63 | 0,87 |
| J5/7 | C3AR1 + IL1R2 + CD177 + CIITA | 0,769 | 0,673 | 0,866 |
| J5/7 | C3AR1 + IL1R2 + CD177 + CIITA + CD3D | 0,76 | 0,652 | 0,867 |
| J5/7 | C3AR1 + IL1R2 + CD177 + CIITA + CD3D + CTLA4 | 0,752 | 0,641 | 0,862 |
| J5/7 | C3AR1 + IL1R2 + CD177 + CIITA + CTLA4 | 0,752 | 0,643 | 0,861 |
| J5/7 | C3AR1 + IL1R2 + CD177 + CIITA + TAP2 | 0,788 | 0,694 | 0,882 |
| J5/7 | C3AR1 + IL1R2 + CD177 + CIITA + TAP2 + CD3D | 0,784 | 0,69 | 0,878 |
| J5/7 | C3AR1 + IL1R2 + CD177 + CIITA + TAP2 + CD3D + CTLA4 | 0,784 | 0,69 | 0,878 |
| J5/7 | C3AR1 + IL1R2 + CD177 + CIITA + TAP2 + CTLA4 | 0,787 | 0,691 | 0,883 |
| J5/7 | C3AR1 + IL1R2 + CD177 + CX3CR1 | 0,775 | 0,67 | 0,879 |
| J5/7 | C3AR1 + IL1R2 + CD177 + CX3CR1 + CD3D | 0,766 | 0,65 | 0,882 |
| J5/7 | C3AR1 + IL1R2 + CD177 + CX3CR1 + CD3D + CTLA4 | 0,754 | 0,634 | 0,874 |
| J5/7 | C3AR1 + IL1R2 + CD177 + CX3CR1 + CIITA | 0,776 | 0,68 | 0,872 |
| J5/7 | C3AR1 + IL1R2 + CD177 + CX3CR1 + CIITA + CD3D | 0,769 | 0,666 | 0,871 |
| J5/7 | C3AR1 + IL1R2 + CD177 + CX3CR1 + CIITA + CD3D + CTLA4 | 0,76 | 0,649 | 0,87 |
| J5/7 | C3AR1 + IL1R2 + CD177 + CX3CR1 + CIITA + CTLA4 | 0,761 | 0,651 | 0,872 |
| J5/7 | C3AR1 + IL1R2 + CD177 + CX3CR1 + CIITA + TAP2 | 0,788 | 0,693 | 0,883 |
| J5/7 | C3AR1 + IL1R2 + CD177 + CX3CR1 + CIITA + TAP2 + CD3D | 0,786 | 0,691 | 0,88 |
| J5/7 | C3AR1 + IL1R2 + CD177 + CX3CR1 + CIITA + TAP2 + CD3D + CTLA4 | 0,785 | 0,691 | 0,88 |
| J5/7 | C3AR1 + IL1R2 + CD177 + CX3CR1 + CIITA + TAP2 + CTLA4 | 0,786 | 0,69 | 0,882 |
| J5/7 | C3AR1 + IL1R2 + CD177 + CX3CR1 + CTLA4 | 0,758 | 0,638 | 0,879 |
| J5/7 | C3AR1 + IL1R2 + CD177 + CX3CR1 + TAP2 | 0,786 | 0,691 | 0,882 |
| J5/7 | C3AR1 + IL1R2 + CD177 + CX3CR1 + TAP2 + CD3D | 0,783 | 0,689 | 0,877 |
| J5/7 | C3AR1 + IL1R2 + CD177 + CX3CR1 + TAP2 + CD3D + CTLA4 | 0,783 | 0,689 | 0,878 |
| J5/7 | C3AR1 + IL1R2 + CD177 + CX3CR1 + TAP2 + CTLA4 | 0,787 | 0,691 | 0,883 |
| J5/7 | C3AR1 + IL1R2 + CD177 + S100A9 | 0,785 | 0,694 | 0,876 |
| J5/7 | C3AR1 + IL1R2 + CD177 + S100A9 + CD3D | 0,768 | 0,657 | 0,88 |
| J5/7 | C3AR1 + IL1R2 + CD177 + S100A9 + CD3D + CTLA4 | 0,764 | 0,649 | 0,88 |
| J5/7 | C3AR1 + IL1R2 + CD177 + S100A9 + CIITA | 0,785 | 0,695 | 0,875 |
| J5/7 | C3AR1 + IL1R2 + CD177 + S100A9 + CIITA + CD3D | 0,772 | 0,665 | 0,879 |
| J5/7 | C3AR1 + IL1R2 + CD177 + S100A9 + CIITA + CD3D + CTLA4 | 0,765 | 0,654 | 0,876 |
| J5/7 | C3AR1 + IL1R2 + CD177 + S100A9 + CIITA + CTLA4 | 0,763 | 0,65 | 0,876 |
| J5/7 | C3AR1 + IL1R2 + CD177 + S100A9 + CIITA + TAP2 | 0,799 | 0,714 | 0,885 |
| J5/7 | C3AR1 + IL1R2 + CD177 + S100A9 + CIITA + TAP2 + CD3D | 0,789 | 0,699 | 0,878 |
| J5/7 | C3AR1 + IL1R2 + CD177 + S100A9 + CIITA + TAP2 + CD3D + CTLA4 | 0,79 | 0,701 | 0,878 |
| J5/7 | C3AR1 + IL1R2 + CD177 + S100A9 + CIITA + TAP2 + CTLA4 | 0,799 | 0,712 | 0,886 |
| J5/7 | C3AR1 + IL1R2 + CD177 + S100A9 + CTLA4 | 0,763 | 0,65 | 0,876 |
| J5/7 | C3AR1 + IL1R2 + CD177 + S100A9 + CX3CR1 | 0,793 | 0,695 | 0,891 |
| J5/7 | C3AR1 + IL1R2 + CD177 + S100A9 + CX3CR1 + CD3D | 0,781 | 0,673 | 0,89 |
| J5/7 | C3AR1 + IL1R2 + CD177 + S100A9 + CX3CR1 + CD3D + CTLA4 | 0,775 | 0,662 | 0,888 |
| J5/7 | C3AR1 + IL1R2 + CD177 + S100A9 + CX3CR1 + CIITA | 0,792 | 0,697 | 0,887 |
| J5/7 | C3AR1 + IL1R2 + CD177 + S100A9 + CX3CR1 + CIITA + CD3D | 0,781 | 0,675 | 0,886 |
| J5/7 | C3AR1 + IL1R2 + CD177 + S100A9 + CX3CR1 + CIITA + CD3D + CTLA4 | 0,778 | 0,667 | 0,889 |
| J5/7 | C3AR1 + IL1R2 + CD177 + S100A9 + CX3CR1 + CIITA + CTLA4 | 0,782 | 0,669 | 0,895 |
| J5/7 | C3AR1 + IL1R2 + CD177 + S100A9 + CX3CR1 + CIITA + TAP2 | 0,803 | 0,718 | 0,889 |
| J5/7 | C3AR1 + IL1R2 + CD177 + S100A9 + CX3CR1 + CIITA + TAP2 + CD3D | 0,787 | 0,697 | 0,878 |
| J5/7 | C3AR1 + IL1R2 + CD177 + S100A9 + CX3CR1 + CIITA + TAP2 + CD3D + CTLA4 | 0,79 | 0,701 | 0,878 |
| J5/7 | C3AR1 + IL1R2 + CD177 + S100A9 + CX3CR1 + CIITA + TAP2 + CTLA4 | 0,797 | 0,709 | 0,885 |
| J5/7 | C3AR1 + IL1R2 + CD177 + S100A9 + CX3CR1 + CTLA4 | 0,781 | 0,666 | 0,896 |
| J5/7 | C3AR1 + IL1R2 + CD177 + S100A9 + CX3CR1 + TAP2 | 0,798 | 0,713 | 0,883 |
| J5/7 | C3AR1 + IL1R2 + CD177 + S100A9 + CX3CR1 + TAP2 + CD3D | 0,787 | 0,699 | 0,876 |
| J5/7 | C3AR1 + IL1R2 + CD177 + S100A9 + CX3CR1 + TAP2 + CD3D + CTLA4 | 0,79 | 0,703 | 0,876 |
| J5/7 | C3AR1 + IL1R2 + CD177 + S100A9 + CX3CR1 + TAP2 + CTLA4 | 0,798 | 0,711 | 0,884 |
| J5/7 | C3AR1 + IL1R2 + CD177 + S100A9 + TAP2 | 0,793 | 0,707 | 0,879 |
| J5/7 | C3AR1 + IL1R2 + CD177 + S100A9 + TAP2 + CD3D | 0,785 | 0,696 | 0,873 |
| J5/7 | C3AR1 + IL1R2 + CD177 + S100A9 + TAP2 + CD3D + CTLA4 | 0,791 | 0,704 | 0,877 |
| J5/7 | C3AR1 + IL1R2 + CD177 + S100A9 + TAP2 + CTLA4 | 0,793 | 0,708 | 0,879 |
| J5/7 | C3AR1 + IL1R2 + CD177 + TAP2 | 0,786 | 0,691 | 0,882 |
| J5/7 | C3AR1 + IL1R2 + CD177 + TAP2 + CD3D | 0,785 | 0,69 | 0,879 |
| J5/7 | C3AR1 + IL1R2 + CD177 + TAP2 + CD3D + CTLA4 | 0,785 | 0,69 | 0,88 |
| J5/7 | C3AR1 + IL1R2 + CD177 + TAP2 + CTLA4 | 0,786 | 0,689 | 0,882 |
| J5/7 | C3AR1 + IL1R2 + CD3D | 0,783 | 0,677 | 0,889 |
| J5/7 | C3AR1 + IL1R2 + CD3D + CTLA4 | 0,775 | 0,663 | 0,887 |
| J5/7 | C3AR1 + IL1R2 + CD74 | 0,805 | 0,728 | 0,881 |
| J5/7 | C3AR1 + IL1R2 + CD74 + CD177 | 0,766 | 0,655 | 0,876 |
| J5/7 | C3AR1 + IL1R2 + CD74 + CD177 + CD3D | 0,765 | 0,655 | 0,875 |
| J5/7 | C3AR1 + IL1R2 + CD74 + CD177 + CD3D + CTLA4 | 0,764 | 0,652 | 0,876 |
| J5/7 | C3AR1 + IL1R2 + CD74 + CD177 + CIITA | 0,758 | 0,636 | 0,881 |
| J5/7 | C3AR1 + IL1R2 + CD74 + CD177 + CIITA + CD3D | 0,758 | 0,639 | 0,877 |
| J5/7 | C3AR1 + IL1R2 + CD74 + CD177 + CIITA + CD3D + CTLA4 | 0,758 | 0,638 | 0,879 |
| J5/7 | C3AR1 + IL1R2 + CD74 + CD177 + CIITA + TAP2 | 0,783 | 0,674 | 0,891 |
| J5/7 | C3AR1 + IL1R2 + CD74 + CD177 + CIITA + TAP2 + CD3D | 0,773 | 0,663 | 0,883 |
| J5/7 | C3AR1 + IL1R2 + CD74 + CD177 + CIITA + TAP2 + CD3D + CTLA4 | 0,775 | 0,665 | 0,885 |
| J5/7 | C3AR1 + IL1R2 + CD74 + CD177 + CIITA + TAP2 + CTLA4 | 0,778 | 0,667 | 0,889 |
| J5/7 | C3AR1 + IL1R2 + CD74 + CD177 + CTLA4 | 0,766 | 0,652 | 0,88 |
| J5/7 | C3AR1 + IL1R2 + CD74 + CD177 + CX3CR1 | 0,769 | 0,66 | 0,879 |
| J5/7 | C3AR1 + IL1R2 + CD74 + CD177 + CX3CR1 + CD3D | 0,767 | 0,655 | 0,88 |
| J5/7 | C3AR1 + IL1R2 + CD74 + CD177 + CX3CR1 + CD3D + CTLA4 | 0,762 | 0,648 | 0,876 |
| J5/7 | C3AR1 + IL1R2 + CD74 + CD177 + CX3CR1 + CIITA | 0,763 | 0,643 | 0,883 |
| J5/7 | C3AR1 + IL1R2 + CD74 + CD177 + CX3CR1 + CIITA + CD3D | 0,758 | 0,639 | 0,877 |
| J5/7 | C3AR1 + IL1R2 + CD74 + CD177 + CX3CR1 + CIITA + CD3D + CTLA4 | 0,76 | 0,638 | 0,882 |
| J5/7 | C3AR1 + IL1R2 + CD74 + CD177 + CX3CR1 + CIITA + CTLA4 | 0,751 | 0,623 | 0,88 |
| J5/7 | C3AR1 + IL1R2 + CD74 + CD177 + CX3CR1 + CIITA + TAP2 | 0,779 | 0,668 | 0,89 |
| J5/7 | C3AR1 + IL1R2 + CD74 + CD177 + CX3CR1 + CIITA + TAP2 + CD3D | 0,771 | 0,66 | 0,882 |
| J5/7 | C3AR1 + IL1R2 + CD74 + CD177 + CX3CR1 + CIITA + TAP2 + CD3D + CTLA4 | 0,771 | 0,66 | 0,882 |
| J5/7 | C3AR1 + IL1R2 + CD74 + CD177 + CX3CR1 + CIITA + TAP2 + CTLA4 | 0,778 | 0,666 | 0,889 |
| J5/7 | C3AR1 + IL1R2 + CD74 + CD177 + CX3CR1 + CTLA4 | 0,767 | 0,653 | 0,881 |
| J5/7 | C3AR1 + IL1R2 + CD74 + CD177 + CX3CR1 + TAP2 | 0,78 | 0,679 | 0,881 |
| J5/7 | C3AR1 + IL1R2 + CD74 + CD177 + CX3CR1 + TAP2 + CD3D | 0,777 | 0,675 | 0,88 |
| J5/7 | C3AR1 + IL1R2 + CD74 + CD177 + CX3CR1 + TAP2 + CD3D + CTLA4 | 0,777 | 0,674 | 0,879 |
| J5/7 | C3AR1 + IL1R2 + CD74 + CD177 + CX3CR1 + TAP2 + CTLA4 | 0,78 | 0,677 | 0,883 |
| J5/7 | C3AR1 + IL1R2 + CD74 + CD177 + S100A9 | 0,77 | 0,67 | 0,871 |
| J5/7 | C3AR1 + IL1R2 + CD74 + CD177 + S100A9 + CD3D | 0,777 | 0,681 | 0,873 |
| J5/7 | C3AR1 + IL1R2 + CD74 + CD177 + S100A9 + CD3D + CTLA4 | 0,779 | 0,683 | 0,874 |
| J5/7 | C3AR1 + IL1R2 + CD74 + CD177 + S100A9 + CIITA | 0,775 | 0,665 | 0,885 |
| J5/7 | C3AR1 + IL1R2 + CD74 + CD177 + S100A9 + CIITA + CD3D | 0,781 | 0,677 | 0,886 |
| J5/7 | C3AR1 + IL1R2 + CD74 + CD177 + S100A9 + CIITA + CD3D + CTLA4 | 0,777 | 0,67 | 0,884 |
| J5/7 | C3AR1 + IL1R2 + CD74 + CD177 + S100A9 + CIITA + CTLA4 | 0,766 | 0,649 | 0,884 |
| J5/7 | C3AR1 + IL1R2 + CD74 + CD177 + S100A9 + CIITA + TAP2 | 0,79 | 0,691 | 0,889 |
| J5/7 | C3AR1 + IL1R2 + CD74 + CD177 + S100A9 + CIITA + TAP2 + CD3D | 0,789 | 0,693 | 0,884 |
| J5/7 | C3AR1 + IL1R2 + CD74 + CD177 + S100A9 + CIITA + TAP2 + CD3D + CTLA4 | 0,79 | 0,695 | 0,885 |
| J5/7 | C3AR1 + IL1R2 + CD74 + CD177 + S100A9 + CIITA + TAP2 + CTLA4 | 0,792 | 0,693 | 0,89 |
| J5/7 | C3AR1 + IL1R2 + CD74 + CD177 + S100A9 + CTLA4 | 0,773 | 0,669 | 0,876 |
| J5/7 | C3AR1 + IL1R2 + CD74 + CD177 + S100A9 + CX3CR1 | 0,791 | 0,7 | 0,882 |
| J5/7 | C3AR1 + IL1R2 + CD74 + CD177 + S100A9 + CX3CR1 + CD3D | 0,786 | 0,689 | 0,883 |
| J5/7 | C3AR1 + IL1R2 + CD74 + CD177 + S100A9 + CX3CR1 + CD3D + CTLA4 | 0,781 | 0,681 | 0,881 |
| J5/7 | C3AR1 + IL1R2 + CD74 + CD177 + S100A9 + CX3CR1 + CIITA | 0,785 | 0,681 | 0,889 |
| J5/7 | C3AR1 + IL1R2 + CD74 + CD177 + S100A9 + CX3CR1 + CIITA + CD3D | 0,784 | 0,681 | 0,888 |
| J5/7 | C3AR1 + IL1R2 + CD74 + CD177 + S100A9 + CX3CR1 + CIITA + CD3D + CTLA4 | 0,779 | 0,672 | 0,886 |
| J5/7 | C3AR1 + IL1R2 + CD74 + CD177 + S100A9 + CX3CR1 + CIITA + CTLA4 | 0,777 | 0,663 | 0,89 |
| J5/7 | C3AR1 + IL1R2 + CD74 + CD177 + S100A9 + CX3CR1 + CIITA + TAP2 | 0,793 | 0,695 | 0,892 |
| J5/7 | C3AR1 + IL1R2 + CD74 + CD177 + S100A9 + CX3CR1 + CIITA + TAP2 + CD3D | 0,787 | 0,692 | 0,883 |
| J5/7 | C3AR1 + IL1R2 + CD74 + CD177 + S100A9 + CX3CR1 + CIITA + TAP2 + CD3D + CTLA4 | 0,791 | 0,698 | 0,885 |
| J5/7 | C3AR1 + IL1R2 + CD74 + CD177 + S100A9 + CX3CR1 + CIITA + TAP2 + CTLA4 | 0,793 | 0,694 | 0,892 |
| J5/7 | C3AR1 + IL1R2 + CD74 + CD177 + S100A9 + CX3CR1 + CTLA4 | 0,782 | 0,682 | 0,882 |
| J5/7 | C3AR1 + IL1R2 + CD74 + CD177 + S100A9 + CX3CR1 + TAP2 | 0,793 | 0,703 | 0,884 |
| J5/7 | C3AR1 + IL1R2 + CD74 + CD177 + S100A9 + CX3CR1 + TAP2 + CD3D | 0,792 | 0,704 | 0,881 |
| J5/7 | C3AR1 + IL1R2 + CD74 + CD177 + S100A9 + CX3CR1 + TAP2 + CD3D + CTLA4 | 0,795 | 0,707 | 0,883 |
| J5/7 | C3AR1 + IL1R2 + CD74 + CD177 + S100A9 + CX3CR1 + TAP2 + CTLA4 | 0,791 | 0,699 | 0,883 |
| J5/7 | C3AR1 + IL1R2 + CD74 + CD177 + S100A9 + TAP2 | 0,791 | 0,699 | 0,883 |
| J5/7 | C3AR1 + IL1R2 + CD74 + CD177 + S100A9 + TAP2 + CD3D | 0,792 | 0,703 | 0,881 |
| J5/7 | C3AR1 + IL1R2 + CD74 + CD177 + S100A9 + TAP2 + CD3D + CTLA4 | 0,795 | 0,707 | 0,883 |
| J5/7 | C3AR1 + IL1R2 + CD74 + CD177 + S100A9 + TAP2 + CTLA4 | 0,792 | 0,7 | 0,885 |
| J5/7 | C3AR1 + IL1R2 + CD74 + CD177 + TAP2 | 0,78 | 0,679 | 0,881 |
| J5/7 | C3AR1 + IL1R2 + CD74 + CD177 + TAP2 + CD3D | 0,779 | 0,677 | 0,881 |
| J5/7 | C3AR1 + IL1R2 + CD74 + CD177 + TAP2 + CD3D + CTLA4 | 0,779 | 0,677 | 0,882 |
| J5/7 | C3AR1 + IL1R2 + CD74 + CD177 + TAP2 + CTLA4 | 0,78 | 0,677 | 0,883 |
| J5/7 | C3AR1 + IL1R2 + CD74 + CD3D | 0,779 | 0,679 | 0,879 |
| J5/7 | C3AR1 + IL1R2 + CD74 + CD3D + CTLA4 | 0,775 | 0,668 | 0,882 |
| J5/7 | C3AR1 + IL1R2 + CD74 + CIITA | 0,771 | 0,651 | 0,891 |
| J5/7 | C3AR1 + IL1R2 + CD74 + CIITA + CD3D | 0,773 | 0,655 | 0,891 |
| J5/7 | C3AR1 + IL1R2 + CD74 + CIITA + CD3D + CTLA4 | 0,762 | 0,637 | 0,886 |
| J5/7 | C3AR1 + IL1R2 + CD74 + CIITA + CTLA4 | 0,762 | 0,636 | 0,887 |
| J5/7 | C3AR1 + IL1R2 + CD74 + CIITA + TAP2 | 0,785 | 0,675 | 0,894 |
| J5/7 | C3AR1 + IL1R2 + CD74 + CIITA + TAP2 + CD3D | 0,783 | 0,671 | 0,894 |
| J5/7 | C3AR1 + IL1R2 + CD74 + CIITA + TAP2 + CD3D + CTLA4 | 0,781 | 0,666 | 0,896 |
| J5/7 | C3AR1 + IL1R2 + CD74 + CIITA + TAP2 + CTLA4 | 0,779 | 0,666 | 0,893 |
| J5/7 | C3AR1 + IL1R2 + CD74 + CTLA4 | 0,776 | 0,668 | 0,885 |
| J5/7 | C3AR1 + IL1R2 + CD74 + CX3CR1 | 0,777 | 0,67 | 0,884 |
| J5/7 | C3AR1 + IL1R2 + CD74 + CX3CR1 + CD3D | 0,78 | 0,683 | 0,878 |
| J5/7 | C3AR1 + IL1R2 + CD74 + CX3CR1 + CD3D + CTLA4 | 0,775 | 0,669 | 0,882 |
| J5/7 | C3AR1 + IL1R2 + CD74 + CX3CR1 + CIITA | 0,772 | 0,652 | 0,893 |
| J5/7 | C3AR1 + IL1R2 + CD74 + CX3CR1 + CIITA + CD3D | 0,767 | 0,648 | 0,886 |
| J5/7 | C3AR1 + IL1R2 + CD74 + CX3CR1 + CIITA + CD3D + CTLA4 | 0,761 | 0,636 | 0,886 |
| J5/7 | C3AR1 + IL1R2 + CD74 + CX3CR1 + CIITA + CTLA4 | 0,762 | 0,636 | 0,888 |
| J5/7 | C3AR1 + IL1R2 + CD74 + CX3CR1 + CIITA + TAP2 | 0,79 | 0,681 | 0,898 |
| J5/7 | C3AR1 + IL1R2 + CD74 + CX3CR1 + CIITA + TAP2 + CD3D | 0,788 | 0,68 | 0,895 |
| J5/7 | C3AR1 + IL1R2 + CD74 + CX3CR1 + CIITA + TAP2 + CD3D + CTLA4 | 0,786 | 0,674 | 0,898 |
| J5/7 | C3AR1 + IL1R2 + CD74 + CX3CR1 + CIITA + TAP2 + CTLA4 | 0,785 | 0,673 | 0,898 |
| J5/7 | C3AR1 + IL1R2 + CD74 + CX3CR1 + CTLA4 | 0,776 | 0,667 | 0,885 |
| J5/7 | C3AR1 + IL1R2 + CD74 + CX3CR1 + TAP2 | 0,793 | 0,694 | 0,892 |
| J5/7 | C3AR1 + IL1R2 + CD74 + CX3CR1 + TAP2 + CD3D | 0,792 | 0,697 | 0,887 |
| J5/7 | C3AR1 + IL1R2 + CD74 + CX3CR1 + TAP2 + CD3D + CTLA4 | 0,794 | 0,696 | 0,892 |
| J5/7 | C3AR1 + IL1R2 + CD74 + CX3CR1 + TAP2 + CTLA4 | 0,793 | 0,693 | 0,893 |
| J5/7 | C3AR1 + IL1R2 + CD74 + IFNG | 0,774 | 0,66 | 0,889 |
| J5/7 | C3AR1 + IL1R2 + CD74 + IFNG + CD177 | 0,759 | 0,638 | 0,88 |
| J5/7 | C3AR1 + IL1R2 + CD74 + IFNG + CD177 + CD3D | 0,758 | 0,629 | 0,886 |
| J5/7 | C3AR1 + IL1R2 + CD74 + IFNG + CD177 + CD3D + CTLA4 | 0,756 | 0,627 | 0,886 |
| J5/7 | C3AR1 + IL1R2 + CD74 + IFNG + CD177 + CIITA | 0,752 | 0,624 | 0,879 |
| J5/7 | C3AR1 + IL1R2 + CD74 + IFNG + CD177 + CIITA + CD3D | 0,758 | 0,629 | 0,887 |
| J5/7 | C3AR1 + IL1R2 + CD74 + IFNG + CD177 + CIITA + CD3D + CTLA4 | 0,756 | 0,625 | 0,886 |
| J5/7 | C3AR1 + IL1R2 + CD74 + IFNG + CD177 + CIITA + CTLA4 | 0,753 | 0,621 | 0,885 |
| J5/7 | C3AR1 + IL1R2 + CD74 + IFNG + CD177 + CIITA + TAP2 | 0,783 | 0,669 | 0,896 |
| J5/7 | C3AR1 + IL1R2 + CD74 + IFNG + CD177 + CIITA + TAP2 + CD3D | 0,778 | 0,664 | 0,892 |
| J5/7 | C3AR1 + IL1R2 + CD74 + IFNG + CD177 + CIITA + TAP2 + CD3D + CTLA4 | 0,782 | 0,67 | 0,894 |
| J5/7 | C3AR1 + IL1R2 + CD74 + IFNG + CD177 + CIITA + TAP2 + CTLA4 | 0,779 | 0,664 | 0,894 |
| J5/7 | C3AR1 + IL1R2 + CD74 + IFNG + CD177 + CTLA4 | 0,755 | 0,628 | 0,883 |
| J5/7 | C3AR1 + IL1R2 + CD74 + IFNG + CD177 + CX3CR1 | 0,756 | 0,629 | 0,883 |
| J5/7 | C3AR1 + IL1R2 + CD74 + IFNG + CD177 + CX3CR1 + CD3D | 0,76 | 0,631 | 0,889 |
| J5/7 | C3AR1 + IL1R2 + CD74 + IFNG + CD177 + CX3CR1 + CD3D + CTLA4 | 0,755 | 0,62 | 0,889 |
| J5/7 | C3AR1 + IL1R2 + CD74 + IFNG + CD177 + CX3CR1 + CIITA | 0,757 | 0,63 | 0,884 |
| J5/7 | C3AR1 + IL1R2 + CD74 + IFNG + CD177 + CX3CR1 + CIITA + CD3D | 0,76 | 0,632 | 0,889 |
| J5/7 | C3AR1 + IL1R2 + CD74 + IFNG + CD177 + CX3CR1 + CIITA + CD3D + CTLA4 | 0,754 | 0,621 | 0,887 |
| J5/7 | C3AR1 + IL1R2 + CD74 + IFNG + CD177 + CX3CR1 + CIITA + CTLA4 | 0,758 | 0,628 | 0,889 |
| J5/7 | C3AR1 + IL1R2 + CD74 + IFNG + CD177 + CX3CR1 + CIITA + TAP2 | 0,784 | 0,67 | 0,897 |
| J5/7 | C3AR1 + IL1R2 + CD74 + IFNG + CD177 + CX3CR1 + CIITA + TAP2 + CD3D | 0,783 | 0,67 | 0,895 |
| J5/7 | C3AR1 + IL1R2 + CD74 + IFNG + CD177 + CX3CR1 + CIITA + TAP2 + CD3D + CTLA4 | 0,781 | 0,668 | 0,895 |
| J5/7 | C3AR1 + IL1R2 + CD74 + IFNG + CD177 + CX3CR1 + CIITA + TAP2 + CTLA4 | 0,78 | 0,665 | 0,894 |
| J5/7 | C3AR1 + IL1R2 + CD74 + IFNG + CD177 + CX3CR1 + CTLA4 | 0,758 | 0,628 | 0,888 |
| J5/7 | C3AR1 + IL1R2 + CD74 + IFNG + CD177 + CX3CR1 + TAP2 | 0,783 | 0,67 | 0,896 |
| J5/7 | C3AR1 + IL1R2 + CD74 + IFNG + CD177 + CX3CR1 + TAP2 + CD3D | 0,783 | 0,67 | 0,895 |
| J5/7 | C3AR1 + IL1R2 + CD74 + IFNG + CD177 + CX3CR1 + TAP2 + CD3D + CTLA4 | 0,781 | 0,668 | 0,895 |
| J5/7 | C3AR1 + IL1R2 + CD74 + IFNG + CD177 + CX3CR1 + TAP2 + CTLA4 | 0,782 | 0,668 | 0,896 |
| J5/7 | C3AR1 + IL1R2 + CD74 + IFNG + CD177 + S100A9 | 0,781 | 0,677 | 0,886 |
| J5/7 | C3AR1 + IL1R2 + CD74 + IFNG + CD177 + S100A9 + CD3D | 0,791 | 0,685 | 0,896 |
| J5/7 | C3AR1 + IL1R2 + CD74 + IFNG + CD177 + S100A9 + CD3D + CTLA4 | 0,791 | 0,686 | 0,896 |
| J5/7 | C3AR1 + IL1R2 + CD74 + IFNG + CD177 + S100A9 + CIITA | 0,776 | 0,665 | 0,887 |
| J5/7 | C3AR1 + IL1R2 + CD74 + IFNG + CD177 + S100A9 + CIITA + CD3D | 0,792 | 0,684 | 0,9 |
| J5/7 | C3AR1 + IL1R2 + CD74 + IFNG + CD177 + S100A9 + CIITA + CD3D + CTLA4 | 0,789 | 0,68 | 0,897 |
| J5/7 | C3AR1 + IL1R2 + CD74 + IFNG + CD177 + S100A9 + CIITA + CTLA4 | 0,769 | 0,649 | 0,89 |
| J5/7 | C3AR1 + IL1R2 + CD74 + IFNG + CD177 + S100A9 + CIITA + TAP2 | 0,798 | 0,701 | 0,895 |
| J5/7 | C3AR1 + IL1R2 + CD74 + IFNG + CD177 + S100A9 + CIITA + TAP2 + CD3D | 0,8 | 0,703 | 0,897 |
| J5/7 | C3AR1 + IL1R2 + CD74 + IFNG + CD177 + S100A9 + CIITA + TAP2 + CD3D + CTLA4 | 0,807 | 0,712 | 0,902 |
| J5/7 | C3AR1 + IL1R2 + CD74 + IFNG + CD177 + S100A9 + CIITA + TAP2 + CTLA4 | 0,799 | 0,701 | 0,897 |
| J5/7 | C3AR1 + IL1R2 + CD74 + IFNG + CD177 + S100A9 + CTLA4 | 0,779 | 0,67 | 0,887 |
| J5/7 | C3AR1 + IL1R2 + CD74 + IFNG + CD177 + S100A9 + CX3CR1 | 0,8 | 0,702 | 0,898 |
| J5/7 | C3AR1 + IL1R2 + CD74 + IFNG + CD177 + S100A9 + CX3CR1 + CD3D | 0,801 | 0,694 | 0,907 |
| J5/7 | C3AR1 + IL1R2 + CD74 + IFNG + CD177 + S100A9 + CX3CR1 + CD3D + CTLA4 | 0,801 | 0,694 | 0,907 |
| J5/7 | C3AR1 + IL1R2 + CD74 + IFNG + CD177 + S100A9 + CX3CR1 + CIITA | 0,798 | 0,699 | 0,898 |
| J5/7 | C3AR1 + IL1R2 + CD74 + IFNG + CD177 + S100A9 + CX3CR1 + CIITA + CD3D | 0,8 | 0,692 | 0,908 |
| J5/7 | C3AR1 + IL1R2 + CD74 + IFNG + CD177 + S100A9 + CX3CR1 + CIITA + CD3D + CTLA4 | 0,801 | 0,693 | 0,908 |
| J5/7 | C3AR1 + IL1R2 + CD74 + IFNG + CD177 + S100A9 + CX3CR1 + CIITA + CTLA4 | 0,795 | 0,683 | 0,907 |
| J5/7 | C3AR1 + IL1R2 + CD74 + IFNG + CD177 + S100A9 + CX3CR1 + CIITA + TAP2 | 0,814 | 0,719 | 0,909 |
| J5/7 | C3AR1 + IL1R2 + CD74 + IFNG + CD177 + S100A9 + CX3CR1 + CIITA + TAP2 + CD3D | 0,809 | 0,711 | 0,908 |
| J5/7 | C3AR1 + IL1R2 + CD74 + IFNG + CD177 + S100A9 + CX3CR1 + CIITA + TAP2 + CD3D + CTLA4 | 0,81 | 0,714 | 0,907 |
| J5/7 | C3AR1 + IL1R2 + CD74 + IFNG + CD177 + S100A9 + CX3CR1 + CIITA + TAP2 + CTLA4 | 0,813 | 0,715 | 0,911 |
| J5/7 | C3AR1 + IL1R2 + CD74 + IFNG + CD177 + S100A9 + CX3CR1 + CTLA4 | 0,797 | 0,692 | 0,903 |
| J5/7 | C3AR1 + IL1R2 + CD74 + IFNG + CD177 + S100A9 + CX3CR1 + TAP2 | 0,82 | 0,728 | 0,911 |
| J5/7 | C3AR1 + IL1R2 + CD74 + IFNG + CD177 + S100A9 + CX3CR1 + TAP2 + CD3D | 0,812 | 0,715 | 0,909 |
| J5/7 | C3AR1 + IL1R2 + CD74 + IFNG + CD177 + S100A9 + CX3CR1 + TAP2 + CD3D + CTLA4 | 0,809 | 0,713 | 0,906 |
| J5/7 | C3AR1 + IL1R2 + CD74 + IFNG + CD177 + S100A9 + CX3CR1 + TAP2 + CTLA4 | 0,819 | 0,725 | 0,912 |
| J5/7 | C3AR1 + IL1R2 + CD74 + IFNG + CD177 + S100A9 + TAP2 | 0,809 | 0,718 | 0,899 |
| J5/7 | C3AR1 + IL1R2 + CD74 + IFNG + CD177 + S100A9 + TAP2 + CD3D | 0,803 | 0,707 | 0,898 |
| J5/7 | C3AR1 + IL1R2 + CD74 + IFNG + CD177 + S100A9 + TAP2 + CD3D + CTLA4 | 0,81 | 0,715 | 0,904 |
| J5/7 | C3AR1 + IL1R2 + CD74 + IFNG + CD177 + S100A9 + TAP2 + CTLA4 | 0,808 | 0,716 | 0,9 |
| J5/7 | C3AR1 + IL1R2 + CD74 + IFNG + CD177 + TAP2 | 0,788 | 0,679 | 0,897 |
| J5/7 | C3AR1 + IL1R2 + CD74 + IFNG + CD177 + TAP2 + CD3D | 0,779 | 0,665 | 0,892 |
| J5/7 | C3AR1 + IL1R2 + CD74 + IFNG + CD177 + TAP2 + CD3D + CTLA4 | 0,782 | 0,67 | 0,894 |
| J5/7 | C3AR1 + IL1R2 + CD74 + IFNG + CD177 + TAP2 + CTLA4 | 0,785 | 0,675 | 0,896 |
| J5/7 | C3AR1 + IL1R2 + CD74 + IFNG + CD3D | 0,771 | 0,652 | 0,889 |
| J5/7 | C3AR1 + IL1R2 + CD74 + IFNG + CD3D + CTLA4 | 0,768 | 0,647 | 0,889 |
| J5/7 | C3AR1 + IL1R2 + CD74 + IFNG + CIITA | 0,765 | 0,642 | 0,889 |
| J5/7 | C3AR1 + IL1R2 + CD74 + IFNG + CIITA + CD3D | 0,768 | 0,645 | 0,89 |
| J5/7 | C3AR1 + IL1R2 + CD74 + IFNG + CIITA + CD3D + CTLA4 | 0,766 | 0,64 | 0,892 |
| J5/7 | C3AR1 + IL1R2 + CD74 + IFNG + CIITA + CTLA4 | 0,768 | 0,64 | 0,895 |
| J5/7 | C3AR1 + IL1R2 + CD74 + IFNG + CIITA + TAP2 | 0,795 | 0,684 | 0,905 |
| J5/7 | C3AR1 + IL1R2 + CD74 + IFNG + CIITA + TAP2 + CD3D | 0,786 | 0,673 | 0,899 |
| J5/7 | C3AR1 + IL1R2 + CD74 + IFNG + CIITA + TAP2 + CD3D + CTLA4 | 0,785 | 0,668 | 0,902 |
| J5/7 | C3AR1 + IL1R2 + CD74 + IFNG + CIITA + TAP2 + CTLA4 | 0,787 | 0,669 | 0,905 |
| J5/7 | C3AR1 + IL1R2 + CD74 + IFNG + CTLA4 | 0,77 | 0,648 | 0,892 |
| J5/7 | C3AR1 + IL1R2 + CD74 + IFNG + CX3CR1 | 0,773 | 0,654 | 0,892 |
| J5/7 | C3AR1 + IL1R2 + CD74 + IFNG + CX3CR1 + CD3D | 0,766 | 0,645 | 0,887 |
| J5/7 | C3AR1 + IL1R2 + CD74 + IFNG + CX3CR1 + CD3D + CTLA4 | 0,767 | 0,644 | 0,89 |
| J5/7 | C3AR1 + IL1R2 + CD74 + IFNG + CX3CR1 + CIITA | 0,771 | 0,646 | 0,896 |
| J5/7 | C3AR1 + IL1R2 + CD74 + IFNG + CX3CR1 + CIITA + CD3D | 0,765 | 0,643 | 0,888 |
| J5/7 | C3AR1 + IL1R2 + CD74 + IFNG + CX3CR1 + CIITA + CD3D + CTLA4 | 0,765 | 0,639 | 0,891 |
| J5/7 | C3AR1 + IL1R2 + CD74 + IFNG + CX3CR1 + CIITA + CTLA4 | 0,767 | 0,64 | 0,894 |
| J5/7 | C3AR1 + IL1R2 + CD74 + IFNG + CX3CR1 + CIITA + TAP2 | 0,796 | 0,686 | 0,905 |
| J5/7 | C3AR1 + IL1R2 + CD74 + IFNG + CX3CR1 + CIITA + TAP2 + CD3D | 0,79 | 0,677 | 0,903 |
| J5/7 | C3AR1 + IL1R2 + CD74 + IFNG + CX3CR1 + CIITA + TAP2 + CD3D + CTLA4 | 0,785 | 0,669 | 0,901 |
| J5/7 | C3AR1 + IL1R2 + CD74 + IFNG + CX3CR1 + CIITA + TAP2 + CTLA4 | 0,789 | 0,673 | 0,904 |
| J5/7 | C3AR1 + IL1R2 + CD74 + IFNG + CX3CR1 + CTLA4 | 0,772 | 0,651 | 0,894 |
| J5/7 | C3AR1 + IL1R2 + CD74 + IFNG + CX3CR1 + TAP2 | 0,8 | 0,695 | 0,904 |
| J5/7 | C3AR1 + IL1R2 + CD74 + IFNG + CX3CR1 + TAP2 + CD3D | 0,792 | 0,684 | 0,9 |
| J5/7 | C3AR1 + IL1R2 + CD74 + IFNG + CX3CR1 + TAP2 + CD3D + CTLA4 | 0,787 | 0,675 | 0,899 |
| J5/7 | C3AR1 + IL1R2 + CD74 + IFNG + CX3CR1 + TAP2 + CTLA4 | 0,788 | 0,676 | 0,899 |
| J5/7 | C3AR1 + IL1R2 + CD74 + IFNG + S100A9 | 0,791 | 0,69 | 0,891 |
| J5/7 | C3AR1 + IL1R2 + CD74 + IFNG + S100A9 + CD3D | 0,799 | 0,701 | 0,898 |
| J5/7 | C3AR1 + IL1R2 + CD74 + IFNG + S100A9 + CD3D + CTLA4 | 0,797 | 0,698 | 0,896 |
| J5/7 | C3AR1 + IL1R2 + CD74 + IFNG + S100A9 + CIITA | 0,787 | 0,679 | 0,894 |
| J5/7 | C3AR1 + IL1R2 + CD74 + IFNG + S100A9 + CIITA + CD3D | 0,789 | 0,682 | 0,897 |
| J5/7 | C3AR1 + IL1R2 + CD74 + IFNG + S100A9 + CIITA + CD3D + CTLA4 | 0,788 | 0,679 | 0,897 |
| J5/7 | C3AR1 + IL1R2 + CD74 + IFNG + S100A9 + CIITA + CTLA4 | 0,78 | 0,663 | 0,897 |
| J5/7 | C3AR1 + IL1R2 + CD74 + IFNG + S100A9 + CIITA + TAP2 | 0,807 | 0,712 | 0,903 |
| J5/7 | C3AR1 + IL1R2 + CD74 + IFNG + S100A9 + CIITA + TAP2 + CD3D | 0,801 | 0,702 | 0,9 |
| J5/7 | C3AR1 + IL1R2 + CD74 + IFNG + S100A9 + CIITA + TAP2 + CD3D + CTLA4 | 0,801 | 0,702 | 0,901 |
| J5/7 | C3AR1 + IL1R2 + CD74 + IFNG + S100A9 + CIITA + TAP2 + CTLA4 | 0,804 | 0,702 | 0,905 |
| J5/7 | C3AR1 + IL1R2 + CD74 + IFNG + S100A9 + CTLA4 | 0,791 | 0,687 | 0,895 |
| J5/7 | C3AR1 + IL1R2 + CD74 + IFNG + S100A9 + CX3CR1 | 0,802 | 0,708 | 0,897 |
| J5/7 | C3AR1 + IL1R2 + CD74 + IFNG + S100A9 + CX3CR1 + CD3D | 0,802 | 0,703 | 0,9 |
| J5/7 | C3AR1 + IL1R2 + CD74 + IFNG + S100A9 + CX3CR1 + CD3D + CTLA4 | 0,798 | 0,696 | 0,901 |
| J5/7 | C3AR1 + IL1R2 + CD74 + IFNG + S100A9 + CX3CR1 + CIITA | 0,796 | 0,692 | 0,9 |
| J5/7 | C3AR1 + IL1R2 + CD74 + IFNG + S100A9 + CX3CR1 + CIITA + CD3D | 0,8 | 0,693 | 0,906 |
| J5/7 | C3AR1 + IL1R2 + CD74 + IFNG + S100A9 + CX3CR1 + CIITA + CD3D + CTLA4 | 0,801 | 0,693 | 0,91 |
| J5/7 | C3AR1 + IL1R2 + CD74 + IFNG + S100A9 + CX3CR1 + CIITA + CTLA4 | 0,794 | 0,684 | 0,905 |
| J5/7 | C3AR1 + IL1R2 + CD74 + IFNG + S100A9 + CX3CR1 + CIITA + TAP2 | 0,808 | 0,713 | 0,903 |
| J5/7 | C3AR1 + IL1R2 + CD74 + IFNG + S100A9 + CX3CR1 + CIITA + TAP2 + CD3D | 0,799 | 0,699 | 0,899 |
| J5/7 | C3AR1 + IL1R2 + CD74 + IFNG + S100A9 + CX3CR1 + CIITA + TAP2 + CD3D + CTLA4 | 0,796 | 0,695 | 0,898 |
| J5/7 | C3AR1 + IL1R2 + CD74 + IFNG + S100A9 + CX3CR1 + CIITA + TAP2 + CTLA4 | 0,801 | 0,699 | 0,903 |
| J5/7 | C3AR1 + IL1R2 + CD74 + IFNG + S100A9 + CX3CR1 + CTLA4 | 0,798 | 0,695 | 0,901 |
| J5/7 | C3AR1 + IL1R2 + CD74 + IFNG + S100A9 + CX3CR1 + TAP2 | 0,812 | 0,723 | 0,901 |
| J5/7 | C3AR1 + IL1R2 + CD74 + IFNG + S100A9 + CX3CR1 + TAP2 + CD3D | 0,808 | 0,716 | 0,9 |
| J5/7 | C3AR1 + IL1R2 + CD74 + IFNG + S100A9 + CX3CR1 + TAP2 + CD3D + CTLA4 | 0,809 | 0,717 | 0,901 |
| J5/7 | C3AR1 + IL1R2 + CD74 + IFNG + S100A9 + CX3CR1 + TAP2 + CTLA4 | 0,807 | 0,712 | 0,901 |
| J5/7 | C3AR1 + IL1R2 + CD74 + IFNG + S100A9 + TAP2 | 0,815 | 0,726 | 0,903 |
| J5/7 | C3AR1 + IL1R2 + CD74 + IFNG + S100A9 + TAP2 + CD3D | 0,808 | 0,717 | 0,9 |
| J5/7 | C3AR1 + IL1R2 + CD74 + IFNG + S100A9 + TAP2 + CD3D + CTLA4 | 0,809 | 0,718 | 0,9 |
| J5/7 | C3AR1 + IL1R2 + CD74 + IFNG + S100A9 + TAP2 + CTLA4 | 0,812 | 0,722 | 0,903 |
| J5/7 | C3AR1 + IL1R2 + CD74 + IFNG + TAP2 | 0,8 | 0,696 | 0,905 |
| J5/7 | C3AR1 + IL1R2 + CD74 + IFNG + TAP2 + CD3D | 0,792 | 0,685 | 0,9 |
| J5/7 | C3AR1 + IL1R2 + CD74 + IFNG + TAP2 + CD3D + CTLA4 | 0,788 | 0,676 | 0,899 |
| J5/7 | C3AR1 + IL1R2 + CD74 + IFNG + TAP2 + CTLA4 | 0,79 | 0,679 | 0,9 |
| J5/7 | C3AR1 + IL1R2 + CD74 + S100A9 | 0,788 | 0,69 | 0,885 |
| J5/7 | C3AR1 + IL1R2 + CD74 + S100A9 + CD3D | 0,793 | 0,705 | 0,882 |
| J5/7 | C3AR1 + IL1R2 + CD74 + S100A9 + CD3D + CTLA4 | 0,788 | 0,694 | 0,881 |
| J5/7 | C3AR1 + IL1R2 + CD74 + S100A9 + CIITA | 0,783 | 0,672 | 0,894 |
| J5/7 | C3AR1 + IL1R2 + CD74 + S100A9 + CIITA + CD3D | 0,784 | 0,676 | 0,892 |
| J5/7 | C3AR1 + IL1R2 + CD74 + S100A9 + CIITA + CD3D + CTLA4 | 0,775 | 0,662 | 0,889 |
| J5/7 | C3AR1 + IL1R2 + CD74 + S100A9 + CIITA + CTLA4 | 0,77 | 0,648 | 0,892 |
| J5/7 | C3AR1 + IL1R2 + CD74 + S100A9 + CIITA + TAP2 | 0,796 | 0,696 | 0,895 |
| J5/7 | C3AR1 + IL1R2 + CD74 + S100A9 + CIITA + TAP2 + CD3D | 0,797 | 0,698 | 0,895 |
| J5/7 | C3AR1 + IL1R2 + CD74 + S100A9 + CIITA + TAP2 + CD3D + CTLA4 | 0,793 | 0,688 | 0,898 |
| J5/7 | C3AR1 + IL1R2 + CD74 + S100A9 + CIITA + TAP2 + CTLA4 | 0,793 | 0,687 | 0,898 |
| J5/7 | C3AR1 + IL1R2 + CD74 + S100A9 + CTLA4 | 0,789 | 0,689 | 0,888 |
| J5/7 | C3AR1 + IL1R2 + CD74 + S100A9 + CX3CR1 | 0,793 | 0,698 | 0,887 |
| J5/7 | C3AR1 + IL1R2 + CD74 + S100A9 + CX3CR1 + CD3D | 0,793 | 0,704 | 0,881 |
| J5/7 | C3AR1 + IL1R2 + CD74 + S100A9 + CX3CR1 + CD3D + CTLA4 | 0,789 | 0,695 | 0,882 |
| J5/7 | C3AR1 + IL1R2 + CD74 + S100A9 + CX3CR1 + CIITA | 0,788 | 0,677 | 0,898 |
| J5/7 | C3AR1 + IL1R2 + CD74 + S100A9 + CX3CR1 + CIITA + CD3D | 0,783 | 0,675 | 0,89 |
| J5/7 | C3AR1 + IL1R2 + CD74 + S100A9 + CX3CR1 + CIITA + CD3D + CTLA4 | 0,775 | 0,662 | 0,889 |
| J5/7 | C3AR1 + IL1R2 + CD74 + S100A9 + CX3CR1 + CIITA + CTLA4 | 0,772 | 0,652 | 0,893 |
| J5/7 | C3AR1 + IL1R2 + CD74 + S100A9 + CX3CR1 + CIITA + TAP2 | 0,798 | 0,698 | 0,897 |
| J5/7 | C3AR1 + IL1R2 + CD74 + S100A9 + CX3CR1 + CIITA + TAP2 + CD3D | 0,796 | 0,698 | 0,894 |
| J5/7 | C3AR1 + IL1R2 + CD74 + S100A9 + CX3CR1 + CIITA + TAP2 + CD3D + CTLA4 | 0,793 | 0,69 | 0,895 |
| J5/7 | C3AR1 + IL1R2 + CD74 + S100A9 + CX3CR1 + CIITA + TAP2 + CTLA4 | 0,793 | 0,688 | 0,897 |
| J5/7 | C3AR1 + IL1R2 + CD74 + S100A9 + CX3CR1 + CTLA4 | 0,787 | 0,687 | 0,886 |
| J5/7 | C3AR1 + IL1R2 + CD74 + S100A9 + CX3CR1 + TAP2 | 0,799 | 0,712 | 0,887 |
| J5/7 | C3AR1 + IL1R2 + CD74 + S100A9 + CX3CR1 + TAP2 + CD3D | 0,797 | 0,71 | 0,883 |
| J5/7 | C3AR1 + IL1R2 + CD74 + S100A9 + CX3CR1 + TAP2 + CD3D + CTLA4 | 0,796 | 0,708 | 0,885 |
| J5/7 | C3AR1 + IL1R2 + CD74 + S100A9 + CX3CR1 + TAP2 + CTLA4 | 0,796 | 0,706 | 0,887 |
| J5/7 | C3AR1 + IL1R2 + CD74 + S100A9 + TAP2 | 0,8 | 0,713 | 0,886 |
| J5/7 | C3AR1 + IL1R2 + CD74 + S100A9 + TAP2 + CD3D | 0,8 | 0,715 | 0,885 |
| J5/7 | C3AR1 + IL1R2 + CD74 + S100A9 + TAP2 + CD3D + CTLA4 | 0,795 | 0,705 | 0,884 |
| J5/7 | C3AR1 + IL1R2 + CD74 + S100A9 + TAP2 + CTLA4 | 0,794 | 0,705 | 0,884 |
| J5/7 | C3AR1 + IL1R2 + CD74 + TAP2 | 0,795 | 0,7 | 0,891 |
| J5/7 | C3AR1 + IL1R2 + CD74 + TAP2 + CD3D | 0,795 | 0,7 | 0,89 |
| J5/7 | C3AR1 + IL1R2 + CD74 + TAP2 + CD3D + CTLA4 | 0,793 | 0,691 | 0,895 |
| J5/7 | C3AR1 + IL1R2 + CD74 + TAP2 + CTLA4 | 0,792 | 0,692 | 0,891 |
| J5/7 | C3AR1 + IL1R2 + CIITA | 0,799 | 0,716 | 0,882 |
| J5/7 | C3AR1 + IL1R2 + CIITA + CD3D | 0,791 | 0,701 | 0,881 |
| J5/7 | C3AR1 + IL1R2 + CIITA + CD3D + CTLA4 | 0,772 | 0,668 | 0,876 |
| J5/7 | C3AR1 + IL1R2 + CIITA + CTLA4 | 0,77 | 0,665 | 0,876 |
| J5/7 | C3AR1 + IL1R2 + CIITA + TAP2 | 0,802 | 0,714 | 0,89 |
| J5/7 | C3AR1 + IL1R2 + CIITA + TAP2 + CD3D | 0,799 | 0,711 | 0,888 |
| J5/7 | C3AR1 + IL1R2 + CIITA + TAP2 + CD3D + CTLA4 | 0,797 | 0,7 | 0,894 |
| J5/7 | C3AR1 + IL1R2 + CIITA + TAP2 + CTLA4 | 0,796 | 0,703 | 0,889 |
| J5/7 | C3AR1 + IL1R2 + CTLA4 | 0,774 | 0,663 | 0,886 |
| J5/7 | C3AR1 + IL1R2 + CX3CR1 | 0,805 | 0,716 | 0,894 |
| J5/7 | C3AR1 + IL1R2 + CX3CR1 + CD3D | 0,787 | 0,683 | 0,891 |
| J5/7 | C3AR1 + IL1R2 + CX3CR1 + CD3D + CTLA4 | 0,774 | 0,66 | 0,889 |
| J5/7 | C3AR1 + IL1R2 + CX3CR1 + CIITA | 0,799 | 0,715 | 0,883 |
| J5/7 | C3AR1 + IL1R2 + CX3CR1 + CIITA + CD3D | 0,792 | 0,702 | 0,881 |
| J5/7 | C3AR1 + IL1R2 + CX3CR1 + CIITA + CD3D + CTLA4 | 0,773 | 0,665 | 0,88 |
| J5/7 | C3AR1 + IL1R2 + CX3CR1 + CIITA + CTLA4 | 0,772 | 0,665 | 0,88 |
| J5/7 | C3AR1 + IL1R2 + CX3CR1 + CIITA + TAP2 | 0,8 | 0,709 | 0,89 |
| J5/7 | C3AR1 + IL1R2 + CX3CR1 + CIITA + TAP2 + CD3D | 0,799 | 0,709 | 0,889 |
| J5/7 | C3AR1 + IL1R2 + CX3CR1 + CIITA + TAP2 + CD3D + CTLA4 | 0,799 | 0,705 | 0,893 |
| J5/7 | C3AR1 + IL1R2 + CX3CR1 + CIITA + TAP2 + CTLA4 | 0,798 | 0,703 | 0,893 |
| J5/7 | C3AR1 + IL1R2 + CX3CR1 + CTLA4 | 0,774 | 0,66 | 0,887 |
| J5/7 | C3AR1 + IL1R2 + CX3CR1 + TAP2 | 0,799 | 0,709 | 0,89 |
| J5/7 | C3AR1 + IL1R2 + CX3CR1 + TAP2 + CD3D | 0,799 | 0,71 | 0,889 |
| J5/7 | C3AR1 + IL1R2 + CX3CR1 + TAP2 + CD3D + CTLA4 | 0,8 | 0,706 | 0,894 |
| J5/7 | C3AR1 + IL1R2 + CX3CR1 + TAP2 + CTLA4 | 0,8 | 0,706 | 0,894 |
| J5/7 | C3AR1 + IL1R2 + IFNG | 0,757 | 0,635 | 0,88 |
| J5/7 | C3AR1 + IL1R2 + IFNG + CD177 + CIITA | 0,753 | 0,648 | 0,858 |
| J5/7 | C3AR1 + IL1R2 + IFNG + CD177 + CIITA + CD3D | 0,751 | 0,632 | 0,869 |
| J5/7 | C3AR1 + IL1R2 + IFNG + CD177 + CIITA + CD3D + CTLA4 | 0,751 | 0,636 | 0,867 |
| J5/7 | C3AR1 + IL1R2 + IFNG + CD177 + CIITA + TAP2 | 0,789 | 0,687 | 0,89 |
| J5/7 | C3AR1 + IL1R2 + IFNG + CD177 + CIITA + TAP2 + CD3D | 0,78 | 0,675 | 0,886 |
| J5/7 | C3AR1 + IL1R2 + IFNG + CD177 + CIITA + TAP2 + CD3D + CTLA4 | 0,781 | 0,678 | 0,885 |
| J5/7 | C3AR1 + IL1R2 + IFNG + CD177 + CIITA + TAP2 + CTLA4 | 0,788 | 0,686 | 0,89 |
| J5/7 | C3AR1 + IL1R2 + IFNG + CD177 + CX3CR1 + CIITA | 0,765 | 0,663 | 0,867 |
| J5/7 | C3AR1 + IL1R2 + IFNG + CD177 + CX3CR1 + CIITA + CD3D | 0,765 | 0,647 | 0,883 |
| J5/7 | C3AR1 + IL1R2 + IFNG + CD177 + CX3CR1 + CIITA + CD3D + CTLA4 | 0,764 | 0,649 | 0,88 |
| J5/7 | C3AR1 + IL1R2 + IFNG + CD177 + CX3CR1 + CIITA + CTLA4 | 0,757 | 0,642 | 0,872 |
| J5/7 | C3AR1 + IL1R2 + IFNG + CD177 + CX3CR1 + CIITA + TAP2 | 0,789 | 0,689 | 0,889 |
| J5/7 | C3AR1 + IL1R2 + IFNG + CD177 + CX3CR1 + CIITA + TAP2 + CD3D | 0,783 | 0,679 | 0,886 |
| J5/7 | C3AR1 + IL1R2 + IFNG + CD177 + CX3CR1 + CIITA + TAP2 + CD3D + CTLA4 | 0,783 | 0,68 | 0,885 |
| J5/7 | C3AR1 + IL1R2 + IFNG + CD177 + CX3CR1 + CIITA + TAP2 + CTLA4 | 0,789 | 0,686 | 0,892 |
| J5/7 | C3AR1 + IL1R2 + IFNG + CD177 + CX3CR1 + TAP2 | 0,784 | 0,681 | 0,887 |
| J5/7 | C3AR1 + IL1R2 + IFNG + CD177 + CX3CR1 + TAP2 + CD3D | 0,769 | 0,657 | 0,882 |
| J5/7 | C3AR1 + IL1R2 + IFNG + CD177 + CX3CR1 + TAP2 + CD3D + CTLA4 | 0,772 | 0,661 | 0,882 |
| J5/7 | C3AR1 + IL1R2 + IFNG + CD177 + CX3CR1 + TAP2 + CTLA4 | 0,777 | 0,668 | 0,885 |
| J5/7 | C3AR1 + IL1R2 + IFNG + CD177 + S100A9 | 0,757 | 0,648 | 0,866 |
| J5/7 | C3AR1 + IL1R2 + IFNG + CD177 + S100A9 + CD3D | 0,77 | 0,652 | 0,888 |
| J5/7 | C3AR1 + IL1R2 + IFNG + CD177 + S100A9 + CD3D + CTLA4 | 0,77 | 0,651 | 0,889 |
| J5/7 | C3AR1 + IL1R2 + IFNG + CD177 + S100A9 + CIITA | 0,772 | 0,672 | 0,872 |
| J5/7 | C3AR1 + IL1R2 + IFNG + CD177 + S100A9 + CIITA + CD3D | 0,771 | 0,659 | 0,883 |
| J5/7 | C3AR1 + IL1R2 + IFNG + CD177 + S100A9 + CIITA + CD3D + CTLA4 | 0,769 | 0,657 | 0,882 |
| J5/7 | C3AR1 + IL1R2 + IFNG + CD177 + S100A9 + CIITA + CTLA4 | 0,758 | 0,644 | 0,873 |
| J5/7 | C3AR1 + IL1R2 + IFNG + CD177 + S100A9 + CIITA + TAP2 | 0,799 | 0,708 | 0,891 |
| J5/7 | C3AR1 + IL1R2 + IFNG + CD177 + S100A9 + CIITA + TAP2 + CD3D | 0,798 | 0,7 | 0,896 |
| J5/7 | C3AR1 + IL1R2 + IFNG + CD177 + S100A9 + CIITA + TAP2 + CD3D + CTLA4 | 0,8 | 0,705 | 0,895 |
| J5/7 | C3AR1 + IL1R2 + IFNG + CD177 + S100A9 + CIITA + TAP2 + CTLA4 | 0,798 | 0,705 | 0,891 |
| J5/7 | C3AR1 + IL1R2 + IFNG + CD177 + S100A9 + CTLA4 | 0,752 | 0,633 | 0,872 |
| J5/7 | C3AR1 + IL1R2 + IFNG + CD177 + S100A9 + CX3CR1 | 0,791 | 0,683 | 0,899 |
| J5/7 | C3AR1 + IL1R2 + IFNG + CD177 + S100A9 + CX3CR1 + CD3D | 0,778 | 0,657 | 0,899 |
| J5/7 | C3AR1 + IL1R2 + IFNG + CD177 + S100A9 + CX3CR1 + CD3D + CTLA4 | 0,78 | 0,657 | 0,904 |
| J5/7 | C3AR1 + IL1R2 + IFNG + CD177 + S100A9 + CX3CR1 + CIITA | 0,796 | 0,698 | 0,893 |
| J5/7 | C3AR1 + IL1R2 + IFNG + CD177 + S100A9 + CX3CR1 + CIITA + CD3D | 0,79 | 0,679 | 0,901 |
| J5/7 | C3AR1 + IL1R2 + IFNG + CD177 + S100A9 + CX3CR1 + CIITA + CD3D + CTLA4 | 0,789 | 0,676 | 0,902 |
| J5/7 | C3AR1 + IL1R2 + IFNG + CD177 + S100A9 + CX3CR1 + CIITA + CTLA4 | 0,792 | 0,683 | 0,902 |
| J5/7 | C3AR1 + IL1R2 + IFNG + CD177 + S100A9 + CX3CR1 + CIITA + TAP2 | 0,81 | 0,717 | 0,904 |
| J5/7 | C3AR1 + IL1R2 + IFNG + CD177 + S100A9 + CX3CR1 + CIITA + TAP2 + CD3D | 0,805 | 0,706 | 0,905 |
| J5/7 | C3AR1 + IL1R2 + IFNG + CD177 + S100A9 + CX3CR1 + CIITA + TAP2 + CD3D + CTLA4 | 0,807 | 0,708 | 0,905 |
| J5/7 | C3AR1 + IL1R2 + IFNG + CD177 + S100A9 + CX3CR1 + CIITA + TAP2 + CTLA4 | 0,809 | 0,715 | 0,902 |
| J5/7 | C3AR1 + IL1R2 + IFNG + CD177 + S100A9 + CX3CR1 + CTLA4 | 0,78 | 0,662 | 0,899 |
| J5/7 | C3AR1 + IL1R2 + IFNG + CD177 + S100A9 + CX3CR1 + TAP2 | 0,81 | 0,716 | 0,904 |
| J5/7 | C3AR1 + IL1R2 + IFNG + CD177 + S100A9 + CX3CR1 + TAP2 + CD3D | 0,804 | 0,701 | 0,908 |
| J5/7 | C3AR1 + IL1R2 + IFNG + CD177 + S100A9 + CX3CR1 + TAP2 + CD3D + CTLA4 | 0,803 | 0,703 | 0,902 |
| J5/7 | C3AR1 + IL1R2 + IFNG + CD177 + S100A9 + CX3CR1 + TAP2 + CTLA4 | 0,806 | 0,711 | 0,901 |
| J5/7 | C3AR1 + IL1R2 + IFNG + CD177 + S100A9 + TAP2 | 0,802 | 0,711 | 0,893 |
| J5/7 | C3AR1 + IL1R2 + IFNG + CD177 + S100A9 + TAP2 + CD3D | 0,799 | 0,702 | 0,897 |
| J5/7 | C3AR1 + IL1R2 + IFNG + CD177 + S100A9 + TAP2 + CD3D + CTLA4 | 0,801 | 0,705 | 0,897 |
| J5/7 | C3AR1 + IL1R2 + IFNG + CD177 + S100A9 + TAP2 + CTLA4 | 0,799 | 0,708 | 0,891 |
| J5/7 | C3AR1 + IL1R2 + IFNG + CD177 + TAP2 | 0,776 | 0,67 | 0,882 |
| J5/7 | C3AR1 + IL1R2 + IFNG + CD177 + TAP2 + CD3D | 0,772 | 0,663 | 0,882 |
| J5/7 | C3AR1 + IL1R2 + IFNG + CD177 + TAP2 + CD3D + CTLA4 | 0,773 | 0,665 | 0,882 |
| J5/7 | C3AR1 + IL1R2 + IFNG + CD177 + TAP2 + CTLA4 | 0,774 | 0,667 | 0,881 |
| J5/7 | C3AR1 + IL1R2 + IFNG + CD3D | 0,754 | 0,62 | 0,887 |
| J5/7 | C3AR1 + IL1R2 + IFNG + CD3D + CTLA4 | 0,749 | 0,617 | 0,881 |
| J5/7 | C3AR1 + IL1R2 + IFNG + CIITA | 0,786 | 0,692 | 0,879 |
| J5/7 | C3AR1 + IL1R2 + IFNG + CIITA + CD3D | 0,78 | 0,672 | 0,888 |
| J5/7 | C3AR1 + IL1R2 + IFNG + CIITA + CD3D + CTLA4 | 0,769 | 0,658 | 0,881 |
| J5/7 | C3AR1 + IL1R2 + IFNG + CIITA + CTLA4 | 0,766 | 0,656 | 0,876 |
| J5/7 | C3AR1 + IL1R2 + IFNG + CIITA + TAP2 | 0,805 | 0,713 | 0,898 |
| J5/7 | C3AR1 + IL1R2 + IFNG + CIITA + TAP2 + CD3D | 0,798 | 0,701 | 0,895 |
| J5/7 | C3AR1 + IL1R2 + IFNG + CIITA + TAP2 + CD3D + CTLA4 | 0,795 | 0,694 | 0,897 |
| J5/7 | C3AR1 + IL1R2 + IFNG + CIITA + TAP2 + CTLA4 | 0,798 | 0,697 | 0,898 |
| J5/7 | C3AR1 + IL1R2 + IFNG + CTLA4 | 0,753 | 0,626 | 0,879 |
| J5/7 | C3AR1 + IL1R2 + IFNG + CX3CR1 | 0,779 | 0,663 | 0,895 |
| J5/7 | C3AR1 + IL1R2 + IFNG + CX3CR1 + CD3D | 0,765 | 0,633 | 0,897 |
| J5/7 | C3AR1 + IL1R2 + IFNG + CX3CR1 + CD3D + CTLA4 | 0,768 | 0,64 | 0,897 |
| J5/7 | C3AR1 + IL1R2 + IFNG + CX3CR1 + CIITA | 0,796 | 0,704 | 0,887 |
| J5/7 | C3AR1 + IL1R2 + IFNG + CX3CR1 + CIITA + CD3D | 0,784 | 0,677 | 0,89 |
| J5/7 | C3AR1 + IL1R2 + IFNG + CX3CR1 + CIITA + CD3D + CTLA4 | 0,778 | 0,67 | 0,886 |
| J5/7 | C3AR1 + IL1R2 + IFNG + CX3CR1 + CIITA + CTLA4 | 0,775 | 0,667 | 0,884 |
| J5/7 | C3AR1 + IL1R2 + IFNG + CX3CR1 + CIITA + TAP2 | 0,804 | 0,709 | 0,899 |
| J5/7 | C3AR1 + IL1R2 + IFNG + CX3CR1 + CIITA + TAP2 + CD3D | 0,8 | 0,703 | 0,896 |
| J5/7 | C3AR1 + IL1R2 + IFNG + CX3CR1 + CIITA + TAP2 + CD3D + CTLA4 | 0,795 | 0,695 | 0,895 |
| J5/7 | C3AR1 + IL1R2 + IFNG + CX3CR1 + CIITA + TAP2 + CTLA4 | 0,796 | 0,696 | 0,896 |
| J5/7 | C3AR1 + IL1R2 + IFNG + CX3CR1 + CTLA4 | 0,77 | 0,643 | 0,897 |
| J5/7 | C3AR1 + IL1R2 + IFNG + CX3CR1 + TAP2 | 0,802 | 0,703 | 0,901 |
| J5/7 | C3AR1 + IL1R2 + IFNG + CX3CR1 + TAP2 + CD3D | 0,795 | 0,694 | 0,897 |
| J5/7 | C3AR1 + IL1R2 + IFNG + CX3CR1 + TAP2 + CD3D + CTLA4 | 0,791 | 0,685 | 0,896 |
| J5/7 | C3AR1 + IL1R2 + IFNG + CX3CR1 + TAP2 + CTLA4 | 0,792 | 0,687 | 0,897 |
| J5/7 | C3AR1 + IL1R2 + IFNG + S100A9 | 0,784 | 0,684 | 0,883 |
| J5/7 | C3AR1 + IL1R2 + IFNG + S100A9 + CD3D | 0,783 | 0,674 | 0,892 |
| J5/7 | C3AR1 + IL1R2 + IFNG + S100A9 + CD3D + CTLA4 | 0,783 | 0,673 | 0,892 |
| J5/7 | C3AR1 + IL1R2 + IFNG + S100A9 + CIITA | 0,786 | 0,689 | 0,882 |
| J5/7 | C3AR1 + IL1R2 + IFNG + S100A9 + CIITA + CD3D | 0,782 | 0,677 | 0,886 |
| J5/7 | C3AR1 + IL1R2 + IFNG + S100A9 + CIITA + CD3D + CTLA4 | 0,783 | 0,675 | 0,891 |
| J5/7 | C3AR1 + IL1R2 + IFNG + S100A9 + CIITA + CTLA4 | 0,771 | 0,66 | 0,882 |
| J5/7 | C3AR1 + IL1R2 + IFNG + S100A9 + CIITA + TAP2 | 0,807 | 0,716 | 0,899 |
| J5/7 | C3AR1 + IL1R2 + IFNG + S100A9 + CIITA + TAP2 + CD3D | 0,807 | 0,712 | 0,902 |
| J5/7 | C3AR1 + IL1R2 + IFNG + S100A9 + CIITA + TAP2 + CD3D + CTLA4 | 0,806 | 0,712 | 0,901 |
| J5/7 | C3AR1 + IL1R2 + IFNG + S100A9 + CIITA + TAP2 + CTLA4 | 0,807 | 0,713 | 0,901 |
| J5/7 | C3AR1 + IL1R2 + IFNG + S100A9 + CTLA4 | 0,77 | 0,655 | 0,885 |
| J5/7 | C3AR1 + IL1R2 + IFNG + S100A9 + CX3CR1 | 0,8 | 0,702 | 0,899 |
| J5/7 | C3AR1 + IL1R2 + IFNG + S100A9 + CX3CR1 + CD3D | 0,789 | 0,681 | 0,898 |
| J5/7 | C3AR1 + IL1R2 + IFNG + S100A9 + CX3CR1 + CD3D + CTLA4 | 0,789 | 0,68 | 0,899 |
| J5/7 | C3AR1 + IL1R2 + IFNG + S100A9 + CX3CR1 + CIITA | 0,804 | 0,71 | 0,899 |
| J5/7 | C3AR1 + IL1R2 + IFNG + S100A9 + CX3CR1 + CIITA + CD3D | 0,794 | 0,691 | 0,897 |
| J5/7 | C3AR1 + IL1R2 + IFNG + S100A9 + CX3CR1 + CIITA + CD3D + CTLA4 | 0,792 | 0,683 | 0,9 |
| J5/7 | C3AR1 + IL1R2 + IFNG + S100A9 + CX3CR1 + CIITA + CTLA4 | 0,79 | 0,681 | 0,898 |
| J5/7 | C3AR1 + IL1R2 + IFNG + S100A9 + CX3CR1 + CIITA + TAP2 | 0,812 | 0,721 | 0,903 |
| J5/7 | C3AR1 + IL1R2 + IFNG + S100A9 + CX3CR1 + CIITA + TAP2 + CD3D | 0,808 | 0,712 | 0,904 |
| J5/7 | C3AR1 + IL1R2 + IFNG + S100A9 + CX3CR1 + CIITA + TAP2 + CD3D + CTLA4 | 0,806 | 0,709 | 0,903 |
| J5/7 | C3AR1 + IL1R2 + IFNG + S100A9 + CX3CR1 + CIITA + TAP2 + CTLA4 | 0,809 | 0,715 | 0,903 |
| J5/7 | C3AR1 + IL1R2 + IFNG + S100A9 + CX3CR1 + CTLA4 | 0,787 | 0,675 | 0,9 |
| J5/7 | C3AR1 + IL1R2 + IFNG + S100A9 + CX3CR1 + TAP2 | 0,813 | 0,724 | 0,902 |
| J5/7 | C3AR1 + IL1R2 + IFNG + S100A9 + CX3CR1 + TAP2 + CD3D | 0,81 | 0,717 | 0,904 |
| J5/7 | C3AR1 + IL1R2 + IFNG + S100A9 + CX3CR1 + TAP2 + CD3D + CTLA4 | 0,807 | 0,712 | 0,902 |
| J5/7 | C3AR1 + IL1R2 + IFNG + S100A9 + CX3CR1 + TAP2 + CTLA4 | 0,809 | 0,716 | 0,902 |
| J5/7 | C3AR1 + IL1R2 + IFNG + S100A9 + TAP2 | 0,813 | 0,727 | 0,899 |
| J5/7 | C3AR1 + IL1R2 + IFNG + S100A9 + TAP2 + CD3D | 0,807 | 0,713 | 0,901 |
| J5/7 | C3AR1 + IL1R2 + IFNG + S100A9 + TAP2 + CD3D + CTLA4 | 0,806 | 0,712 | 0,899 |
| J5/7 | C3AR1 + IL1R2 + IFNG + S100A9 + TAP2 + CTLA4 | 0,808 | 0,718 | 0,899 |
| J5/7 | C3AR1 + IL1R2 + IFNG + TAP2 | 0,794 | 0,695 | 0,894 |
| J5/7 | C3AR1 + IL1R2 + IFNG + TAP2 + CD3D | 0,791 | 0,688 | 0,893 |
| J5/7 | C3AR1 + IL1R2 + IFNG + TAP2 + CD3D + CTLA4 | 0,797 | 0,692 | 0,901 |
| J5/7 | C3AR1 + IL1R2 + IFNG + TAP2 + CTLA4 | 0,795 | 0,69 | 0,901 |
| J5/7 | C3AR1 + IL1R2 + S100A9 | 0,798 | 0,711 | 0,885 |
| J5/7 | C3AR1 + IL1R2 + S100A9 + CD3D | 0,788 | 0,694 | 0,881 |
| J5/7 | C3AR1 + IL1R2 + S100A9 + CD3D + CTLA4 | 0,775 | 0,67 | 0,88 |
| J5/7 | C3AR1 + IL1R2 + S100A9 + CIITA | 0,8 | 0,713 | 0,887 |
| J5/7 | C3AR1 + IL1R2 + S100A9 + CIITA + CD3D | 0,788 | 0,687 | 0,888 |
| J5/7 | C3AR1 + IL1R2 + S100A9 + CIITA + CD3D + CTLA4 | 0,775 | 0,665 | 0,886 |
| J5/7 | C3AR1 + IL1R2 + S100A9 + CIITA + CTLA4 | 0,774 | 0,661 | 0,888 |
| J5/7 | C3AR1 + IL1R2 + S100A9 + CIITA + TAP2 | 0,803 | 0,716 | 0,889 |
| J5/7 | C3AR1 + IL1R2 + S100A9 + CIITA + TAP2 + CD3D | 0,8 | 0,71 | 0,891 |
| J5/7 | C3AR1 + IL1R2 + S100A9 + CIITA + TAP2 + CD3D + CTLA4 | 0,797 | 0,703 | 0,891 |
| J5/7 | C3AR1 + IL1R2 + S100A9 + CIITA + TAP2 + CTLA4 | 0,799 | 0,706 | 0,892 |
| J5/7 | C3AR1 + IL1R2 + S100A9 + CTLA4 | 0,776 | 0,668 | 0,884 |
| J5/7 | C3AR1 + IL1R2 + S100A9 + CX3CR1 | 0,805 | 0,718 | 0,892 |
| J5/7 | C3AR1 + IL1R2 + S100A9 + CX3CR1 + CD3D | 0,794 | 0,701 | 0,887 |
| J5/7 | C3AR1 + IL1R2 + S100A9 + CX3CR1 + CD3D + CTLA4 | 0,779 | 0,673 | 0,886 |
| J5/7 | C3AR1 + IL1R2 + S100A9 + CX3CR1 + CIITA | 0,799 | 0,708 | 0,891 |
| J5/7 | C3AR1 + IL1R2 + S100A9 + CX3CR1 + CIITA + CD3D | 0,787 | 0,686 | 0,888 |
| J5/7 | C3AR1 + IL1R2 + S100A9 + CX3CR1 + CIITA + CD3D + CTLA4 | 0,78 | 0,669 | 0,892 |
| J5/7 | C3AR1 + IL1R2 + S100A9 + CX3CR1 + CIITA + CTLA4 | 0,783 | 0,671 | 0,894 |
| J5/7 | C3AR1 + IL1R2 + S100A9 + CX3CR1 + CIITA + TAP2 | 0,806 | 0,721 | 0,892 |
| J5/7 | C3AR1 + IL1R2 + S100A9 + CX3CR1 + CIITA + TAP2 + CD3D | 0,802 | 0,714 | 0,889 |
| J5/7 | C3AR1 + IL1R2 + S100A9 + CX3CR1 + CIITA + TAP2 + CD3D + CTLA4 | 0,801 | 0,708 | 0,894 |
| J5/7 | C3AR1 + IL1R2 + S100A9 + CX3CR1 + CIITA + TAP2 + CTLA4 | 0,801 | 0,709 | 0,893 |
| J5/7 | C3AR1 + IL1R2 + S100A9 + CX3CR1 + CTLA4 | 0,784 | 0,676 | 0,891 |
| J5/7 | C3AR1 + IL1R2 + S100A9 + CX3CR1 + TAP2 | 0,801 | 0,719 | 0,884 |
| J5/7 | C3AR1 + IL1R2 + S100A9 + CX3CR1 + TAP2 + CD3D | 0,8 | 0,718 | 0,882 |
| J5/7 | C3AR1 + IL1R2 + S100A9 + CX3CR1 + TAP2 + CD3D + CTLA4 | 0,797 | 0,714 | 0,881 |
| J5/7 | C3AR1 + IL1R2 + S100A9 + CX3CR1 + TAP2 + CTLA4 | 0,797 | 0,713 | 0,882 |
| J5/7 | C3AR1 + IL1R2 + S100A9 + TAP2 | 0,801 | 0,719 | 0,884 |
| J5/7 | C3AR1 + IL1R2 + S100A9 + TAP2 + CD3D | 0,799 | 0,716 | 0,881 |
| J5/7 | C3AR1 + IL1R2 + S100A9 + TAP2 + CD3D + CTLA4 | 0,798 | 0,713 | 0,884 |
| J5/7 | C3AR1 + IL1R2 + S100A9 + TAP2 + CTLA4 | 0,798 | 0,712 | 0,884 |
| J5/7 | C3AR1 + IL1R2 + TAP2 | 0,801 | 0,714 | 0,889 |
| J5/7 | C3AR1 + IL1R2 + TAP2 + CD3D | 0,8 | 0,711 | 0,888 |
| J5/7 | C3AR1 + IL1R2 + TAP2 + CD3D + CTLA4 | 0,797 | 0,701 | 0,893 |
| J5/7 | C3AR1 + IL1R2 + TAP2 + CTLA4 | 0,796 | 0,703 | 0,89 |
| J5/7 | C3AR1 + S100A9 | 0,77 | 0,678 | 0,862 |
| J5/7 | C3AR1 + S100A9 + CD3D | 0,776 | 0,683 | 0,87 |
| J5/7 | C3AR1 + S100A9 + CD3D + CTLA4 | 0,764 | 0,661 | 0,867 |
| J5/7 | C3AR1 + S100A9 + CIITA | 0,77 | 0,678 | 0,862 |
| J5/7 | C3AR1 + S100A9 + CIITA + CD3D | 0,774 | 0,676 | 0,872 |
| J5/7 | C3AR1 + S100A9 + CIITA + CD3D + CTLA4 | 0,763 | 0,658 | 0,869 |
| J5/7 | C3AR1 + S100A9 + CIITA + CTLA4 | 0,755 | 0,645 | 0,864 |
| J5/7 | C3AR1 + S100A9 + CIITA + TAP2 | 0,793 | 0,708 | 0,878 |
| J5/7 | C3AR1 + S100A9 + CIITA + TAP2 + CD3D | 0,789 | 0,7 | 0,878 |
| J5/7 | C3AR1 + S100A9 + CIITA + TAP2 + CD3D + CTLA4 | 0,79 | 0,698 | 0,881 |
| J5/7 | C3AR1 + S100A9 + CIITA + TAP2 + CTLA4 | 0,79 | 0,699 | 0,881 |
| J5/7 | C3AR1 + S100A9 + CTLA4 | 0,758 | 0,653 | 0,863 |
| J5/7 | C3AR1 + S100A9 + CX3CR1 | 0,79 | 0,701 | 0,88 |
| J5/7 | C3AR1 + S100A9 + CX3CR1 + CD3D | 0,785 | 0,693 | 0,877 |
| J5/7 | C3AR1 + S100A9 + CX3CR1 + CD3D + CTLA4 | 0,775 | 0,67 | 0,881 |
| J5/7 | C3AR1 + S100A9 + CX3CR1 + CIITA | 0,794 | 0,703 | 0,884 |
| J5/7 | C3AR1 + S100A9 + CX3CR1 + CIITA + CD3D | 0,782 | 0,682 | 0,881 |
| J5/7 | C3AR1 + S100A9 + CX3CR1 + CIITA + CD3D + CTLA4 | 0,773 | 0,664 | 0,883 |
| J5/7 | C3AR1 + S100A9 + CX3CR1 + CIITA + CTLA4 | 0,773 | 0,662 | 0,884 |
| J5/7 | C3AR1 + S100A9 + CX3CR1 + CIITA + TAP2 | 0,791 | 0,705 | 0,878 |
| J5/7 | C3AR1 + S100A9 + CX3CR1 + CIITA + TAP2 + CD3D | 0,789 | 0,7 | 0,878 |
| J5/7 | C3AR1 + S100A9 + CX3CR1 + CIITA + TAP2 + CD3D + CTLA4 | 0,789 | 0,697 | 0,88 |
| J5/7 | C3AR1 + S100A9 + CX3CR1 + CIITA + TAP2 + CTLA4 | 0,788 | 0,696 | 0,88 |
| J5/7 | C3AR1 + S100A9 + CX3CR1 + CTLA4 | 0,776 | 0,67 | 0,882 |
| J5/7 | C3AR1 + S100A9 + CX3CR1 + TAP2 | 0,789 | 0,706 | 0,871 |
| J5/7 | C3AR1 + S100A9 + CX3CR1 + TAP2 + CD3D | 0,791 | 0,71 | 0,873 |
| J5/7 | C3AR1 + S100A9 + CX3CR1 + TAP2 + CD3D + CTLA4 | 0,784 | 0,698 | 0,87 |
| J5/7 | C3AR1 + S100A9 + CX3CR1 + TAP2 + CTLA4 | 0,786 | 0,7 | 0,872 |
| J5/7 | C3AR1 + S100A9 + TAP2 | 0,788 | 0,705 | 0,871 |
| J5/7 | C3AR1 + S100A9 + TAP2 + CD3D | 0,791 | 0,708 | 0,873 |
| J5/7 | C3AR1 + S100A9 + TAP2 + CD3D + CTLA4 | 0,784 | 0,698 | 0,869 |
| J5/7 | C3AR1 + S100A9 + TAP2 + CTLA4 | 0,787 | 0,702 | 0,873 |
| J5/7 | C3AR1 + TAP2 | 0,785 | 0,694 | 0,875 |
| J5/7 | C3AR1 + TAP2 + CD3D | 0,783 | 0,692 | 0,873 |
| J5/7 | C3AR1 + TAP2 + CD3D + CTLA4 | 0,775 | 0,677 | 0,873 |
| J5/7 | C3AR1 + TAP2 + CTLA4 | 0,776 | 0,681 | 0,871 |

### [Tableau 7]

**Tableau 7. Performance (AUC et intervalle de confiance à 95%, avec les bornes AUC.2.5 et AUC.97.5) de la mesure de l'expression de C3AR1, en combinaison avec un ou plusieurs autre(s) biomarqueur(s) (analyse multivariée), pour la prédiction de survenue d'une infection associée aux soins dans les 4 jours ou dans les 7 jours suivant le prélèvement d'échantillon.**

| **Délai maximum entre le prélèvement et la survenue d'infection associée aux soins** | **Biomarqueurs** | **AUC** | **AUC.2.5** | **AUC.97.5** |
|---|---|---|---|---|
| 4 jours | C3AR1 + 130360601-HERV0808 | 0,661 | 0,546 | 0,776 |
| 4 jours | C3AR1 + IL18RAP | 0,662 | 0,563 | 0,761 |
| 4 jours | C3AR1 + IFIH1 | 0,663 | 0,535 | 0,79 |
| 4 jours | C3AR1 + 121601801-HERV0492 | 0,664 | 0,563 | 0,765 |
| 4 jours | C3AR1 + 050286701-HERV0513 | 0,665 | 0,567 | 0,764 |
| 4 jours | C3AR1 + IL18 | 0,665 | 0,566 | 0,764 |
| 4 jours | C3AR1 + CD74 | 0,666 | 0,568 | 0,763 |
| 4 jours | C3AR1 + OAS3 | 0,666 | 0,567 | 0,765 |
| 4 jours | C3AR1 + CD274 | 0,669 | 0,57 | 0,768 |
| 4 jours | C3AR1 + IL18R1 | 0,671 | 0,573 | 0,769 |
| 4 jours | C3AR1 + TNFSF4 | 0,673 | 0,574 | 0,772 |
| 4 jours | C3AR1 + 011052202-HERV1033 | 0,676 | 0,578 | 0,773 |
| 4 jours | C3AR1 + 011052702-MALR1044 | 0,677 | 0,579 | 0,776 |
| 4 jours | C3AR1 + IL10 | 0,681 | 0,582 | 0,779 |
| 4 jours | C3AR1 + IL1RN | 0,683 | 0,585 | 0,782 |
| 4 jours | C3AR1 + 220247002-HERV0797 | 0,687 | 0,59 | 0,783 |
| 4 jours | C3AR1 + CX3CR1 | 0,689 | 0,593 | 0,786 |
| 4 jours | C3AR1 + 021456001-MALR1017 | 0,691 | 0,594 | 0,787 |
| 4 jours | C3AR1 + IL7R | 0,693 | 0,596 | 0,79 |
| 4 jours | C3AR1 + IL15 | 0,694 | 0,588 | 0,8 |
| 4 jours | C3AR1 + IL1R2 | 0,695 | 0,599 | 0,791 |
| 4 jours | C3AR1 + 170369402HE41 | 0,696 | 0,6 | 0,793 |
| 4 jours | C3AR1 + HP | 0,715 | 0,622 | 0,808 |
| 4 jours | C3AR1 + IFNG | 0,723 | 0,629 | 0,816 |
| 4 jours | C3AR1 + CD177 | 0,68 | 0,489 | 0,87 |
| 4 jours | C3AR1 + CD177 + CD3D + CTLA4 | 0,664 | 0,463 | 0,865 |
| 4 jours | C3AR1 + CD177 + CIITA | 0,684 | 0,493 | 0,874 |
| 4 jours | C3AR1 + CD177 + CIITA + CD3D | 0,668 | 0,472 | 0,864 |
| 4 jours | C3AR1 + CD177 + CIITA + CD3D + CTLA4 | 0,68 | 0,486 | 0,873 |
| 4 jours | C3AR1 + CD177 + CIITA + CTLA4 | 0,676 | 0,482 | 0,87 |
| 4 jours | C3AR1 + CD177 + CIITA + TAP2 | 0,684 | 0,492 | 0,875 |
| 4 jours | C3AR1 + CD177 + CIITA + TAP2 + CD3D | 0,684 | 0,492 | 0,876 |
| 4 jours | C3AR1 + CD177 + CIITA + TAP2 + CD3D + CTLA4 | 0,707 | 0,518 | 0,896 |
| 4 jours | C3AR1 + CD177 + CIITA + TAP2 + CTLA4 | 0,699 | 0,509 | 0,889 |
| 4 jours | C3AR1 + CD177 + CX3CR1 | 0,703 | 0,515 | 0,891 |
| 4 jours | C3AR1 + CD177 + CX3CR1 + CD3D | 0,695 | 0,508 | 0,883 |
| 4 jours | C3AR1 + CD177 + CX3CR1 + CD3D + CTLA4 | 0,699 | 0,511 | 0,888 |
| 4 jours | C3AR1 + CD177 + CX3CR1 + CIITA | 0,734 | 0,552 | 0,917 |
| 4 jours | C3AR1 + CD177 + CX3CR1 + CIITA + CD3D | 0,719 | 0,531 | 0,907 |
| 4 jours | C3AR1 + CD177 + CX3CR1 + CIITA + CD3D + CTLA4 | 0,715 | 0,525 | 0,905 |
| 4 jours | C3AR1 + CD177 + CX3CR1 + CIITA + CTLA4 | 0,715 | 0,524 | 0,906 |
| 4 jours | C3AR1 + CD177 + CX3CR1 + CIITA + TAP2 | 0,773 | 0,611 | 0,936 |
| 4 jours | C3AR1 + CD177 + CX3CR1 + CIITA + TAP2 + CD3D | 0,773 | 0,61 | 0,937 |
| 4 jours | C3AR1 + CD177 + CX3CR1 + CIITA + TAP2 + CD3D + CTLA4 | 0,77 | 0,605 | 0,934 |
| 4 jours | C3AR1 + CD177 + CX3CR1 + CIITA + TAP2 + CTLA4 | 0,762 | 0,594 | 0,929 |
| 4 jours | C3AR1 + CD177 + CX3CR1 + CTLA4 | 0,703 | 0,513 | 0,893 |
| 4 jours | C3AR1 + CD177 + CX3CR1 + TAP2 | 0,73 | 0,553 | 0,908 |
| 4 jours | C3AR1 + CD177 + CX3CR1 + TAP2 + CD3D | 0,746 | 0,575 | 0,917 |
| 4 jours | C3AR1 + CD177 + CX3CR1 + TAP2 + CD3D + CTLA4 | 0,758 | 0,59 | 0,926 |
| 4 jours | C3AR1 + CD177 + CX3CR1 + TAP2 + CTLA4 | 0,754 | 0,585 | 0,923 |
| 4 jours | C3AR1 + CD177 + S100A9 | 0,781 | 0,614 | 0,948 |
| 4 jours | C3AR1 + CD177 + S100A9 + CD3D | 0,781 | 0,617 | 0,946 |
| 4 jours | C3AR1 + CD177 + S100A9 + CD3D + CTLA4 | 0,781 | 0,615 | 0,947 |
| 4 jours | C3AR1 + CD177 + S100A9 + CIITA | 0,793 | 0,625 | 0,961 |
| 4 jours | C3AR1 + CD177 + S100A9 + CIITA + CD3D | 0,781 | 0,615 | 0,948 |
| 4 jours | C3AR1 + CD177 + S100A9 + CIITA + CD3D + CTLA4 | 0,801 | 0,64 | 0,961 |
| 4 jours | C3AR1 + CD177 + S100A9 + CIITA + CTLA4 | 0,797 | 0,632 | 0,962 |
| 4 jours | C3AR1 + CD177 + S100A9 + CIITA + TAP2 | 0,805 | 0,632 | 0,977 |
| 4 jours | C3AR1 + CD177 + S100A9 + CIITA + TAP2 + CD3D | 0,809 | 0,647 | 0,971 |
| 4 jours | C3AR1 + CD177 + S100A9 + CIITA + TAP2 + CD3D + CTLA4 | 0,805 | 0,642 | 0,967 |
| 4 jours | C3AR1 + CD177 + S100A9 + CIITA + TAP2 + CTLA4 | 0,816 | 0,656 | 0,976 |
| 4 jours | C3AR1 + CD177 + S100A9 + CTLA4 | 0,77 | 0,6 | 0,939 |
| 4 jours | C3AR1 + CD177 + S100A9 + CX3CR1 | 0,859 | 0,711 | 1 |
| 4 jours | C3AR1 + CD177 + S100A9 + CX3CR1 + CD3D | 0,887 | 0,755 | 1 |
| 4 jours | C3AR1 + CD177 + S100A9 + CX3CR1 + CD3D + CTLA4 | 0,879 | 0,749 | 1 |
| 4 jours | C3AR1 + CD177 + S100A9 + CX3CR1 + CIITA | 0,879 | 0,743 | 1 |
| 4 jours | C3AR1 + CD177 + S100A9 + CX3CR1 + CIITA + CD3D | 0,887 | 0,755 | 1 |
| 4 jours | C3AR1 + CD177 + S100A9 + CX3CR1 + CIITA + CD3D + CTLA4 | 0,887 | 0,762 | 1 |
| 4 jours | C3AR1 + CD177 + S100A9 + CX3CR1 + CIITA + CTLA4 | 0,871 | 0,729 | 1 |
| 4 jours | C3AR1 + CD177 + S100A9 + CX3CR1 + CIITA + TAP2 | 0,883 | 0,743 | 1 |
| 4 jours | C3AR1 + CD177 + S100A9 + CX3CR1 + CIITA + TAP2 + CD3D | 0,887 | 0,755 | 1 |
| 4 jours | C3AR1 + CD177 + S100A9 + CX3CR1 + CIITA + TAP2 + CD3D + CTLA4 | 0,887 | 0,76 | 1 |
| 4 jours | C3AR1 + CD177 + S100A9 + CX3CR1 + CIITA + TAP2 + CTLA4 | 0,879 | 0,735 | 1 |
| 4 jours | C3AR1 + CD177 + S100A9 + CX3CR1 + CTLA4 | 0,859 | 0,712 | 1 |
| 4 jours | C3AR1 + CD177 + S100A9 + CX3CR1 + TAP2 | 0,883 | 0,743 | 1 |
| 4 jours | C3AR1 + CD177 + S100A9 + CX3CR1 + TAP2 + CD3D | 0,887 | 0,755 | 1 |
| 4 jours | C3AR1 + CD177 + S100A9 + CX3CR1 + TAP2 + CD3D + CTLA4 | 0,879 | 0,751 | 1 |
| 4 jours | C3AR1 + CD177 + S100A9 + CX3CR1 + TAP2 + CTLA4 | 0,883 | 0,739 | 1 |
| 4 jours | C3AR1 + CD177 + S100A9 + TAP2 | 0,801 | 0,635 | 0,967 |
| 4 jours | C3AR1 + CD177 + S100A9 + TAP2 + CD3D | 0,801 | 0,642 | 0,959 |
| 4 jours | C3AR1 + CD177 + S100A9 + TAP2 + CD3D + CTLA4 | 0,805 | 0,648 | 0,961 |
| 4 jours | C3AR1 + CD177 + S100A9 + TAP2 + CTLA4 | 0,797 | 0,634 | 0,96 |
| 4 jours | C3AR1 + CD177 + TAP2 | 0,699 | 0,511 | 0,888 |
| 4 jours | C3AR1 + CD177 + TAP2 + CD3D | 0,695 | 0,504 | 0,886 |
| 4 jours | C3AR1 + CD177 + TAP2 + CD3D + CTLA4 | 0,703 | 0,512 | 0,894 |
| 4 jours | C3AR1 + CD177 + TAP2 + CTLA4 | 0,699 | 0,509 | 0,89 |
| 4 jours | C3AR1 + CD74 + CD177 | 0,707 | 0,521 | 0,893 |
| 4 jours | C3AR1 + CD74 + CD177 + CD3D | 0,719 | 0,536 | 0,902 |
| 4 jours | C3AR1 + CD74 + CD177 + CD3D + CTLA4 | 0,73 | 0,551 | 0,91 |
| 4 jours | C3AR1 + CD74 + CD177 + CIITA | 0,723 | 0,539 | 0,906 |
| 4 jours | C3AR1 + CD74 + CD177 + CIITA + CD3D | 0,723 | 0,542 | 0,904 |
| 4 jours | C3AR1 + CD74 + CD177 + CIITA + CD3D + CTLA4 | 0,727 | 0,546 | 0,907 |
| 4 jours | C3AR1 + CD74 + CD177 + CIITA + CTLA4 | 0,727 | 0,546 | 0,907 |
| 4 jours | C3AR1 + CD74 + CD177 + CIITA + TAP2 | 0,719 | 0,534 | 0,903 |
| 4 jours | C3AR1 + CD74 + CD177 + CIITA + TAP2 + CD3D | 0,723 | 0,541 | 0,904 |
| 4 jours | C3AR1 + CD74 + CD177 + CIITA + TAP2 + CD3D + CTLA4 | 0,742 | 0,564 | 0,92 |
| 4 jours | C3AR1 + CD74 + CD177 + CIITA + TAP2 + CTLA4 | 0,738 | 0,558 | 0,919 |
| 4 jours | C3AR1 + CD74 + CD177 + CTLA4 | 0,73 | 0,551 | 0,91 |
| 4 jours | C3AR1 + CD74 + CD177 + CX3CR1 | 0,734 | 0,553 | 0,915 |
| 4 jours | C3AR1 + CD74 + CD177 + CX3CR1 + CD3D | 0,742 | 0,567 | 0,917 |
| 4 jours | C3AR1 + CD74 + CD177 + CX3CR1 + CD3D + CTLA4 | 0,75 | 0,576 | 0,924 |
| 4 jours | C3AR1 + CD74 + CD177 + CX3CR1 + CIITA | 0,762 | 0,588 | 0,936 |
| 4 jours | C3AR1 + CD74 + CD177 + CX3CR1 + CIITA + CD3D | 0,762 | 0,592 | 0,931 |
| 4 jours | C3AR1 + CD74 + CD177 + CX3CR1 + CIITA + CD3D + CTLA4 | 0,758 | 0,586 | 0,929 |
| 4 jours | C3AR1 + CD74 + CD177 + CX3CR1 + CIITA + CTLA4 | 0,754 | 0,579 | 0,929 |
| 4 jours | C3AR1 + CD74 + CD177 + CX3CR1 + CIITA + TAP2 | 0,77 | 0,603 | 0,936 |
| 4 jours | C3AR1 + CD74 + CD177 + CX3CR1 + CIITA + TAP2 + CD3D | 0,781 | 0,617 | 0,945 |
| 4 jours | C3AR1 + CD74 + CD177 + CX3CR1 + CIITA + TAP2 + CD3D + CTLA4 | 0,785 | 0,622 | 0,948 |
| 4 jours | C3AR1 + CD74 + CD177 + CX3CR1 + CIITA + TAP2 + CTLA4 | 0,77 | 0,601 | 0,938 |
| 4 jours | C3AR1 + CD74 + CD177 + CX3CR1 + CTLA4 | 0,754 | 0,576 | 0,932 |
| 4 jours | C3AR1 + CD74 + CD177 + CX3CR1 + TAP2 | 0,777 | 0,612 | 0,943 |
| 4 jours | C3AR1 + CD74 + CD177 + CX3CR1 + TAP2 + CD3D | 0,777 | 0,61 | 0,945 |
| 4 jours | C3AR1 + CD74 + CD177 + CX3CR1 + TAP2 + CD3D + CTLA4 | 0,809 | 0,649 | 0,968 |
| 4 jours | C3AR1 + CD74 + CD177 + CX3CR1 + TAP2 + CTLA4 | 0,805 | 0,646 | 0,963 |
| 4 jours | C3AR1 + CD74 + CD177 + S100A9 | 0,777 | 0,61 | 0,945 |
| 4 jours | C3AR1 + CD74 + CD177 + S100A9 + CD3D | 0,785 | 0,622 | 0,948 |
| 4 jours | C3AR1 + CD74 + CD177 + S100A9 + CD3D + CTLA4 | 0,781 | 0,615 | 0,947 |
| 4 jours | C3AR1 + CD74 + CD177 + S100A9 + CIITA | 0,801 | 0,634 | 0,968 |
| 4 jours | C3AR1 + CD74 + CD177 + S100A9 + CIITA + CD3D | 0,789 | 0,622 | 0,956 |
| 4 jours | C3AR1 + CD74 + CD177 + S100A9 + CIITA + CD3D + CTLA4 | 0,805 | 0,644 | 0,965 |
| 4 jours | C3AR1 + CD74 + CD177 + S100A9 + CIITA + CTLA4 | 0,797 | 0,632 | 0,962 |
| 4 jours | C3AR1 + CD74 + CD177 + S100A9 + CIITA + TAP2 | 0,809 | 0,638 | 0,98 |
| 4 jours | C3AR1 + CD74 + CD177 + S100A9 + CIITA + TAP2 + CD3D | 0,809 | 0,644 | 0,973 |
| 4 jours | C3AR1 + CD74 + CD177 + S100A9 + CIITA + TAP2 + CD3D + CTLA4 | 0,805 | 0,637 | 0,972 |
| 4 jours | C3AR1 + CD74 + CD177 + S100A9 + CIITA + TAP2 + CTLA4 | 0,816 | 0,652 | 0,981 |
| 4 jours | C3AR1 + CD74 + CD177 + S100A9 + CTLA4 | 0,773 | 0,605 | 0,942 |
| 4 jours | C3AR1 + CD74 + CD177 + S100A9 + CX3CR1 | 0,871 | 0,726 | 1 |
| 4 jours | C3AR1 + CD74 + CD177 + S100A9 + CX3CR1 + CD3D | 0,887 | 0,75 | 1 |
| 4 jours | C3AR1 + CD74 + CD177 + S100A9 + CX3CR1 + CD3D + CTLA4 | 0,887 | 0,759 | 1 |
| 4 jours | C3AR1 + CD74 + CD177 + S100A9 + CX3CR1 + CIITA | 0,879 | 0,743 | 1 |
| 4 jours | C3AR1 + CD74 + CD177 + S100A9 + CX3CR1 + CIITA + CD3D | 0,887 | 0,755 | 1 |
| 4 jours | C3AR1 + CD74 + CD177 + S100A9 + CX3CR1 + CIITA + CD3D + CTLA4 | 0,883 | 0,754 | 1 |
| 4 jours | C3AR1 + CD74 + CD177 + S100A9 + CX3CR1 + CIITA + CTLA4 | 0,875 | 0,738 | 1 |
| 4 jours | C3AR1 + CD74 + CD177 + S100A9 + CX3CR1 + CIITA + TAP2 | 0,887 | 0,747 | 1 |
| 4 jours | C3AR1 + CD74 + CD177 + S100A9 + CX3CR1 + CIITA + TAP2 + CD3D | 0,887 | 0,755 | 1 |
| 4 jours | C3AR1 + CD74 + CD177 + S100A9 + CX3CR1 + CIITA + TAP2 + CD3D + CTLA4 | 0,891 | 0,767 | 1 |
| 4 jours | C3AR1 + CD74 + CD177 + S100A9 + CX3CR1 + CIITA + TAP2 + CTLA4 | 0,883 | 0,739 | 1 |
| 4 jours | C3AR1 + CD74 + CD177 + S100A9 + CX3CR1 + CTLA4 | 0,867 | 0,721 | 1 |
| 4 jours | C3AR1 + CD74 + CD177 + S100A9 + CX3CR1 + TAP2 | 0,883 | 0,743 | 1 |
| 4 jours | C3AR1 + CD74 + CD177 + S100A9 + CX3CR1 + TAP2 + CD3D | 0,887 | 0,75 | 1 |
| 4 jours | C3AR1 + CD74 + CD177 + S100A9 + CX3CR1 + TAP2 + CD3D + CTLA4 | 0,879 | 0,749 | 1 |
| 4 jours | C3AR1 + CD74 + CD177 + S100A9 + CX3CR1 + TAP2 + CTLA4 | 0,883 | 0,739 | 1 |
| 4 jours | C3AR1 + CD74 + CD177 + S100A9 + TAP2 | 0,801 | 0,635 | 0,967 |
| 4 jours | C3AR1 + CD74 + CD177 + S100A9 + TAP2 + CD3D | 0,805 | 0,647 | 0,963 |
| 4 jours | C3AR1 + CD74 + CD177 + S100A9 + TAP2 + CD3D + CTLA4 | 0,805 | 0,648 | 0,961 |
| 4 jours | C3AR1 + CD74 + CD177 + S100A9 + TAP2 + CTLA4 | 0,797 | 0,634 | 0,96 |
| 4 jours | C3AR1 + CD74 + CD177 + TAP2 | 0,719 | 0,535 | 0,903 |
| 4 jours | C3AR1 + CD74 + CD177 + TAP2 + CD3D | 0,719 | 0,535 | 0,903 |
| 4 jours | C3AR1 + CD74 + CD177 + TAP2 + CD3D + CTLA4 | 0,742 | 0,564 | 0,92 |
| 4 jours | C3AR1 + CD74 + CD177 + TAP2 + CTLA4 | 0,738 | 0,558 | 0,919 |
| 4 jours | C3AR1 + CD74 + CD3D + CTLA4 | 0,668 | 0,474 | 0,862 |
| 4 jours | C3AR1 + CD74 + CIITA + CD3D + CTLA4 | 0,672 | 0,479 | 0,865 |
| 4 jours | C3AR1 + CD74 + CIITA + CTLA4 | 0,664 | 0,47 | 0,858 |
| 4 jours | C3AR1 + CD74 + CIITA + TAP2 + CD3D + CTLA4 | 0,699 | 0,503 | 0,895 |
| 4 jours | C3AR1 + CD74 + CIITA + TAP2 + CTLA4 | 0,695 | 0,505 | 0,885 |
| 4 jours | C3AR1 + CD74 + CTLA4 | 0,668 | 0,475 | 0,861 |
| 4 jours | C3AR1 + CD74 + CX3CR1 | 0,742 | 0,552 | 0,933 |
| 4 jours | C3AR1 + CD74 + CX3CR1 + CD3D | 0,711 | 0,524 | 0,898 |
| 4 jours | C3AR1 + CD74 + CX3CR1 + CD3D + CTLA4 | 0,734 | 0,552 | 0,916 |
| 4 jours | C3AR1 + CD74 + CX3CR1 + CIITA | 0,754 | 0,568 | 0,939 |
| 4 jours | C3AR1 + CD74 + CX3CR1 + CIITA + CD3D | 0,734 | 0,545 | 0,924 |
| 4 jours | C3AR1 + CD74 + CX3CR1 + CIITA + CD3D + CTLA4 | 0,73 | 0,539 | 0,922 |
| 4 jours | C3AR1 + CD74 + CX3CR1 + CIITA + CTLA4 | 0,73 | 0,539 | 0,922 |
| 4 jours | C3AR1 + CD74 + CX3CR1 + CIITA + TAP2 | 0,777 | 0,606 | 0,949 |
| 4 jours | C3AR1 + CD74 + CX3CR1 + CIITA + TAP2 + CD3D | 0,797 | 0,635 | 0,959 |
| 4 jours | C3AR1 + CD74 + CX3CR1 + CIITA + TAP2 + CD3D + CTLA4 | 0,789 | 0,619 | 0,959 |
| 4 jours | C3AR1 + CD74 + CX3CR1 + CIITA + TAP2 + CTLA4 | 0,793 | 0,626 | 0,96 |
| 4 jours | C3AR1 + CD74 + CX3CR1 + CTLA4 | 0,734 | 0,551 | 0,918 |
| 4 jours | C3AR1 + CD74 + CX3CR1 + TAP2 | 0,773 | 0,596 | 0,951 |
| 4 jours | C3AR1 + CD74 + CX3CR1 + TAP2 + CD3D | 0,727 | 0,542 | 0,912 |
| 4 jours | C3AR1 + CD74 + CX3CR1 + TAP2 + CD3D + CTLA4 | 0,785 | 0,622 | 0,948 |
| 4 jours | C3AR1 + CD74 + CX3CR1 + TAP2 + CTLA4 | 0,781 | 0,614 | 0,948 |
| 4 jours | C3AR1 + CD74 + IFNG + CD177 | 0,727 | 0,548 | 0,905 |
| 4 jours | C3AR1 + CD74 + IFNG + CD177 + CD3D | 0,727 | 0,545 | 0,909 |
| 4 jours | C3AR1 + CD74 + IFNG + CD177 + CD3D + CTLA4 | 0,723 | 0,541 | 0,905 |
| 4 jours | C3AR1 + CD74 + IFNG + CD177 + CIITA | 0,727 | 0,548 | 0,905 |
| 4 jours | C3AR1 + CD74 + IFNG + CD177 + CIITA + CD3D | 0,727 | 0,545 | 0,909 |
| 4 jours | C3AR1 + CD74 + IFNG + CD177 + CIITA + CD3D + CTLA4 | 0,715 | 0,527 | 0,903 |
| 4 jours | C3AR1 + CD74 + IFNG + CD177 + CIITA + CTLA4 | 0,723 | 0,537 | 0,909 |
| 4 jours | C3AR1 + CD74 + IFNG + CD177 + CIITA + TAP2 | 0,723 | 0,543 | 0,902 |
| 4 jours | C3AR1 + CD74 + IFNG + CD177 + CIITA + TAP2 + CD3D | 0,707 | 0,523 | 0,891 |
| 4 jours | C3AR1 + CD74 + IFNG + CD177 + CIITA + TAP2 + CD3D + CTLA4 | 0,719 | 0,532 | 0,905 |
| 4 jours | C3AR1 + CD74 + IFNG + CD177 + CIITA + TAP2 + CTLA4 | 0,73 | 0,548 | 0,913 |
| 4 jours | C3AR1 + CD74 + IFNG + CD177 + CTLA4 | 0,723 | 0,538 | 0,907 |
| 4 jours | C3AR1 + CD74 + IFNG + CD177 + CX3CR1 | 0,855 | 0,714 | 0,997 |
| 4 jours | C3AR1 + CD74 + IFNG + CD177 + CX3CR1 + CD3D | 0,871 | 0,748 | 0,994 |
| 4 jours | C3AR1 + CD74 + IFNG + CD177 + CX3CR1 + CD3D + CTLA4 | 0,891 | 0,778 | 1 |
| 4 jours | C3AR1 + CD74 + IFNG + CD177 + CX3CR1 + CIITA | 0,879 | 0,754 | 1 |
| 4 jours | C3AR1 + CD74 + IFNG + CD177 + CX3CR1 + CIITA + CD3D | 0,887 | 0,776 | 0,998 |
| 4 jours | C3AR1 + CD74 + IFNG + CD177 + CX3CR1 + CIITA + CD3D + CTLA4 | 0,895 | 0,784 | 1 |
| 4 jours | C3AR1 + CD74 + IFNG + CD177 + CX3CR1 + CIITA + CTLA4 | 0,895 | 0,784 | 1 |
| 4 jours | C3AR1 + CD74 + IFNG + CD177 + CX3CR1 + CIITA + TAP2 | 0,871 | 0,744 | 0,998 |
| 4 jours | C3AR1 + CD74 + IFNG + CD177 + CX3CR1 + CIITA + TAP2 + CD3D | 0,875 | 0,758 | 0,992 |
| 4 jours | C3AR1 + CD74 + IFNG + CD177 + CX3CR1 + CIITA + TAP2 + CD3D + CTLA4 | 0,867 | 0,74 | 0,994 |
| 4 jours | C3AR1 + CD74 + IFNG + CD177 + CX3CR1 + CIITA + TAP2 + CTLA4 | 0,867 | 0,742 | 0,992 |
| 4 jours | C3AR1 + CD74 + IFNG + CD177 + CX3CR1 + CTLA4 | 0,898 | 0,79 | 1 |
| 4 jours | C3AR1 + CD74 + IFNG + CD177 + CX3CR1 + TAP2 | 0,852 | 0,712 | 0,992 |
| 4 jours | C3AR1 + CD74 + IFNG + CD177 + CX3CR1 + TAP2 + CD3D | 0,883 | 0,766 | 0,999 |
| 4 jours | C3AR1 + CD74 + IFNG + CD177 + CX3CR1 + TAP2 + CD3D + CTLA4 | 0,867 | 0,74 | 0,994 |
| 4 jours | C3AR1 + CD74 + IFNG + CD177 + CX3CR1 + TAP2 + CTLA4 | 0,871 | 0,748 | 0,995 |
| 4 jours | C3AR1 + CD74 + IFNG + CD177 + S100A9 | 0,789 | 0,624 | 0,954 |
| 4 jours | C3AR1 + CD74 + IFNG + CD177 + S100A9 + CD3D | 0,785 | 0,62 | 0,951 |
| 4 jours | C3AR1 + CD74 + IFNG + CD177 + S100A9 + CD3D + CTLA4 | 0,785 | 0,62 | 0,95 |
| 4 jours | C3AR1 + CD74 + IFNG + CD177 + S100A9 + CIITA | 0,793 | 0,625 | 0,961 |
| 4 jours | C3AR1 + CD74 + IFNG + CD177 + S100A9 + CIITA + CD3D | 0,793 | 0,628 | 0,958 |
| 4 jours | C3AR1 + CD74 + IFNG + CD177 + S100A9 + CIITA + CD3D + CTLA4 | 0,797 | 0,633 | 0,961 |
| 4 jours | C3AR1 + CD74 + IFNG + CD177 + S100A9 + CIITA + CTLA4 | 0,785 | 0,617 | 0,953 |
| 4 jours | C3AR1 + CD74 + IFNG + CD177 + S100A9 + CIITA + TAP2 | 0,801 | 0,628 | 0,974 |
| 4 jours | C3AR1 + CD74 + IFNG + CD177 + S100A9 + CIITA + TAP2 + CD3D | 0,801 | 0,636 | 0,966 |
| 4 jours | C3AR1 + CD74 + IFNG + CD177 + S100A9 + CIITA + TAP2 + CD3D + CTLA4 | 0,809 | 0,647 | 0,97 |
| 4 jours | C3AR1 + CD74 + IFNG + CD177 + S100A9 + CIITA + TAP2 + CTLA4 | 0,797 | 0,631 | 0,963 |
| 4 jours | C3AR1 + CD74 + IFNG + CD177 + S100A9 + CTLA4 | 0,773 | 0,605 | 0,942 |
| 4 jours | C3AR1 + CD74 + IFNG + CD177 + S100A9 + CX3CR1 | 0,938 | 0,851 | 1 |
| 4 jours | C3AR1 + CD74 + IFNG + CD177 + S100A9 + CX3CR1 + CD3D | 0,953 | 0,891 | 1 |
| 4 jours | C3AR1 + CD74 + IFNG + CD177 + S100A9 + CX3CR1 + CD3D + CTLA4 | 0,953 | 0,891 | 1 |
| 4 jours | C3AR1 + CD74 + IFNG + CD177 + S100A9 + CX3CR1 + CIITA | 0,941 | 0,857 | 1 |
| 4 jours | C3AR1 + CD74 + IFNG + CD177 + S100A9 + CX3CR1 + CIITA + CD3D | 0,953 | 0,891 | 1 |
| 4 jours | C3AR1 + CD74 + IFNG + CD177 + S100A9 + CX3CR1 + CIITA + CD3D + CTLA4 | 0,953 | 0,891 | 1 |
| 4 jours | C3AR1 + CD74 + IFNG + CD177 + S100A9 + CX3CR1 + CIITA + CTLA4 | 0,961 | 0,905 | 1 |
| 4 jours | C3AR1 + CD74 + IFNG + CD177 + S100A9 + CX3CR1 + CIITA + TAP2 | 0,934 | 0,845 | 1 |
| 4 jours | C3AR1 + CD74 + IFNG + CD177 + S100A9 + CX3CR1 + CIITA + TAP2 + CD3D | 0,957 | 0,897 | 1 |
| 4 jours | C3AR1 + CD74 + IFNG + CD177 + S100A9 + CX3CR1 + CIITA + TAP2 + CD3D + CTLA4 | 0,965 | 0,914 | 1 |
| 4 jours | C3AR1 + CD74 + IFNG + CD177 + S100A9 + CX3CR1 + CIITA + TAP2 + CTLA4 | 0,953 | 0,889 | 1 |
| 4 jours | C3AR1 + CD74 + IFNG + CD177 + S100A9 + CX3CR1 + CTLA4 | 0,945 | 0,871 | 1 |
| 4 jours | C3AR1 + CD74 + IFNG + CD177 + S100A9 + CX3CR1 + TAP2 | 0,934 | 0,845 | 1 |
| 4 jours | C3AR1 + CD74 + IFNG + CD177 + S100A9 + CX3CR1 + TAP2 + CD3D | 0,957 | 0,897 | 1 |
| 4 jours | C3AR1 + CD74 + IFNG + CD177 + S100A9 + CX3CR1 + TAP2 + CD3D + CTLA4 | 0,965 | 0,914 | 1 |
| 4 jours | C3AR1 + CD74 + IFNG + CD177 + S100A9 + CX3CR1 + TAP2 + CTLA4 | 0,938 | 0,849 | 1 |
| 4 jours | C3AR1 + CD74 + IFNG + CD177 + S100A9 + TAP2 | 0,801 | 0,635 | 0,967 |
| 4 jours | C3AR1 + CD74 + IFNG + CD177 + S100A9 + TAP2 + CD3D | 0,805 | 0,647 | 0,963 |
| 4 jours | C3AR1 + CD74 + IFNG + CD177 + S100A9 + TAP2 + CD3D + CTLA4 | 0,805 | 0,648 | 0,961 |
| 4 jours | C3AR1 + CD74 + IFNG + CD177 + S100A9 + TAP2 + CTLA4 | 0,789 | 0,626 | 0,952 |
| 4 jours | C3AR1 + CD74 + IFNG + CD177 + TAP2 | 0,719 | 0,538 | 0,899 |
| 4 jours | C3AR1 + CD74 + IFNG + CD177 + TAP2 + CD3D | 0,715 | 0,532 | 0,898 |
| 4 jours | C3AR1 + CD74 + IFNG + CD177 + TAP2 + CD3D + CTLA4 | 0,719 | 0,536 | 0,901 |
| 4 jours | C3AR1 + CD74 + IFNG + CD177 + TAP2 + CTLA4 | 0,715 | 0,53 | 0,9 |
| 4 jours | C3AR1 + CD74 + IFNG + CD3D + CTLA4 | 0,684 | 0,49 | 0,877 |
| 4 jours | C3AR1 + CD74 + IFNG + CIITA + CD3D + CTLA4 | 0,684 | 0,49 | 0,878 |
| 4 jours | C3AR1 + CD74 + IFNG + CIITA + TAP2 + CD3D + CTLA4 | 0,68 | 0,484 | 0,875 |
| 4 jours | C3AR1 + CD74 + IFNG + CTLA4 | 0,68 | 0,491 | 0,868 |
| 4 jours | C3AR1 + CD74 + IFNG + CX3CR1 | 0,855 | 0,711 | 1 |
| 4 jours | C3AR1 + CD74 + IFNG + CX3CR1 + CD3D | 0,871 | 0,748 | 0,994 |
| 4 jours | C3AR1 + CD74 + IFNG + CX3CR1 + CD3D + CTLA4 | 0,891 | 0,783 | 0,998 |
| 4 jours | C3AR1 + CD74 + IFNG + CX3CR1 + CIITA | 0,883 | 0,76 | 1 |
| 4 jours | C3AR1 + CD74 + IFNG + CX3CR1 + CIITA + CD3D | 0,883 | 0,769 | 0,996 |
| 4 jours | C3AR1 + CD74 + IFNG + CX3CR1 + CIITA + CD3D + CTLA4 | 0,898 | 0,795 | 1 |
| 4 jours | C3AR1 + CD74 + IFNG + CX3CR1 + CIITA + CTLA4 | 0,887 | 0,776 | 0,998 |
| 4 jours | C3AR1 + CD74 + IFNG + CX3CR1 + CIITA + TAP2 | 0,867 | 0,739 | 0,996 |
| 4 jours | C3AR1 + CD74 + IFNG + CX3CR1 + CIITA + TAP2 + CD3D | 0,883 | 0,77 | 0,996 |
| 4 jours | C3AR1 + CD74 + IFNG + CX3CR1 + CIITA + TAP2 + CD3D + CTLA4 | 0,891 | 0,783 | 0,998 |
| 4 jours | C3AR1 + CD74 + IFNG + CX3CR1 + CIITA + TAP2 + CTLA4 | 0,887 | 0,776 | 0,997 |
| 4 jours | C3AR1 + CD74 + IFNG + CX3CR1 + CTLA4 | 0,887 | 0,776 | 0,998 |
| 4 jours | C3AR1 + CD74 + IFNG + CX3CR1 + TAP2 | 0,852 | 0,712 | 0,992 |
| 4 jours | C3AR1 + CD74 + IFNG + CX3CR1 + TAP2 + CD3D | 0,883 | 0,766 | 0,999 |
| 4 jours | C3AR1 + CD74 + IFNG + CX3CR1 + TAP2 + CD3D + CTLA4 | 0,887 | 0,777 | 0,997 |
| 4 jours | C3AR1 + CD74 + IFNG + CX3CR1 + TAP2 + CTLA4 | 0,887 | 0,776 | 0,997 |
| 4 jours | C3AR1 + CD74 + IFNG + S100A9 | 0,797 | 0,633 | 0,961 |
| 4 jours | C3AR1 + CD74 + IFNG + S100A9 + CD3D | 0,789 | 0,626 | 0,953 |
| 4 jours | C3AR1 + CD74 + IFNG + S100A9 + CD3D + CTLA4 | 0,789 | 0,628 | 0,95 |
| 4 jours | C3AR1 + CD74 + IFNG + S100A9 + CIITA | 0,797 | 0,63 | 0,964 |
| 4 jours | C3AR1 + CD74 + IFNG + S100A9 + CIITA + CD3D | 0,785 | 0,617 | 0,953 |
| 4 jours | C3AR1 + CD74 + IFNG + S100A9 + CIITA + CD3D + CTLA4 | 0,793 | 0,629 | 0,957 |
| 4 jours | C3AR1 + CD74 + IFNG + S100A9 + CIITA + CTLA4 | 0,793 | 0,628 | 0,958 |
| 4 jours | C3AR1 + CD74 + IFNG + S100A9 + CIITA + TAP2 | 0,805 | 0,632 | 0,977 |
| 4 jours | C3AR1 + CD74 + IFNG + S100A9 + CIITA + TAP2 + CD3D | 0,793 | 0,624 | 0,962 |
| 4 jours | C3AR1 + CD74 + IFNG + S100A9 + CIITA + TAP2 + CD3D + CTLA4 | 0,805 | 0,642 | 0,967 |
| 4 jours | C3AR1 + CD74 + IFNG + S100A9 + CIITA + TAP2 + CTLA4 | 0,805 | 0,64 | 0,97 |
| 4 jours | C3AR1 + CD74 + IFNG + S100A9 + CTLA4 | 0,793 | 0,632 | 0,954 |
| 4 jours | C3AR1 + CD74 + IFNG + S100A9 + CX3CR1 | 0,922 | 0,827 | 1 |
| 4 jours | C3AR1 + CD74 + IFNG + S100A9 + CX3CR1 + CD3D | 0,938 | 0,862 | 1 |
| 4 jours | C3AR1 + CD74 + IFNG + S100A9 + CX3CR1 + CD3D + CTLA4 | 0,941 | 0,867 | 1 |
| 4 jours | C3AR1 + CD74 + IFNG + S100A9 + CX3CR1 + CIITA | 0,922 | 0,827 | 1 |
| 4 jours | C3AR1 + CD74 + IFNG + S100A9 + CX3CR1 + CIITA + CD3D | 0,949 | 0,883 | 1 |
| 4 jours | C3AR1 + CD74 + IFNG + S100A9 + CX3CR1 + CIITA + CD3D + CTLA4 | 0,945 | 0,876 | 1 |
| 4 jours | C3AR1 + CD74 + IFNG + S100A9 + CX3CR1 + CIITA + CTLA4 | 0,922 | 0,832 | 1 |
| 4 jours | C3AR1 + CD74 + IFNG + S100A9 + CX3CR1 + CIITA + TAP2 | 0,918 | 0,818 | 1 |
| 4 jours | C3AR1 + CD74 + IFNG + S100A9 + CX3CR1 + CIITA + TAP2 + CD3D | 0,945 | 0,874 | 1 |
| 4 jours | C3AR1 + CD74 + IFNG + S100A9 + CX3CR1 + CIITA + TAP2 + CD3D + CTLA4 | 0,949 | 0,877 | 1 |
| 4 jours | C3AR1 + CD74 + IFNG + S100A9 + CX3CR1 + CIITA + TAP2 + CTLA4 | 0,918 | 0,82 | 1 |
| 4 jours | C3AR1 + CD74 + IFNG + S100A9 + CX3CR1 + CTLA4 | 0,914 | 0,817 | 1 |
| 4 jours | C3AR1 + CD74 + IFNG + S100A9 + CX3CR1 + TAP2 | 0,918 | 0,818 | 1 |
| 4 jours | C3AR1 + CD74 + IFNG + S100A9 + CX3CR1 + TAP2 + CD3D | 0,941 | 0,866 | 1 |
| 4 jours | C3AR1 + CD74 + IFNG + S100A9 + CX3CR1 + TAP2 + CD3D + CTLA4 | 0,953 | 0,883 | 1 |
| 4 jours | C3AR1 + CD74 + IFNG + S100A9 + CX3CR1 + TAP2 + CTLA4 | 0,922 | 0,832 | 1 |
| 4 jours | C3AR1 + CD74 + IFNG + S100A9 + TAP2 | 0,809 | 0,647 | 0,97 |
| 4 jours | C3AR1 + CD74 + IFNG + S100A9 + TAP2 + CD3D | 0,797 | 0,633 | 0,961 |
| 4 jours | C3AR1 + CD74 + IFNG + S100A9 + TAP2 + CD3D + CTLA4 | 0,809 | 0,649 | 0,968 |
| 4 jours | C3AR1 + CD74 + IFNG + S100A9 + TAP2 + CTLA4 | 0,805 | 0,645 | 0,965 |
| 4 jours | C3AR1 + CD74 + IFNG + TAP2 + CD3D + CTLA4 | 0,684 | 0,49 | 0,877 |
| 4 jours | C3AR1 + CD74 + IFNG + TAP2 + CTLA4 | 0,684 | 0,496 | 0,871 |
| 4 jours | C3AR1 + CD74 + S100A9 | 0,797 | 0,636 | 0,958 |
| 4 jours | C3AR1 + CD74 + S100A9 + CD3D | 0,781 | 0,617 | 0,946 |
| 4 jours | C3AR1 + CD74 + S100A9 + CD3D + CTLA4 | 0,785 | 0,622 | 0,948 |
| 4 jours | C3AR1 + CD74 + S100A9 + CIITA | 0,805 | 0,639 | 0,97 |
| 4 jours | C3AR1 + CD74 + S100A9 + CIITA + CD3D | 0,789 | 0,624 | 0,954 |
| 4 jours | C3AR1 + CD74 + S100A9 + CIITA + CD3D + CTLA4 | 0,785 | 0,616 | 0,954 |
| 4 jours | C3AR1 + CD74 + S100A9 + CIITA + CTLA4 | 0,797 | 0,631 | 0,962 |
| 4 jours | C3AR1 + CD74 + S100A9 + CIITA + TAP2 | 0,809 | 0,634 | 0,983 |
| 4 jours | C3AR1 + CD74 + S100A9 + CIITA + TAP2 + CD3D | 0,793 | 0,626 | 0,96 |
| 4 jours | C3AR1 + CD74 + S100A9 + CIITA + TAP2 + CD3D + CTLA4 | 0,805 | 0,639 | 0,97 |
| 4 jours | C3AR1 + CD74 + S100A9 + CIITA + TAP2 + CTLA4 | 0,816 | 0,653 | 0,98 |
| 4 jours | C3AR1 + CD74 + S100A9 + CTLA4 | 0,789 | 0,627 | 0,951 |
| 4 jours | C3AR1 + CD74 + S100A9 + CX3CR1 | 0,867 | 0,727 | 1 |
| 4 jours | C3AR1 + CD74 + S100A9 + CX3CR1 + CD3D | 0,887 | 0,747 | 1 |
| 4 jours | C3AR1 + CD74 + S100A9 + CX3CR1 + CD3D + CTLA4 | 0,879 | 0,744 | 1 |
| 4 jours | C3AR1 + CD74 + S100A9 + CX3CR1 + CIITA | 0,871 | 0,736 | 1 |
| 4 jours | C3AR1 + CD74 + S100A9 + CX3CR1 + CIITA + CD3D | 0,883 | 0,747 | 1 |
| 4 jours | C3AR1 + CD74 + S100A9 + CX3CR1 + CIITA + CD3D + CTLA4 | 0,895 | 0,773 | 1 |
| 4 jours | C3AR1 + CD74 + S100A9 + CX3CR1 + CIITA + CTLA4 | 0,875 | 0,741 | 1 |
| 4 jours | C3AR1 + CD74 + S100A9 + CX3CR1 + CIITA + TAP2 | 0,879 | 0,743 | 1 |
| 4 jours | C3AR1 + CD74 + S100A9 + CX3CR1 + CIITA + TAP2 + CD3D | 0,883 | 0,747 | 1 |
| 4 jours | C3AR1 + CD74 + S100A9 + CX3CR1 + CIITA + TAP2 + CD3D + CTLA4 | 0,891 | 0,767 | 1 |
| 4 jours | C3AR1 + CD74 + S100A9 + CX3CR1 + CIITA + TAP2 + CTLA4 | 0,879 | 0,739 | 1 |
| 4 jours | C3AR1 + CD74 + S100A9 + CX3CR1 + CTLA4 | 0,875 | 0,737 | 1 |
| 4 jours | C3AR1 + CD74 + S100A9 + CX3CR1 + TAP2 | 0,871 | 0,726 | 1 |
| 4 jours | C3AR1 + CD74 + S100A9 + CX3CR1 + TAP2 + CD3D | 0,887 | 0,747 | 1 |
| 4 jours | C3AR1 + CD74 + S100A9 + CX3CR1 + TAP2 + CD3D + CTLA4 | 0,883 | 0,753 | 1 |
| 4 jours | C3AR1 + CD74 + S100A9 + CX3CR1 + TAP2 + CTLA4 | 0,875 | 0,73 | 1 |
| 4 jours | C3AR1 + CD74 + S100A9 + TAP2 | 0,812 | 0,651 | 0,974 |
| 4 jours | C3AR1 + CD74 + S100A9 + TAP2 + CD3D | 0,801 | 0,637 | 0,965 |
| 4 jours | C3AR1 + CD74 + S100A9 + TAP2 + CD3D + CTLA4 | 0,805 | 0,642 | 0,967 |
| 4 jours | C3AR1 + CD74 + S100A9 + TAP2 + CTLA4 | 0,816 | 0,66 | 0,973 |
| 4 jours | C3AR1 + CD74 + TAP2 + CD3D + CTLA4 | 0,695 | 0,5 | 0,891 |
| 4 jours | C3AR1 + CD74 + TAP2 + CTLA4 | 0,688 | 0,496 | 0,879 |
| 4 jours | C3AR1 + CIITA + TAP2 + CD3D + CTLA4 | 0,688 | 0,498 | 0,877 |
| 4 jours | C3AR1 + CIITA + TAP2 + CTLA4 | 0,664 | 0,469 | 0,859 |
| 4 jours | C3AR1 + CX3CR1 + CD3D + CTLA4 | 0,668 | 0,474 | 0,862 |
| 4 jours | C3AR1 + CX3CR1 + CIITA | 0,711 | 0,52 | 0,902 |
| 4 jours | C3AR1 + CX3CR1 + CIITA + CD3D | 0,676 | 0,478 | 0,873 |
| 4 jours | C3AR1 + CX3CR1 + CIITA + CD3D + CTLA4 | 0,688 | 0,492 | 0,883 |
| 4 jours | C3AR1 + CX3CR1 + CIITA + CTLA4 | 0,719 | 0,528 | 0,909 |
| 4 jours | C3AR1 + CX3CR1 + CIITA + TAP2 | 0,754 | 0,578 | 0,93 |
| 4 jours | C3AR1 + CX3CR1 + CIITA + TAP2 + CD3D | 0,75 | 0,579 | 0,921 |
| 4 jours | C3AR1 + CX3CR1 + CIITA + TAP2 + CD3D + CTLA4 | 0,75 | 0,578 | 0,922 |
| 4 jours | C3AR1 + CX3CR1 + CIITA + TAP2 + CTLA4 | 0,762 | 0,595 | 0,928 |
| 4 jours | C3AR1 + CX3CR1 + CTLA4 | 0,668 | 0,475 | 0,861 |
| 4 jours | C3AR1 + CX3CR1 + TAP2 | 0,672 | 0,482 | 0,862 |
| 4 jours | C3AR1 + CX3CR1 + TAP2 + CD3D | 0,676 | 0,484 | 0,868 |
| 4 jours | C3AR1 + CX3CR1 + TAP2 + CD3D + CTLA4 | 0,695 | 0,509 | 0,881 |
| 4 jours | C3AR1 + CX3CR1 + TAP2 + CTLA4 | 0,691 | 0,505 | 0,878 |
| 4 jours | C3AR1 + IFNG + CD177 | 0,703 | 0,517 | 0,89 |
| 4 jours | C3AR1 + IFNG + CD177 + CD3D | 0,707 | 0,523 | 0,891 |
| 4 jours | C3AR1 + IFNG + CD177 + CD3D + CTLA4 | 0,711 | 0,526 | 0,896 |
| 4 jours | C3AR1 + IFNG + CD177 + CIITA | 0,707 | 0,524 | 0,89 |
| 4 jours | C3AR1 + IFNG + CD177 + CIITA + CD3D | 0,699 | 0,514 | 0,884 |
| 4 jours | C3AR1 + IFNG + CD177 + CIITA + CD3D + CTLA4 | 0,699 | 0,509 | 0,889 |
| 4 jours | C3AR1 + IFNG + CD177 + CIITA + CTLA4 | 0,734 | 0,552 | 0,916 |
| 4 jours | C3AR1 + IFNG + CD177 + CIITA + TAP2 | 0,703 | 0,518 | 0,888 |
| 4 jours | C3AR1 + IFNG + CD177 + CIITA + TAP2 + CD3D | 0,703 | 0,518 | 0,888 |
| 4 jours | C3AR1 + IFNG + CD177 + CIITA + TAP2 + CD3D + CTLA4 | 0,703 | 0,512 | 0,895 |
| 4 jours | C3AR1 + IFNG + CD177 + CIITA + TAP2 + CTLA4 | 0,734 | 0,552 | 0,916 |
| 4 jours | C3AR1 + IFNG + CD177 + CTLA4 | 0,727 | 0,546 | 0,907 |
| 4 jours | C3AR1 + IFNG + CD177 + CX3CR1 | 0,844 | 0,696 | 0,992 |
| 4 jours | C3AR1 + IFNG + CD177 + CX3CR1 + CD3D | 0,852 | 0,716 | 0,987 |
| 4 jours | C3AR1 + IFNG + CD177 + CX3CR1 + CD3D + CTLA4 | 0,895 | 0,778 | 1 |
| 4 jours | C3AR1 + IFNG + CD177 + CX3CR1 + CIITA | 0,863 | 0,722 | 1 |
| 4 jours | C3AR1 + IFNG + CD177 + CX3CR1 + CIITA + CD3D | 0,863 | 0,734 | 0,993 |
| 4 jours | C3AR1 + IFNG + CD177 + CX3CR1 + CIITA + CD3D + CTLA4 | 0,883 | 0,757 | 1 |
| 4 jours | C3AR1 + IFNG + CD177 + CX3CR1 + CIITA + CTLA4 | 0,895 | 0,781 | 1 |
| 4 jours | C3AR1 + IFNG + CD177 + CX3CR1 + CIITA + TAP2 | 0,867 | 0,736 | 0,998 |
| 4 jours | C3AR1 + IFNG + CD177 + CX3CR1 + CIITA + TAP2 + CD3D | 0,863 | 0,732 | 0,995 |
| 4 jours | C3AR1 + IFNG + CD177 + CX3CR1 + CIITA + TAP2 + CD3D + CTLA4 | 0,879 | 0,75 | 1 |
| 4 jours | C3AR1 + IFNG + CD177 + CX3CR1 + CIITA + TAP2 + CTLA4 | 0,875 | 0,746 | 1 |
| 4 jours | C3AR1 + IFNG + CD177 + CX3CR1 + CTLA4 | 0,883 | 0,765 | 1 |
| 4 jours | C3AR1 + IFNG + CD177 + CX3CR1 + TAP2 | 0,855 | 0,722 | 0,989 |
| 4 jours | C3AR1 + IFNG + CD177 + CX3CR1 + TAP2 + CD3D | 0,859 | 0,731 | 0,987 |
| 4 jours | C3AR1 + IFNG + CD177 + CX3CR1 + TAP2 + CD3D + CTLA4 | 0,883 | 0,757 | 1 |
| 4 jours | C3AR1 + IFNG + CD177 + CX3CR1 + TAP2 + CTLA4 | 0,879 | 0,752 | 1 |
| 4 jours | C3AR1 + IFNG + CD177 + S100A9 | 0,789 | 0,624 | 0,954 |
| 4 jours | C3AR1 + IFNG + CD177 + S100A9 + CD3D | 0,781 | 0,615 | 0,947 |
| 4 jours | C3AR1 + IFNG + CD177 + S100A9 + CD3D + CTLA4 | 0,785 | 0,62 | 0,95 |
| 4 jours | C3AR1 + IFNG + CD177 + S100A9 + CIITA | 0,793 | 0,625 | 0,961 |
| 4 jours | C3AR1 + IFNG + CD177 + S100A9 + CIITA + CD3D | 0,789 | 0,624 | 0,954 |
| 4 jours | C3AR1 + IFNG + CD177 + S100A9 + CIITA + CD3D + CTLA4 | 0,785 | 0,62 | 0,951 |
| 4 jours | C3AR1 + IFNG + CD177 + S100A9 + CIITA + CTLA4 | 0,785 | 0,619 | 0,952 |
| 4 jours | C3AR1 + IFNG + CD177 + S100A9 + CIITA + TAP2 | 0,801 | 0,628 | 0,974 |
| 4 jours | C3AR1 + IFNG + CD177 + S100A9 + CIITA + TAP2 + CD3D | 0,797 | 0,636 | 0,957 |
| 4 jours | C3AR1 + IFNG + CD177 + S100A9 + CIITA + TAP2 + CD3D + CTLA4 | 0,797 | 0,635 | 0,959 |
| 4 jours | C3AR1 + IFNG + CD177 + S100A9 + CIITA + TAP2 + CTLA4 | 0,812 | 0,653 | 0,972 |
| 4 jours | C3AR1 + IFNG + CD177 + S100A9 + CTLA4 | 0,77 | 0,6 | 0,939 |
| 4 jours | C3AR1 + IFNG + CD177 + S100A9 + CX3CR1 | 0,93 | 0,838 | 1 |
| 4 jours | C3AR1 + IFNG + CD177 + S100A9 + CX3CR1 + CD3D | 0,941 | 0,866 | 1 |
| 4 jours | C3AR1 + IFNG + CD177 + S100A9 + CX3CR1 + CD3D + CTLA4 | 0,957 | 0,897 | 1 |
| 4 jours | C3AR1 + IFNG + CD177 + S100A9 + CX3CR1 + CIITA | 0,93 | 0,838 | 1 |
| 4 jours | C3AR1 + IFNG + CD177 + S100A9 + CX3CR1 + CIITA + CD3D | 0,941 | 0,866 | 1 |
| 4 jours | C3AR1 + IFNG + CD177 + S100A9 + CX3CR1 + CIITA + CD3D + CTLA4 | 0,957 | 0,897 | 1 |
| 4 jours | C3AR1 + IFNG + CD177 + S100A9 + CX3CR1 + CIITA + CTLA4 | 0,945 | 0,871 | 1 |
| 4 jours | C3AR1 + IFNG + CD177 + S100A9 + CX3CR1 + CIITA + TAP2 | 0,934 | 0,845 | 1 |
| 4 jours | C3AR1 + IFNG + CD177 + S100A9 + CX3CR1 + CIITA + TAP2 + CD3D | 0,949 | 0,882 | 1 |
| 4 jours | C3AR1 + IFNG + CD177 + S100A9 + CX3CR1 + CIITA + TAP2 + CD3D + CTLA4 | 0,953 | 0,889 | 1 |
| 4 jours | C3AR1 + IFNG + CD177 + S100A9 + CX3CR1 + CIITA + TAP2 + CTLA4 | 0,941 | 0,863 | 1 |
| 4 jours | C3AR1 + IFNG + CD177 + S100A9 + CX3CR1 + CTLA4 | 0,941 | 0,864 | 1 |
| 4 jours | C3AR1 + IFNG + CD177 + S100A9 + CX3CR1 + TAP2 | 0,934 | 0,845 | 1 |
| 4 jours | C3AR1 + IFNG + CD177 + S100A9 + CX3CR1 + TAP2 + CD3D | 0,949 | 0,882 | 1 |
| 4 jours | C3AR1 + IFNG + CD177 + S100A9 + CX3CR1 + TAP2 + CD3D + CTLA4 | 0,953 | 0,889 | 1 |
| 4 jours | C3AR1 + IFNG + CD177 + S100A9 + CX3CR1 + TAP2 + CTLA4 | 0,938 | 0,852 | 1 |
| 4 jours | C3AR1 + IFNG + CD177 + S100A9 + TAP2 | 0,801 | 0,635 | 0,967 |
| 4 jours | C3AR1 + IFNG + CD177 + S100A9 + TAP2 + CD3D | 0,801 | 0,642 | 0,959 |
| 4 jours | C3AR1 + IFNG + CD177 + S100A9 + TAP2 + CD3D + CTLA4 | 0,805 | 0,648 | 0,961 |
| 4 jours | C3AR1 + IFNG + CD177 + S100A9 + TAP2 + CTLA4 | 0,785 | 0,621 | 0,949 |
| 4 jours | C3AR1 + IFNG + CD177 + TAP2 | 0,719 | 0,535 | 0,902 |
| 4 jours | C3AR1 + IFNG + CD177 + TAP2 + CD3D | 0,719 | 0,536 | 0,902 |
| 4 jours | C3AR1 + IFNG + CD177 + TAP2 + CD3D + CTLA4 | 0,707 | 0,521 | 0,893 |
| 4 jours | C3AR1 + IFNG + CD177 + TAP2 + CTLA4 | 0,727 | 0,546 | 0,907 |
| 4 jours | C3AR1 + IFNG + CD3D | 0,684 | 0,491 | 0,876 |
| 4 jours | C3AR1 + IFNG + CIITA + CTLA4 | 0,684 | 0,495 | 0,873 |
| 4 jours | C3AR1 + IFNG + CIITA + TAP2 | 0,668 | 0,471 | 0,865 |
| 4 jours | C3AR1 + IFNG + CIITA + TAP2 + CD3D | 0,668 | 0,471 | 0,865 |
| 4 jours | C3AR1 + IFNG + CIITA + TAP2 + CTLA4 | 0,691 | 0,503 | 0,88 |
| 4 jours | C3AR1 + IFNG + CTLA4 | 0,668 | 0,477 | 0,859 |
| 4 jours | C3AR1 + IFNG + CX3CR1 | 0,84 | 0,685 | 0,994 |
| 4 jours | C3AR1 + IFNG + CX3CR1 + CD3D | 0,852 | 0,716 | 0,987 |
| 4 jours | C3AR1 + IFNG + CX3CR1 + CD3D + CTLA4 | 0,879 | 0,762 | 0,996 |
| 4 jours | C3AR1 + IFNG + CX3CR1 + CIITA | 0,863 | 0,723 | 1 |
| 4 jours | C3AR1 + IFNG + CX3CR1 + CIITA + CD3D | 0,863 | 0,734 | 0,993 |
| 4 jours | C3AR1 + IFNG + CX3CR1 + CIITA + CD3D + CTLA4 | 0,891 | 0,779 | 1 |
| 4 jours | C3AR1 + IFNG + CX3CR1 + CIITA + CTLA4 | 0,883 | 0,767 | 0,998 |
| 4 jours | C3AR1 + IFNG + CX3CR1 + CIITA + TAP2 | 0,867 | 0,736 | 0,998 |
| 4 jours | C3AR1 + IFNG + CX3CR1 + CIITA + TAP2 + CD3D | 0,863 | 0,733 | 0,993 |
| 4 jours | C3AR1 + IFNG + CX3CR1 + CIITA + TAP2 + CD3D + CTLA4 | 0,883 | 0,768 | 0,997 |
| 4 jours | C3AR1 + IFNG + CX3CR1 + CIITA + TAP2 + CTLA4 | 0,887 | 0,774 | 1 |
| 4 jours | C3AR1 + IFNG + CX3CR1 + CTLA4 | 0,867 | 0,745 | 0,99 |
| 4 jours | C3AR1 + IFNG + CX3CR1 + TAP2 | 0,855 | 0,721 | 0,99 |
| 4 jours | C3AR1 + IFNG + CX3CR1 + TAP2 + CD3D | 0,859 | 0,731 | 0,987 |
| 4 jours | C3AR1 + IFNG + CX3CR1 + TAP2 + CD3D + CTLA4 | 0,879 | 0,76 | 0,998 |
| 4 jours | C3AR1 + IFNG + CX3CR1 + TAP2 + CTLA4 | 0,875 | 0,753 | 0,997 |
| 4 jours | C3AR1 + IFNG + S100A9 | 0,801 | 0,637 | 0,964 |
| 4 jours | C3AR1 + IFNG + S100A9 + CD3D | 0,781 | 0,613 | 0,95 |
| 4 jours | C3AR1 + IFNG + S100A9 + CD3D + CTLA4 | 0,797 | 0,639 | 0,955 |
| 4 jours | C3AR1 + IFNG + S100A9 + CIITA | 0,797 | 0,629 | 0,965 |
| 4 jours | C3AR1 + IFNG + S100A9 + CIITA + CD3D | 0,77 | 0,598 | 0,941 |
| 4 jours | C3AR1 + IFNG + S100A9 + CIITA + CD3D + CTLA4 | 0,777 | 0,609 | 0,946 |
| 4 jours | C3AR1 + IFNG + S100A9 + CIITA + CTLA4 | 0,777 | 0,606 | 0,948 |
| 4 jours | C3AR1 + IFNG + S100A9 + CIITA + TAP2 | 0,809 | 0,639 | 0,979 |
| 4 jours | C3AR1 + IFNG + S100A9 + CIITA + TAP2 + CD3D | 0,789 | 0,621 | 0,957 |
| 4 jours | C3AR1 + IFNG + S100A9 + CIITA + TAP2 + CD3D + CTLA4 | 0,789 | 0,62 | 0,958 |
| 4 jours | C3AR1 + IFNG + S100A9 + CIITA + TAP2 + CTLA4 | 0,797 | 0,632 | 0,962 |
| 4 jours | C3AR1 + IFNG + S100A9 + CTLA4 | 0,793 | 0,632 | 0,954 |
| 4 jours | C3AR1 + IFNG + S100A9 + CX3CR1 | 0,922 | 0,827 | 1 |
| 4 jours | C3AR1 + IFNG + S100A9 + CX3CR1 + CD3D | 0,934 | 0,854 | 1 |
| 4 jours | C3AR1 + IFNG + S100A9 + CX3CR1 + CD3D + CTLA4 | 0,938 | 0,861 | 1 |
| 4 jours | C3AR1 + IFNG + S100A9 + CX3CR1 + CIITA | 0,918 | 0,82 | 1 |
| 4 jours | C3AR1 + IFNG + S100A9 + CX3CR1 + CIITA + CD3D | 0,934 | 0,852 | 1 |
| 4 jours | C3AR1 + IFNG + S100A9 + CX3CR1 + CIITA + CD3D + CTLA4 | 0,926 | 0,837 | 1 |
| 4 jours | C3AR1 + IFNG + S100A9 + CX3CR1 + CIITA + CTLA4 | 0,922 | 0,832 | 1 |
| 4 jours | C3AR1 + IFNG + S100A9 + CX3CR1 + CIITA + TAP2 | 0,918 | 0,82 | 1 |
| 4 jours | C3AR1 + IFNG + S100A9 + CX3CR1 + CIITA + TAP2 + CD3D | 0,938 | 0,86 | 1 |
| 4 jours | C3AR1 + IFNG + S100A9 + CX3CR1 + CIITA + TAP2 + CD3D + CTLA4 | 0,938 | 0,858 | 1 |
| 4 jours | C3AR1 + IFNG + S100A9 + CX3CR1 + CIITA + TAP2 + CTLA4 | 0,922 | 0,827 | 1 |
| 4 jours | C3AR1 + IFNG + S100A9 + CX3CR1 + CTLA4 | 0,918 | 0,825 | 1 |
| 4 jours | C3AR1 + IFNG + S100A9 + CX3CR1 + TAP2 | 0,918 | 0,82 | 1 |
| 4 jours | C3AR1 + IFNG + S100A9 + CX3CR1 + TAP2 + CD3D | 0,941 | 0,866 | 1 |
| 4 jours | C3AR1 + IFNG + S100A9 + CX3CR1 + TAP2 + CD3D + CTLA4 | 0,941 | 0,865 | 1 |
| 4 jours | C3AR1 + IFNG + S100A9 + CX3CR1 + TAP2 + CTLA4 | 0,922 | 0,832 | 1 |
| 4 jours | C3AR1 + IFNG + S100A9 + TAP2 | 0,809 | 0,647 | 0,97 |
| 4 jours | C3AR1 + IFNG + S100A9 + TAP2 + CD3D | 0,797 | 0,631 | 0,963 |
| 4 jours | C3AR1 + IFNG + S100A9 + TAP2 + CD3D + CTLA4 | 0,801 | 0,637 | 0,965 |
| 4 jours | C3AR1 + IFNG + S100A9 + TAP2 + CTLA4 | 0,805 | 0,645 | 0,965 |
| 4 jours | C3AR1 + IFNG + TAP2 | 0,68 | 0,484 | 0,875 |
| 4 jours | C3AR1 + IFNG + TAP2 + CD3D | 0,691 | 0,496 | 0,886 |
| 4 jours | C3AR1 + IFNG + TAP2 + CTLA4 | 0,668 | 0,477 | 0,859 |
| 4 jours | C3AR1 + IL1R2 + CD177 | 0,676 | 0,483 | 0,869 |
| 4 jours | C3AR1 + IL1R2 + CD177 + CD3D | 0,691 | 0,501 | 0,882 |
| 4 jours | C3AR1 + IL1R2 + CD177 + CD3D + CTLA4 | 0,699 | 0,508 | 0,89 |
| 4 jours | C3AR1 + IL1R2 + CD177 + CIITA | 0,734 | 0,554 | 0,915 |
| 4 jours | C3AR1 + IL1R2 + CD177 + CIITA + CD3D | 0,734 | 0,551 | 0,918 |
| 4 jours | C3AR1 + IL1R2 + CD177 + CIITA + CD3D + CTLA4 | 0,719 | 0,535 | 0,902 |
| 4 jours | C3AR1 + IL1R2 + CD177 + CIITA + CTLA4 | 0,703 | 0,515 | 0,891 |
| 4 jours | C3AR1 + IL1R2 + CD177 + CIITA + TAP2 | 0,715 | 0,525 | 0,905 |
| 4 jours | C3AR1 + IL1R2 + CD177 + CIITA + TAP2 + CD3D | 0,742 | 0,561 | 0,924 |
| 4 jours | C3AR1 + IL1R2 + CD177 + CIITA + TAP2 + CD3D + CTLA4 | 0,723 | 0,539 | 0,907 |
| 4 jours | C3AR1 + IL1R2 + CD177 + CIITA + TAP2 + CTLA4 | 0,727 | 0,544 | 0,91 |
| 4 jours | C3AR1 + IL1R2 + CD177 + CTLA4 | 0,691 | 0,499 | 0,884 |
| 4 jours | C3AR1 + IL1R2 + CD177 + CX3CR1 | 0,691 | 0,501 | 0,881 |
| 4 jours | C3AR1 + IL1R2 + CD177 + CX3CR1 + CD3D | 0,699 | 0,51 | 0,888 |
| 4 jours | C3AR1 + IL1R2 + CD177 + CX3CR1 + CD3D + CTLA4 | 0,688 | 0,493 | 0,882 |
| 4 jours | C3AR1 + IL1R2 + CD177 + CX3CR1 + CIITA | 0,77 | 0,597 | 0,942 |
| 4 jours | C3AR1 + IL1R2 + CD177 + CX3CR1 + CIITA + CD3D | 0,715 | 0,519 | 0,911 |
| 4 jours | C3AR1 + IL1R2 + CD177 + CX3CR1 + CIITA + CD3D + CTLA4 | 0,715 | 0,519 | 0,911 |
| 4 jours | C3AR1 + IL1R2 + CD177 + CX3CR1 + CIITA + CTLA4 | 0,711 | 0,52 | 0,902 |
| 4 jours | C3AR1 + IL1R2 + CD177 + CX3CR1 + CIITA + TAP2 | 0,777 | 0,615 | 0,939 |
| 4 jours | C3AR1 + IL1R2 + CD177 + CX3CR1 + CIITA + TAP2 + CD3D | 0,789 | 0,629 | 0,949 |
| 4 jours | C3AR1 + IL1R2 + CD177 + CX3CR1 + CIITA + TAP2 + CD3D + CTLA4 | 0,777 | 0,612 | 0,942 |
| 4 jours | C3AR1 + IL1R2 + CD177 + CX3CR1 + CIITA + TAP2 + CTLA4 | 0,781 | 0,62 | 0,943 |
| 4 jours | C3AR1 + IL1R2 + CD177 + CX3CR1 + CTLA4 | 0,711 | 0,522 | 0,9 |
| 4 jours | C3AR1 + IL1R2 + CD177 + CX3CR1 + TAP2 | 0,727 | 0,547 | 0,906 |
| 4 jours | C3AR1 + IL1R2 + CD177 + CX3CR1 + TAP2 + CD3D | 0,734 | 0,559 | 0,909 |
| 4 jours | C3AR1 + IL1R2 + CD177 + CX3CR1 + TAP2 + CD3D + CTLA4 | 0,734 | 0,56 | 0,908 |
| 4 jours | C3AR1 + IL1R2 + CD177 + CX3CR1 + TAP2 + CTLA4 | 0,734 | 0,56 | 0,908 |
| 4 jours | C3AR1 + IL1R2 + CD177 + S100A9 | 0,82 | 0,663 | 0,978 |
| 4 jours | C3AR1 + IL1R2 + CD177 + S100A9 + CD3D | 0,824 | 0,676 | 0,972 |
| 4 jours | C3AR1 + IL1R2 + CD177 + S100A9 + CD3D + CTLA4 | 0,82 | 0,67 | 0,971 |
| 4 jours | C3AR1 + IL1R2 + CD177 + S100A9 + CIITA | 0,816 | 0,658 | 0,975 |
| 4 jours | C3AR1 + IL1R2 + CD177 + S100A9 + CIITA + CD3D | 0,832 | 0,686 | 0,978 |
| 4 jours | C3AR1 + IL1R2 + CD177 + S100A9 + CIITA + CD3D + CTLA4 | 0,836 | 0,692 | 0,979 |
| 4 jours | C3AR1 + IL1R2 + CD177 + S100A9 + CIITA + CTLA4 | 0,828 | 0,677 | 0,979 |
| 4 jours | C3AR1 + IL1R2 + CD177 + S100A9 + CIITA + TAP2 | 0,832 | 0,679 | 0,985 |
| 4 jours | C3AR1 + IL1R2 + CD177 + S100A9 + CIITA + TAP2 + CD3D | 0,832 | 0,688 | 0,976 |
| 4 jours | C3AR1 + IL1R2 + CD177 + S100A9 + CIITA + TAP2 + CD3D + CTLA4 | 0,844 | 0,705 | 0,983 |
| 4 jours | C3AR1 + IL1R2 + CD177 + S100A9 + CIITA + TAP2 + CTLA4 | 0,828 | 0,677 | 0,979 |
| 4 jours | C3AR1 + IL1R2 + CD177 + S100A9 + CTLA4 | 0,828 | 0,677 | 0,979 |
| 4 jours | C3AR1 + IL1R2 + CD177 + S100A9 + CX3CR1 | 0,859 | 0,716 | 1 |
| 4 jours | C3AR1 + IL1R2 + CD177 + S100A9 + CX3CR1 + CD3D | 0,891 | 0,766 | 1 |
| 4 jours | C3AR1 + IL1R2 + CD177 + S100A9 + CX3CR1 + CD3D + CTLA4 | 0,887 | 0,764 | 1 |
| 4 jours | C3AR1 + IL1R2 + CD177 + S100A9 + CX3CR1 + CIITA | 0,867 | 0,731 | 1 |
| 4 jours | C3AR1 + IL1R2 + CD177 + S100A9 + CX3CR1 + CIITA + CD3D | 0,895 | 0,774 | 1 |
| 4 jours | C3AR1 + IL1R2 + CD177 + S100A9 + CX3CR1 + CIITA + CD3D + CTLA4 | 0,895 | 0,779 | 1 |
| 4 jours | C3AR1 + IL1R2 + CD177 + S100A9 + CX3CR1 + CIITA + CTLA4 | 0,867 | 0,728 | 1 |
| 4 jours | C3AR1 + IL1R2 + CD177 + S100A9 + CX3CR1 + CIITA + TAP2 | 0,883 | 0,751 | 1 |
| 4 jours | C3AR1 + IL1R2 + CD177 + S100A9 + CX3CR1 + CIITA + TAP2 + CD3D | 0,906 | 0,796 | 1 |
| 4 jours | C3AR1 + IL1R2 + CD177 + S100A9 + CX3CR1 + CIITA + TAP2 + CD3D + CTLA4 | 0,898 | 0,79 | 1 |
| 4 jours | C3AR1 + IL1R2 + CD177 + S100A9 + CX3CR1 + CIITA + TAP2 + CTLA4 | 0,883 | 0,751 | 1 |
| 4 jours | C3AR1 + IL1R2 + CD177 + S100A9 + CX3CR1 + CTLA4 | 0,863 | 0,722 | 1 |
| 4 jours | C3AR1 + IL1R2 + CD177 + S100A9 + CX3CR1 + TAP2 | 0,871 | 0,735 | 1 |
| 4 jours | C3AR1 + IL1R2 + CD177 + S100A9 + CX3CR1 + TAP2 + CD3D | 0,898 | 0,781 | 1 |
| 4 jours | C3AR1 + IL1R2 + CD177 + S100A9 + CX3CR1 + TAP2 + CD3D + CTLA4 | 0,895 | 0,782 | 1 |
| 4 jours | C3AR1 + IL1R2 + CD177 + S100A9 + CX3CR1 + TAP2 + CTLA4 | 0,875 | 0,737 | 1 |
| 4 jours | C3AR1 + IL1R2 + CD177 + S100A9 + TAP2 | 0,832 | 0,679 | 0,985 |
| 4 jours | C3AR1 + IL1R2 + CD177 + S100A9 + TAP2 + CD3D | 0,832 | 0,69 | 0,975 |
| 4 jours | C3AR1 + IL1R2 + CD177 + S100A9 + TAP2 + CD3D + CTLA4 | 0,848 | 0,712 | 0,983 |
| 4 jours | C3AR1 + IL1R2 + CD177 + S100A9 + TAP2 + CTLA4 | 0,82 | 0,667 | 0,974 |
| 4 jours | C3AR1 + IL1R2 + CD177 + TAP2 | 0,707 | 0,512 | 0,902 |
| 4 jours | C3AR1 + IL1R2 + CD177 + TAP2 + CD3D | 0,707 | 0,513 | 0,902 |
| 4 jours | C3AR1 + IL1R2 + CD177 + TAP2 + CD3D + CTLA4 | 0,73 | 0,551 | 0,91 |
| 4 jours | C3AR1 + IL1R2 + CD177 + TAP2 + CTLA4 | 0,73 | 0,552 | 0,909 |
| 4 jours | C3AR1 + IL1R2 + CD74 | 0,666 | 0,568 | 0,763 |
| 4 jours | C3AR1 + IL1R2 + CD74 + CD177 | 0,723 | 0,541 | 0,904 |
| 4 jours | C3AR1 + IL1R2 + CD74 + CD177 + CD3D | 0,75 | 0,579 | 0,921 |
| 4 jours | C3AR1 + IL1R2 + CD74 + CD177 + CD3D + CTLA4 | 0,758 | 0,589 | 0,926 |
| 4 jours | C3AR1 + IL1R2 + CD74 + CD177 + CIITA | 0,73 | 0,549 | 0,912 |
| 4 jours | C3AR1 + IL1R2 + CD74 + CD177 + CIITA + CD3D | 0,738 | 0,565 | 0,912 |
| 4 jours | C3AR1 + IL1R2 + CD74 + CD177 + CIITA + CD3D + CTLA4 | 0,742 | 0,569 | 0,915 |
| 4 jours | C3AR1 + IL1R2 + CD74 + CD177 + CIITA + CTLA4 | 0,746 | 0,573 | 0,919 |
| 4 jours | C3AR1 + IL1R2 + CD74 + CD177 + CIITA + TAP2 | 0,738 | 0,562 | 0,915 |
| 4 jours | C3AR1 + IL1R2 + CD74 + CD177 + CIITA + TAP2 + CD3D | 0,742 | 0,568 | 0,916 |
| 4 jours | C3AR1 + IL1R2 + CD74 + CD177 + CIITA + TAP2 + CD3D + CTLA4 | 0,754 | 0,585 | 0,922 |
| 4 jours | C3AR1 + IL1R2 + CD74 + CD177 + CIITA + TAP2 + CTLA4 | 0,734 | 0,557 | 0,912 |
| 4 jours | C3AR1 + IL1R2 + CD74 + CD177 + CTLA4 | 0,738 | 0,561 | 0,916 |
| 4 jours | C3AR1 + IL1R2 + CD74 + CD177 + CX3CR1 | 0,738 | 0,561 | 0,915 |
| 4 jours | C3AR1 + IL1R2 + CD74 + CD177 + CX3CR1 + CD3D | 0,758 | 0,588 | 0,927 |
| 4 jours | C3AR1 + IL1R2 + CD74 + CD177 + CX3CR1 + CD3D + CTLA4 | 0,758 | 0,586 | 0,929 |
| 4 jours | C3AR1 + IL1R2 + CD74 + CD177 + CX3CR1 + CIITA | 0,746 | 0,568 | 0,924 |
| 4 jours | C3AR1 + IL1R2 + CD74 + CD177 + CX3CR1 + CIITA + CD3D | 0,762 | 0,593 | 0,931 |
| 4 jours | C3AR1 + IL1R2 + CD74 + CD177 + CX3CR1 + CIITA + CD3D + CTLA4 | 0,766 | 0,601 | 0,93 |
| 4 jours | C3AR1 + IL1R2 + CD74 + CD177 + CX3CR1 + CIITA + CTLA4 | 0,738 | 0,561 | 0,916 |
| 4 jours | C3AR1 + IL1R2 + CD74 + CD177 + CX3CR1 + CIITA + TAP2 | 0,77 | 0,602 | 0,937 |
| 4 jours | C3AR1 + IL1R2 + CD74 + CD177 + CX3CR1 + CIITA + TAP2 + CD3D | 0,789 | 0,625 | 0,953 |
| 4 jours | C3AR1 + IL1R2 + CD74 + CD177 + CX3CR1 + CIITA + TAP2 + CD3D + CTLA4 | 0,789 | 0,625 | 0,953 |
| 4 jours | C3AR1 + IL1R2 + CD74 + CD177 + CX3CR1 + CIITA + TAP2 + CTLA4 | 0,781 | 0,618 | 0,944 |
| 4 jours | C3AR1 + IL1R2 + CD74 + CD177 + CX3CR1 + CTLA4 | 0,75 | 0,573 | 0,927 |
| 4 jours | C3AR1 + IL1R2 + CD74 + CD177 + CX3CR1 + TAP2 | 0,777 | 0,611 | 0,944 |
| 4 jours | C3AR1 + IL1R2 + CD74 + CD177 + CX3CR1 + TAP2 + CD3D | 0,777 | 0,614 | 0,941 |
| 4 jours | C3AR1 + IL1R2 + CD74 + CD177 + CX3CR1 + TAP2 + CD3D + CTLA4 | 0,812 | 0,656 | 0,969 |
| 4 jours | C3AR1 + IL1R2 + CD74 + CD177 + CX3CR1 + TAP2 + CTLA4 | 0,797 | 0,638 | 0,955 |
| 4 jours | C3AR1 + IL1R2 + CD74 + CD177 + S100A9 | 0,828 | 0,674 | 0,982 |
| 4 jours | C3AR1 + IL1R2 + CD74 + CD177 + S100A9 + CD3D | 0,828 | 0,683 | 0,973 |
| 4 jours | C3AR1 + IL1R2 + CD74 + CD177 + S100A9 + CD3D + CTLA4 | 0,828 | 0,683 | 0,973 |
| 4 jours | C3AR1 + IL1R2 + CD74 + CD177 + S100A9 + CIITA | 0,828 | 0,676 | 0,98 |
| 4 jours | C3AR1 + IL1R2 + CD74 + CD177 + S100A9 + CIITA + CD3D | 0,824 | 0,676 | 0,972 |
| 4 jours | C3AR1 + IL1R2 + CD74 + CD177 + S100A9 + CIITA + CD3D + CTLA4 | 0,824 | 0,676 | 0,972 |
| 4 jours | C3AR1 + IL1R2 + CD74 + CD177 + S100A9 + CIITA + CTLA4 | 0,828 | 0,677 | 0,979 |
| 4 jours | C3AR1 + IL1R2 + CD74 + CD177 + S100A9 + CIITA + TAP2 | 0,828 | 0,674 | 0,982 |
| 4 jours | C3AR1 + IL1R2 + CD74 + CD177 + S100A9 + CIITA + TAP2 + CD3D | 0,836 | 0,694 | 0,978 |
| 4 jours | C3AR1 + IL1R2 + CD74 + CD177 + S100A9 + CIITA + TAP2 + CD3D + CTLA4 | 0,844 | 0,705 | 0,983 |
| 4 jours | C3AR1 + IL1R2 + CD74 + CD177 + S100A9 + CIITA + TAP2 + CTLA4 | 0,828 | 0,677 | 0,979 |
| 4 jours | C3AR1 + IL1R2 + CD74 + CD177 + S100A9 + CTLA4 | 0,828 | 0,677 | 0,979 |
| 4 jours | C3AR1 + IL1R2 + CD74 + CD177 + S100A9 + CX3CR1 | 0,859 | 0,713 | 1 |
| 4 jours | C3AR1 + IL1R2 + CD74 + CD177 + S100A9 + CX3CR1 + CD3D | 0,891 | 0,766 | 1 |
| 4 jours | C3AR1 + IL1R2 + CD74 + CD177 + S100A9 + CX3CR1 + CD3D + CTLA4 | 0,895 | 0,775 | 1 |
| 4 jours | C3AR1 + IL1R2 + CD74 + CD177 + S100A9 + CX3CR1 + CIITA | 0,867 | 0,734 | 1 |
| 4 jours | C3AR1 + IL1R2 + CD74 + CD177 + S100A9 + CX3CR1 + CIITA + CD3D | 0,902 | 0,788 | 1 |
| 4 jours | C3AR1 + IL1R2 + CD74 + CD177 + S100A9 + CX3CR1 + CIITA + CD3D + CTLA4 | 0,895 | 0,782 | 1 |
| 4 jours | C3AR1 + IL1R2 + CD74 + CD177 + S100A9 + CX3CR1 + CIITA + CTLA4 | 0,871 | 0,74 | 1 |
| 4 jours | C3AR1 + IL1R2 + CD74 + CD177 + S100A9 + CX3CR1 + CIITA + TAP2 | 0,887 | 0,762 | 1 |
| 4 jours | C3AR1 + IL1R2 + CD74 + CD177 + S100A9 + CX3CR1 + CIITA + TAP2 + CD3D | 0,902 | 0,792 | 1 |
| 4 jours | C3AR1 + IL1R2 + CD74 + CD177 + S100A9 + CX3CR1 + CIITA + TAP2 + CD3D + CTLA4 | 0,906 | 0,804 | 1 |
| 4 jours | C3AR1 + IL1R2 + CD74 + CD177 + S100A9 + CX3CR1 + CIITA + TAP2 + CTLA4 | 0,887 | 0,759 | 1 |
| 4 jours | C3AR1 + IL1R2 + CD74 + CD177 + S100A9 + CX3CR1 + CTLA4 | 0,863 | 0,722 | 1 |
| 4 jours | C3AR1 + IL1R2 + CD74 + CD177 + S100A9 + CX3CR1 + TAP2 | 0,871 | 0,735 | 1 |
| 4 jours | C3AR1 + IL1R2 + CD74 + CD177 + S100A9 + CX3CR1 + TAP2 + CD3D | 0,898 | 0,781 | 1 |
| 4 jours | C3AR1 + IL1R2 + CD74 + CD177 + S100A9 + CX3CR1 + TAP2 + CD3D + CTLA4 | 0,891 | 0,772 | 1 |
| 4 jours | C3AR1 + IL1R2 + CD74 + CD177 + S100A9 + CX3CR1 + TAP2 + CTLA4 | 0,875 | 0,737 | 1 |
| 4 jours | C3AR1 + IL1R2 + CD74 + CD177 + S100A9 + TAP2 | 0,824 | 0,669 | 0,979 |
| 4 jours | C3AR1 + IL1R2 + CD74 + CD177 + S100A9 + TAP2 + CD3D | 0,836 | 0,694 | 0,978 |
| 4 jours | C3AR1 + IL1R2 + CD74 + CD177 + S100A9 + TAP2 + CD3D + CTLA4 | 0,848 | 0,712 | 0,983 |
| 4 jours | C3AR1 + IL1R2 + CD74 + CD177 + S100A9 + TAP2 + CTLA4 | 0,828 | 0,677 | 0,979 |
| 4 jours | C3AR1 + IL1R2 + CD74 + CD177 + TAP2 | 0,727 | 0,544 | 0,909 |
| 4 jours | C3AR1 + IL1R2 + CD74 + CD177 + TAP2 + CD3D | 0,746 | 0,573 | 0,92 |
| 4 jours | C3AR1 + IL1R2 + CD74 + CD177 + TAP2 + CD3D + CTLA4 | 0,734 | 0,556 | 0,913 |
| 4 jours | C3AR1 + IL1R2 + CD74 + CD177 + TAP2 + CTLA4 | 0,738 | 0,56 | 0,916 |
| 4 jours | C3AR1 + IL1R2 + CD74 + CD3D | 0,672 | 0,48 | 0,863 |
| 4 jours | C3AR1 + IL1R2 + CD74 + CD3D + CTLA4 | 0,703 | 0,52 | 0,886 |
| 4 jours | C3AR1 + IL1R2 + CD74 + CIITA | 0,691 | 0,503 | 0,88 |
| 4 jours | C3AR1 + IL1R2 + CD74 + CIITA + CD3D | 0,684 | 0,495 | 0,873 |
| 4 jours | C3AR1 + IL1R2 + CD74 + CIITA + CD3D + CTLA4 | 0,711 | 0,529 | 0,893 |
| 4 jours | C3AR1 + IL1R2 + CD74 + CIITA + CTLA4 | 0,691 | 0,506 | 0,877 |
| 4 jours | C3AR1 + IL1R2 + CD74 + CIITA + TAP2 | 0,707 | 0,522 | 0,892 |
| 4 jours | C3AR1 + IL1R2 + CD74 + CIITA + TAP2 + CD3D | 0,711 | 0,528 | 0,894 |
| 4 jours | C3AR1 + IL1R2 + CD74 + CIITA + TAP2 + CD3D + CTLA4 | 0,723 | 0,543 | 0,902 |
| 4 jours | C3AR1 + IL1R2 + CD74 + CIITA + TAP2 + CTLA4 | 0,711 | 0,528 | 0,894 |
| 4 jours | C3AR1 + IL1R2 + CD74 + CTLA4 | 0,68 | 0,49 | 0,869 |
| 4 jours | C3AR1 + IL1R2 + CD74 + CX3CR1 | 0,711 | 0,514 | 0,908 |
| 4 jours | C3AR1 + IL1R2 + CD74 + CX3CR1 + CD3D | 0,715 | 0,532 | 0,897 |
| 4 jours | C3AR1 + IL1R2 + CD74 + CX3CR1 + CD3D + CTLA4 | 0,73 | 0,55 | 0,911 |
| 4 jours | C3AR1 + IL1R2 + CD74 + CX3CR1 + CIITA | 0,75 | 0,56 | 0,94 |
| 4 jours | C3AR1 + IL1R2 + CD74 + CX3CR1 + CIITA + CD3D | 0,75 | 0,571 | 0,929 |
| 4 jours | C3AR1 + IL1R2 + CD74 + CX3CR1 + CIITA + CD3D + CTLA4 | 0,754 | 0,577 | 0,93 |
| 4 jours | C3AR1 + IL1R2 + CD74 + CX3CR1 + CIITA + CTLA4 | 0,738 | 0,551 | 0,926 |
| 4 jours | C3AR1 + IL1R2 + CD74 + CX3CR1 + CIITA + TAP2 | 0,781 | 0,611 | 0,952 |
| 4 jours | C3AR1 + IL1R2 + CD74 + CX3CR1 + CIITA + TAP2 + CD3D | 0,77 | 0,598 | 0,941 |
| 4 jours | C3AR1 + IL1R2 + CD74 + CX3CR1 + CIITA + TAP2 + CD3D + CTLA4 | 0,785 | 0,62 | 0,95 |
| 4 jours | C3AR1 + IL1R2 + CD74 + CX3CR1 + CIITA + TAP2 + CTLA4 | 0,77 | 0,595 | 0,944 |
| 4 jours | C3AR1 + IL1R2 + CD74 + CX3CR1 + CTLA4 | 0,734 | 0,551 | 0,918 |
| 4 jours | C3AR1 + IL1R2 + CD74 + CX3CR1 + TAP2 | 0,754 | 0,579 | 0,929 |
| 4 jours | C3AR1 + IL1R2 + CD74 + CX3CR1 + TAP2 + CD3D | 0,742 | 0,567 | 0,917 |
| 4 jours | C3AR1 + IL1R2 + CD74 + CX3CR1 + TAP2 + CD3D + CTLA4 | 0,77 | 0,604 | 0,936 |
| 4 jours | C3AR1 + IL1R2 + CD74 + CX3CR1 + TAP2 + CTLA4 | 0,781 | 0,615 | 0,947 |
| 4 jours | C3AR1 + IL1R2 + CD74 + IFNG | 0,727 | 0,547 | 0,906 |
| 4 jours | C3AR1 + IL1R2 + CD74 + IFNG + CD177 | 0,738 | 0,563 | 0,914 |
| 4 jours | C3AR1 + IL1R2 + CD74 + IFNG + CD177 + CD3D | 0,75 | 0,577 | 0,923 |
| 4 jours | C3AR1 + IL1R2 + CD74 + IFNG + CD177 + CD3D + CTLA4 | 0,762 | 0,594 | 0,929 |
| 4 jours | C3AR1 + IL1R2 + CD74 + IFNG + CD177 + CIITA | 0,738 | 0,563 | 0,913 |
| 4 jours | C3AR1 + IL1R2 + CD74 + IFNG + CD177 + CIITA + CD3D | 0,75 | 0,578 | 0,922 |
| 4 jours | C3AR1 + IL1R2 + CD74 + IFNG + CD177 + CIITA + CD3D + CTLA4 | 0,758 | 0,584 | 0,932 |
| 4 jours | C3AR1 + IL1R2 + CD74 + IFNG + CD177 + CIITA + CTLA4 | 0,754 | 0,58 | 0,928 |
| 4 jours | C3AR1 + IL1R2 + CD74 + IFNG + CD177 + CIITA + TAP2 | 0,742 | 0,568 | 0,917 |
| 4 jours | C3AR1 + IL1R2 + CD74 + IFNG + CD177 + CIITA + TAP2 + CD3D | 0,754 | 0,584 | 0,924 |
| 4 jours | C3AR1 + IL1R2 + CD74 + IFNG + CD177 + CIITA + TAP2 + CD3D + CTLA4 | 0,758 | 0,584 | 0,932 |
| 4 jours | C3AR1 + IL1R2 + CD74 + IFNG + CD177 + CIITA + TAP2 + CTLA4 | 0,754 | 0,58 | 0,928 |
| 4 jours | C3AR1 + IL1R2 + CD74 + IFNG + CD177 + CTLA4 | 0,758 | 0,589 | 0,927 |
| 4 jours | C3AR1 + IL1R2 + CD74 + IFNG + CD177 + CX3CR1 | 0,867 | 0,737 | 0,997 |
| 4 jours | C3AR1 + IL1R2 + CD74 + IFNG + CD177 + CX3CR1 + CD3D | 0,871 | 0,752 | 0,99 |
| 4 jours | C3AR1 + IL1R2 + CD74 + IFNG + CD177 + CX3CR1 + CD3D + CTLA4 | 0,914 | 0,821 | 1 |
| 4 jours | C3AR1 + IL1R2 + CD74 + IFNG + CD177 + CX3CR1 + CIITA | 0,875 | 0,753 | 0,997 |
| 4 jours | C3AR1 + IL1R2 + CD74 + IFNG + CD177 + CX3CR1 + CIITA + CD3D | 0,871 | 0,753 | 0,989 |
| 4 jours | C3AR1 + IL1R2 + CD74 + IFNG + CD177 + CX3CR1 + CIITA + CD3D + CTLA4 | 0,918 | 0,828 | 1 |
| 4 jours | C3AR1 + IL1R2 + CD74 + IFNG + CD177 + CX3CR1 + CIITA + CTLA4 | 0,914 | 0,82 | 1 |
| 4 jours | C3AR1 + IL1R2 + CD74 + IFNG + CD177 + CX3CR1 + CIITA + TAP2 | 0,855 | 0,725 | 0,986 |
| 4 jours | C3AR1 + IL1R2 + CD74 + IFNG + CD177 + CX3CR1 + CIITA + TAP2 + CD3D | 0,871 | 0,752 | 0,99 |
| 4 jours | C3AR1 + IL1R2 + CD74 + IFNG + CD177 + CX3CR1 + CIITA + TAP2 + CD3D + CTLA4 | 0,891 | 0,782 | 1 |
| 4 jours | C3AR1 + IL1R2 + CD74 + IFNG + CD177 + CX3CR1 + CIITA + TAP2 + CTLA4 | 0,895 | 0,788 | 1 |
| 4 jours | C3AR1 + IL1R2 + CD74 + IFNG + CD177 + CX3CR1 + CTLA4 | 0,902 | 0,801 | 1 |
| 4 jours | C3AR1 + IL1R2 + CD74 + IFNG + CD177 + CX3CR1 + TAP2 | 0,863 | 0,738 | 0,989 |
| 4 jours | C3AR1 + IL1R2 + CD74 + IFNG + CD177 + CX3CR1 + TAP2 + CD3D | 0,887 | 0,777 | 0,997 |
| 4 jours | C3AR1 + IL1R2 + CD74 + IFNG + CD177 + CX3CR1 + TAP2 + CD3D + CTLA4 | 0,891 | 0,78 | 1 |
| 4 jours | C3AR1 + IL1R2 + CD74 + IFNG + CD177 + CX3CR1 + TAP2 + CTLA4 | 0,891 | 0,78 | 1 |
| 4 jours | C3AR1 + IL1R2 + CD74 + IFNG + CD177 + S100A9 | 0,828 | 0,683 | 0,973 |
| 4 jours | C3AR1 + IL1R2 + CD74 + IFNG + CD177 + S100A9 + CD3D | 0,836 | 0,697 | 0,975 |
| 4 jours | C3AR1 + IL1R2 + CD74 + IFNG + CD177 + S100A9 + CD3D + CTLA4 | 0,844 | 0,71 | 0,977 |
| 4 jours | C3AR1 + IL1R2 + CD74 + IFNG + CD177 + S100A9 + CIITA | 0,824 | 0,673 | 0,975 |
| 4 jours | C3AR1 + IL1R2 + CD74 + IFNG + CD177 + S100A9 + CIITA + CD3D | 0,844 | 0,71 | 0,978 |
| 4 jours | C3AR1 + IL1R2 + CD74 + IFNG + CD177 + S100A9 + CIITA + CD3D + CTLA4 | 0,84 | 0,705 | 0,975 |
| 4 jours | C3AR1 + IL1R2 + CD74 + IFNG + CD177 + S100A9 + CIITA + CTLA4 | 0,82 | 0,675 | 0,966 |
| 4 jours | C3AR1 + IL1R2 + CD74 + IFNG + CD177 + S100A9 + CIITA + TAP2 | 0,855 | 0,718 | 0,993 |
| 4 jours | C3AR1 + IL1R2 + CD74 + IFNG + CD177 + S100A9 + CIITA + TAP2 + CD3D | 0,855 | 0,726 | 0,985 |
| 4 jours | C3AR1 + IL1R2 + CD74 + IFNG + CD177 + S100A9 + CIITA + TAP2 + CD3D + CTLA4 | 0,848 | 0,713 | 0,982 |
| 4 jours | C3AR1 + IL1R2 + CD74 + IFNG + CD177 + S100A9 + CIITA + TAP2 + CTLA4 | 0,84 | 0,701 | 0,979 |
| 4 jours | C3AR1 + IL1R2 + CD74 + IFNG + CD177 + S100A9 + CTLA4 | 0,828 | 0,688 | 0,969 |
| 4 jours | C3AR1 + IL1R2 + CD74 + IFNG + CD177 + S100A9 + CX3CR1 | 0,941 | 0,864 | 1 |
| 4 jours | C3AR1 + IL1R2 + CD74 + IFNG + CD177 + S100A9 + CX3CR1 + CD3D | 0,977 | 0,928 | 1 |
| 4 jours | C3AR1 + IL1R2 + CD74 + IFNG + CD177 + S100A9 + CX3CR1 + CIITA | 0,945 | 0,871 | 1 |
| 4 jours | C3AR1 + IL1R2 + CD74 + IFNG + CD177 + S100A9 + CX3CR1 + CIITA + CD3D | 0,984 | 0,951 | 1 |
| 4 jours | C3AR1 + IL1R2 + CD74 + IFNG + CD177 + S100A9 + CX3CR1 + CIITA + TAP2 | 0,945 | 0,871 | 1 |
| 4 jours | C3AR1 + IL1R2 + CD74 + IFNG + CD177 + S100A9 + CX3CR1 + TAP2 | 0,941 | 0,864 | 1 |
| 4 jours | C3AR1 + IL1R2 + CD74 + IFNG + CD177 + S100A9 + TAP2 | 0,84 | 0,7 | 0,98 |
| 4 jours | C3AR1 + IL1R2 + CD74 + IFNG + CD177 + S100A9 + TAP2 + CD3D | 0,848 | 0,714 | 0,981 |
| 4 jours | C3AR1 + IL1R2 + CD74 + IFNG + CD177 + S100A9 + TAP2 + CD3D + CTLA4 | 0,844 | 0,709 | 0,979 |
| 4 jours | C3AR1 + IL1R2 + CD74 + IFNG + CD177 + S100A9 + TAP2 + CTLA4 | 0,824 | 0,68 | 0,969 |
| 4 jours | C3AR1 + IL1R2 + CD74 + IFNG + CD177 + TAP2 | 0,738 | 0,563 | 0,914 |
| 4 jours | C3AR1 + IL1R2 + CD74 + IFNG + CD177 + TAP2 + CD3D | 0,746 | 0,572 | 0,92 |
| 4 jours | C3AR1 + IL1R2 + CD74 + IFNG + CD177 + TAP2 + CD3D + CTLA4 | 0,762 | 0,593 | 0,93 |
| 4 jours | C3AR1 + IL1R2 + CD74 + IFNG + CD177 + TAP2 + CTLA4 | 0,758 | 0,589 | 0,927 |
| 4 jours | C3AR1 + IL1R2 + CD74 + IFNG + CD3D | 0,727 | 0,546 | 0,907 |
| 4 jours | C3AR1 + IL1R2 + CD74 + IFNG + CD3D + CTLA4 | 0,762 | 0,593 | 0,93 |
| 4 jours | C3AR1 + IL1R2 + CD74 + IFNG + CIITA | 0,727 | 0,548 | 0,905 |
| 4 jours | C3AR1 + IL1R2 + CD74 + IFNG + CIITA + CD3D | 0,734 | 0,558 | 0,911 |
| 4 jours | C3AR1 + IL1R2 + CD74 + IFNG + CIITA + CD3D + CTLA4 | 0,758 | 0,584 | 0,932 |
| 4 jours | C3AR1 + IL1R2 + CD74 + IFNG + CIITA + CTLA4 | 0,762 | 0,59 | 0,934 |
| 4 jours | C3AR1 + IL1R2 + CD74 + IFNG + CIITA + TAP2 | 0,719 | 0,538 | 0,9 |
| 4 jours | C3AR1 + IL1R2 + CD74 + IFNG + CIITA + TAP2 + CD3D | 0,73 | 0,553 | 0,908 |
| 4 jours | C3AR1 + IL1R2 + CD74 + IFNG + CIITA + TAP2 + CD3D + CTLA4 | 0,758 | 0,584 | 0,932 |
| 4 jours | C3AR1 + IL1R2 + CD74 + IFNG + CIITA + TAP2 + CTLA4 | 0,762 | 0,59 | 0,934 |
| 4 jours | C3AR1 + IL1R2 + CD74 + IFNG + CTLA4 | 0,758 | 0,587 | 0,928 |
| 4 jours | C3AR1 + IL1R2 + CD74 + IFNG + CX3CR1 | 0,867 | 0,737 | 0,997 |
| 4 jours | C3AR1 + IL1R2 + CD74 + IFNG + CX3CR1 + CD3D | 0,875 | 0,758 | 0,992 |
| 4 jours | C3AR1 + IL1R2 + CD74 + IFNG + CX3CR1 + CD3D + CTLA4 | 0,887 | 0,776 | 0,997 |
| 4 jours | C3AR1 + IL1R2 + CD74 + IFNG + CX3CR1 + CIITA | 0,875 | 0,753 | 0,997 |
| 4 jours | C3AR1 + IL1R2 + CD74 + IFNG + CX3CR1 + CIITA + CD3D | 0,875 | 0,758 | 0,992 |
| 4 jours | C3AR1 + IL1R2 + CD74 + IFNG + CX3CR1 + CIITA + CD3D + CTLA4 | 0,883 | 0,769 | 0,996 |
| 4 jours | C3AR1 + IL1R2 + CD74 + IFNG + CX3CR1 + CIITA + CTLA4 | 0,879 | 0,764 | 0,993 |
| 4 jours | C3AR1 + IL1R2 + CD74 + IFNG + CX3CR1 + CIITA + TAP2 | 0,867 | 0,742 | 0,993 |
| 4 jours | C3AR1 + IL1R2 + CD74 + IFNG + CX3CR1 + CIITA + TAP2 + CD3D | 0,879 | 0,763 | 0,995 |
| 4 jours | C3AR1 + IL1R2 + CD74 + IFNG + CX3CR1 + CIITA + TAP2 + CD3D + CTLA4 | 0,879 | 0,763 | 0,995 |
| 4 jours | C3AR1 + IL1R2 + CD74 + IFNG + CX3CR1 + CIITA + TAP2 + CTLA4 | 0,887 | 0,776 | 0,998 |
| 4 jours | C3AR1 + IL1R2 + CD74 + IFNG + CX3CR1 + CTLA4 | 0,879 | 0,764 | 0,993 |
| 4 jours | C3AR1 + IL1R2 + CD74 + IFNG + CX3CR1 + TAP2 | 0,859 | 0,732 | 0,986 |
| 4 jours | C3AR1 + IL1R2 + CD74 + IFNG + CX3CR1 + TAP2 + CD3D | 0,883 | 0,771 | 0,995 |
| 4 jours | C3AR1 + IL1R2 + CD74 + IFNG + CX3CR1 + TAP2 + CD3D + CTLA4 | 0,875 | 0,757 | 0,993 |
| 4 jours | C3AR1 + IL1R2 + CD74 + IFNG + CX3CR1 + TAP2 + CTLA4 | 0,887 | 0,776 | 0,998 |
| 4 jours | C3AR1 + IL1R2 + CD74 + IFNG + S100A9 | 0,816 | 0,666 | 0,967 |
| 4 jours | C3AR1 + IL1R2 + CD74 + IFNG + S100A9 + CD3D | 0,852 | 0,719 | 0,984 |
| 4 jours | C3AR1 + IL1R2 + CD74 + IFNG + S100A9 + CD3D + CTLA4 | 0,848 | 0,713 | 0,982 |
| 4 jours | C3AR1 + IL1R2 + CD74 + IFNG + S100A9 + CIITA | 0,828 | 0,681 | 0,975 |
| 4 jours | C3AR1 + IL1R2 + CD74 + IFNG + S100A9 + CIITA + CD3D | 0,848 | 0,712 | 0,983 |
| 4 jours | C3AR1 + IL1R2 + CD74 + IFNG + S100A9 + CIITA + CD3D + CTLA4 | 0,844 | 0,706 | 0,982 |
| 4 jours | C3AR1 + IL1R2 + CD74 + IFNG + S100A9 + CIITA + CTLA4 | 0,816 | 0,668 | 0,965 |
| 4 jours | C3AR1 + IL1R2 + CD74 + IFNG + S100A9 + CIITA + TAP2 | 0,844 | 0,699 | 0,989 |
| 4 jours | C3AR1 + IL1R2 + CD74 + IFNG + S100A9 + CIITA + TAP2 + CD3D | 0,852 | 0,716 | 0,987 |
| 4 jours | C3AR1 + IL1R2 + CD74 + IFNG + S100A9 + CIITA + TAP2 + CD3D + CTLA4 | 0,848 | 0,711 | 0,984 |
| 4 jours | C3AR1 + IL1R2 + CD74 + IFNG + S100A9 + CIITA + TAP2 + CTLA4 | 0,828 | 0,681 | 0,975 |
| 4 jours | C3AR1 + IL1R2 + CD74 + IFNG + S100A9 + CTLA4 | 0,816 | 0,669 | 0,963 |
| 4 jours | C3AR1 + IL1R2 + CD74 + IFNG + S100A9 + CX3CR1 | 0,926 | 0,838 | 1 |
| 4 jours | C3AR1 + IL1R2 + CD74 + IFNG + S100A9 + CX3CR1 + CD3D | 0,945 | 0,876 | 1 |
| 4 jours | C3AR1 + IL1R2 + CD74 + IFNG + S100A9 + CX3CR1 + CD3D + CTLA4 | 0,945 | 0,876 | 1 |
| 4 jours | C3AR1 + IL1R2 + CD74 + IFNG + S100A9 + CX3CR1 + CIITA | 0,926 | 0,838 | 1 |
| 4 jours | C3AR1 + IL1R2 + CD74 + IFNG + S100A9 + CX3CR1 + CIITA + CD3D | 0,945 | 0,873 | 1 |
| 4 jours | C3AR1 + IL1R2 + CD74 + IFNG + S100A9 + CX3CR1 + CIITA + CD3D + CTLA4 | 0,949 | 0,882 | 1 |
| 4 jours | C3AR1 + IL1R2 + CD74 + IFNG + S100A9 + CX3CR1 + CIITA + CTLA4 | 0,918 | 0,825 | 1 |
| 4 jours | C3AR1 + IL1R2 + CD74 + IFNG + S100A9 + CX3CR1 + CIITA + TAP2 | 0,93 | 0,843 | 1 |
| 4 jours | C3AR1 + IL1R2 + CD74 + IFNG + S100A9 + CX3CR1 + CIITA + TAP2 + CD3D | 0,949 | 0,883 | 1 |
| 4 jours | C3AR1 + IL1R2 + CD74 + IFNG + S100A9 + CX3CR1 + CIITA + TAP2 + CD3D + CTLA4 | 0,949 | 0,881 | 1 |
| 4 jours | C3AR1 + IL1R2 + CD74 + IFNG + S100A9 + CX3CR1 + CIITA + TAP2 + CTLA4 | 0,914 | 0,815 | 1 |
| 4 jours | C3AR1 + IL1R2 + CD74 + IFNG + S100A9 + CX3CR1 + CTLA4 | 0,922 | 0,833 | 1 |
| 4 jours | C3AR1 + IL1R2 + CD74 + IFNG + S100A9 + CX3CR1 + TAP2 | 0,93 | 0,843 | 1 |
| 4 jours | C3AR1 + IL1R2 + CD74 + IFNG + S100A9 + CX3CR1 + TAP2 + CD3D | 0,938 | 0,862 | 1 |
| 4 jours | C3AR1 + IL1R2 + CD74 + IFNG + S100A9 + CX3CR1 + TAP2 + CD3D + CTLA4 | 0,953 | 0,887 | 1 |
| 4 jours | C3AR1 + IL1R2 + CD74 + IFNG + S100A9 + CX3CR1 + TAP2 + CTLA4 | 0,918 | 0,825 | 1 |
| 4 jours | C3AR1 + IL1R2 + CD74 + IFNG + S100A9 + TAP2 | 0,832 | 0,685 | 0,979 |
| 4 jours | C3AR1 + IL1R2 + CD74 + IFNG + S100A9 + TAP2 + CD3D | 0,848 | 0,709 | 0,986 |
| 4 jours | C3AR1 + IL1R2 + CD74 + IFNG + S100A9 + TAP2 + CD3D + CTLA4 | 0,852 | 0,717 | 0,986 |
| 4 jours | C3AR1 + IL1R2 + CD74 + IFNG + S100A9 + TAP2 + CTLA4 | 0,812 | 0,661 | 0,964 |
| 4 jours | C3AR1 + IL1R2 + CD74 + IFNG + TAP2 | 0,719 | 0,537 | 0,9 |
| 4 jours | C3AR1 + IL1R2 + CD74 + IFNG + TAP2 + CD3D | 0,73 | 0,552 | 0,909 |
| 4 jours | C3AR1 + IL1R2 + CD74 + IFNG + TAP2 + CD3D + CTLA4 | 0,762 | 0,592 | 0,932 |
| 4 jours | C3AR1 + IL1R2 + CD74 + IFNG + TAP2 + CTLA4 | 0,758 | 0,587 | 0,928 |
| 4 jours | C3AR1 + IL1R2 + CD74 + S100A9 | 0,824 | 0,67 | 0,979 |
| 4 jours | C3AR1 + IL1R2 + CD74 + S100A9 + CD3D | 0,844 | 0,708 | 0,979 |
| 4 jours | C3AR1 + IL1R2 + CD74 + S100A9 + CD3D + CTLA4 | 0,844 | 0,708 | 0,979 |
| 4 jours | C3AR1 + IL1R2 + CD74 + S100A9 + CIITA | 0,824 | 0,67 | 0,979 |
| 4 jours | C3AR1 + IL1R2 + CD74 + S100A9 + CIITA + CD3D | 0,844 | 0,708 | 0,979 |
| 4 jours | C3AR1 + IL1R2 + CD74 + S100A9 + CIITA + CD3D + CTLA4 | 0,84 | 0,703 | 0,977 |
| 4 jours | C3AR1 + IL1R2 + CD74 + S100A9 + CIITA + CTLA4 | 0,816 | 0,661 | 0,972 |
| 4 jours | C3AR1 + IL1R2 + CD74 + S100A9 + CIITA + TAP2 | 0,824 | 0,672 | 0,977 |
| 4 jours | C3AR1 + IL1R2 + CD74 + S100A9 + CIITA + TAP2 + CD3D | 0,844 | 0,706 | 0,981 |
| 4 jours | C3AR1 + IL1R2 + CD74 + S100A9 + CIITA + TAP2 + CD3D + CTLA4 | 0,844 | 0,706 | 0,982 |
| 4 jours | C3AR1 + IL1R2 + CD74 + S100A9 + CIITA + TAP2 + CTLA4 | 0,816 | 0,661 | 0,972 |
| 4 jours | C3AR1 + IL1R2 + CD74 + S100A9 + CTLA4 | 0,816 | 0,661 | 0,972 |
| 4 jours | C3AR1 + IL1R2 + CD74 + S100A9 + CX3CR1 | 0,871 | 0,733 | 1 |
| 4 jours | C3AR1 + IL1R2 + CD74 + S100A9 + CX3CR1 + CD3D | 0,898 | 0,781 | 1 |
| 4 jours | C3AR1 + IL1R2 + CD74 + S100A9 + CX3CR1 + CD3D + CTLA4 | 0,891 | 0,766 | 1 |
| 4 jours | C3AR1 + IL1R2 + CD74 + S100A9 + CX3CR1 + CIITA | 0,867 | 0,732 | 1 |
| 4 jours | C3AR1 + IL1R2 + CD74 + S100A9 + CX3CR1 + CIITA + CD3D | 0,898 | 0,786 | 1 |
| 4 jours | C3AR1 + IL1R2 + CD74 + S100A9 + CX3CR1 + CIITA + CD3D + CTLA4 | 0,902 | 0,791 | 1 |
| 4 jours | C3AR1 + IL1R2 + CD74 + S100A9 + CX3CR1 + CIITA + CTLA4 | 0,875 | 0,742 | 1 |
| 4 jours | C3AR1 + IL1R2 + CD74 + S100A9 + CX3CR1 + CIITA + TAP2 | 0,879 | 0,746 | 1 |
| 4 jours | C3AR1 + IL1R2 + CD74 + S100A9 + CX3CR1 + CIITA + TAP2 + CD3D | 0,898 | 0,786 | 1 |
| 4 jours | C3AR1 + IL1R2 + CD74 + S100A9 + CX3CR1 + CIITA + TAP2 + CD3D + CTLA4 | 0,922 | 0,826 | 1 |
| 4 jours | C3AR1 + IL1R2 + CD74 + S100A9 + CX3CR1 + CIITA + TAP2 + CTLA4 | 0,883 | 0,754 | 1 |
| 4 jours | C3AR1 + IL1R2 + CD74 + S100A9 + CX3CR1 + CTLA4 | 0,879 | 0,747 | 1 |
| 4 jours | C3AR1 + IL1R2 + CD74 + S100A9 + CX3CR1 + TAP2 | 0,871 | 0,736 | 1 |
| 4 jours | C3AR1 + IL1R2 + CD74 + S100A9 + CX3CR1 + TAP2 + CD3D | 0,898 | 0,781 | 1 |
| 4 jours | C3AR1 + IL1R2 + CD74 + S100A9 + CX3CR1 + TAP2 + CD3D + CTLA4 | 0,891 | 0,768 | 1 |
| 4 jours | C3AR1 + IL1R2 + CD74 + S100A9 + CX3CR1 + TAP2 + CTLA4 | 0,883 | 0,751 | 1 |
| 4 jours | C3AR1 + IL1R2 + CD74 + S100A9 + TAP2 | 0,816 | 0,658 | 0,975 |
| 4 jours | C3AR1 + IL1R2 + CD74 + S100A9 + TAP2 + CD3D | 0,844 | 0,706 | 0,981 |
| 4 jours | C3AR1 + IL1R2 + CD74 + S100A9 + TAP2 + CD3D + CTLA4 | 0,848 | 0,713 | 0,983 |
| 4 jours | C3AR1 + IL1R2 + CD74 + S100A9 + TAP2 + CTLA4 | 0,816 | 0,661 | 0,972 |
| 4 jours | C3AR1 + IL1R2 + CD74 + TAP2 | 0,703 | 0,52 | 0,886 |
| 4 jours | C3AR1 + IL1R2 + CD74 + TAP2 + CD3D | 0,711 | 0,529 | 0,893 |
| 4 jours | C3AR1 + IL1R2 + CD74 + TAP2 + CD3D + CTLA4 | 0,715 | 0,531 | 0,898 |
| 4 jours | C3AR1 + IL1R2 + CD74 + TAP2 + CTLA4 | 0,707 | 0,521 | 0,893 |
| 4 jours | C3AR1 + IL1R2 + CIITA | 0,723 | 0,538 | 0,907 |
| 4 jours | C3AR1 + IL1R2 + CIITA + CD3D | 0,707 | 0,52 | 0,894 |
| 4 jours | C3AR1 + IL1R2 + CIITA + CD3D + CTLA4 | 0,676 | 0,481 | 0,871 |
| 4 jours | C3AR1 + IL1R2 + CIITA + CTLA4 | 0,668 | 0,474 | 0,862 |
| 4 jours | C3AR1 + IL1R2 + CIITA + TAP2 | 0,715 | 0,531 | 0,899 |
| 4 jours | C3AR1 + IL1R2 + CIITA + TAP2 + CD3D | 0,723 | 0,543 | 0,903 |
| 4 jours | C3AR1 + IL1R2 + CIITA + TAP2 + CD3D + CTLA4 | 0,707 | 0,523 | 0,891 |
| 4 jours | C3AR1 + IL1R2 + CIITA + TAP2 + CTLA4 | 0,707 | 0,523 | 0,891 |
| 4 jours | C3AR1 + IL1R2 + CX3CR1 | 0,676 | 0,479 | 0,872 |
| 4 jours | C3AR1 + IL1R2 + CX3CR1 + CD3D | 0,664 | 0,465 | 0,863 |
| 4 jours | C3AR1 + IL1R2 + CX3CR1 + CD3D + CTLA4 | 0,672 | 0,48 | 0,864 |
| 4 jours | C3AR1 + IL1R2 + CX3CR1 + CIITA | 0,73 | 0,542 | 0,919 |
| 4 jours | C3AR1 + IL1R2 + CX3CR1 + CIITA + CD3D | 0,703 | 0,51 | 0,896 |
| 4 jours | C3AR1 + IL1R2 + CX3CR1 + CIITA + CD3D + CTLA4 | 0,711 | 0,52 | 0,902 |
| 4 jours | C3AR1 + IL1R2 + CX3CR1 + CIITA + CTLA4 | 0,723 | 0,53 | 0,915 |
| 4 jours | C3AR1 + IL1R2 + CX3CR1 + CIITA + TAP2 | 0,758 | 0,584 | 0,932 |
| 4 jours | C3AR1 + IL1R2 + CX3CR1 + CIITA + TAP2 + CD3D | 0,777 | 0,615 | 0,94 |
| 4 jours | C3AR1 + IL1R2 + CX3CR1 + CIITA + TAP2 + CD3D + CTLA4 | 0,766 | 0,599 | 0,933 |
| 4 jours | C3AR1 + IL1R2 + CX3CR1 + CIITA + TAP2 + CTLA4 | 0,754 | 0,584 | 0,924 |
| 4 jours | C3AR1 + IL1R2 + CX3CR1 + CTLA4 | 0,672 | 0,479 | 0,864 |
| 4 jours | C3AR1 + IL1R2 + CX3CR1 + TAP2 | 0,707 | 0,52 | 0,894 |
| 4 jours | C3AR1 + IL1R2 + CX3CR1 + TAP2 + CD3D | 0,699 | 0,515 | 0,883 |
| 4 jours | C3AR1 + IL1R2 + CX3CR1 + TAP2 + CD3D + CTLA4 | 0,715 | 0,535 | 0,894 |
| 4 jours | C3AR1 + IL1R2 + CX3CR1 + TAP2 + CTLA4 | 0,719 | 0,54 | 0,897 |
| 4 jours | C3AR1 + IL1R2 + IFNG | 0,715 | 0,527 | 0,902 |
| 4 jours | C3AR1 + IL1R2 + IFNG + CD177 | 0,73 | 0,548 | 0,913 |
| 4 jours | C3AR1 + IL1R2 + IFNG + CD177 + CD3D | 0,754 | 0,577 | 0,931 |
| 4 jours | C3AR1 + IL1R2 + IFNG + CD177 + CD3D + CTLA4 | 0,75 | 0,579 | 0,921 |
| 4 jours | C3AR1 + IL1R2 + IFNG + CD177 + CIITA | 0,734 | 0,556 | 0,913 |
| 4 jours | C3AR1 + IL1R2 + IFNG + CD177 + CIITA + CD3D | 0,746 | 0,567 | 0,925 |
| 4 jours | C3AR1 + IL1R2 + IFNG + CD177 + CIITA + CD3D + CTLA4 | 0,75 | 0,579 | 0,921 |
| 4 jours | C3AR1 + IL1R2 + IFNG + CD177 + CIITA + CTLA4 | 0,75 | 0,58 | 0,92 |
| 4 jours | C3AR1 + IL1R2 + IFNG + CD177 + CIITA + TAP2 | 0,746 | 0,568 | 0,924 |
| 4 jours | C3AR1 + IL1R2 + IFNG + CD177 + CIITA + TAP2 + CD3D | 0,75 | 0,574 | 0,926 |
| 4 jours | C3AR1 + IL1R2 + IFNG + CD177 + CIITA + TAP2 + CD3D + CTLA4 | 0,75 | 0,578 | 0,922 |
| 4 jours | C3AR1 + IL1R2 + IFNG + CD177 + CIITA + TAP2 + CTLA4 | 0,766 | 0,597 | 0,934 |
| 4 jours | C3AR1 + IL1R2 + IFNG + CD177 + CTLA4 | 0,75 | 0,58 | 0,92 |
| 4 jours | C3AR1 + IL1R2 + IFNG + CD177 + CX3CR1 | 0,844 | 0,699 | 0,988 |
| 4 jours | C3AR1 + IL1R2 + IFNG + CD177 + CX3CR1 + CD3D | 0,863 | 0,74 | 0,987 |
| 4 jours | C3AR1 + IL1R2 + IFNG + CD177 + CX3CR1 + CD3D + CTLA4 | 0,895 | 0,784 | 1 |
| 4 jours | C3AR1 + IL1R2 + IFNG + CD177 + CX3CR1 + CIITA | 0,863 | 0,727 | 1 |
| 4 jours | C3AR1 + IL1R2 + IFNG + CD177 + CX3CR1 + CIITA + CD3D | 0,875 | 0,754 | 0,996 |
| 4 jours | C3AR1 + IL1R2 + IFNG + CD177 + CX3CR1 + CIITA + CD3D + CTLA4 | 0,891 | 0,778 | 1 |
| 4 jours | C3AR1 + IL1R2 + IFNG + CD177 + CX3CR1 + CIITA + CTLA4 | 0,891 | 0,778 | 1 |
| 4 jours | C3AR1 + IL1R2 + IFNG + CD177 + CX3CR1 + CIITA + TAP2 | 0,871 | 0,746 | 0,996 |
| 4 jours | C3AR1 + IL1R2 + IFNG + CD177 + CX3CR1 + CIITA + TAP2 + CD3D | 0,871 | 0,749 | 0,994 |
| 4 jours | C3AR1 + IL1R2 + IFNG + CD177 + CX3CR1 + CIITA + TAP2 + CD3D + CTLA4 | 0,895 | 0,779 | 1 |
| 4 jours | C3AR1 + IL1R2 + IFNG + CD177 + CX3CR1 + CIITA + TAP2 + CTLA4 | 0,895 | 0,781 | 1 |
| 4 jours | C3AR1 + IL1R2 + IFNG + CD177 + CX3CR1 + CTLA4 | 0,895 | 0,784 | 1 |
| 4 jours | C3AR1 + IL1R2 + IFNG + CD177 + CX3CR1 + TAP2 | 0,852 | 0,72 | 0,983 |
| 4 jours | C3AR1 + IL1R2 + IFNG + CD177 + CX3CR1 + TAP2 + CD3D | 0,871 | 0,749 | 0,994 |
| 4 jours | C3AR1 + IL1R2 + IFNG + CD177 + CX3CR1 + TAP2 + CD3D + CTLA4 | 0,895 | 0,779 | 1 |
| 4 jours | C3AR1 + IL1R2 + IFNG + CD177 + CX3CR1 + TAP2 + CTLA4 | 0,906 | 0,797 | 1 |
| 4 jours | C3AR1 + IL1R2 + IFNG + CD177 + S100A9 | 0,828 | 0,681 | 0,976 |
| 4 jours | C3AR1 + IL1R2 + IFNG + CD177 + S100A9 + CD3D | 0,836 | 0,698 | 0,974 |
| 4 jours | C3AR1 + IL1R2 + IFNG + CD177 + S100A9 + CD3D + CTLA4 | 0,836 | 0,699 | 0,973 |
| 4 jours | C3AR1 + IL1R2 + IFNG + CD177 + S100A9 + CIITA | 0,82 | 0,668 | 0,972 |
| 4 jours | C3AR1 + IL1R2 + IFNG + CD177 + S100A9 + CIITA + CD3D | 0,844 | 0,71 | 0,978 |
| 4 jours | C3AR1 + IL1R2 + IFNG + CD177 + S100A9 + CIITA + CD3D + CTLA4 | 0,84 | 0,705 | 0,975 |
| 4 jours | C3AR1 + IL1R2 + IFNG + CD177 + S100A9 + CIITA + CTLA4 | 0,82 | 0,675 | 0,966 |
| 4 jours | C3AR1 + IL1R2 + IFNG + CD177 + S100A9 + CIITA + TAP2 | 0,852 | 0,713 | 0,99 |
| 4 jours | C3AR1 + IL1R2 + IFNG + CD177 + S100A9 + CIITA + TAP2 + CD3D | 0,855 | 0,726 | 0,985 |
| 4 jours | C3AR1 + IL1R2 + IFNG + CD177 + S100A9 + CIITA + TAP2 + CD3D + CTLA4 | 0,848 | 0,713 | 0,982 |
| 4 jours | C3AR1 + IL1R2 + IFNG + CD177 + S100A9 + CIITA + TAP2 + CTLA4 | 0,84 | 0,701 | 0,979 |
| 4 jours | C3AR1 + IL1R2 + IFNG + CD177 + S100A9 + CTLA4 | 0,824 | 0,681 | 0,968 |
| 4 jours | C3AR1 + IL1R2 + IFNG + CD177 + S100A9 + CX3CR1 | 0,941 | 0,864 | 1 |
| 4 jours | C3AR1 + IL1R2 + IFNG + CD177 + S100A9 + CX3CR1 + CD3D | 0,977 | 0,928 | 1 |
| 4 jours | C3AR1 + IL1R2 + IFNG + CD177 + S100A9 + CX3CR1 + CIITA | 0,938 | 0,857 | 1 |
| 4 jours | C3AR1 + IL1R2 + IFNG + CD177 + S100A9 + CX3CR1 + CIITA + CD3D | 0,98 | 0,939 | 1 |
| 4 jours | C3AR1 + IL1R2 + IFNG + CD177 + S100A9 + CX3CR1 + CIITA + TAP2 | 0,938 | 0,857 | 1 |
| 4 jours | C3AR1 + IL1R2 + IFNG + CD177 + S100A9 + CX3CR1 + TAP2 | 0,941 | 0,864 | 1 |
| 4 jours | C3AR1 + IL1R2 + IFNG + CD177 + S100A9 + CX3CR1 + TAP2 + CD3D | 0,984 | 0,951 | 1 |
| 4 jours | C3AR1 + IL1R2 + IFNG + CD177 + S100A9 + TAP2 | 0,84 | 0,696 | 0,984 |
| 4 jours | C3AR1 + IL1R2 + IFNG + CD177 + S100A9 + TAP2 + CD3D | 0,84 | 0,703 | 0,976 |
| 4 jours | C3AR1 + IL1R2 + IFNG + CD177 + S100A9 + TAP2 + CD3D + CTLA4 | 0,84 | 0,703 | 0,976 |
| 4 jours | C3AR1 + IL1R2 + IFNG + CD177 + S100A9 + TAP2 + CTLA4 | 0,824 | 0,68 | 0,969 |
| 4 jours | C3AR1 + IL1R2 + IFNG + CD177 + TAP2 | 0,742 | 0,562 | 0,923 |
| 4 jours | C3AR1 + IL1R2 + IFNG + CD177 + TAP2 + CD3D | 0,75 | 0,571 | 0,929 |
| 4 jours | C3AR1 + IL1R2 + IFNG + CD177 + TAP2 + CD3D + CTLA4 | 0,758 | 0,588 | 0,928 |
| 4 jours | C3AR1 + IL1R2 + IFNG + CD177 + TAP2 + CTLA4 | 0,766 | 0,597 | 0,934 |
| 4 jours | C3AR1 + IL1R2 + IFNG + CD3D | 0,73 | 0,552 | 0,909 |
| 4 jours | C3AR1 + IL1R2 + IFNG + CD3D + CTLA4 | 0,742 | 0,57 | 0,915 |
| 4 jours | C3AR1 + IL1R2 + IFNG + CIITA | 0,715 | 0,528 | 0,901 |
| 4 jours | C3AR1 + IL1R2 + IFNG + CIITA + CD3D | 0,738 | 0,561 | 0,916 |
| 4 jours | C3AR1 + IL1R2 + IFNG + CIITA + CD3D + CTLA4 | 0,734 | 0,56 | 0,909 |
| 4 jours | C3AR1 + IL1R2 + IFNG + CIITA + CTLA4 | 0,742 | 0,57 | 0,915 |
| 4 jours | C3AR1 + IL1R2 + IFNG + CIITA + TAP2 | 0,734 | 0,555 | 0,914 |
| 4 jours | C3AR1 + IL1R2 + IFNG + CIITA + TAP2 + CD3D | 0,746 | 0,57 | 0,923 |
| 4 jours | C3AR1 + IL1R2 + IFNG + CIITA + TAP2 + CD3D + CTLA4 | 0,742 | 0,567 | 0,917 |
| 4 jours | C3AR1 + IL1R2 + IFNG + CIITA + TAP2 + CTLA4 | 0,754 | 0,583 | 0,925 |
| 4 jours | C3AR1 + IL1R2 + IFNG + CTLA4 | 0,742 | 0,57 | 0,915 |
| 4 jours | C3AR1 + IL1R2 + IFNG + CX3CR1 | 0,844 | 0,701 | 0,986 |
| 4 jours | C3AR1 + IL1R2 + IFNG + CX3CR1 + CD3D | 0,844 | 0,71 | 0,977 |
| 4 jours | C3AR1 + IL1R2 + IFNG + CX3CR1 + CD3D + CTLA4 | 0,867 | 0,746 | 0,988 |
| 4 jours | C3AR1 + IL1R2 + IFNG + CX3CR1 + CIITA | 0,859 | 0,722 | 0,997 |
| 4 jours | C3AR1 + IL1R2 + IFNG + CX3CR1 + CIITA + CD3D | 0,883 | 0,769 | 0,996 |
| 4 jours | C3AR1 + IL1R2 + IFNG + CX3CR1 + CIITA + CD3D + CTLA4 | 0,883 | 0,771 | 0,994 |
| 4 jours | C3AR1 + IL1R2 + IFNG + CX3CR1 + CIITA + CTLA4 | 0,883 | 0,771 | 0,994 |
| 4 jours | C3AR1 + IL1R2 + IFNG + CX3CR1 + CIITA + TAP2 | 0,875 | 0,751 | 0,999 |
| 4 jours | C3AR1 + IL1R2 + IFNG + CX3CR1 + CIITA + TAP2 + CD3D | 0,871 | 0,751 | 0,992 |
| 4 jours | C3AR1 + IL1R2 + IFNG + CX3CR1 + CIITA + TAP2 + CD3D + CTLA4 | 0,879 | 0,764 | 0,993 |
| 4 jours | C3AR1 + IL1R2 + IFNG + CX3CR1 + CIITA + TAP2 + CTLA4 | 0,879 | 0,764 | 0,993 |
| 4 jours | C3AR1 + IL1R2 + IFNG + CX3CR1 + CTLA4 | 0,871 | 0,752 | 0,99 |
| 4 jours | C3AR1 + IL1R2 + IFNG + CX3CR1 + TAP2 | 0,852 | 0,722 | 0,981 |
| 4 jours | C3AR1 + IL1R2 + IFNG + CX3CR1 + TAP2 + CD3D | 0,867 | 0,743 | 0,992 |
| 4 jours | C3AR1 + IL1R2 + IFNG + CX3CR1 + TAP2 + CD3D + CTLA4 | 0,875 | 0,758 | 0,992 |
| 4 jours | C3AR1 + IL1R2 + IFNG + CX3CR1 + TAP2 + CTLA4 | 0,863 | 0,739 | 0,988 |
| 4 jours | C3AR1 + IL1R2 + IFNG + S100A9 | 0,816 | 0,666 | 0,967 |
| 4 jours | C3AR1 + IL1R2 + IFNG + S100A9 + CD3D | 0,852 | 0,718 | 0,985 |
| 4 jours | C3AR1 + IL1R2 + IFNG + S100A9 + CD3D + CTLA4 | 0,852 | 0,718 | 0,985 |
| 4 jours | C3AR1 + IL1R2 + IFNG + S100A9 + CIITA | 0,824 | 0,675 | 0,973 |
| 4 jours | C3AR1 + IL1R2 + IFNG + S100A9 + CIITA + CD3D | 0,848 | 0,712 | 0,983 |
| 4 jours | C3AR1 + IL1R2 + IFNG + S100A9 + CIITA + CD3D + CTLA4 | 0,848 | 0,712 | 0,983 |
| 4 jours | C3AR1 + IL1R2 + IFNG + S100A9 + CIITA + CTLA4 | 0,816 | 0,668 | 0,965 |
| 4 jours | C3AR1 + IL1R2 + IFNG + S100A9 + CIITA + TAP2 | 0,844 | 0,699 | 0,989 |
| 4 jours | C3AR1 + IL1R2 + IFNG + S100A9 + CIITA + TAP2 + CD3D | 0,844 | 0,704 | 0,983 |
| 4 jours | C3AR1 + IL1R2 + IFNG + S100A9 + CIITA + TAP2 + CD3D + CTLA4 | 0,848 | 0,711 | 0,984 |
| 4 jours | C3AR1 + IL1R2 + IFNG + S100A9 + CIITA + TAP2 + CTLA4 | 0,828 | 0,681 | 0,975 |
| 4 jours | C3AR1 + IL1R2 + IFNG + S100A9 + CTLA4 | 0,816 | 0,669 | 0,964 |
| 4 jours | C3AR1 + IL1R2 + IFNG + S100A9 + CX3CR1 | 0,918 | 0,82 | 1 |
| 4 jours | C3AR1 + IL1R2 + IFNG + S100A9 + CX3CR1 + CD3D | 0,941 | 0,867 | 1 |
| 4 jours | C3AR1 + IL1R2 + IFNG + S100A9 + CX3CR1 + CD3D + CTLA4 | 0,938 | 0,861 | 1 |
| 4 jours | C3AR1 + IL1R2 + IFNG + S100A9 + CX3CR1 + CIITA | 0,918 | 0,82 | 1 |
| 4 jours | C3AR1 + IL1R2 + IFNG + S100A9 + CX3CR1 + CIITA + CD3D | 0,941 | 0,867 | 1 |
| 4 jours | C3AR1 + IL1R2 + IFNG + S100A9 + CX3CR1 + CIITA + CD3D + CTLA4 | 0,941 | 0,867 | 1 |
| 4 jours | C3AR1 + IL1R2 + IFNG + S100A9 + CX3CR1 + CIITA + CTLA4 | 0,922 | 0,832 | 1 |
| 4 jours | C3AR1 + IL1R2 + IFNG + S100A9 + CX3CR1 + CIITA + TAP2 | 0,918 | 0,82 | 1 |
| 4 jours | C3AR1 + IL1R2 + IFNG + S100A9 + CX3CR1 + CIITA + TAP2 + CD3D | 0,941 | 0,868 | 1 |
| 4 jours | C3AR1 + IL1R2 + IFNG + S100A9 + CX3CR1 + CIITA + TAP2 + CD3D + CTLA4 | 0,938 | 0,858 | 1 |
| 4 jours | C3AR1 + IL1R2 + IFNG + S100A9 + CX3CR1 + CIITA + TAP2 + CTLA4 | 0,918 | 0,82 | 1 |
| 4 jours | C3AR1 + IL1R2 + IFNG + S100A9 + CX3CR1 + CTLA4 | 0,922 | 0,833 | 1 |
| 4 jours | C3AR1 + IL1R2 + IFNG + S100A9 + CX3CR1 + TAP2 | 0,918 | 0,82 | 1 |
| 4 jours | C3AR1 + IL1R2 + IFNG + S100A9 + CX3CR1 + TAP2 + CD3D | 0,941 | 0,868 | 1 |
| 4 jours | C3AR1 + IL1R2 + IFNG + S100A9 + CX3CR1 + TAP2 + CD3D + CTLA4 | 0,941 | 0,867 | 1 |
| 4 jours | C3AR1 + IL1R2 + IFNG + S100A9 + CX3CR1 + TAP2 + CTLA4 | 0,914 | 0,817 | 1 |
| 4 jours | C3AR1 + IL1R2 + IFNG + S100A9 + TAP2 | 0,832 | 0,685 | 0,979 |
| 4 jours | C3AR1 + IL1R2 + IFNG + S100A9 + TAP2 + CD3D | 0,852 | 0,718 | 0,985 |
| 4 jours | C3AR1 + IL1R2 + IFNG + S100A9 + TAP2 + CD3D + CTLA4 | 0,848 | 0,713 | 0,983 |
| 4 jours | C3AR1 + IL1R2 + IFNG + S100A9 + TAP2 + CTLA4 | 0,812 | 0,661 | 0,964 |
| 4 jours | C3AR1 + IL1R2 + IFNG + TAP2 | 0,711 | 0,524 | 0,898 |
| 4 jours | C3AR1 + IL1R2 + IFNG + TAP2 + CD3D | 0,723 | 0,538 | 0,907 |
| 4 jours | C3AR1 + IL1R2 + IFNG + TAP2 + CD3D + CTLA4 | 0,742 | 0,567 | 0,917 |
| 4 jours | C3AR1 + IL1R2 + IFNG + TAP2 + CTLA4 | 0,754 | 0,583 | 0,925 |
| 4 jours | C3AR1 + IL1R2 + S100A9 | 0,824 | 0,67 | 0,979 |
| 4 jours | C3AR1 + IL1R2 + S100A9 + CD3D | 0,84 | 0,7 | 0,979 |
| 4 jours | C3AR1 + IL1R2 + S100A9 + CD3D + CTLA4 | 0,844 | 0,705 | 0,982 |
| 4 jours | C3AR1 + IL1R2 + S100A9 + CIITA | 0,824 | 0,67 | 0,979 |
| 4 jours | C3AR1 + IL1R2 + S100A9 + CIITA + CD3D | 0,848 | 0,715 | 0,98 |
| 4 jours | C3AR1 + IL1R2 + S100A9 + CIITA + CD3D + CTLA4 | 0,832 | 0,689 | 0,975 |
| 4 jours | C3AR1 + IL1R2 + S100A9 + CIITA + CTLA4 | 0,816 | 0,661 | 0,972 |
| 4 jours | C3AR1 + IL1R2 + S100A9 + CIITA + TAP2 | 0,82 | 0,665 | 0,976 |
| 4 jours | C3AR1 + IL1R2 + S100A9 + CIITA + TAP2 + CD3D | 0,844 | 0,706 | 0,981 |
| 4 jours | C3AR1 + IL1R2 + S100A9 + CIITA + TAP2 + CD3D + CTLA4 | 0,844 | 0,706 | 0,982 |
| 4 jours | C3AR1 + IL1R2 + S100A9 + CIITA + TAP2 + CTLA4 | 0,816 | 0,661 | 0,972 |
| 4 jours | C3AR1 + IL1R2 + S100A9 + CTLA4 | 0,82 | 0,667 | 0,974 |
| 4 jours | C3AR1 + IL1R2 + S100A9 + CX3CR1 | 0,852 | 0,71 | 0,993 |
| 4 jours | C3AR1 + IL1R2 + S100A9 + CX3CR1 + CD3D | 0,898 | 0,781 | 1 |
| 4 jours | C3AR1 + IL1R2 + S100A9 + CX3CR1 + CD3D + CTLA4 | 0,887 | 0,762 | 1 |
| 4 jours | C3AR1 + IL1R2 + S100A9 + CX3CR1 + CIITA | 0,867 | 0,732 | 1 |
| 4 jours | C3AR1 + IL1R2 + S100A9 + CX3CR1 + CIITA + CD3D | 0,895 | 0,779 | 1 |
| 4 jours | C3AR1 + IL1R2 + S100A9 + CX3CR1 + CIITA + CD3D + CTLA4 | 0,902 | 0,795 | 1 |
| 4 jours | C3AR1 + IL1R2 + S100A9 + CX3CR1 + CIITA + CTLA4 | 0,883 | 0,751 | 1 |
| 4 jours | C3AR1 + IL1R2 + S100A9 + CX3CR1 + CIITA + TAP2 | 0,883 | 0,751 | 1 |
| 4 jours | C3AR1 + IL1R2 + S100A9 + CX3CR1 + CIITA + TAP2 + CD3D | 0,898 | 0,786 | 1 |
| 4 jours | C3AR1 + IL1R2 + S100A9 + CX3CR1 + CIITA + TAP2 + CD3D + CTLA4 | 0,914 | 0,815 | 1 |
| 4 jours | C3AR1 + IL1R2 + S100A9 + CX3CR1 + CIITA + TAP2 + CTLA4 | 0,883 | 0,751 | 1 |
| 4 jours | C3AR1 + IL1R2 + S100A9 + CX3CR1 + CTLA4 | 0,863 | 0,727 | 1 |
| 4 jours | C3AR1 + IL1R2 + S100A9 + CX3CR1 + TAP2 | 0,875 | 0,745 | 1 |
| 4 jours | C3AR1 + IL1R2 + S100A9 + CX3CR1 + TAP2 + CD3D | 0,895 | 0,773 | 1 |
| 4 jours | C3AR1 + IL1R2 + S100A9 + CX3CR1 + TAP2 + CD3D + CTLA4 | 0,875 | 0,746 | 1 |
| 4 jours | C3AR1 + IL1R2 + S100A9 + CX3CR1 + TAP2 + CTLA4 | 0,879 | 0,747 | 1 |
| 4 jours | C3AR1 + IL1R2 + S100A9 + TAP2 | 0,816 | 0,658 | 0,975 |
| 4 jours | C3AR1 + IL1R2 + S100A9 + TAP2 + CD3D | 0,84 | 0,701 | 0,979 |
| 4 jours | C3AR1 + IL1R2 + S100A9 + TAP2 + CD3D + CTLA4 | 0,848 | 0,713 | 0,983 |
| 4 jours | C3AR1 + IL1R2 + S100A9 + TAP2 + CTLA4 | 0,816 | 0,661 | 0,972 |
| 4 jours | C3AR1 + IL1R2 + TAP2 | 0,68 | 0,488 | 0,871 |
| 4 jours | C3AR1 + IL1R2 + TAP2 + CD3D | 0,715 | 0,523 | 0,907 |
| 4 jours | C3AR1 + IL1R2 + TAP2 + CD3D + CTLA4 | 0,715 | 0,535 | 0,895 |
| 4 jours | C3AR1 + IL1R2 + TAP2 + CTLA4 | 0,711 | 0,53 | 0,892 |
| 4 jours | C3AR1 + S100A9 | 0,793 | 0,63 | 0,956 |
| 4 jours | C3AR1 + S100A9 + CD3D | 0,781 | 0,617 | 0,946 |
| 4 jours | C3AR1 + S100A9 + CD3D + CTLA4 | 0,789 | 0,628 | 0,95 |
| 4 jours | C3AR1 + S100A9 + CIITA | 0,801 | 0,637 | 0,965 |
| 4 jours | C3AR1 + S100A9 + CIITA + CD3D | 0,773 | 0,603 | 0,944 |
| 4 jours | C3AR1 + S100A9 + CIITA + CD3D + CTLA4 | 0,785 | 0,618 | 0,952 |
| 4 jours | C3AR1 + S100A9 + CIITA + CTLA4 | 0,785 | 0,616 | 0,954 |
| 4 jours | C3AR1 + S100A9 + CIITA + TAP2 | 0,805 | 0,63 | 0,979 |
| 4 jours | C3AR1 + S100A9 + CIITA + TAP2 + CD3D | 0,785 | 0,616 | 0,954 |
| 4 jours | C3AR1 + S100A9 + CIITA + TAP2 + CD3D + CTLA4 | 0,797 | 0,631 | 0,963 |
| 4 jours | C3AR1 + S100A9 + CIITA + TAP2 + CTLA4 | 0,812 | 0,649 | 0,976 |
| 4 jours | C3AR1 + S100A9 + CTLA4 | 0,789 | 0,627 | 0,951 |
| 4 jours | C3AR1 + S100A9 + CX3CR1 | 0,863 | 0,719 | 1 |
| 4 jours | C3AR1 + S100A9 + CX3CR1 + CD3D | 0,887 | 0,756 | 1 |
| 4 jours | C3AR1 + S100A9 + CX3CR1 + CD3D + CTLA4 | 0,879 | 0,748 | 1 |
| 4 jours | C3AR1 + S100A9 + CX3CR1 + CIITA | 0,871 | 0,736 | 1 |
| 4 jours | C3AR1 + S100A9 + CX3CR1 + CIITA + CD3D | 0,879 | 0,742 | 1 |
| 4 jours | C3AR1 + S100A9 + CX3CR1 + CIITA + CD3D + CTLA4 | 0,891 | 0,768 | 1 |
| 4 jours | C3AR1 + S100A9 + CX3CR1 + CIITA + CTLA4 | 0,875 | 0,741 | 1 |
| 4 jours | C3AR1 + S100A9 + CX3CR1 + CIITA + TAP2 | 0,875 | 0,734 | 1 |
| 4 jours | C3AR1 + S100A9 + CX3CR1 + CIITA + TAP2 + CD3D | 0,883 | 0,747 | 1 |
| 4 jours | C3AR1 + S100A9 + CX3CR1 + CIITA + TAP2 + CD3D + CTLA4 | 0,887 | 0,76 | 1 |
| 4 jours | C3AR1 + S100A9 + CX3CR1 + CIITA + TAP2 + CTLA4 | 0,875 | 0,73 | 1 |
| 4 jours | C3AR1 + S100A9 + CX3CR1 + CTLA4 | 0,871 | 0,729 | 1 |
| 4 jours | C3AR1 + S100A9 + CX3CR1 + TAP2 | 0,871 | 0,726 | 1 |
| 4 jours | C3AR1 + S100A9 + CX3CR1 + TAP2 + CD3D | 0,887 | 0,756 | 1 |
| 4 jours | C3AR1 + S100A9 + CX3CR1 + TAP2 + CD3D + CTLA4 | 0,875 | 0,743 | 1 |
| 4 jours | C3AR1 + S100A9 + CX3CR1 + TAP2 + CTLA4 | 0,875 | 0,73 | 1 |
| 4 jours | C3AR1 + S100A9 + TAP2 | 0,812 | 0,651 | 0,974 |
| 4 jours | C3AR1 + S100A9 + TAP2 + CD3D | 0,797 | 0,631 | 0,963 |
| 4 jours | C3AR1 + S100A9 + TAP2 + CD3D + CTLA4 | 0,801 | 0,637 | 0,965 |
| 4 jours | C3AR1 + S100A9 + TAP2 + CTLA4 | 0,816 | 0,66 | 0,973 |
| 4 jours | C3AR1 + TAP2 + CD3D + CTLA4 | 0,695 | 0,505 | 0,885 |
| 7 jours | C3AR1 + 121601801-HERV0492 | 0,633 | 0,54 | 0,726 |
| 7 jours | C3AR1 + IL18RAP | 0,633 | 0,541 | 0,725 |
| 7 jours | C3AR1 + IL18 | 0,634 | 0,542 | 0,726 |
| 7 jours | C3AR1 + BTLA | 0,635 | 0,543 | 0,727 |
| 7 jours | C3AR1 + 050286701-HERV0513 | 0,635 | 0,544 | 0,727 |
| 7 jours | C3AR1 + OAS3 | 0,636 | 0,544 | 0,728 |
| 7 jours | C3AR1 + 011052202-HERV1033 | 0,643 | 0,552 | 0,734 |
| 7 jours | C3AR1 + 011052702-MALR1044 | 0,644 | 0,552 | 0,736 |
| 7 jours | C3AR1 + CD3D | 0,646 | 0,555 | 0,737 |
| 7 jours | C3AR1 + IL7R | 0,646 | 0,555 | 0,738 |
| 7 jours | C3AR1 + STAT4 | 0,649 | 0,558 | 0,741 |
| 7 jours | C3AR1 + CD274 | 0,651 | 0,559 | 0,742 |
| 7 jours | C3AR1 + CD40LG | 0,655 | 0,565 | 0,746 |
| 7 jours | C3AR1 + 190665001-HERV0376 | 0,658 | 0,568 | 0,748 |
| 7 jours | C3AR1 + IL10 | 0,665 | 0,572 | 0,757 |
| 7 jours | C3AR1 + 170369402HE41 | 0,666 | 0,575 | 0,757 |
| 7 jours | C3AR1 + 220247002-HERV0797 | 0,667 | 0,577 | 0,757 |
| 7 jours | C3AR1 + IL1RN | 0,667 | 0,576 | 0,758 |
| 7 jours | C3AR1 + 130360601-HERV0808 | 0,677 | 0,579 | 0,774 |
| 7 jours | C3AR1 + CD74 | 0,682 | 0,595 | 0,769 |
| 7 jours | C3AR1 + S100A9 | 0,686 | 0,598 | 0,773 |
| 7 jours | C3AR1 + CX3CR1 | 0,688 | 0,601 | 0,776 |
| 7 jours | C3AR1 + 021456001-MALR1017 | 0,69 | 0,602 | 0,778 |
| 7 jours | C3AR1 + IL18R1 | 0,69 | 0,603 | 0,777 |
| 7 jours | C3AR1 + IL15 | 0,691 | 0,597 | 0,785 |
| 7 jours | C3AR1 + IFNG | 0,692 | 0,603 | 0,781 |
| 7 jours | C3AR1 + IFIH1 | 0,7 | 0,589 | 0,811 |
| 7 jours | C3AR1 + IL1R2 | 0,706 | 0,62 | 0,793 |
| 7 jours | C3AR1 + HP | 0,709 | 0,624 | 0,794 |
| 7 jours | C3AR1 + CD177 | 0,723 | 0,561 | 0,885 |
| 7 jours | C3AR1 + CD177 + CD3D | 0,726 | 0,565 | 0,887 |
| 7 jours | C3AR1 + CD177 + CD3D + CTLA4 | 0,731 | 0,571 | 0,892 |
| 7 jours | C3AR1 + CD177 + CIITA | 0,85 | 0,725 | 0,976 |
| 7 jours | C3AR1 + CD177 + CIITA + CD3D | 0,856 | 0,733 | 0,979 |
| 7 jours | C3AR1 + CD177 + CIITA + CD3D + CTLA4 | 0,886 | 0,783 | 0,989 |
| 7 jours | C3AR1 + CD177 + CIITA + CTLA4 | 0,875 | 0,764 | 0,987 |
| 7 jours | C3AR1 + CD177 + CIITA + TAP2 | 0,85 | 0,724 | 0,977 |
| 7 jours | C3AR1 + CD177 + CIITA + TAP2 + CD3D | 0,859 | 0,737 | 0,98 |
| 7 jours | C3AR1 + CD177 + CIITA + TAP2 + CD3D + CTLA4 | 0,881 | 0,774 | 0,988 |
| 7 jours | C3AR1 + CD177 + CIITA + TAP2 + CTLA4 | 0,875 | 0,764 | 0,987 |
| 7 jours | C3AR1 + CD177 + CTLA4 | 0,717 | 0,552 | 0,883 |
| 7 jours | C3AR1 + CD177 + CX3CR1 | 0,734 | 0,573 | 0,895 |
| 7 jours | C3AR1 + CD177 + CX3CR1 + CD3D | 0,734 | 0,575 | 0,893 |
| 7 jours | C3AR1 + CD177 + CX3CR1 + CD3D + CTLA4 | 0,723 | 0,56 | 0,886 |
| 7 jours | C3AR1 + CD177 + CX3CR1 + CIITA | 0,853 | 0,727 | 0,979 |
| 7 jours | C3AR1 + CD177 + CX3CR1 + CIITA + CD3D | 0,853 | 0,729 | 0,977 |
| 7 jours | C3AR1 + CD177 + CX3CR1 + CIITA + CD3D + CTLA4 | 0,889 | 0,786 | 0,992 |
| 7 jours | C3AR1 + CD177 + CX3CR1 + CIITA + CTLA4 | 0,87 | 0,756 | 0,984 |
| 7 jours | C3AR1 + CD177 + CX3CR1 + CIITA + TAP2 | 0,856 | 0,731 | 0,981 |
| 7 jours | C3AR1 + CD177 + CX3CR1 + CIITA + TAP2 + CD3D | 0,853 | 0,727 | 0,979 |
| 7 jours | C3AR1 + CD177 + CX3CR1 + CIITA + TAP2 + CD3D + CTLA4 | 0,892 | 0,79 | 0,994 |
| 7 jours | C3AR1 + CD177 + CX3CR1 + CIITA + TAP2 + CTLA4 | 0,873 | 0,759 | 0,986 |
| 7 jours | C3AR1 + CD177 + CX3CR1 + CTLA4 | 0,731 | 0,569 | 0,894 |
| 7 jours | C3AR1 + CD177 + CX3CR1 + TAP2 | 0,77 | 0,614 | 0,926 |
| 7 jours | C3AR1 + CD177 + CX3CR1 + TAP2 + CD3D | 0,767 | 0,613 | 0,922 |
| 7 jours | C3AR1 + CD177 + CX3CR1 + TAP2 + CD3D + CTLA4 | 0,77 | 0,614 | 0,926 |
| 7 jours | C3AR1 + CD177 + CX3CR1 + TAP2 + CTLA4 | 0,767 | 0,609 | 0,926 |
| 7 jours | C3AR1 + CD177 + S100A9 | 0,762 | 0,605 | 0,918 |
| 7 jours | C3AR1 + CD177 + S100A9 + CD3D | 0,767 | 0,615 | 0,92 |
| 7 jours | C3AR1 + CD177 + S100A9 + CD3D + CTLA4 | 0,756 | 0,602 | 0,91 |
| 7 jours | C3AR1 + CD177 + S100A9 + CIITA | 0,853 | 0,729 | 0,977 |
| 7 jours | C3AR1 + CD177 + S100A9 + CIITA + CD3D | 0,859 | 0,736 | 0,981 |
| 7 jours | C3AR1 + CD177 + S100A9 + CIITA + CD3D + CTLA4 | 0,878 | 0,77 | 0,986 |
| 7 jours | C3AR1 + CD177 + S100A9 + CIITA + CTLA4 | 0,875 | 0,764 | 0,987 |
| 7 jours | C3AR1 + CD177 + S100A9 + CIITA + TAP2 | 0,856 | 0,734 | 0,978 |
| 7 jours | C3AR1 + CD177 + S100A9 + CIITA + TAP2 + CD3D | 0,859 | 0,736 | 0,981 |
| 7 jours | C3AR1 + CD177 + S100A9 + CIITA + TAP2 + CD3D + CTLA4 | 0,886 | 0,783 | 0,99 |
| 7 jours | C3AR1 + CD177 + S100A9 + CIITA + TAP2 + CTLA4 | 0,878 | 0,768 | 0,988 |
| 7 jours | C3AR1 + CD177 + S100A9 + CTLA4 | 0,765 | 0,608 | 0,921 |
| 7 jours | C3AR1 + CD177 + S100A9 + CX3CR1 | 0,77 | 0,616 | 0,924 |
| 7 jours | C3AR1 + CD177 + S100A9 + CX3CR1 + CD3D | 0,784 | 0,636 | 0,932 |
| 7 jours | C3AR1 + CD177 + S100A9 + CX3CR1 + CD3D + CTLA4 | 0,773 | 0,621 | 0,925 |
| 7 jours | C3AR1 + CD177 + S100A9 + CX3CR1 + CIITA | 0,856 | 0,733 | 0,979 |
| 7 jours | C3AR1 + CD177 + S100A9 + CX3CR1 + CIITA + CD3D | 0,867 | 0,747 | 0,987 |
| 7 jours | C3AR1 + CD177 + S100A9 + CX3CR1 + CIITA + CD3D + CTLA4 | 0,898 | 0,799 | 0,996 |
| 7 jours | C3AR1 + CD177 + S100A9 + CX3CR1 + CIITA + CTLA4 | 0,873 | 0,761 | 0,985 |
| 7 jours | C3AR1 + CD177 + S100A9 + CX3CR1 + CIITA + TAP2 | 0,859 | 0,735 | 0,982 |
| 7 jours | C3AR1 + CD177 + S100A9 + CX3CR1 + CIITA + TAP2 + CD3D | 0,856 | 0,733 | 0,979 |
| 7 jours | C3AR1 + CD177 + S100A9 + CX3CR1 + CIITA + TAP2 + CD3D + CTLA4 | 0,898 | 0,799 | 0,996 |
| 7 jours | C3AR1 + CD177 + S100A9 + CX3CR1 + CIITA + TAP2 + CTLA4 | 0,881 | 0,774 | 0,988 |
| 7 jours | C3AR1 + CD177 + S100A9 + CX3CR1 + CTLA4 | 0,77 | 0,616 | 0,924 |
| 7 jours | C3AR1 + CD177 + S100A9 + CX3CR1 + TAP2 | 0,781 | 0,629 | 0,933 |
| 7 jours | C3AR1 + CD177 + S100A9 + CX3CR1 + TAP2 + CD3D | 0,781 | 0,631 | 0,932 |
| 7 jours | C3AR1 + CD177 + S100A9 + CX3CR1 + TAP2 + CD3D + CTLA4 | 0,784 | 0,637 | 0,931 |
| 7 jours | C3AR1 + CD177 + S100A9 + CX3CR1 + TAP2 + CTLA4 | 0,789 | 0,64 | 0,939 |
| 7 jours | C3AR1 + CD177 + S100A9 + TAP2 | 0,792 | 0,643 | 0,941 |
| 7 jours | C3AR1 + CD177 + S100A9 + TAP2 + CD3D | 0,789 | 0,644 | 0,935 |
| 7 jours | C3AR1 + CD177 + S100A9 + TAP2 + CD3D + CTLA4 | 0,789 | 0,644 | 0,935 |
| 7 jours | C3AR1 + CD177 + S100A9 + TAP2 + CTLA4 | 0,792 | 0,643 | 0,941 |
| 7 jours | C3AR1 + CD177 + TAP2 | 0,767 | 0,611 | 0,924 |
| 7 jours | C3AR1 + CD177 + TAP2 + CD3D | 0,762 | 0,606 | 0,918 |
| 7 jours | C3AR1 + CD177 + TAP2 + CD3D + CTLA4 | 0,77 | 0,615 | 0,925 |
| 7 jours | C3AR1 + CD177 + TAP2 + CTLA4 | 0,767 | 0,612 | 0,923 |
| 7 jours | C3AR1 + CD74 + CD177 | 0,812 | 0,669 | 0,954 |
| 7 jours | C3AR1 + CD74 + CD177 + CD3D | 0,803 | 0,661 | 0,946 |
| 7 jours | C3AR1 + CD74 + CD177 + CD3D + CTLA4 | 0,803 | 0,661 | 0,946 |
| 7 jours | C3AR1 + CD74 + CD177 + CIITA | 0,87 | 0,752 | 0,987 |
| 7 jours | C3AR1 + CD74 + CD177 + CIITA + CD3D | 0,875 | 0,76 | 0,991 |
| 7 jours | C3AR1 + CD74 + CD177 + CIITA + CD3D + CTLA4 | 0,889 | 0,787 | 0,992 |
| 7 jours | C3AR1 + CD74 + CD177 + CIITA + CTLA4 | 0,875 | 0,765 | 0,985 |
| 7 jours | C3AR1 + CD74 + CD177 + CIITA + TAP2 | 0,87 | 0,752 | 0,987 |
| 7 jours | C3AR1 + CD74 + CD177 + CIITA + TAP2 + CD3D | 0,87 | 0,753 | 0,987 |
| 7 jours | C3AR1 + CD74 + CD177 + CIITA + TAP2 + CD3D + CTLA4 | 0,898 | 0,8 | 0,995 |
| 7 jours | C3AR1 + CD74 + CD177 + CIITA + TAP2 + CTLA4 | 0,873 | 0,761 | 0,985 |
| 7 jours | C3AR1 + CD74 + CD177 + CTLA4 | 0,817 | 0,678 | 0,956 |
| 7 jours | C3AR1 + CD74 + CD177 + CX3CR1 | 0,823 | 0,684 | 0,961 |
| 7 jours | C3AR1 + CD74 + CD177 + CX3CR1 + CD3D | 0,812 | 0,671 | 0,952 |
| 7 jours | C3AR1 + CD74 + CD177 + CX3CR1 + CD3D + CTLA4 | 0,812 | 0,672 | 0,951 |
| 7 jours | C3AR1 + CD74 + CD177 + CX3CR1 + CIITA | 0,878 | 0,767 | 0,99 |
| 7 jours | C3AR1 + CD74 + CD177 + CX3CR1 + CIITA + CD3D | 0,878 | 0,767 | 0,989 |
| 7 jours | C3AR1 + CD74 + CD177 + CX3CR1 + CIITA + CD3D + CTLA4 | 0,892 | 0,792 | 0,992 |
| 7 jours | C3AR1 + CD74 + CD177 + CX3CR1 + CIITA + CTLA4 | 0,875 | 0,763 | 0,988 |
| 7 jours | C3AR1 + CD74 + CD177 + CX3CR1 + CIITA + TAP2 | 0,875 | 0,762 | 0,989 |
| 7 jours | C3AR1 + CD74 + CD177 + CX3CR1 + CIITA + TAP2 + CD3D | 0,873 | 0,758 | 0,987 |
| 7 jours | C3AR1 + CD74 + CD177 + CX3CR1 + CIITA + TAP2 + CD3D + CTLA4 | 0,898 | 0,801 | 0,994 |
| 7 jours | C3AR1 + CD74 + CD177 + CX3CR1 + CIITA + TAP2 + CTLA4 | 0,875 | 0,763 | 0,987 |
| 7 jours | C3AR1 + CD74 + CD177 + CX3CR1 + CTLA4 | 0,823 | 0,683 | 0,962 |
| 7 jours | C3AR1 + CD74 + CD177 + CX3CR1 + TAP2 | 0,817 | 0,675 | 0,96 |
| 7 jours | C3AR1 + CD74 + CD177 + CX3CR1 + TAP2 + CD3D | 0,82 | 0,684 | 0,956 |
| 7 jours | C3AR1 + CD74 + CD177 + CX3CR1 + TAP2 + CD3D + CTLA4 | 0,82 | 0,684 | 0,956 |
| 7 jours | C3AR1 + CD74 + CD177 + CX3CR1 + TAP2 + CTLA4 | 0,825 | 0,685 | 0,966 |
| 7 jours | C3AR1 + CD74 + CD177 + S100A9 | 0,825 | 0,685 | 0,966 |
| 7 jours | C3AR1 + CD74 + CD177 + S100A9 + CD3D | 0,831 | 0,696 | 0,966 |
| 7 jours | C3AR1 + CD74 + CD177 + S100A9 + CD3D + CTLA4 | 0,828 | 0,692 | 0,964 |
| 7 jours | C3AR1 + CD74 + CD177 + S100A9 + CIITA | 0,873 | 0,757 | 0,988 |
| 7 jours | C3AR1 + CD74 + CD177 + S100A9 + CIITA + CD3D | 0,87 | 0,753 | 0,987 |
| 7 jours | C3AR1 + CD74 + CD177 + S100A9 + CIITA + CD3D + CTLA4 | 0,889 | 0,787 | 0,992 |
| 7 jours | C3AR1 + CD74 + CD177 + S100A9 + CIITA + CTLA4 | 0,878 | 0,77 | 0,987 |
| 7 jours | C3AR1 + CD74 + CD177 + S100A9 + CIITA + TAP2 | 0,878 | 0,765 | 0,991 |
| 7 jours | C3AR1 + CD74 + CD177 + S100A9 + CIITA + TAP2 + CD3D | 0,875 | 0,761 | 0,99 |
| 7 jours | C3AR1 + CD74 + CD177 + S100A9 + CIITA + TAP2 + CD3D + CTLA4 | 0,9 | 0,802 | 0,998 |
| 7 jours | C3AR1 + CD74 + CD177 + S100A9 + CIITA + TAP2 + CTLA4 | 0,878 | 0,77 | 0,987 |
| 7 jours | C3AR1 + CD74 + CD177 + S100A9 + CTLA4 | 0,825 | 0,685 | 0,966 |
| 7 jours | C3AR1 + CD74 + CD177 + S100A9 + CX3CR1 | 0,853 | 0,718 | 0,989 |
| 7 jours | C3AR1 + CD74 + CD177 + S100A9 + CX3CR1 + CD3D | 0,845 | 0,708 | 0,981 |
| 7 jours | C3AR1 + CD74 + CD177 + S100A9 + CX3CR1 + CD3D + CTLA4 | 0,85 | 0,715 | 0,986 |
| 7 jours | C3AR1 + CD74 + CD177 + S100A9 + CX3CR1 + CIITA | 0,881 | 0,766 | 0,995 |
| 7 jours | C3AR1 + CD74 + CD177 + S100A9 + CX3CR1 + CIITA + CD3D | 0,875 | 0,758 | 0,993 |
| 7 jours | C3AR1 + CD74 + CD177 + S100A9 + CX3CR1 + CIITA + CD3D + CTLA4 | 0,889 | 0,784 | 0,994 |
| 7 jours | C3AR1 + CD74 + CD177 + S100A9 + CX3CR1 + CIITA + CTLA4 | 0,875 | 0,763 | 0,988 |
| 7 jours | C3AR1 + CD74 + CD177 + S100A9 + CX3CR1 + CIITA + TAP2 | 0,881 | 0,769 | 0,993 |
| 7 jours | C3AR1 + CD74 + CD177 + S100A9 + CX3CR1 + CIITA + TAP2 + CD3D | 0,878 | 0,761 | 0,995 |
| 7 jours | C3AR1 + CD74 + CD177 + S100A9 + CX3CR1 + CIITA + TAP2 + CD3D + CTLA4 | 0,898 | 0,8 | 0,995 |
| 7 jours | C3AR1 + CD74 + CD177 + S100A9 + CX3CR1 + CIITA + TAP2 + CTLA4 | 0,884 | 0,774 | 0,993 |
| 7 jours | C3AR1 + CD74 + CD177 + S100A9 + CX3CR1 + CTLA4 | 0,856 | 0,722 | 0,99 |
| 7 jours | C3AR1 + CD74 + CD177 + S100A9 + CX3CR1 + TAP2 | 0,856 | 0,72 | 0,992 |
| 7 jours | C3AR1 + CD74 + CD177 + S100A9 + CX3CR1 + TAP2 + CD3D | 0,853 | 0,719 | 0,987 |
| 7 jours | C3AR1 + CD74 + CD177 + S100A9 + CX3CR1 + TAP2 + CD3D + CTLA4 | 0,85 | 0,715 | 0,986 |
| 7 jours | C3AR1 + CD74 + CD177 + S100A9 + CX3CR1 + TAP2 + CTLA4 | 0,859 | 0,723 | 0,994 |
| 7 jours | C3AR1 + CD74 + CD177 + S100A9 + TAP2 | 0,823 | 0,68 | 0,966 |
| 7 jours | C3AR1 + CD74 + CD177 + S100A9 + TAP2 + CD3D | 0,831 | 0,696 | 0,966 |
| 7 jours | C3AR1 + CD74 + CD177 + S100A9 + TAP2 + CD3D + CTLA4 | 0,828 | 0,693 | 0,964 |
| 7 jours | C3AR1 + CD74 + CD177 + S100A9 + TAP2 + CTLA4 | 0,823 | 0,68 | 0,966 |
| 7 jours | C3AR1 + CD74 + CD177 + TAP2 | 0,82 | 0,682 | 0,958 |
| 7 jours | C3AR1 + CD74 + CD177 + TAP2 + CD3D | 0,814 | 0,675 | 0,954 |
| 7 jours | C3AR1 + CD74 + CD177 + TAP2 + CD3D + CTLA4 | 0,809 | 0,667 | 0,95 |
| 7 jours | C3AR1 + CD74 + CD177 + TAP2 + CTLA4 | 0,817 | 0,677 | 0,958 |
| 7 jours | C3AR1 + CD74 + CD3D | 0,745 | 0,586 | 0,905 |
| 7 jours | C3AR1 + CD74 + CD3D + CTLA4 | 0,745 | 0,586 | 0,904 |
| 7 jours | C3AR1 + CD74 + CIITA | 0,861 | 0,74 | 0,983 |
| 7 jours | C3AR1 + CD74 + CIITA + CD3D | 0,853 | 0,728 | 0,979 |
| 7 jours | C3AR1 + CD74 + CIITA + CD3D + CTLA4 | 0,867 | 0,753 | 0,981 |
| 7 jours | C3AR1 + CD74 + CIITA + CTLA4 | 0,878 | 0,766 | 0,99 |
| 7 jours | C3AR1 + CD74 + CIITA + TAP2 | 0,859 | 0,736 | 0,981 |
| 7 jours | C3AR1 + CD74 + CIITA + TAP2 + CD3D | 0,853 | 0,728 | 0,979 |
| 7 jours | C3AR1 + CD74 + CIITA + TAP2 + CD3D + CTLA4 | 0,87 | 0,757 | 0,983 |
| 7 jours | C3AR1 + CD74 + CIITA + TAP2 + CTLA4 | 0,881 | 0,769 | 0,992 |
| 7 jours | C3AR1 + CD74 + CTLA4 | 0,765 | 0,61 | 0,919 |
| 7 jours | C3AR1 + CD74 + CX3CR1 | 0,776 | 0,619 | 0,932 |
| 7 jours | C3AR1 + CD74 + CX3CR1 + CD3D | 0,767 | 0,616 | 0,919 |
| 7 jours | C3AR1 + CD74 + CX3CR1 + CD3D + CTLA4 | 0,767 | 0,616 | 0,919 |
| 7 jours | C3AR1 + CD74 + CX3CR1 + CIITA | 0,867 | 0,744 | 0,99 |
| 7 jours | C3AR1 + CD74 + CX3CR1 + CIITA + CD3D | 0,867 | 0,747 | 0,987 |
| 7 jours | C3AR1 + CD74 + CX3CR1 + CIITA + CD3D + CTLA4 | 0,87 | 0,756 | 0,984 |
| 7 jours | C3AR1 + CD74 + CX3CR1 + CIITA + CTLA4 | 0,873 | 0,754 | 0,991 |
| 7 jours | C3AR1 + CD74 + CX3CR1 + CIITA + TAP2 | 0,864 | 0,74 | 0,989 |
| 7 jours | C3AR1 + CD74 + CX3CR1 + CIITA + TAP2 + CD3D | 0,864 | 0,743 | 0,986 |
| 7 jours | C3AR1 + CD74 + CX3CR1 + CIITA + TAP2 + CD3D + CTLA4 | 0,87 | 0,756 | 0,984 |
| 7 jours | C3AR1 + CD74 + CX3CR1 + CIITA + TAP2 + CTLA4 | 0,87 | 0,749 | 0,991 |
| 7 jours | C3AR1 + CD74 + CX3CR1 + CTLA4 | 0,776 | 0,623 | 0,929 |
| 7 jours | C3AR1 + CD74 + CX3CR1 + TAP2 | 0,781 | 0,627 | 0,936 |
| 7 jours | C3AR1 + CD74 + CX3CR1 + TAP2 + CD3D | 0,778 | 0,626 | 0,931 |
| 7 jours | C3AR1 + CD74 + CX3CR1 + TAP2 + CD3D + CTLA4 | 0,792 | 0,644 | 0,94 |
| 7 jours | C3AR1 + CD74 + CX3CR1 + TAP2 + CTLA4 | 0,798 | 0,647 | 0,948 |
| 7 jours | C3AR1 + CD74 + IFNG | 0,823 | 0,692 | 0,954 |
| 7 jours | C3AR1 + CD74 + IFNG + CD177 | 0,842 | 0,719 | 0,965 |
| 7 jours | C3AR1 + CD74 + IFNG + CD177 + CD3D | 0,839 | 0,715 | 0,964 |
| 7 jours | C3AR1 + CD74 + IFNG + CD177 + CD3D + CTLA4 | 0,845 | 0,723 | 0,967 |
| 7 jours | C3AR1 + CD74 + IFNG + CD177 + CIITA | 0,892 | 0,791 | 0,993 |
| 7 jours | C3AR1 + CD74 + IFNG + CD177 + CIITA + CD3D | 0,892 | 0,791 | 0,993 |
| 7 jours | C3AR1 + CD74 + IFNG + CD177 + CIITA + CD3D + CTLA4 | 0,892 | 0,795 | 0,989 |
| 7 jours | C3AR1 + CD74 + IFNG + CD177 + CIITA + CTLA4 | 0,886 | 0,786 | 0,987 |
| 7 jours | C3AR1 + CD74 + IFNG + CD177 + CIITA + TAP2 | 0,892 | 0,792 | 0,992 |
| 7 jours | C3AR1 + CD74 + IFNG + CD177 + CIITA + TAP2 + CD3D | 0,892 | 0,792 | 0,992 |
| 7 jours | C3AR1 + CD74 + IFNG + CD177 + CIITA + TAP2 + CD3D + CTLA4 | 0,892 | 0,794 | 0,99 |
| 7 jours | C3AR1 + CD74 + IFNG + CD177 + CIITA + TAP2 + CTLA4 | 0,9 | 0,807 | 0,994 |
| 7 jours | C3AR1 + CD74 + IFNG + CD177 + CTLA4 | 0,839 | 0,715 | 0,963 |
| 7 jours | C3AR1 + CD74 + IFNG + CD177 + CX3CR1 | 0,881 | 0,775 | 0,986 |
| 7 jours | C3AR1 + CD74 + IFNG + CD177 + CX3CR1 + CD3D | 0,886 | 0,784 | 0,989 |
| 7 jours | C3AR1 + CD74 + IFNG + CD177 + CX3CR1 + CD3D + CTLA4 | 0,886 | 0,782 | 0,991 |
| 7 jours | C3AR1 + CD74 + IFNG + CD177 + CX3CR1 + CIITA | 0,917 | 0,827 | 1 |
| 7 jours | C3AR1 + CD74 + IFNG + CD177 + CX3CR1 + CIITA + CD3D | 0,906 | 0,808 | 1 |
| 7 jours | C3AR1 + CD74 + IFNG + CD177 + CX3CR1 + CIITA + CD3D + CTLA4 | 0,911 | 0,817 | 1 |
| 7 jours | C3AR1 + CD74 + IFNG + CD177 + CX3CR1 + CIITA + CTLA4 | 0,917 | 0,827 | 1 |
| 7 jours | C3AR1 + CD74 + IFNG + CD177 + CX3CR1 + CIITA + TAP2 | 0,917 | 0,827 | 1 |
| 7 jours | C3AR1 + CD74 + IFNG + CD177 + CX3CR1 + CIITA + TAP2 + CD3D | 0,914 | 0,822 | 1 |
| 7 jours | C3AR1 + CD74 + IFNG + CD177 + CX3CR1 + CIITA + TAP2 + CD3D + CTLA4 | 0,928 | 0,844 | 1 |
| 7 jours | C3AR1 + CD74 + IFNG + CD177 + CX3CR1 + CIITA + TAP2 + CTLA4 | 0,914 | 0,823 | 1 |
| 7 jours | C3AR1 + CD74 + IFNG + CD177 + CX3CR1 + CTLA4 | 0,881 | 0,774 | 0,988 |
| 7 jours | C3AR1 + CD74 + IFNG + CD177 + CX3CR1 + TAP2 | 0,881 | 0,775 | 0,986 |
| 7 jours | C3AR1 + CD74 + IFNG + CD177 + CX3CR1 + TAP2 + CD3D | 0,892 | 0,79 | 0,994 |
| 7 jours | C3AR1 + CD74 + IFNG + CD177 + CX3CR1 + TAP2 + CD3D + CTLA4 | 0,889 | 0,786 | 0,992 |
| 7 jours | C3AR1 + CD74 + IFNG + CD177 + CX3CR1 + TAP2 + CTLA4 | 0,878 | 0,768 | 0,988 |
| 7 jours | C3AR1 + CD74 + IFNG + CD177 + S100A9 | 0,842 | 0,72 | 0,965 |
| 7 jours | C3AR1 + CD74 + IFNG + CD177 + S100A9 + CD3D | 0,845 | 0,723 | 0,967 |
| 7 jours | C3AR1 + CD74 + IFNG + CD177 + S100A9 + CD3D + CTLA4 | 0,842 | 0,719 | 0,965 |
| 7 jours | C3AR1 + CD74 + IFNG + CD177 + S100A9 + CIITA | 0,889 | 0,789 | 0,989 |
| 7 jours | C3AR1 + CD74 + IFNG + CD177 + S100A9 + CIITA + CD3D | 0,884 | 0,781 | 0,986 |
| 7 jours | C3AR1 + CD74 + IFNG + CD177 + S100A9 + CIITA + CD3D + CTLA4 | 0,886 | 0,786 | 0,987 |
| 7 jours | C3AR1 + CD74 + IFNG + CD177 + S100A9 + CIITA + CTLA4 | 0,889 | 0,79 | 0,988 |
| 7 jours | C3AR1 + CD74 + IFNG + CD177 + S100A9 + CIITA + TAP2 | 0,906 | 0,814 | 0,997 |
| 7 jours | C3AR1 + CD74 + IFNG + CD177 + S100A9 + CIITA + TAP2 + CD3D | 0,906 | 0,814 | 0,997 |
| 7 jours | C3AR1 + CD74 + IFNG + CD177 + S100A9 + CIITA + TAP2 + CD3D + CTLA4 | 0,892 | 0,794 | 0,99 |
| 7 jours | C3AR1 + CD74 + IFNG + CD177 + S100A9 + CIITA + TAP2 + CTLA4 | 0,895 | 0,798 | 0,992 |
| 7 jours | C3AR1 + CD74 + IFNG + CD177 + S100A9 + CTLA4 | 0,842 | 0,72 | 0,965 |
| 7 jours | C3AR1 + CD74 + IFNG + CD177 + S100A9 + CX3CR1 | 0,881 | 0,777 | 0,985 |
| 7 jours | C3AR1 + CD74 + IFNG + CD177 + S100A9 + CX3CR1 + CD3D | 0,881 | 0,776 | 0,986 |
| 7 jours | C3AR1 + CD74 + IFNG + CD177 + S100A9 + CX3CR1 + CD3D + CTLA4 | 0,886 | 0,784 | 0,989 |
| 7 jours | C3AR1 + CD74 + IFNG + CD177 + S100A9 + CX3CR1 + CIITA | 0,92 | 0,831 | 1 |
| 7 jours | C3AR1 + CD74 + IFNG + CD177 + S100A9 + CX3CR1 + CIITA + CD3D | 0,914 | 0,821 | 1 |
| 7 jours | C3AR1 + CD74 + IFNG + CD177 + S100A9 + CX3CR1 + CIITA + CD3D + CTLA4 | 0,909 | 0,813 | 1 |
| 7 jours | C3AR1 + CD74 + IFNG + CD177 + S100A9 + CX3CR1 + CIITA + CTLA4 | 0,922 | 0,835 | 1 |
| 7 jours | C3AR1 + CD74 + IFNG + CD177 + S100A9 + CX3CR1 + CIITA + TAP2 | 0,922 | 0,834 | 1 |
| 7 jours | C3AR1 + CD74 + IFNG + CD177 + S100A9 + CX3CR1 + CIITA + TAP2 + CD3D | 0,922 | 0,834 | 1 |
| 7 jours | C3AR1 + CD74 + IFNG + CD177 + S100A9 + CX3CR1 + CIITA + TAP2 + CD3D + CTLA4 | 0,925 | 0,84 | 1 |
| 7 jours | C3AR1 + CD74 + IFNG + CD177 + S100A9 + CX3CR1 + CIITA + TAP2 + CTLA4 | 0,922 | 0,835 | 1 |
| 7 jours | C3AR1 + CD74 + IFNG + CD177 + S100A9 + CX3CR1 + CTLA4 | 0,884 | 0,778 | 0,989 |
| 7 jours | C3AR1 + CD74 + IFNG + CD177 + S100A9 + CX3CR1 + TAP2 | 0,881 | 0,777 | 0,985 |
| 7 jours | C3AR1 + CD74 + IFNG + CD177 + S100A9 + CX3CR1 + TAP2 + CD3D | 0,878 | 0,771 | 0,985 |
| 7 jours | C3AR1 + CD74 + IFNG + CD177 + S100A9 + CX3CR1 + TAP2 + CD3D + CTLA4 | 0,881 | 0,775 | 0,987 |
| 7 jours | C3AR1 + CD74 + IFNG + CD177 + S100A9 + CX3CR1 + TAP2 + CTLA4 | 0,878 | 0,769 | 0,987 |
| 7 jours | C3AR1 + CD74 + IFNG + CD177 + S100A9 + TAP2 | 0,842 | 0,718 | 0,967 |
| 7 jours | C3AR1 + CD74 + IFNG + CD177 + S100A9 + TAP2 + CD3D | 0,834 | 0,707 | 0,96 |
| 7 jours | C3AR1 + CD74 + IFNG + CD177 + S100A9 + TAP2 + CD3D + CTLA4 | 0,839 | 0,715 | 0,964 |
| 7 jours | C3AR1 + CD74 + IFNG + CD177 + S100A9 + TAP2 + CTLA4 | 0,839 | 0,714 | 0,964 |
| 7 jours | C3AR1 + CD74 + IFNG + CD177 + TAP2 | 0,845 | 0,722 | 0,968 |
| 7 jours | C3AR1 + CD74 + IFNG + CD177 + TAP2 + CD3D | 0,839 | 0,715 | 0,964 |
| 7 jours | C3AR1 + CD74 + IFNG + CD177 + TAP2 + CD3D + CTLA4 | 0,839 | 0,715 | 0,964 |
| 7 jours | C3AR1 + CD74 + IFNG + CD177 + TAP2 + CTLA4 | 0,839 | 0,714 | 0,964 |
| 7 jours | C3AR1 + CD74 + IFNG + CD3D | 0,823 | 0,692 | 0,954 |
| 7 jours | C3AR1 + CD74 + IFNG + CD3D + CTLA4 | 0,831 | 0,703 | 0,959 |
| 7 jours | C3AR1 + CD74 + IFNG + CIITA | 0,87 | 0,758 | 0,981 |
| 7 jours | C3AR1 + CD74 + IFNG + CIITA + CD3D | 0,878 | 0,769 | 0,987 |
| 7 jours | C3AR1 + CD74 + IFNG + CIITA + CD3D + CTLA4 | 0,875 | 0,765 | 0,985 |
| 7 jours | C3AR1 + CD74 + IFNG + CIITA + CTLA4 | 0,881 | 0,775 | 0,987 |
| 7 jours | C3AR1 + CD74 + IFNG + CIITA + TAP2 | 0,878 | 0,769 | 0,987 |
| 7 jours | C3AR1 + CD74 + IFNG + CIITA + TAP2 + CD3D | 0,878 | 0,769 | 0,987 |
| 7 jours | C3AR1 + CD74 + IFNG + CIITA + TAP2 + CD3D + CTLA4 | 0,884 | 0,777 | 0,99 |
| 7 jours | C3AR1 + CD74 + IFNG + CIITA + TAP2 + CTLA4 | 0,884 | 0,777 | 0,991 |
| 7 jours | C3AR1 + CD74 + IFNG + CTLA4 | 0,825 | 0,695 | 0,956 |
| 7 jours | C3AR1 + CD74 + IFNG + CX3CR1 | 0,878 | 0,767 | 0,99 |
| 7 jours | C3AR1 + CD74 + IFNG + CX3CR1 + CD3D | 0,873 | 0,758 | 0,987 |
| 7 jours | C3AR1 + CD74 + IFNG + CX3CR1 + CD3D + CTLA4 | 0,873 | 0,756 | 0,989 |
| 7 jours | C3AR1 + CD74 + IFNG + CX3CR1 + CIITA | 0,917 | 0,824 | 1 |
| 7 jours | C3AR1 + CD74 + IFNG + CX3CR1 + CIITA + CD3D | 0,914 | 0,819 | 1 |
| 7 jours | C3AR1 + CD74 + IFNG + CX3CR1 + CIITA + CD3D + CTLA4 | 0,925 | 0,835 | 1 |
| 7 jours | C3AR1 + CD74 + IFNG + CX3CR1 + CIITA + CTLA4 | 0,92 | 0,83 | 1 |
| 7 jours | C3AR1 + CD74 + IFNG + CX3CR1 + CIITA + TAP2 | 0,92 | 0,83 | 1 |
| 7 jours | C3AR1 + CD74 + IFNG + CX3CR1 + CIITA + TAP2 + CD3D | 0,911 | 0,814 | 1 |
| 7 jours | C3AR1 + CD74 + IFNG + CX3CR1 + CIITA + TAP2 + CD3D + CTLA4 | 0,931 | 0,844 | 1 |
| 7 jours | C3AR1 + CD74 + IFNG + CX3CR1 + CIITA + TAP2 + CTLA4 | 0,917 | 0,826 | 1 |
| 7 jours | C3AR1 + CD74 + IFNG + CX3CR1 + CTLA4 | 0,873 | 0,758 | 0,987 |
| 7 jours | C3AR1 + CD74 + IFNG + CX3CR1 + TAP2 | 0,878 | 0,766 | 0,99 |
| 7 jours | C3AR1 + CD74 + IFNG + CX3CR1 + TAP2 + CD3D | 0,873 | 0,757 | 0,988 |
| 7 jours | C3AR1 + CD74 + IFNG + CX3CR1 + TAP2 + CD3D + CTLA4 | 0,87 | 0,752 | 0,988 |
| 7 jours | C3AR1 + CD74 + IFNG + CX3CR1 + TAP2 + CTLA4 | 0,873 | 0,758 | 0,987 |
| 7 jours | C3AR1 + CD74 + IFNG + S100A9 | 0,828 | 0,699 | 0,957 |
| 7 jours | C3AR1 + CD74 + IFNG + S100A9 + CD3D | 0,828 | 0,699 | 0,957 |
| 7 jours | C3AR1 + CD74 + IFNG + S100A9 + CD3D + CTLA4 | 0,823 | 0,691 | 0,955 |
| 7 jours | C3AR1 + CD74 + IFNG + S100A9 + CIITA | 0,867 | 0,754 | 0,98 |
| 7 jours | C3AR1 + CD74 + IFNG + S100A9 + CIITA + CD3D | 0,867 | 0,753 | 0,981 |
| 7 jours | C3AR1 + CD74 + IFNG + S100A9 + CIITA + CD3D + CTLA4 | 0,87 | 0,758 | 0,982 |
| 7 jours | C3AR1 + CD74 + IFNG + S100A9 + CIITA + CTLA4 | 0,884 | 0,779 | 0,989 |
| 7 jours | C3AR1 + CD74 + IFNG + S100A9 + CIITA + TAP2 | 0,878 | 0,769 | 0,987 |
| 7 jours | C3AR1 + CD74 + IFNG + S100A9 + CIITA + TAP2 + CD3D | 0,875 | 0,765 | 0,986 |
| 7 jours | C3AR1 + CD74 + IFNG + S100A9 + CIITA + TAP2 + CD3D + CTLA4 | 0,881 | 0,773 | 0,989 |
| 7 jours | C3AR1 + CD74 + IFNG + S100A9 + CIITA + TAP2 + CTLA4 | 0,884 | 0,777 | 0,991 |
| 7 jours | C3AR1 + CD74 + IFNG + S100A9 + CTLA4 | 0,823 | 0,691 | 0,955 |
| 7 jours | C3AR1 + CD74 + IFNG + S100A9 + CX3CR1 | 0,859 | 0,74 | 0,977 |
| 7 jours | C3AR1 + CD74 + IFNG + S100A9 + CX3CR1 + CD3D | 0,875 | 0,766 | 0,985 |
| 7 jours | C3AR1 + CD74 + IFNG + S100A9 + CX3CR1 + CD3D + CTLA4 | 0,878 | 0,767 | 0,99 |
| 7 jours | C3AR1 + CD74 + IFNG + S100A9 + CX3CR1 + CIITA | 0,917 | 0,824 | 1 |
| 7 jours | C3AR1 + CD74 + IFNG + S100A9 + CX3CR1 + CIITA + CD3D | 0,914 | 0,819 | 1 |
| 7 jours | C3AR1 + CD74 + IFNG + S100A9 + CX3CR1 + CIITA + CD3D + CTLA4 | 0,92 | 0,828 | 1 |
| 7 jours | C3AR1 + CD74 + IFNG + S100A9 + CX3CR1 + CIITA + CTLA4 | 0,922 | 0,832 | 1 |
| 7 jours | C3AR1 + CD74 + IFNG + S100A9 + CX3CR1 + CIITA + TAP2 | 0,922 | 0,832 | 1 |
| 7 jours | C3AR1 + CD74 + IFNG + S100A9 + CX3CR1 + CIITA + TAP2 + CD3D | 0,911 | 0,814 | 1 |
| 7 jours | C3AR1 + CD74 + IFNG + S100A9 + CX3CR1 + CIITA + TAP2 + CD3D + CTLA4 | 0,925 | 0,838 | 1 |
| 7 jours | C3AR1 + CD74 + IFNG + S100A9 + CX3CR1 + CIITA + TAP2 + CTLA4 | 0,925 | 0,839 | 1 |
| 7 jours | C3AR1 + CD74 + IFNG + S100A9 + CX3CR1 + CTLA4 | 0,881 | 0,772 | 0,99 |
| 7 jours | C3AR1 + CD74 + IFNG + S100A9 + CX3CR1 + TAP2 | 0,861 | 0,744 | 0,979 |
| 7 jours | C3AR1 + CD74 + IFNG + S100A9 + CX3CR1 + TAP2 + CD3D | 0,878 | 0,77 | 0,987 |
| 7 jours | C3AR1 + CD74 + IFNG + S100A9 + CX3CR1 + TAP2 + CD3D + CTLA4 | 0,873 | 0,759 | 0,986 |
| 7 jours | C3AR1 + CD74 + IFNG + S100A9 + CX3CR1 + TAP2 + CTLA4 | 0,881 | 0,772 | 0,99 |
| 7 jours | C3AR1 + CD74 + IFNG + S100A9 + TAP2 | 0,845 | 0,721 | 0,969 |
| 7 jours | C3AR1 + CD74 + IFNG + S100A9 + TAP2 + CD3D | 0,839 | 0,714 | 0,965 |
| 7 jours | C3AR1 + CD74 + IFNG + S100A9 + TAP2 + CD3D + CTLA4 | 0,839 | 0,714 | 0,965 |
| 7 jours | C3AR1 + CD74 + IFNG + S100A9 + TAP2 + CTLA4 | 0,831 | 0,702 | 0,96 |
| 7 jours | C3AR1 + CD74 + IFNG + TAP2 | 0,828 | 0,699 | 0,957 |
| 7 jours | C3AR1 + CD74 + IFNG + TAP2 + CD3D | 0,825 | 0,696 | 0,955 |
| 7 jours | C3AR1 + CD74 + IFNG + TAP2 + CD3D + CTLA4 | 0,834 | 0,706 | 0,962 |
| 7 jours | C3AR1 + CD74 + IFNG + TAP2 + CTLA4 | 0,831 | 0,702 | 0,96 |
| 7 jours | C3AR1 + CD74 + S100A9 | 0,828 | 0,687 | 0,97 |
| 7 jours | C3AR1 + CD74 + S100A9 + CD3D | 0,814 | 0,672 | 0,957 |
| 7 jours | C3AR1 + CD74 + S100A9 + CD3D + CTLA4 | 0,823 | 0,684 | 0,962 |
| 7 jours | C3AR1 + CD74 + S100A9 + CIITA | 0,886 | 0,778 | 0,995 |
| 7 jours | C3AR1 + CD74 + S100A9 + CIITA + CD3D | 0,878 | 0,766 | 0,99 |
| 7 jours | C3AR1 + CD74 + S100A9 + CIITA + CD3D + CTLA4 | 0,867 | 0,754 | 0,98 |
| 7 jours | C3AR1 + CD74 + S100A9 + CIITA + CTLA4 | 0,878 | 0,765 | 0,992 |
| 7 jours | C3AR1 + CD74 + S100A9 + CIITA + TAP2 | 0,886 | 0,778 | 0,995 |
| 7 jours | C3AR1 + CD74 + S100A9 + CIITA + TAP2 + CD3D | 0,886 | 0,779 | 0,994 |
| 7 jours | C3AR1 + CD74 + S100A9 + CIITA + TAP2 + CD3D + CTLA4 | 0,884 | 0,778 | 0,99 |
| 7 jours | C3AR1 + CD74 + S100A9 + CIITA + TAP2 + CTLA4 | 0,881 | 0,768 | 0,994 |
| 7 jours | C3AR1 + CD74 + S100A9 + CTLA4 | 0,828 | 0,687 | 0,97 |
| 7 jours | C3AR1 + CD74 + S100A9 + CX3CR1 | 0,848 | 0,711 | 0,984 |
| 7 jours | C3AR1 + CD74 + S100A9 + CX3CR1 + CD3D | 0,856 | 0,722 | 0,99 |
| 7 jours | C3AR1 + CD74 + S100A9 + CX3CR1 + CD3D + CTLA4 | 0,856 | 0,722 | 0,99 |
| 7 jours | C3AR1 + CD74 + S100A9 + CX3CR1 + CIITA | 0,881 | 0,764 | 0,998 |
| 7 jours | C3AR1 + CD74 + S100A9 + CX3CR1 + CIITA + CD3D | 0,884 | 0,769 | 0,998 |
| 7 jours | C3AR1 + CD74 + S100A9 + CX3CR1 + CIITA + CD3D + CTLA4 | 0,889 | 0,785 | 0,994 |
| 7 jours | C3AR1 + CD74 + S100A9 + CX3CR1 + CIITA + CTLA4 | 0,886 | 0,773 | 1 |
| 7 jours | C3AR1 + CD74 + S100A9 + CX3CR1 + CIITA + TAP2 | 0,881 | 0,763 | 0,998 |
| 7 jours | C3AR1 + CD74 + S100A9 + CX3CR1 + CIITA + TAP2 + CD3D | 0,884 | 0,769 | 0,998 |
| 7 jours | C3AR1 + CD74 + S100A9 + CX3CR1 + CIITA + TAP2 + CD3D + CTLA4 | 0,892 | 0,79 | 0,994 |
| 7 jours | C3AR1 + CD74 + S100A9 + CX3CR1 + CIITA + TAP2 + CTLA4 | 0,884 | 0,768 | 0,999 |
| 7 jours | C3AR1 + CD74 + S100A9 + CX3CR1 + CTLA4 | 0,856 | 0,724 | 0,988 |
| 7 jours | C3AR1 + CD74 + S100A9 + CX3CR1 + TAP2 | 0,853 | 0,714 | 0,992 |
| 7 jours | C3AR1 + CD74 + S100A9 + CX3CR1 + TAP2 + CD3D | 0,856 | 0,722 | 0,99 |
| 7 jours | C3AR1 + CD74 + S100A9 + CX3CR1 + TAP2 + CD3D + CTLA4 | 0,853 | 0,717 | 0,99 |
| 7 jours | C3AR1 + CD74 + S100A9 + CX3CR1 + TAP2 + CTLA4 | 0,85 | 0,711 | 0,989 |
| 7 jours | C3AR1 + CD74 + S100A9 + TAP2 | 0,825 | 0,684 | 0,967 |
| 7 jours | C3AR1 + CD74 + S100A9 + TAP2 + CD3D | 0,823 | 0,682 | 0,963 |
| 7 jours | C3AR1 + CD74 + S100A9 + TAP2 + CD3D + CTLA4 | 0,831 | 0,695 | 0,967 |
| 7 jours | C3AR1 + CD74 + S100A9 + TAP2 + CTLA4 | 0,825 | 0,684 | 0,967 |
| 7 jours | C3AR1 + CD74 + TAP2 | 0,773 | 0,621 | 0,925 |
| 7 jours | C3AR1 + CD74 + TAP2 + CD3D | 0,773 | 0,623 | 0,922 |
| 7 jours | C3AR1 + CD74 + TAP2 + CD3D + CTLA4 | 0,767 | 0,615 | 0,919 |
| 7 jours | C3AR1 + CD74 + TAP2 + CTLA4 | 0,789 | 0,641 | 0,938 |
| 7 jours | C3AR1 + CIITA | 0,825 | 0,694 | 0,957 |
| 7 jours | C3AR1 + CIITA + CD3D | 0,825 | 0,692 | 0,959 |
| 7 jours | C3AR1 + CIITA + CD3D + CTLA4 | 0,839 | 0,716 | 0,963 |
| 7 jours | C3AR1 + CIITA + CTLA4 | 0,839 | 0,715 | 0,964 |
| 7 jours | C3AR1 + CIITA + TAP2 | 0,834 | 0,705 | 0,963 |
| 7 jours | C3AR1 + CIITA + TAP2 + CD3D | 0,828 | 0,698 | 0,959 |
| 7 jours | C3AR1 + CIITA + TAP2 + CD3D + CTLA4 | 0,831 | 0,704 | 0,958 |
| 7 jours | C3AR1 + CIITA + TAP2 + CTLA4 | 0,842 | 0,719 | 0,965 |
| 7 jours | C3AR1 + CX3CR1 + CD3D | 0,64 | 0,459 | 0,821 |
| 7 jours | C3AR1 + CX3CR1 + CD3D + CTLA4 | 0,632 | 0,449 | 0,815 |
| 7 jours | C3AR1 + CX3CR1 + CIITA | 0,817 | 0,682 | 0,952 |
| 7 jours | C3AR1 + CX3CR1 + CIITA + CD3D | 0,837 | 0,711 | 0,962 |
| 7 jours | C3AR1 + CX3CR1 + CIITA + CD3D + CTLA4 | 0,831 | 0,704 | 0,958 |
| 7 jours | C3AR1 + CX3CR1 + CIITA + CTLA4 | 0,837 | 0,712 | 0,961 |
| 7 jours | C3AR1 + CX3CR1 + CIITA + TAP2 | 0,831 | 0,699 | 0,963 |
| 7 jours | C3AR1 + CX3CR1 + CIITA + TAP2 + CD3D | 0,839 | 0,714 | 0,965 |
| 7 jours | C3AR1 + CX3CR1 + CIITA + TAP2 + CD3D + CTLA4 | 0,853 | 0,736 | 0,97 |
| 7 jours | C3AR1 + CX3CR1 + CIITA + TAP2 + CTLA4 | 0,845 | 0,72 | 0,969 |
| 7 jours | C3AR1 + CX3CR1 + TAP2 | 0,693 | 0,516 | 0,869 |
| 7 jours | C3AR1 + CX3CR1 + TAP2 + CD3D | 0,693 | 0,516 | 0,87 |
| 7 jours | C3AR1 + CX3CR1 + TAP2 + CD3D + CTLA4 | 0,695 | 0,518 | 0,873 |
| 7 jours | C3AR1 + CX3CR1 + TAP2 + CTLA4 | 0,693 | 0,515 | 0,87 |
| 7 jours | C3AR1 + IFNG | 0,734 | 0,571 | 0,897 |
| 7 jours | C3AR1 + IFNG + CD177 | 0,806 | 0,665 | 0,947 |
| 7 jours | C3AR1 + IFNG + CD177 + CD3D | 0,812 | 0,674 | 0,949 |
| 7 jours | C3AR1 + IFNG + CD177 + CD3D + CTLA4 | 0,809 | 0,671 | 0,947 |
| 7 jours | C3AR1 + IFNG + CD177 + CIITA | 0,875 | 0,766 | 0,985 |
| 7 jours | C3AR1 + IFNG + CD177 + CIITA + CD3D | 0,873 | 0,761 | 0,984 |
| 7 jours | C3AR1 + IFNG + CD177 + CIITA + CD3D + CTLA4 | 0,881 | 0,776 | 0,985 |
| 7 jours | C3AR1 + IFNG + CD177 + CIITA + CTLA4 | 0,878 | 0,774 | 0,983 |
| 7 jours | C3AR1 + IFNG + CD177 + CIITA + TAP2 | 0,878 | 0,77 | 0,987 |
| 7 jours | C3AR1 + IFNG + CD177 + CIITA + TAP2 + CD3D | 0,878 | 0,77 | 0,987 |
| 7 jours | C3AR1 + IFNG + CD177 + CIITA + TAP2 + CD3D + CTLA4 | 0,892 | 0,795 | 0,989 |
| 7 jours | C3AR1 + IFNG + CD177 + CIITA + TAP2 + CTLA4 | 0,881 | 0,778 | 0,984 |
| 7 jours | C3AR1 + IFNG + CD177 + CTLA4 | 0,809 | 0,671 | 0,947 |
| 7 jours | C3AR1 + IFNG + CD177 + CX3CR1 | 0,803 | 0,66 | 0,947 |
| 7 jours | C3AR1 + IFNG + CD177 + CX3CR1 + CD3D | 0,814 | 0,676 | 0,953 |
| 7 jours | C3AR1 + IFNG + CD177 + CX3CR1 + CD3D + CTLA4 | 0,792 | 0,648 | 0,937 |
| 7 jours | C3AR1 + IFNG + CD177 + CX3CR1 + CIITA | 0,884 | 0,78 | 0,987 |
| 7 jours | C3AR1 + IFNG + CD177 + CX3CR1 + CIITA + CD3D | 0,881 | 0,775 | 0,987 |
| 7 jours | C3AR1 + IFNG + CD177 + CX3CR1 + CIITA + CD3D + CTLA4 | 0,9 | 0,805 | 0,996 |
| 7 jours | C3AR1 + IFNG + CD177 + CX3CR1 + CIITA + CTLA4 | 0,889 | 0,79 | 0,988 |
| 7 jours | C3AR1 + IFNG + CD177 + CX3CR1 + CIITA + TAP2 | 0,889 | 0,79 | 0,988 |
| 7 jours | C3AR1 + IFNG + CD177 + CX3CR1 + CIITA + TAP2 + CD3D | 0,884 | 0,781 | 0,987 |
| 7 jours | C3AR1 + IFNG + CD177 + CX3CR1 + CIITA + TAP2 + CD3D + CTLA4 | 0,9 | 0,803 | 0,997 |
| 7 jours | C3AR1 + IFNG + CD177 + CX3CR1 + CIITA + TAP2 + CTLA4 | 0,886 | 0,784 | 0,989 |
| 7 jours | C3AR1 + IFNG + CD177 + CX3CR1 + CTLA4 | 0,801 | 0,659 | 0,942 |
| 7 jours | C3AR1 + IFNG + CD177 + CX3CR1 + TAP2 | 0,817 | 0,68 | 0,954 |
| 7 jours | C3AR1 + IFNG + CD177 + CX3CR1 + TAP2 + CD3D | 0,82 | 0,684 | 0,956 |
| 7 jours | C3AR1 + IFNG + CD177 + CX3CR1 + TAP2 + CD3D + CTLA4 | 0,825 | 0,693 | 0,958 |
| 7 jours | C3AR1 + IFNG + CD177 + CX3CR1 + TAP2 + CTLA4 | 0,825 | 0,693 | 0,958 |
| 7 jours | C3AR1 + IFNG + CD177 + S100A9 | 0,798 | 0,655 | 0,94 |
| 7 jours | C3AR1 + IFNG + CD177 + S100A9 + CD3D | 0,795 | 0,651 | 0,939 |
| 7 jours | C3AR1 + IFNG + CD177 + S100A9 + CD3D + CTLA4 | 0,801 | 0,659 | 0,942 |
| 7 jours | C3AR1 + IFNG + CD177 + S100A9 + CIITA | 0,873 | 0,762 | 0,983 |
| 7 jours | C3AR1 + IFNG + CD177 + S100A9 + CIITA + CD3D | 0,875 | 0,766 | 0,985 |
| 7 jours | C3AR1 + IFNG + CD177 + S100A9 + CIITA + CD3D + CTLA4 | 0,881 | 0,777 | 0,985 |
| 7 jours | C3AR1 + IFNG + CD177 + S100A9 + CIITA + CTLA4 | 0,873 | 0,765 | 0,98 |
| 7 jours | C3AR1 + IFNG + CD177 + S100A9 + CIITA + TAP2 | 0,873 | 0,763 | 0,982 |
| 7 jours | C3AR1 + IFNG + CD177 + S100A9 + CIITA + TAP2 + CD3D | 0,873 | 0,762 | 0,983 |
| 7 jours | C3AR1 + IFNG + CD177 + S100A9 + CIITA + TAP2 + CD3D + CTLA4 | 0,892 | 0,795 | 0,989 |
| 7 jours | C3AR1 + IFNG + CD177 + S100A9 + CIITA + TAP2 + CTLA4 | 0,881 | 0,777 | 0,985 |
| 7 jours | C3AR1 + IFNG + CD177 + S100A9 + CTLA4 | 0,801 | 0,659 | 0,942 |
| 7 jours | C3AR1 + IFNG + CD177 + S100A9 + CX3CR1 | 0,795 | 0,652 | 0,938 |
| 7 jours | C3AR1 + IFNG + CD177 + S100A9 + CX3CR1 + CD3D | 0,789 | 0,646 | 0,933 |
| 7 jours | C3AR1 + IFNG + CD177 + S100A9 + CX3CR1 + CD3D + CTLA4 | 0,789 | 0,646 | 0,933 |
| 7 jours | C3AR1 + IFNG + CD177 + S100A9 + CX3CR1 + CIITA | 0,884 | 0,778 | 0,989 |
| 7 jours | C3AR1 + IFNG + CD177 + S100A9 + CX3CR1 + CIITA + CD3D | 0,881 | 0,775 | 0,987 |
| 7 jours | C3AR1 + IFNG + CD177 + S100A9 + CX3CR1 + CIITA + CD3D + CTLA4 | 0,895 | 0,797 | 0,993 |
| 7 jours | C3AR1 + IFNG + CD177 + S100A9 + CX3CR1 + CIITA + CTLA4 | 0,889 | 0,788 | 0,991 |
| 7 jours | C3AR1 + IFNG + CD177 + S100A9 + CX3CR1 + CIITA + TAP2 | 0,903 | 0,807 | 0,999 |
| 7 jours | C3AR1 + IFNG + CD177 + S100A9 + CX3CR1 + CIITA + TAP2 + CD3D | 0,9 | 0,803 | 0,997 |
| 7 jours | C3AR1 + IFNG + CD177 + S100A9 + CX3CR1 + CIITA + TAP2 + CD3D + CTLA4 | 0,892 | 0,792 | 0,992 |
| 7 jours | C3AR1 + IFNG + CD177 + S100A9 + CX3CR1 + CIITA + TAP2 + CTLA4 | 0,892 | 0,789 | 0,995 |
| 7 jours | C3AR1 + IFNG + CD177 + S100A9 + CX3CR1 + CTLA4 | 0,806 | 0,667 | 0,945 |
| 7 jours | C3AR1 + IFNG + CD177 + S100A9 + CX3CR1 + TAP2 | 0,82 | 0,688 | 0,952 |
| 7 jours | C3AR1 + IFNG + CD177 + S100A9 + CX3CR1 + TAP2 + CD3D | 0,809 | 0,672 | 0,945 |
| 7 jours | C3AR1 + IFNG + CD177 + S100A9 + CX3CR1 + TAP2 + CD3D + CTLA4 | 0,817 | 0,684 | 0,951 |
| 7 jours | C3AR1 + IFNG + CD177 + S100A9 + CX3CR1 + TAP2 + CTLA4 | 0,823 | 0,691 | 0,954 |
| 7 jours | C3AR1 + IFNG + CD177 + S100A9 + TAP2 | 0,801 | 0,66 | 0,941 |
| 7 jours | C3AR1 + IFNG + CD177 + S100A9 + TAP2 + CD3D | 0,798 | 0,656 | 0,94 |
| 7 jours | C3AR1 + IFNG + CD177 + S100A9 + TAP2 + CD3D + CTLA4 | 0,806 | 0,667 | 0,945 |
| 7 jours | C3AR1 + IFNG + CD177 + S100A9 + TAP2 + CTLA4 | 0,798 | 0,656 | 0,94 |
| 7 jours | C3AR1 + IFNG + CD177 + TAP2 | 0,809 | 0,669 | 0,949 |
| 7 jours | C3AR1 + IFNG + CD177 + TAP2 + CD3D | 0,812 | 0,673 | 0,95 |
| 7 jours | C3AR1 + IFNG + CD177 + TAP2 + CD3D + CTLA4 | 0,806 | 0,666 | 0,947 |
| 7 jours | C3AR1 + IFNG + CD177 + TAP2 + CTLA4 | 0,812 | 0,673 | 0,95 |
| 7 jours | C3AR1 + IFNG + CD3D | 0,748 | 0,591 | 0,905 |
| 7 jours | C3AR1 + IFNG + CD3D + CTLA4 | 0,745 | 0,586 | 0,904 |
| 7 jours | C3AR1 + IFNG + CIITA | 0,85 | 0,73 | 0,97 |
| 7 jours | C3AR1 + IFNG + CIITA + CD3D | 0,85 | 0,73 | 0,97 |
| 7 jours | C3AR1 + IFNG + CIITA + CD3D + CTLA4 | 0,859 | 0,742 | 0,975 |
| 7 jours | C3AR1 + IFNG + CIITA + CTLA4 | 0,859 | 0,743 | 0,975 |
| 7 jours | C3AR1 + IFNG + CIITA + TAP2 | 0,848 | 0,727 | 0,969 |
| 7 jours | C3AR1 + IFNG + CIITA + TAP2 + CD3D | 0,845 | 0,723 | 0,966 |
| 7 jours | C3AR1 + IFNG + CIITA + TAP2 + CD3D + CTLA4 | 0,856 | 0,738 | 0,974 |
| 7 jours | C3AR1 + IFNG + CIITA + TAP2 + CTLA4 | 0,856 | 0,738 | 0,974 |
| 7 jours | C3AR1 + IFNG + CTLA4 | 0,748 | 0,589 | 0,907 |
| 7 jours | C3AR1 + IFNG + CX3CR1 | 0,742 | 0,583 | 0,901 |
| 7 jours | C3AR1 + IFNG + CX3CR1 + CD3D | 0,748 | 0,588 | 0,908 |
| 7 jours | C3AR1 + IFNG + CX3CR1 + CD3D + CTLA4 | 0,77 | 0,616 | 0,924 |
| 7 jours | C3AR1 + IFNG + CX3CR1 + CIITA | 0,861 | 0,745 | 0,978 |
| 7 jours | C3AR1 + IFNG + CX3CR1 + CIITA + CD3D | 0,864 | 0,749 | 0,98 |
| 7 jours | C3AR1 + IFNG + CX3CR1 + CIITA + CD3D + CTLA4 | 0,867 | 0,751 | 0,983 |
| 7 jours | C3AR1 + IFNG + CX3CR1 + CIITA + CTLA4 | 0,859 | 0,738 | 0,979 |
| 7 jours | C3AR1 + IFNG + CX3CR1 + CIITA + TAP2 | 0,875 | 0,766 | 0,984 |
| 7 jours | C3AR1 + IFNG + CX3CR1 + CIITA + TAP2 + CD3D | 0,878 | 0,771 | 0,986 |
| 7 jours | C3AR1 + IFNG + CX3CR1 + CIITA + TAP2 + CD3D + CTLA4 | 0,889 | 0,787 | 0,992 |
| 7 jours | C3AR1 + IFNG + CX3CR1 + CIITA + TAP2 + CTLA4 | 0,886 | 0,779 | 0,994 |
| 7 jours | C3AR1 + IFNG + CX3CR1 + CTLA4 | 0,77 | 0,616 | 0,924 |
| 7 jours | C3AR1 + IFNG + CX3CR1 + TAP2 | 0,776 | 0,622 | 0,929 |
| 7 jours | C3AR1 + IFNG + CX3CR1 + TAP2 + CD3D | 0,784 | 0,635 | 0,933 |
| 7 jours | C3AR1 + IFNG + CX3CR1 + TAP2 + CD3D + CTLA4 | 0,787 | 0,641 | 0,932 |
| 7 jours | C3AR1 + IFNG + CX3CR1 + TAP2 + CTLA4 | 0,784 | 0,637 | 0,931 |
| 7 jours | C3AR1 + IFNG + S100A9 | 0,762 | 0,609 | 0,915 |
| 7 jours | C3AR1 + IFNG + S100A9 + CD3D | 0,762 | 0,609 | 0,914 |
| 7 jours | C3AR1 + IFNG + S100A9 + CD3D + CTLA4 | 0,759 | 0,606 | 0,912 |
| 7 jours | C3AR1 + IFNG + S100A9 + CIITA | 0,859 | 0,743 | 0,974 |
| 7 jours | C3AR1 + IFNG + S100A9 + CIITA + CD3D | 0,859 | 0,743 | 0,974 |
| 7 jours | C3AR1 + IFNG + S100A9 + CIITA + CD3D + CTLA4 | 0,853 | 0,736 | 0,971 |
| 7 jours | C3AR1 + IFNG + S100A9 + CIITA + CTLA4 | 0,859 | 0,744 | 0,974 |
| 7 jours | C3AR1 + IFNG + S100A9 + CIITA + TAP2 | 0,856 | 0,739 | 0,973 |
| 7 jours | C3AR1 + IFNG + S100A9 + CIITA + TAP2 + CD3D | 0,856 | 0,739 | 0,973 |
| 7 jours | C3AR1 + IFNG + S100A9 + CIITA + TAP2 + CD3D + CTLA4 | 0,853 | 0,736 | 0,971 |
| 7 jours | C3AR1 + IFNG + S100A9 + CIITA + TAP2 + CTLA4 | 0,85 | 0,731 | 0,97 |
| 7 jours | C3AR1 + IFNG + S100A9 + CTLA4 | 0,745 | 0,587 | 0,903 |
| 7 jours | C3AR1 + IFNG + S100A9 + CX3CR1 | 0,781 | 0,633 | 0,929 |
| 7 jours | C3AR1 + IFNG + S100A9 + CX3CR1 + CD3D | 0,789 | 0,643 | 0,936 |
| 7 jours | C3AR1 + IFNG + S100A9 + CX3CR1 + CD3D + CTLA4 | 0,787 | 0,639 | 0,934 |
| 7 jours | C3AR1 + IFNG + S100A9 + CX3CR1 + CIITA | 0,873 | 0,762 | 0,983 |
| 7 jours | C3AR1 + IFNG + S100A9 + CX3CR1 + CIITA + CD3D | 0,875 | 0,765 | 0,986 |
| 7 jours | C3AR1 + IFNG + S100A9 + CX3CR1 + CIITA + CD3D + CTLA4 | 0,884 | 0,78 | 0,987 |
| 7 jours | C3AR1 + IFNG + S100A9 + CX3CR1 + CIITA + CTLA4 | 0,87 | 0,759 | 0,981 |
| 7 jours | C3AR1 + IFNG + S100A9 + CX3CR1 + CIITA + TAP2 | 0,884 | 0,78 | 0,987 |
| 7 jours | C3AR1 + IFNG + S100A9 + CX3CR1 + CIITA + TAP2 + CD3D | 0,889 | 0,789 | 0,989 |
| 7 jours | C3AR1 + IFNG + S100A9 + CX3CR1 + CIITA + TAP2 + CD3D + CTLA4 | 0,881 | 0,777 | 0,985 |
| 7 jours | C3AR1 + IFNG + S100A9 + CX3CR1 + CIITA + TAP2 + CTLA4 | 0,886 | 0,784 | 0,989 |
| 7 jours | C3AR1 + IFNG + S100A9 + CX3CR1 + CTLA4 | 0,787 | 0,64 | 0,934 |
| 7 jours | C3AR1 + IFNG + S100A9 + CX3CR1 + TAP2 | 0,787 | 0,642 | 0,932 |
| 7 jours | C3AR1 + IFNG + S100A9 + CX3CR1 + TAP2 + CD3D | 0,787 | 0,641 | 0,932 |
| 7 jours | C3AR1 + IFNG + S100A9 + CX3CR1 + TAP2 + CD3D + CTLA4 | 0,792 | 0,649 | 0,936 |
| 7 jours | C3AR1 + IFNG + S100A9 + CX3CR1 + TAP2 + CTLA4 | 0,781 | 0,633 | 0,929 |
| 7 jours | C3AR1 + IFNG + S100A9 + TAP2 | 0,762 | 0,608 | 0,915 |
| 7 jours | C3AR1 + IFNG + S100A9 + TAP2 + CD3D | 0,762 | 0,608 | 0,916 |
| 7 jours | C3AR1 + IFNG + S100A9 + TAP2 + CD3D + CTLA4 | 0,762 | 0,608 | 0,915 |
| 7 jours | C3AR1 + IFNG + S100A9 + TAP2 + CTLA4 | 0,762 | 0,608 | 0,915 |
| 7 jours | C3AR1 + IFNG + TAP2 | 0,753 | 0,594 | 0,913 |
| 7 jours | C3AR1 + IFNG + TAP2 + CD3D | 0,748 | 0,587 | 0,908 |
| 7 jours | C3AR1 + IFNG + TAP2 + CD3D + CTLA4 | 0,748 | 0,588 | 0,907 |
| 7 jours | C3AR1 + IFNG + TAP2 + CTLA4 | 0,748 | 0,587 | 0,909 |
| 7 jours | C3AR1 + IL1R2 + CD177 | 0,726 | 0,564 | 0,887 |
| 7 jours | C3AR1 + IL1R2 + CD177 + CD3D | 0,726 | 0,564 | 0,887 |
| 7 jours | C3AR1 + IL1R2 + CD177 + CD3D + CTLA4 | 0,726 | 0,564 | 0,887 |
| 7 jours | C3AR1 + IL1R2 + CD177 + CIITA | 0,853 | 0,729 | 0,977 |
| 7 jours | C3AR1 + IL1R2 + CD177 + CIITA + CD3D | 0,859 | 0,736 | 0,981 |
| 7 jours | C3AR1 + IL1R2 + CD177 + CIITA + CD3D + CTLA4 | 0,878 | 0,771 | 0,985 |
| 7 jours | C3AR1 + IL1R2 + CD177 + CIITA + CTLA4 | 0,875 | 0,764 | 0,987 |
| 7 jours | C3AR1 + IL1R2 + CD177 + CIITA + TAP2 | 0,856 | 0,732 | 0,98 |
| 7 jours | C3AR1 + IL1R2 + CD177 + CIITA + TAP2 + CD3D | 0,861 | 0,74 | 0,983 |
| 7 jours | C3AR1 + IL1R2 + CD177 + CIITA + TAP2 + CD3D + CTLA4 | 0,875 | 0,765 | 0,986 |
| 7 jours | C3AR1 + IL1R2 + CD177 + CIITA + TAP2 + CTLA4 | 0,878 | 0,769 | 0,987 |
| 7 jours | C3AR1 + IL1R2 + CD177 + CTLA4 | 0,726 | 0,564 | 0,887 |
| 7 jours | C3AR1 + IL1R2 + CD177 + CX3CR1 | 0,723 | 0,561 | 0,885 |
| 7 jours | C3AR1 + IL1R2 + CD177 + CX3CR1 + CD3D | 0,72 | 0,557 | 0,883 |
| 7 jours | C3AR1 + IL1R2 + CD177 + CX3CR1 + CD3D + CTLA4 | 0,723 | 0,561 | 0,885 |
| 7 jours | C3AR1 + IL1R2 + CD177 + CX3CR1 + CIITA | 0,856 | 0,732 | 0,98 |
| 7 jours | C3AR1 + IL1R2 + CD177 + CX3CR1 + CIITA + CD3D | 0,853 | 0,729 | 0,977 |
| 7 jours | C3AR1 + IL1R2 + CD177 + CX3CR1 + CIITA + CD3D + CTLA4 | 0,884 | 0,776 | 0,991 |
| 7 jours | C3AR1 + IL1R2 + CD177 + CX3CR1 + CIITA + CTLA4 | 0,87 | 0,756 | 0,984 |
| 7 jours | C3AR1 + IL1R2 + CD177 + CX3CR1 + CIITA + TAP2 | 0,856 | 0,731 | 0,981 |
| 7 jours | C3AR1 + IL1R2 + CD177 + CX3CR1 + CIITA + TAP2 + CD3D | 0,856 | 0,732 | 0,98 |
| 7 jours | C3AR1 + IL1R2 + CD177 + CX3CR1 + CIITA + TAP2 + CD3D + CTLA4 | 0,884 | 0,776 | 0,991 |
| 7 jours | C3AR1 + IL1R2 + CD177 + CX3CR1 + CIITA + TAP2 + CTLA4 | 0,873 | 0,759 | 0,986 |
| 7 jours | C3AR1 + IL1R2 + CD177 + CX3CR1 + CTLA4 | 0,731 | 0,571 | 0,892 |
| 7 jours | C3AR1 + IL1R2 + CD177 + CX3CR1 + TAP2 | 0,767 | 0,612 | 0,922 |
| 7 jours | C3AR1 + IL1R2 + CD177 + CX3CR1 + TAP2 + CD3D | 0,767 | 0,614 | 0,921 |
| 7 jours | C3AR1 + IL1R2 + CD177 + CX3CR1 + TAP2 + CD3D + CTLA4 | 0,77 | 0,613 | 0,927 |
| 7 jours | C3AR1 + IL1R2 + CD177 + CX3CR1 + TAP2 + CTLA4 | 0,767 | 0,609 | 0,926 |
| 7 jours | C3AR1 + IL1R2 + CD177 + S100A9 | 0,767 | 0,615 | 0,92 |
| 7 jours | C3AR1 + IL1R2 + CD177 + S100A9 + CD3D | 0,776 | 0,626 | 0,926 |
| 7 jours | C3AR1 + IL1R2 + CD177 + S100A9 + CD3D + CTLA4 | 0,767 | 0,616 | 0,918 |
| 7 jours | C3AR1 + IL1R2 + CD177 + S100A9 + CIITA | 0,859 | 0,739 | 0,979 |
| 7 jours | C3AR1 + IL1R2 + CD177 + S100A9 + CIITA + CD3D | 0,864 | 0,747 | 0,982 |
| 7 jours | C3AR1 + IL1R2 + CD177 + S100A9 + CIITA + CD3D + CTLA4 | 0,892 | 0,792 | 0,992 |
| 7 jours | C3AR1 + IL1R2 + CD177 + S100A9 + CIITA + CTLA4 | 0,884 | 0,778 | 0,989 |
| 7 jours | C3AR1 + IL1R2 + CD177 + S100A9 + CIITA + TAP2 | 0,861 | 0,743 | 0,98 |
| 7 jours | C3AR1 + IL1R2 + CD177 + S100A9 + CIITA + TAP2 + CD3D | 0,864 | 0,747 | 0,982 |
| 7 jours | C3AR1 + IL1R2 + CD177 + S100A9 + CIITA + TAP2 + CD3D + CTLA4 | 0,889 | 0,789 | 0,989 |
| 7 jours | C3AR1 + IL1R2 + CD177 + S100A9 + CIITA + TAP2 + CTLA4 | 0,881 | 0,773 | 0,988 |
| 7 jours | C3AR1 + IL1R2 + CD177 + S100A9 + CTLA4 | 0,767 | 0,614 | 0,92 |
| 7 jours | C3AR1 + IL1R2 + CD177 + S100A9 + CX3CR1 | 0,773 | 0,622 | 0,924 |
| 7 jours | C3AR1 + IL1R2 + CD177 + S100A9 + CX3CR1 + CD3D | 0,789 | 0,642 | 0,937 |
| 7 jours | C3AR1 + IL1R2 + CD177 + S100A9 + CX3CR1 + CD3D + CTLA4 | 0,767 | 0,613 | 0,921 |
| 7 jours | C3AR1 + IL1R2 + CD177 + S100A9 + CX3CR1 + CIITA | 0,856 | 0,733 | 0,979 |
| 7 jours | C3AR1 + IL1R2 + CD177 + S100A9 + CX3CR1 + CIITA + CD3D | 0,861 | 0,742 | 0,981 |
| 7 jours | C3AR1 + IL1R2 + CD177 + S100A9 + CX3CR1 + CIITA + CD3D + CTLA4 | 0,9 | 0,804 | 0,996 |
| 7 jours | C3AR1 + IL1R2 + CD177 + S100A9 + CX3CR1 + CIITA + CTLA4 | 0,873 | 0,761 | 0,984 |
| 7 jours | C3AR1 + IL1R2 + CD177 + S100A9 + CX3CR1 + CIITA + TAP2 | 0,861 | 0,74 | 0,983 |
| 7 jours | C3AR1 + IL1R2 + CD177 + S100A9 + CX3CR1 + CIITA + TAP2 + CD3D | 0,859 | 0,737 | 0,98 |
| 7 jours | C3AR1 + IL1R2 + CD177 + S100A9 + CX3CR1 + CIITA + TAP2 + CD3D + CTLA4 | 0,9 | 0,806 | 0,995 |
| 7 jours | C3AR1 + IL1R2 + CD177 + S100A9 + CX3CR1 + CIITA + TAP2 + CTLA4 | 0,875 | 0,765 | 0,985 |
| 7 jours | C3AR1 + IL1R2 + CD177 + S100A9 + CX3CR1 + CTLA4 | 0,776 | 0,625 | 0,926 |
| 7 jours | C3AR1 + IL1R2 + CD177 + S100A9 + CX3CR1 + TAP2 | 0,778 | 0,626 | 0,93 |
| 7 jours | C3AR1 + IL1R2 + CD177 + S100A9 + CX3CR1 + TAP2 + CD3D | 0,781 | 0,631 | 0,932 |
| 7 jours | C3AR1 + IL1R2 + CD177 + S100A9 + CX3CR1 + TAP2 + CD3D + CTLA4 | 0,784 | 0,637 | 0,931 |
| 7 jours | C3AR1 + IL1R2 + CD177 + S100A9 + CX3CR1 + TAP2 + CTLA4 | 0,781 | 0,63 | 0,932 |
| 7 jours | C3AR1 + IL1R2 + CD177 + S100A9 + TAP2 | 0,795 | 0,649 | 0,941 |
| 7 jours | C3AR1 + IL1R2 + CD177 + S100A9 + TAP2 + CD3D | 0,787 | 0,639 | 0,934 |
| 7 jours | C3AR1 + IL1R2 + CD177 + S100A9 + TAP2 + CD3D + CTLA4 | 0,787 | 0,64 | 0,933 |
| 7 jours | C3AR1 + IL1R2 + CD177 + S100A9 + TAP2 + CTLA4 | 0,795 | 0,649 | 0,941 |
| 7 jours | C3AR1 + IL1R2 + CD177 + TAP2 | 0,767 | 0,612 | 0,922 |
| 7 jours | C3AR1 + IL1R2 + CD177 + TAP2 + CD3D | 0,765 | 0,609 | 0,92 |
| 7 jours | C3AR1 + IL1R2 + CD177 + TAP2 + CD3D + CTLA4 | 0,773 | 0,62 | 0,926 |
| 7 jours | C3AR1 + IL1R2 + CD177 + TAP2 + CTLA4 | 0,765 | 0,61 | 0,919 |
| 7 jours | C3AR1 + IL1R2 + CD74 | 0,688 | 0,601 | 0,775 |
| 7 jours | C3AR1 + IL1R2 + CD74 + CD177 | 0,812 | 0,67 | 0,953 |
| 7 jours | C3AR1 + IL1R2 + CD74 + CD177 + CD3D | 0,809 | 0,668 | 0,95 |
| 7 jours | C3AR1 + IL1R2 + CD74 + CD177 + CD3D + CTLA4 | 0,806 | 0,664 | 0,948 |
| 7 jours | C3AR1 + IL1R2 + CD74 + CD177 + CIITA | 0,875 | 0,763 | 0,988 |
| 7 jours | C3AR1 + IL1R2 + CD74 + CD177 + CIITA + CD3D | 0,878 | 0,768 | 0,988 |
| 7 jours | C3AR1 + IL1R2 + CD74 + CD177 + CIITA + CD3D + CTLA4 | 0,881 | 0,775 | 0,986 |
| 7 jours | C3AR1 + IL1R2 + CD74 + CD177 + CIITA + CTLA4 | 0,873 | 0,761 | 0,985 |
| 7 jours | C3AR1 + IL1R2 + CD74 + CD177 + CIITA + TAP2 | 0,875 | 0,763 | 0,988 |
| 7 jours | C3AR1 + IL1R2 + CD74 + CD177 + CIITA + TAP2 + CD3D | 0,875 | 0,764 | 0,986 |
| 7 jours | C3AR1 + IL1R2 + CD74 + CD177 + CIITA + TAP2 + CD3D + CTLA4 | 0,881 | 0,775 | 0,987 |
| 7 jours | C3AR1 + IL1R2 + CD74 + CD177 + CIITA + TAP2 + CTLA4 | 0,873 | 0,761 | 0,985 |
| 7 jours | C3AR1 + IL1R2 + CD74 + CD177 + CTLA4 | 0,817 | 0,678 | 0,957 |
| 7 jours | C3AR1 + IL1R2 + CD74 + CD177 + CX3CR1 | 0,828 | 0,688 | 0,969 |
| 7 jours | C3AR1 + IL1R2 + CD74 + CD177 + CX3CR1 + CD3D | 0,814 | 0,674 | 0,955 |
| 7 jours | C3AR1 + IL1R2 + CD74 + CD177 + CX3CR1 + CD3D + CTLA4 | 0,817 | 0,678 | 0,957 |
| 7 jours | C3AR1 + IL1R2 + CD74 + CD177 + CX3CR1 + CIITA | 0,881 | 0,771 | 0,991 |
| 7 jours | C3AR1 + IL1R2 + CD74 + CD177 + CX3CR1 + CIITA + CD3D | 0,875 | 0,763 | 0,988 |
| 7 jours | C3AR1 + IL1R2 + CD74 + CD177 + CX3CR1 + CIITA + CD3D + CTLA4 | 0,889 | 0,787 | 0,991 |
| 7 jours | C3AR1 + IL1R2 + CD74 + CD177 + CX3CR1 + CIITA + CTLA4 | 0,881 | 0,771 | 0,991 |
| 7 jours | C3AR1 + IL1R2 + CD74 + CD177 + CX3CR1 + CIITA + TAP2 | 0,875 | 0,762 | 0,989 |
| 7 jours | C3AR1 + IL1R2 + CD74 + CD177 + CX3CR1 + CIITA + TAP2 + CD3D | 0,873 | 0,758 | 0,987 |
| 7 jours | C3AR1 + IL1R2 + CD74 + CD177 + CX3CR1 + CIITA + TAP2 + CD3D + CTLA4 | 0,898 | 0,801 | 0,994 |
| 7 jours | C3AR1 + IL1R2 + CD74 + CD177 + CX3CR1 + CIITA + TAP2 + CTLA4 | 0,884 | 0,775 | 0,992 |
| 7 jours | C3AR1 + IL1R2 + CD74 + CD177 + CX3CR1 + CTLA4 | 0,834 | 0,697 | 0,97 |
| 7 jours | C3AR1 + IL1R2 + CD74 + CD177 + CX3CR1 + TAP2 | 0,82 | 0,676 | 0,964 |
| 7 jours | C3AR1 + IL1R2 + CD74 + CD177 + CX3CR1 + TAP2 + CD3D | 0,823 | 0,682 | 0,963 |
| 7 jours | C3AR1 + IL1R2 + CD74 + CD177 + CX3CR1 + TAP2 + CD3D + CTLA4 | 0,823 | 0,682 | 0,963 |
| 7 jours | C3AR1 + IL1R2 + CD74 + CD177 + CX3CR1 + TAP2 + CTLA4 | 0,831 | 0,692 | 0,97 |
| 7 jours | C3AR1 + IL1R2 + CD74 + CD177 + S100A9 | 0,828 | 0,691 | 0,965 |
| 7 jours | C3AR1 + IL1R2 + CD74 + CD177 + S100A9 + CD3D | 0,839 | 0,706 | 0,973 |
| 7 jours | C3AR1 + IL1R2 + CD74 + CD177 + S100A9 + CD3D + CTLA4 | 0,837 | 0,706 | 0,967 |
| 7 jours | C3AR1 + IL1R2 + CD74 + CD177 + S100A9 + CIITA | 0,884 | 0,775 | 0,992 |
| 7 jours | C3AR1 + IL1R2 + CD74 + CD177 + S100A9 + CIITA + CD3D | 0,884 | 0,776 | 0,992 |
| 7 jours | C3AR1 + IL1R2 + CD74 + CD177 + S100A9 + CIITA + CD3D + CTLA4 | 0,892 | 0,791 | 0,993 |
| 7 jours | C3AR1 + IL1R2 + CD74 + CD177 + S100A9 + CIITA + CTLA4 | 0,875 | 0,766 | 0,985 |
| 7 jours | C3AR1 + IL1R2 + CD74 + CD177 + S100A9 + CIITA + TAP2 | 0,881 | 0,771 | 0,99 |
| 7 jours | C3AR1 + IL1R2 + CD74 + CD177 + S100A9 + CIITA + TAP2 + CD3D | 0,884 | 0,775 | 0,992 |
| 7 jours | C3AR1 + IL1R2 + CD74 + CD177 + S100A9 + CIITA + TAP2 + CD3D + CTLA4 | 0,903 | 0,809 | 0,997 |
| 7 jours | C3AR1 + IL1R2 + CD74 + CD177 + S100A9 + CIITA + TAP2 + CTLA4 | 0,875 | 0,766 | 0,985 |
| 7 jours | C3AR1 + IL1R2 + CD74 + CD177 + S100A9 + CTLA4 | 0,828 | 0,691 | 0,965 |
| 7 jours | C3AR1 + IL1R2 + CD74 + CD177 + S100A9 + CX3CR1 | 0,859 | 0,726 | 0,992 |
| 7 jours | C3AR1 + IL1R2 + CD74 + CD177 + S100A9 + CX3CR1 + CD3D | 0,848 | 0,712 | 0,983 |
| 7 jours | C3AR1 + IL1R2 + CD74 + CD177 + S100A9 + CX3CR1 + CD3D + CTLA4 | 0,845 | 0,708 | 0,981 |
| 7 jours | C3AR1 + IL1R2 + CD74 + CD177 + S100A9 + CX3CR1 + CIITA | 0,881 | 0,766 | 0,995 |
| 7 jours | C3AR1 + IL1R2 + CD74 + CD177 + S100A9 + CX3CR1 + CIITA + CD3D | 0,884 | 0,771 | 0,996 |
| 7 jours | C3AR1 + IL1R2 + CD74 + CD177 + S100A9 + CX3CR1 + CIITA + CD3D + CTLA4 | 0,9 | 0,802 | 0,999 |
| 7 jours | C3AR1 + IL1R2 + CD74 + CD177 + S100A9 + CX3CR1 + CIITA + CTLA4 | 0,878 | 0,767 | 0,989 |
| 7 jours | C3AR1 + IL1R2 + CD74 + CD177 + S100A9 + CX3CR1 + CIITA + TAP2 | 0,881 | 0,769 | 0,993 |
| 7 jours | C3AR1 + IL1R2 + CD74 + CD177 + S100A9 + CX3CR1 + CIITA + TAP2 + CD3D | 0,881 | 0,768 | 0,993 |
| 7 jours | C3AR1 + IL1R2 + CD74 + CD177 + S100A9 + CX3CR1 + CIITA + TAP2 + CD3D + CTLA4 | 0,909 | 0,814 | 1 |
| 7 jours | C3AR1 + IL1R2 + CD74 + CD177 + S100A9 + CX3CR1 + CIITA + TAP2 + CTLA4 | 0,884 | 0,774 | 0,993 |
| 7 jours | C3AR1 + IL1R2 + CD74 + CD177 + S100A9 + CX3CR1 + CTLA4 | 0,861 | 0,731 | 0,992 |
| 7 jours | C3AR1 + IL1R2 + CD74 + CD177 + S100A9 + CX3CR1 + TAP2 | 0,859 | 0,725 | 0,992 |
| 7 jours | C3AR1 + IL1R2 + CD74 + CD177 + S100A9 + CX3CR1 + TAP2 + CD3D | 0,85 | 0,716 | 0,985 |
| 7 jours | C3AR1 + IL1R2 + CD74 + CD177 + S100A9 + CX3CR1 + TAP2 + CD3D + CTLA4 | 0,845 | 0,708 | 0,981 |
| 7 jours | C3AR1 + IL1R2 + CD74 + CD177 + S100A9 + CX3CR1 + TAP2 + CTLA4 | 0,859 | 0,725 | 0,992 |
| 7 jours | C3AR1 + IL1R2 + CD74 + CD177 + S100A9 + TAP2 | 0,828 | 0,69 | 0,966 |
| 7 jours | C3AR1 + IL1R2 + CD74 + CD177 + S100A9 + TAP2 + CD3D | 0,831 | 0,696 | 0,966 |
| 7 jours | C3AR1 + IL1R2 + CD74 + CD177 + S100A9 + TAP2 + CD3D + CTLA4 | 0,839 | 0,709 | 0,969 |
| 7 jours | C3AR1 + IL1R2 + CD74 + CD177 + S100A9 + TAP2 + CTLA4 | 0,828 | 0,69 | 0,966 |
| 7 jours | C3AR1 + IL1R2 + CD74 + CD177 + TAP2 | 0,817 | 0,677 | 0,958 |
| 7 jours | C3AR1 + IL1R2 + CD74 + CD177 + TAP2 + CD3D | 0,814 | 0,674 | 0,955 |
| 7 jours | C3AR1 + IL1R2 + CD74 + CD177 + TAP2 + CD3D + CTLA4 | 0,812 | 0,67 | 0,953 |
| 7 jours | C3AR1 + IL1R2 + CD74 + CD177 + TAP2 + CTLA4 | 0,814 | 0,672 | 0,956 |
| 7 jours | C3AR1 + IL1R2 + CD74 + CD3D | 0,759 | 0,607 | 0,911 |
| 7 jours | C3AR1 + IL1R2 + CD74 + CD3D + CTLA4 | 0,756 | 0,602 | 0,91 |
| 7 jours | C3AR1 + IL1R2 + CD74 + CIITA | 0,873 | 0,758 | 0,987 |
| 7 jours | C3AR1 + IL1R2 + CD74 + CIITA + CD3D | 0,861 | 0,743 | 0,98 |
| 7 jours | C3AR1 + IL1R2 + CD74 + CIITA + CD3D + CTLA4 | 0,856 | 0,737 | 0,975 |
| 7 jours | C3AR1 + IL1R2 + CD74 + CIITA + CTLA4 | 0,878 | 0,766 | 0,99 |
| 7 jours | C3AR1 + IL1R2 + CD74 + CIITA + TAP2 | 0,864 | 0,745 | 0,983 |
| 7 jours | C3AR1 + IL1R2 + CD74 + CIITA + TAP2 + CD3D | 0,861 | 0,743 | 0,98 |
| 7 jours | C3AR1 + IL1R2 + CD74 + CIITA + TAP2 + CD3D + CTLA4 | 0,856 | 0,737 | 0,975 |
| 7 jours | C3AR1 + IL1R2 + CD74 + CIITA + TAP2 + CTLA4 | 0,878 | 0,766 | 0,991 |
| 7 jours | C3AR1 + IL1R2 + CD74 + CTLA4 | 0,767 | 0,614 | 0,921 |
| 7 jours | C3AR1 + IL1R2 + CD74 + CX3CR1 | 0,776 | 0,616 | 0,935 |
| 7 jours | C3AR1 + IL1R2 + CD74 + CX3CR1 + CD3D | 0,773 | 0,622 | 0,924 |
| 7 jours | C3AR1 + IL1R2 + CD74 + CX3CR1 + CD3D + CTLA4 | 0,776 | 0,627 | 0,924 |
| 7 jours | C3AR1 + IL1R2 + CD74 + CX3CR1 + CIITA | 0,873 | 0,756 | 0,989 |
| 7 jours | C3AR1 + IL1R2 + CD74 + CX3CR1 + CIITA + CD3D | 0,867 | 0,75 | 0,984 |
| 7 jours | C3AR1 + IL1R2 + CD74 + CX3CR1 + CIITA + CD3D + CTLA4 | 0,867 | 0,753 | 0,981 |
| 7 jours | C3AR1 + IL1R2 + CD74 + CX3CR1 + CIITA + CTLA4 | 0,884 | 0,773 | 0,995 |
| 7 jours | C3AR1 + IL1R2 + CD74 + CX3CR1 + CIITA + TAP2 | 0,873 | 0,753 | 0,992 |
| 7 jours | C3AR1 + IL1R2 + CD74 + CX3CR1 + CIITA + TAP2 + CD3D | 0,87 | 0,754 | 0,986 |
| 7 jours | C3AR1 + IL1R2 + CD74 + CX3CR1 + CIITA + TAP2 + CD3D + CTLA4 | 0,864 | 0,749 | 0,979 |
| 7 jours | C3AR1 + IL1R2 + CD74 + CX3CR1 + CIITA + TAP2 + CTLA4 | 0,87 | 0,752 | 0,988 |
| 7 jours | C3AR1 + IL1R2 + CD74 + CX3CR1 + CTLA4 | 0,776 | 0,623 | 0,929 |
| 7 jours | C3AR1 + IL1R2 + CD74 + CX3CR1 + TAP2 | 0,781 | 0,626 | 0,936 |
| 7 jours | C3AR1 + IL1R2 + CD74 + CX3CR1 + TAP2 + CD3D | 0,784 | 0,634 | 0,934 |
| 7 jours | C3AR1 + IL1R2 + CD74 + CX3CR1 + TAP2 + CD3D + CTLA4 | 0,798 | 0,652 | 0,944 |
| 7 jours | C3AR1 + IL1R2 + CD74 + CX3CR1 + TAP2 + CTLA4 | 0,798 | 0,647 | 0,948 |
| 7 jours | C3AR1 + IL1R2 + CD74 + IFNG | 0,823 | 0,689 | 0,956 |
| 7 jours | C3AR1 + IL1R2 + CD74 + IFNG + CD177 | 0,842 | 0,719 | 0,965 |
| 7 jours | C3AR1 + IL1R2 + CD74 + IFNG + CD177 + CD3D | 0,839 | 0,715 | 0,963 |
| 7 jours | C3AR1 + IL1R2 + CD74 + IFNG + CD177 + CD3D + CTLA4 | 0,839 | 0,715 | 0,963 |
| 7 jours | C3AR1 + IL1R2 + CD74 + IFNG + CD177 + CIITA | 0,895 | 0,798 | 0,991 |
| 7 jours | C3AR1 + IL1R2 + CD74 + IFNG + CD177 + CIITA + CD3D | 0,898 | 0,803 | 0,992 |
| 7 jours | C3AR1 + IL1R2 + CD74 + IFNG + CD177 + CIITA + CD3D + CTLA4 | 0,9 | 0,807 | 0,993 |
| 7 jours | C3AR1 + IL1R2 + CD74 + IFNG + CD177 + CIITA + CTLA4 | 0,898 | 0,803 | 0,992 |
| 7 jours | C3AR1 + IL1R2 + CD74 + IFNG + CD177 + CIITA + TAP2 | 0,895 | 0,796 | 0,994 |
| 7 jours | C3AR1 + IL1R2 + CD74 + IFNG + CD177 + CIITA + TAP2 + CD3D | 0,895 | 0,797 | 0,992 |
| 7 jours | C3AR1 + IL1R2 + CD74 + IFNG + CD177 + CIITA + TAP2 + CD3D + CTLA4 | 0,9 | 0,806 | 0,994 |
| 7 jours | C3AR1 + IL1R2 + CD74 + IFNG + CD177 + CIITA + TAP2 + CTLA4 | 0,889 | 0,788 | 0,991 |
| 7 jours | C3AR1 + IL1R2 + CD74 + IFNG + CD177 + CTLA4 | 0,839 | 0,715 | 0,963 |
| 7 jours | C3AR1 + IL1R2 + CD74 + IFNG + CD177 + CX3CR1 | 0,881 | 0,775 | 0,987 |
| 7 jours | C3AR1 + IL1R2 + CD74 + IFNG + CD177 + CX3CR1 + CD3D | 0,892 | 0,792 | 0,992 |
| 7 jours | C3AR1 + IL1R2 + CD74 + IFNG + CD177 + CX3CR1 + CD3D + CTLA4 | 0,889 | 0,786 | 0,993 |
| 7 jours | C3AR1 + IL1R2 + CD74 + IFNG + CD177 + CX3CR1 + CIITA | 0,917 | 0,827 | 1 |
| 7 jours | C3AR1 + IL1R2 + CD74 + IFNG + CD177 + CX3CR1 + CIITA + CD3D | 0,928 | 0,84 | 1 |
| 7 jours | C3AR1 + IL1R2 + CD74 + IFNG + CD177 + CX3CR1 + CIITA + CD3D + CTLA4 | 0,925 | 0,836 | 1 |
| 7 jours | C3AR1 + IL1R2 + CD74 + IFNG + CD177 + CX3CR1 + CIITA + CTLA4 | 0,92 | 0,831 | 1 |
| 7 jours | C3AR1 + IL1R2 + CD74 + IFNG + CD177 + CX3CR1 + CIITA + TAP2 | 0,917 | 0,827 | 1 |
| 7 jours | C3AR1 + IL1R2 + CD74 + IFNG + CD177 + CX3CR1 + CIITA + TAP2 + CD3D | 0,934 | 0,848 | 1 |
| 7 jours | C3AR1 + IL1R2 + CD74 + IFNG + CD177 + CX3CR1 + CIITA + TAP2 + CD3D + CTLA4 | 0,939 | 0,86 | 1 |
| 7 jours | C3AR1 + IL1R2 + CD74 + IFNG + CD177 + CX3CR1 + CIITA + TAP2 + CTLA4 | 0,922 | 0,835 | 1 |
| 7 jours | C3AR1 + IL1R2 + CD74 + IFNG + CD177 + CX3CR1 + CTLA4 | 0,886 | 0,783 | 0,99 |
| 7 jours | C3AR1 + IL1R2 + CD74 + IFNG + CD177 + CX3CR1 + TAP2 | 0,881 | 0,775 | 0,987 |
| 7 jours | C3AR1 + IL1R2 + CD74 + IFNG + CD177 + CX3CR1 + TAP2 + CD3D | 0,892 | 0,79 | 0,994 |
| 7 jours | C3AR1 + IL1R2 + CD74 + IFNG + CD177 + CX3CR1 + TAP2 + CD3D + CTLA4 | 0,889 | 0,786 | 0,992 |
| 7 jours | C3AR1 + IL1R2 + CD74 + IFNG + CD177 + CX3CR1 + TAP2 + CTLA4 | 0,881 | 0,774 | 0,988 |
| 7 jours | C3AR1 + IL1R2 + CD74 + IFNG + CD177 + S100A9 | 0,839 | 0,715 | 0,963 |
| 7 jours | C3AR1 + IL1R2 + CD74 + IFNG + CD177 + S100A9 + CD3D | 0,839 | 0,715 | 0,963 |
| 7 jours | C3AR1 + IL1R2 + CD74 + IFNG + CD177 + S100A9 + CD3D + CTLA4 | 0,837 | 0,711 | 0,962 |
| 7 jours | C3AR1 + IL1R2 + CD74 + IFNG + CD177 + S100A9 + CIITA | 0,889 | 0,79 | 0,989 |
| 7 jours | C3AR1 + IL1R2 + CD74 + IFNG + CD177 + S100A9 + CIITA + CD3D | 0,892 | 0,794 | 0,99 |
| 7 jours | C3AR1 + IL1R2 + CD74 + IFNG + CD177 + S100A9 + CIITA + CD3D + CTLA4 | 0,889 | 0,79 | 0,988 |
| 7 jours | C3AR1 + IL1R2 + CD74 + IFNG + CD177 + S100A9 + CIITA + CTLA4 | 0,9 | 0,808 | 0,993 |
| 7 jours | C3AR1 + IL1R2 + CD74 + IFNG + CD177 + S100A9 + CIITA + TAP2 | 0,898 | 0,801 | 0,994 |
| 7 jours | C3AR1 + IL1R2 + CD74 + IFNG + CD177 + S100A9 + CIITA + TAP2 + CD3D | 0,898 | 0,802 | 0,993 |
| 7 jours | C3AR1 + IL1R2 + CD74 + IFNG + CD177 + S100A9 + CIITA + TAP2 + CD3D + CTLA4 | 0,909 | 0,819 | 0,998 |
| 7 jours | C3AR1 + IL1R2 + CD74 + IFNG + CD177 + S100A9 + CIITA + TAP2 + CTLA4 | 0,886 | 0,784 | 0,989 |
| 7 jours | C3AR1 + IL1R2 + CD74 + IFNG + CD177 + S100A9 + CTLA4 | 0,837 | 0,712 | 0,961 |
| 7 jours | C3AR1 + IL1R2 + CD74 + IFNG + CD177 + S100A9 + CX3CR1 | 0,878 | 0,77 | 0,986 |
| 7 jours | C3AR1 + IL1R2 + CD74 + IFNG + CD177 + S100A9 + CX3CR1 + CD3D | 0,881 | 0,776 | 0,986 |
| 7 jours | C3AR1 + IL1R2 + CD74 + IFNG + CD177 + S100A9 + CX3CR1 + CD3D + CTLA4 | 0,886 | 0,784 | 0,989 |
| 7 jours | C3AR1 + IL1R2 + CD74 + IFNG + CD177 + S100A9 + CX3CR1 + CIITA | 0,925 | 0,839 | 1 |
| 7 jours | C3AR1 + IL1R2 + CD74 + IFNG + CD177 + S100A9 + CX3CR1 + CIITA + CD3D | 0,928 | 0,84 | 1 |
| 7 jours | C3AR1 + IL1R2 + CD74 + IFNG + CD177 + S100A9 + CX3CR1 + CIITA + CD3D + CTLA4 | 0,925 | 0,84 | 1 |
| 7 jours | C3AR1 + IL1R2 + CD74 + IFNG + CD177 + S100A9 + CX3CR1 + CIITA + CTLA4 | 0,925 | 0,839 | 1 |
| 7 jours | C3AR1 + IL1R2 + CD74 + IFNG + CD177 + S100A9 + CX3CR1 + CIITA + TAP2 | 0,928 | 0,842 | 1 |
| 7 jours | C3AR1 + IL1R2 + CD74 + IFNG + CD177 + S100A9 + CX3CR1 + CIITA + TAP2 + CD3D | 0,934 | 0,849 | 1 |
| 7 jours | C3AR1 + IL1R2 + CD74 + IFNG + CD177 + S100A9 + CX3CR1 + CIITA + TAP2 + CD3D + CTLA4 | 0,936 | 0,855 | 1 |
| 7 jours | C3AR1 + IL1R2 + CD74 + IFNG + CD177 + S100A9 + CX3CR1 + CIITA + TAP2 + CTLA4 | 0,928 | 0,844 | 1 |
| 7 jours | C3AR1 + IL1R2 + CD74 + IFNG + CD177 + S100A9 + CX3CR1 + CTLA4 | 0,886 | 0,783 | 0,99 |
| 7 jours | C3AR1 + IL1R2 + CD74 + IFNG + CD177 + S100A9 + CX3CR1 + TAP2 | 0,878 | 0,77 | 0,986 |
| 7 jours | C3AR1 + IL1R2 + CD74 + IFNG + CD177 + S100A9 + CX3CR1 + TAP2 + CD3D | 0,878 | 0,771 | 0,985 |
| 7 jours | C3AR1 + IL1R2 + CD74 + IFNG + CD177 + S100A9 + CX3CR1 + TAP2 + CD3D + CTLA4 | 0,881 | 0,775 | 0,987 |
| 7 jours | C3AR1 + IL1R2 + CD74 + IFNG + CD177 + S100A9 + CX3CR1 + TAP2 + CTLA4 | 0,881 | 0,774 | 0,988 |
| 7 jours | C3AR1 + IL1R2 + CD74 + IFNG + CD177 + S100A9 + TAP2 | 0,842 | 0,717 | 0,967 |
| 7 jours | C3AR1 + IL1R2 + CD74 + IFNG + CD177 + S100A9 + TAP2 + CD3D | 0,842 | 0,718 | 0,966 |
| 7 jours | C3AR1 + IL1R2 + CD74 + IFNG + CD177 + S100A9 + TAP2 + CD3D + CTLA4 | 0,842 | 0,718 | 0,966 |
| 7 jours | C3AR1 + IL1R2 + CD74 + IFNG + CD177 + S100A9 + TAP2 + CTLA4 | 0,839 | 0,714 | 0,965 |
| 7 jours | C3AR1 + IL1R2 + CD74 + IFNG + CD177 + TAP2 | 0,848 | 0,725 | 0,97 |
| 7 jours | C3AR1 + IL1R2 + CD74 + IFNG + CD177 + TAP2 + CD3D | 0,839 | 0,714 | 0,964 |
| 7 jours | C3AR1 + IL1R2 + CD74 + IFNG + CD177 + TAP2 + CD3D + CTLA4 | 0,839 | 0,714 | 0,964 |
| 7 jours | C3AR1 + IL1R2 + CD74 + IFNG + CD177 + TAP2 + CTLA4 | 0,837 | 0,709 | 0,964 |
| 7 jours | C3AR1 + IL1R2 + CD74 + IFNG + CD3D | 0,823 | 0,69 | 0,955 |
| 7 jours | C3AR1 + IL1R2 + CD74 + IFNG + CD3D + CTLA4 | 0,828 | 0,698 | 0,958 |
| 7 jours | C3AR1 + IL1R2 + CD74 + IFNG + CIITA | 0,886 | 0,784 | 0,989 |
| 7 jours | C3AR1 + IL1R2 + CD74 + IFNG + CIITA + CD3D | 0,886 | 0,785 | 0,988 |
| 7 jours | C3AR1 + IL1R2 + CD74 + IFNG + CIITA + CD3D + CTLA4 | 0,884 | 0,779 | 0,988 |
| 7 jours | C3AR1 + IL1R2 + CD74 + IFNG + CIITA + CTLA4 | 0,881 | 0,775 | 0,987 |
| 7 jours | C3AR1 + IL1R2 + CD74 + IFNG + CIITA + TAP2 | 0,898 | 0,799 | 0,996 |
| 7 jours | C3AR1 + IL1R2 + CD74 + IFNG + CIITA + TAP2 + CD3D | 0,889 | 0,787 | 0,991 |
| 7 jours | C3AR1 + IL1R2 + CD74 + IFNG + CIITA + TAP2 + CD3D + CTLA4 | 0,895 | 0,791 | 0,998 |
| 7 jours | C3AR1 + IL1R2 + CD74 + IFNG + CIITA + TAP2 + CTLA4 | 0,892 | 0,787 | 0,997 |
| 7 jours | C3AR1 + IL1R2 + CD74 + IFNG + CTLA4 | 0,828 | 0,698 | 0,958 |
| 7 jours | C3AR1 + IL1R2 + CD74 + IFNG + CX3CR1 | 0,875 | 0,761 | 0,989 |
| 7 jours | C3AR1 + IL1R2 + CD74 + IFNG + CX3CR1 + CD3D | 0,87 | 0,754 | 0,986 |
| 7 jours | C3AR1 + IL1R2 + CD74 + IFNG + CX3CR1 + CD3D + CTLA4 | 0,886 | 0,779 | 0,994 |
| 7 jours | C3AR1 + IL1R2 + CD74 + IFNG + CX3CR1 + CIITA | 0,925 | 0,836 | 1 |
| 7 jours | C3AR1 + IL1R2 + CD74 + IFNG + CX3CR1 + CIITA + CD3D | 0,928 | 0,84 | 1 |
| 7 jours | C3AR1 + IL1R2 + CD74 + IFNG + CX3CR1 + CIITA + CD3D + CTLA4 | 0,939 | 0,857 | 1 |
| 7 jours | C3AR1 + IL1R2 + CD74 + IFNG + CX3CR1 + CIITA + CTLA4 | 0,925 | 0,838 | 1 |
| 7 jours | C3AR1 + IL1R2 + CD74 + IFNG + CX3CR1 + CIITA + TAP2 | 0,922 | 0,834 | 1 |
| 7 jours | C3AR1 + IL1R2 + CD74 + IFNG + CX3CR1 + CIITA + TAP2 + CD3D | 0,934 | 0,848 | 1 |
| 7 jours | C3AR1 + IL1R2 + CD74 + IFNG + CX3CR1 + CIITA + TAP2 + CD3D + CTLA4 | 0,945 | 0,868 | 1 |
| 7 jours | C3AR1 + IL1R2 + CD74 + IFNG + CX3CR1 + CIITA + TAP2 + CTLA4 | 0,925 | 0,838 | 1 |
| 7 jours | C3AR1 + IL1R2 + CD74 + IFNG + CX3CR1 + CTLA4 | 0,878 | 0,767 | 0,99 |
| 7 jours | C3AR1 + IL1R2 + CD74 + IFNG + CX3CR1 + TAP2 | 0,878 | 0,766 | 0,99 |
| 7 jours | C3AR1 + IL1R2 + CD74 + IFNG + CX3CR1 + TAP2 + CD3D | 0,881 | 0,771 | 0,991 |
| 7 jours | C3AR1 + IL1R2 + CD74 + IFNG + CX3CR1 + TAP2 + CD3D + CTLA4 | 0,881 | 0,77 | 0,992 |
| 7 jours | C3AR1 + IL1R2 + CD74 + IFNG + CX3CR1 + TAP2 + CTLA4 | 0,878 | 0,767 | 0,99 |
| 7 jours | C3AR1 + IL1R2 + CD74 + IFNG + S100A9 | 0,842 | 0,716 | 0,968 |
| 7 jours | C3AR1 + IL1R2 + CD74 + IFNG + S100A9 + CD3D | 0,842 | 0,718 | 0,966 |
| 7 jours | C3AR1 + IL1R2 + CD74 + IFNG + S100A9 + CD3D + CTLA4 | 0,845 | 0,722 | 0,968 |
| 7 jours | C3AR1 + IL1R2 + CD74 + IFNG + S100A9 + CIITA | 0,886 | 0,785 | 0,988 |
| 7 jours | C3AR1 + IL1R2 + CD74 + IFNG + S100A9 + CIITA + CD3D | 0,886 | 0,785 | 0,988 |
| 7 jours | C3AR1 + IL1R2 + CD74 + IFNG + S100A9 + CIITA + CD3D + CTLA4 | 0,884 | 0,78 | 0,987 |
| 7 jours | C3AR1 + IL1R2 + CD74 + IFNG + S100A9 + CIITA + CTLA4 | 0,875 | 0,767 | 0,984 |
| 7 jours | C3AR1 + IL1R2 + CD74 + IFNG + S100A9 + CIITA + TAP2 | 0,895 | 0,795 | 0,994 |
| 7 jours | C3AR1 + IL1R2 + CD74 + IFNG + S100A9 + CIITA + TAP2 + CD3D | 0,895 | 0,797 | 0,992 |
| 7 jours | C3AR1 + IL1R2 + CD74 + IFNG + S100A9 + CIITA + TAP2 + CD3D + CTLA4 | 0,906 | 0,814 | 0,998 |
| 7 jours | C3AR1 + IL1R2 + CD74 + IFNG + S100A9 + CIITA + TAP2 + CTLA4 | 0,889 | 0,785 | 0,994 |
| 7 jours | C3AR1 + IL1R2 + CD74 + IFNG + S100A9 + CTLA4 | 0,839 | 0,713 | 0,965 |
| 7 jours | C3AR1 + IL1R2 + CD74 + IFNG + S100A9 + CX3CR1 | 0,861 | 0,745 | 0,978 |
| 7 jours | C3AR1 + IL1R2 + CD74 + IFNG + S100A9 + CX3CR1 + CD3D | 0,878 | 0,772 | 0,985 |
| 7 jours | C3AR1 + IL1R2 + CD74 + IFNG + S100A9 + CX3CR1 + CD3D + CTLA4 | 0,884 | 0,776 | 0,991 |
| 7 jours | C3AR1 + IL1R2 + CD74 + IFNG + S100A9 + CX3CR1 + CIITA | 0,931 | 0,844 | 1 |
| 7 jours | C3AR1 + IL1R2 + CD74 + IFNG + S100A9 + CX3CR1 + CIITA + CD3D | 0,928 | 0,84 | 1 |
| 7 jours | C3AR1 + IL1R2 + CD74 + IFNG + S100A9 + CX3CR1 + CIITA + CD3D + CTLA4 | 0,925 | 0,84 | 1 |
| 7 jours | C3AR1 + IL1R2 + CD74 + IFNG + S100A9 + CX3CR1 + CIITA + CTLA4 | 0,934 | 0,851 | 1 |
| 7 jours | C3AR1 + IL1R2 + CD74 + IFNG + S100A9 + CX3CR1 + CIITA + TAP2 | 0,925 | 0,836 | 1 |
| 7 jours | C3AR1 + IL1R2 + CD74 + IFNG + S100A9 + CX3CR1 + CIITA + TAP2 + CD3D | 0,931 | 0,845 | 1 |
| 7 jours | C3AR1 + IL1R2 + CD74 + IFNG + S100A9 + CX3CR1 + CIITA + TAP2 + CD3D + CTLA4 | 0,936 | 0,856 | 1 |
| 7 jours | C3AR1 + IL1R2 + CD74 + IFNG + S100A9 + CX3CR1 + CIITA + TAP2 + CTLA4 | 0,931 | 0,847 | 1 |
| 7 jours | C3AR1 + IL1R2 + CD74 + IFNG + S100A9 + CX3CR1 + CTLA4 | 0,878 | 0,769 | 0,987 |
| 7 jours | C3AR1 + IL1R2 + CD74 + IFNG + S100A9 + CX3CR1 + TAP2 | 0,867 | 0,753 | 0,982 |
| 7 jours | C3AR1 + IL1R2 + CD74 + IFNG + S100A9 + CX3CR1 + TAP2 + CD3D | 0,886 | 0,783 | 0,99 |
| 7 jours | C3AR1 + IL1R2 + CD74 + IFNG + S100A9 + CX3CR1 + TAP2 + CD3D + CTLA4 | 0,886 | 0,783 | 0,99 |
| 7 jours | C3AR1 + IL1R2 + CD74 + IFNG + S100A9 + CX3CR1 + TAP2 + CTLA4 | 0,881 | 0,771 | 0,991 |
| 7 jours | C3AR1 + IL1R2 + CD74 + IFNG + S100A9 + TAP2 | 0,845 | 0,717 | 0,972 |
| 7 jours | C3AR1 + IL1R2 + CD74 + IFNG + S100A9 + TAP2 + CD3D | 0,842 | 0,716 | 0,968 |
| 7 jours | C3AR1 + IL1R2 + CD74 + IFNG + S100A9 + TAP2 + CD3D + CTLA4 | 0,845 | 0,721 | 0,969 |
| 7 jours | C3AR1 + IL1R2 + CD74 + IFNG + S100A9 + TAP2 + CTLA4 | 0,848 | 0,722 | 0,973 |
| 7 jours | C3AR1 + IL1R2 + CD74 + IFNG + TAP2 | 0,839 | 0,712 | 0,966 |
| 7 jours | C3AR1 + IL1R2 + CD74 + IFNG + TAP2 + CD3D | 0,837 | 0,711 | 0,962 |
| 7 jours | C3AR1 + IL1R2 + CD74 + IFNG + TAP2 + CD3D + CTLA4 | 0,845 | 0,721 | 0,969 |
| 7 jours | C3AR1 + IL1R2 + CD74 + IFNG + TAP2 + CTLA4 | 0,85 | 0,727 | 0,974 |
| 7 jours | C3AR1 + IL1R2 + CD74 + S100A9 | 0,831 | 0,69 | 0,972 |
| 7 jours | C3AR1 + IL1R2 + CD74 + S100A9 + CD3D | 0,825 | 0,685 | 0,966 |
| 7 jours | C3AR1 + IL1R2 + CD74 + S100A9 + CD3D + CTLA4 | 0,828 | 0,694 | 0,963 |
| 7 jours | C3AR1 + IL1R2 + CD74 + S100A9 + CIITA | 0,886 | 0,776 | 0,997 |
| 7 jours | C3AR1 + IL1R2 + CD74 + S100A9 + CIITA + CD3D | 0,898 | 0,798 | 0,997 |
| 7 jours | C3AR1 + IL1R2 + CD74 + S100A9 + CIITA + CD3D + CTLA4 | 0,892 | 0,791 | 0,993 |
| 7 jours | C3AR1 + IL1R2 + CD74 + S100A9 + CIITA + CTLA4 | 0,884 | 0,772 | 0,995 |
| 7 jours | C3AR1 + IL1R2 + CD74 + S100A9 + CIITA + TAP2 | 0,884 | 0,772 | 0,995 |
| 7 jours | C3AR1 + IL1R2 + CD74 + S100A9 + CIITA + TAP2 + CD3D | 0,895 | 0,792 | 0,997 |
| 7 jours | C3AR1 + IL1R2 + CD74 + S100A9 + CIITA + TAP2 + CD3D + CTLA4 | 0,9 | 0,804 | 0,996 |
| 7 jours | C3AR1 + IL1R2 + CD74 + S100A9 + CIITA + TAP2 + CTLA4 | 0,881 | 0,771 | 0,991 |
| 7 jours | C3AR1 + IL1R2 + CD74 + S100A9 + CTLA4 | 0,831 | 0,69 | 0,972 |
| 7 jours | C3AR1 + IL1R2 + CD74 + S100A9 + CX3CR1 | 0,848 | 0,711 | 0,984 |
| 7 jours | C3AR1 + IL1R2 + CD74 + S100A9 + CX3CR1 + CD3D | 0,848 | 0,712 | 0,983 |
| 7 jours | C3AR1 + IL1R2 + CD74 + S100A9 + CX3CR1 + CD3D + CTLA4 | 0,842 | 0,703 | 0,981 |
| 7 jours | C3AR1 + IL1R2 + CD74 + S100A9 + CX3CR1 + CIITA | 0,881 | 0,764 | 0,997 |
| 7 jours | C3AR1 + IL1R2 + CD74 + S100A9 + CX3CR1 + CIITA + CD3D | 0,881 | 0,767 | 0,995 |
| 7 jours | C3AR1 + IL1R2 + CD74 + S100A9 + CX3CR1 + CIITA + CD3D + CTLA4 | 0,892 | 0,79 | 0,994 |
| 7 jours | C3AR1 + IL1R2 + CD74 + S100A9 + CX3CR1 + CIITA + CTLA4 | 0,889 | 0,78 | 0,998 |
| 7 jours | C3AR1 + IL1R2 + CD74 + S100A9 + CX3CR1 + CIITA + TAP2 | 0,878 | 0,762 | 0,994 |
| 7 jours | C3AR1 + IL1R2 + CD74 + S100A9 + CX3CR1 + CIITA + TAP2 + CD3D | 0,886 | 0,776 | 0,997 |
| 7 jours | C3AR1 + IL1R2 + CD74 + S100A9 + CX3CR1 + CIITA + TAP2 + CD3D + CTLA4 | 0,906 | 0,81 | 1 |
| 7 jours | C3AR1 + IL1R2 + CD74 + S100A9 + CX3CR1 + CIITA + TAP2 + CTLA4 | 0,884 | 0,769 | 0,999 |
| 7 jours | C3AR1 + IL1R2 + CD74 + S100A9 + CX3CR1 + CTLA4 | 0,853 | 0,72 | 0,986 |
| 7 jours | C3AR1 + IL1R2 + CD74 + S100A9 + CX3CR1 + TAP2 | 0,856 | 0,717 | 0,995 |
| 7 jours | C3AR1 + IL1R2 + CD74 + S100A9 + CX3CR1 + TAP2 + CD3D | 0,842 | 0,704 | 0,98 |
| 7 jours | C3AR1 + IL1R2 + CD74 + S100A9 + CX3CR1 + TAP2 + CD3D + CTLA4 | 0,845 | 0,708 | 0,982 |
| 7 jours | C3AR1 + IL1R2 + CD74 + S100A9 + CX3CR1 + TAP2 + CTLA4 | 0,85 | 0,711 | 0,989 |
| 7 jours | C3AR1 + IL1R2 + CD74 + S100A9 + TAP2 | 0,828 | 0,687 | 0,969 |
| 7 jours | C3AR1 + IL1R2 + CD74 + S100A9 + TAP2 + CD3D | 0,839 | 0,705 | 0,974 |
| 7 jours | C3AR1 + IL1R2 + CD74 + S100A9 + TAP2 + CD3D + CTLA4 | 0,839 | 0,708 | 0,97 |
| 7 jours | C3AR1 + IL1R2 + CD74 + S100A9 + TAP2 + CTLA4 | 0,828 | 0,687 | 0,969 |
| 7 jours | C3AR1 + IL1R2 + CD74 + TAP2 | 0,767 | 0,614 | 0,921 |
| 7 jours | C3AR1 + IL1R2 + CD74 + TAP2 + CD3D | 0,776 | 0,627 | 0,924 |
| 7 jours | C3AR1 + IL1R2 + CD74 + TAP2 + CD3D + CTLA4 | 0,778 | 0,629 | 0,928 |
| 7 jours | C3AR1 + IL1R2 + CD74 + TAP2 + CTLA4 | 0,789 | 0,64 | 0,939 |
| 7 jours | C3AR1 + IL1R2 + CIITA | 0,837 | 0,707 | 0,966 |
| 7 jours | C3AR1 + IL1R2 + CIITA + CD3D | 0,839 | 0,713 | 0,966 |
| 7 jours | C3AR1 + IL1R2 + CIITA + CD3D + CTLA4 | 0,853 | 0,736 | 0,97 |
| 7 jours | C3AR1 + IL1R2 + CIITA + CTLA4 | 0,848 | 0,725 | 0,97 |
| 7 jours | C3AR1 + IL1R2 + CIITA + TAP2 | 0,845 | 0,719 | 0,971 |
| 7 jours | C3AR1 + IL1R2 + CIITA + TAP2 + CD3D | 0,845 | 0,722 | 0,968 |
| 7 jours | C3AR1 + IL1R2 + CIITA + TAP2 + CD3D + CTLA4 | 0,848 | 0,728 | 0,967 |
| 7 jours | C3AR1 + IL1R2 + CIITA + TAP2 + CTLA4 | 0,842 | 0,716 | 0,968 |
| 7 jours | C3AR1 + IL1R2 + CX3CR1 + CIITA | 0,839 | 0,711 | 0,967 |
| 7 jours | C3AR1 + IL1R2 + CX3CR1 + CIITA + CD3D | 0,834 | 0,706 | 0,962 |
| 7 jours | C3AR1 + IL1R2 + CX3CR1 + CIITA + CD3D + CTLA4 | 0,853 | 0,736 | 0,97 |
| 7 jours | C3AR1 + IL1R2 + CX3CR1 + CIITA + CTLA4 | 0,842 | 0,718 | 0,966 |
| 7 jours | C3AR1 + IL1R2 + CX3CR1 + CIITA + TAP2 | 0,853 | 0,732 | 0,974 |
| 7 jours | C3AR1 + IL1R2 + CX3CR1 + CIITA + TAP2 + CD3D | 0,842 | 0,718 | 0,966 |
| 7 jours | C3AR1 + IL1R2 + CX3CR1 + CIITA + TAP2 + CD3D + CTLA4 | 0,856 | 0,74 | 0,972 |
| 7 jours | C3AR1 + IL1R2 + CX3CR1 + CIITA + TAP2 + CTLA4 | 0,85 | 0,727 | 0,974 |
| 7 jours | C3AR1 + IL1R2 + CX3CR1 + TAP2 | 0,695 | 0,52 | 0,871 |
| 7 jours | C3AR1 + IL1R2 + CX3CR1 + TAP2 + CD3D | 0,69 | 0,512 | 0,868 |
| 7 jours | C3AR1 + IL1R2 + CX3CR1 + TAP2 + CD3D + CTLA4 | 0,695 | 0,518 | 0,873 |
| 7 jours | C3AR1 + IL1R2 + CX3CR1 + TAP2 + CTLA4 | 0,695 | 0,518 | 0,873 |
| 7 jours | C3AR1 + IL1R2 + IFNG | 0,726 | 0,562 | 0,89 |
| 7 jours | C3AR1 + IL1R2 + IFNG + CD177 | 0,801 | 0,658 | 0,943 |
| 7 jours | C3AR1 + IL1R2 + IFNG + CD177 + CD3D | 0,803 | 0,663 | 0,943 |
| 7 jours | C3AR1 + IL1R2 + IFNG + CD177 + CD3D + CTLA4 | 0,812 | 0,675 | 0,948 |
| 7 jours | C3AR1 + IL1R2 + IFNG + CD177 + CIITA | 0,881 | 0,777 | 0,985 |
| 7 jours | C3AR1 + IL1R2 + IFNG + CD177 + CIITA + CD3D | 0,875 | 0,768 | 0,982 |
| 7 jours | C3AR1 + IL1R2 + IFNG + CD177 + CIITA + CD3D + CTLA4 | 0,884 | 0,781 | 0,986 |
| 7 jours | C3AR1 + IL1R2 + IFNG + CD177 + CIITA + CTLA4 | 0,878 | 0,773 | 0,983 |
| 7 jours | C3AR1 + IL1R2 + IFNG + CD177 + CIITA + TAP2 | 0,878 | 0,773 | 0,983 |
| 7 jours | C3AR1 + IL1R2 + IFNG + CD177 + CIITA + TAP2 + CD3D | 0,878 | 0,772 | 0,984 |
| 7 jours | C3AR1 + IL1R2 + IFNG + CD177 + CIITA + TAP2 + CD3D + CTLA4 | 0,889 | 0,789 | 0,989 |
| 7 jours | C3AR1 + IL1R2 + IFNG + CD177 + CIITA + TAP2 + CTLA4 | 0,878 | 0,773 | 0,983 |
| 7 jours | C3AR1 + IL1R2 + IFNG + CD177 + CTLA4 | 0,809 | 0,671 | 0,947 |
| 7 jours | C3AR1 + IL1R2 + IFNG + CD177 + CX3CR1 | 0,806 | 0,664 | 0,949 |
| 7 jours | C3AR1 + IL1R2 + IFNG + CD177 + CX3CR1 + CD3D | 0,812 | 0,673 | 0,951 |
| 7 jours | C3AR1 + IL1R2 + IFNG + CD177 + CX3CR1 + CD3D + CTLA4 | 0,806 | 0,665 | 0,947 |
| 7 jours | C3AR1 + IL1R2 + IFNG + CD177 + CX3CR1 + CIITA | 0,875 | 0,768 | 0,982 |
| 7 jours | C3AR1 + IL1R2 + IFNG + CD177 + CX3CR1 + CIITA + CD3D | 0,881 | 0,775 | 0,987 |
| 7 jours | C3AR1 + IL1R2 + IFNG + CD177 + CX3CR1 + CIITA + CD3D + CTLA4 | 0,903 | 0,808 | 0,999 |
| 7 jours | C3AR1 + IL1R2 + IFNG + CD177 + CX3CR1 + CIITA + CTLA4 | 0,886 | 0,786 | 0,987 |
| 7 jours | C3AR1 + IL1R2 + IFNG + CD177 + CX3CR1 + CIITA + TAP2 | 0,878 | 0,772 | 0,984 |
| 7 jours | C3AR1 + IL1R2 + IFNG + CD177 + CX3CR1 + CIITA + TAP2 + CD3D | 0,892 | 0,793 | 0,991 |
| 7 jours | C3AR1 + IL1R2 + IFNG + CD177 + CX3CR1 + CIITA + TAP2 + CD3D + CTLA4 | 0,903 | 0,808 | 0,999 |
| 7 jours | C3AR1 + IL1R2 + IFNG + CD177 + CX3CR1 + CIITA + TAP2 + CTLA4 | 0,892 | 0,793 | 0,991 |
| 7 jours | C3AR1 + IL1R2 + IFNG + CD177 + CX3CR1 + CTLA4 | 0,806 | 0,665 | 0,948 |
| 7 jours | C3AR1 + IL1R2 + IFNG + CD177 + CX3CR1 + TAP2 | 0,82 | 0,684 | 0,956 |
| 7 jours | C3AR1 + IL1R2 + IFNG + CD177 + CX3CR1 + TAP2 + CD3D | 0,817 | 0,68 | 0,954 |
| 7 jours | C3AR1 + IL1R2 + IFNG + CD177 + CX3CR1 + TAP2 + CD3D + CTLA4 | 0,82 | 0,684 | 0,956 |
| 7 jours | C3AR1 + IL1R2 + IFNG + CD177 + CX3CR1 + TAP2 + CTLA4 | 0,82 | 0,685 | 0,955 |
| 7 jours | C3AR1 + IL1R2 + IFNG + CD177 + S100A9 | 0,795 | 0,651 | 0,939 |
| 7 jours | C3AR1 + IL1R2 + IFNG + CD177 + S100A9 + CD3D | 0,795 | 0,651 | 0,939 |
| 7 jours | C3AR1 + IL1R2 + IFNG + CD177 + S100A9 + CD3D + CTLA4 | 0,801 | 0,659 | 0,942 |
| 7 jours | C3AR1 + IL1R2 + IFNG + CD177 + S100A9 + CIITA | 0,875 | 0,768 | 0,982 |
| 7 jours | C3AR1 + IL1R2 + IFNG + CD177 + S100A9 + CIITA + CD3D | 0,875 | 0,768 | 0,982 |
| 7 jours | C3AR1 + IL1R2 + IFNG + CD177 + S100A9 + CIITA + CD3D + CTLA4 | 0,875 | 0,769 | 0,982 |
| 7 jours | C3AR1 + IL1R2 + IFNG + CD177 + S100A9 + CIITA + CTLA4 | 0,878 | 0,773 | 0,983 |
| 7 jours | C3AR1 + IL1R2 + IFNG + CD177 + S100A9 + CIITA + TAP2 | 0,878 | 0,772 | 0,984 |
| 7 jours | C3AR1 + IL1R2 + IFNG + CD177 + S100A9 + CIITA + TAP2 + CD3D | 0,878 | 0,772 | 0,984 |
| 7 jours | C3AR1 + IL1R2 + IFNG + CD177 + S100A9 + CIITA + TAP2 + CD3D + CTLA4 | 0,884 | 0,782 | 0,985 |
| 7 jours | C3AR1 + IL1R2 + IFNG + CD177 + S100A9 + CIITA + TAP2 + CTLA4 | 0,875 | 0,769 | 0,982 |
| 7 jours | C3AR1 + IL1R2 + IFNG + CD177 + S100A9 + CTLA4 | 0,801 | 0,659 | 0,942 |
| 7 jours | C3AR1 + IL1R2 + IFNG + CD177 + S100A9 + CX3CR1 | 0,789 | 0,644 | 0,935 |
| 7 jours | C3AR1 + IL1R2 + IFNG + CD177 + S100A9 + CX3CR1 + CD3D | 0,798 | 0,657 | 0,939 |
| 7 jours | C3AR1 + IL1R2 + IFNG + CD177 + S100A9 + CX3CR1 + CD3D + CTLA4 | 0,792 | 0,65 | 0,935 |
| 7 jours | C3AR1 + IL1R2 + IFNG + CD177 + S100A9 + CX3CR1 + CIITA | 0,881 | 0,775 | 0,986 |
| 7 jours | C3AR1 + IL1R2 + IFNG + CD177 + S100A9 + CX3CR1 + CIITA + CD3D | 0,884 | 0,78 | 0,988 |
| 7 jours | C3AR1 + IL1R2 + IFNG + CD177 + S100A9 + CX3CR1 + CIITA + CD3D + CTLA4 | 0,889 | 0,788 | 0,991 |
| 7 jours | C3AR1 + IL1R2 + IFNG + CD177 + S100A9 + CX3CR1 + CIITA + CTLA4 | 0,889 | 0,789 | 0,989 |
| 7 jours | C3AR1 + IL1R2 + IFNG + CD177 + S100A9 + CX3CR1 + CIITA + TAP2 | 0,898 | 0,798 | 0,997 |
| 7 jours | C3AR1 + IL1R2 + IFNG + CD177 + S100A9 + CX3CR1 + CIITA + TAP2 + CD3D | 0,892 | 0,793 | 0,991 |
| 7 jours | C3AR1 + IL1R2 + IFNG + CD177 + S100A9 + CX3CR1 + CIITA + TAP2 + CD3D + CTLA4 | 0,9 | 0,806 | 0,994 |
| 7 jours | C3AR1 + IL1R2 + IFNG + CD177 + S100A9 + CX3CR1 + CIITA + TAP2 + CTLA4 | 0,889 | 0,787 | 0,992 |
| 7 jours | C3AR1 + IL1R2 + IFNG + CD177 + S100A9 + CX3CR1 + CTLA4 | 0,806 | 0,666 | 0,946 |
| 7 jours | C3AR1 + IL1R2 + IFNG + CD177 + S100A9 + CX3CR1 + TAP2 | 0,814 | 0,679 | 0,95 |
| 7 jours | C3AR1 + IL1R2 + IFNG + CD177 + S100A9 + CX3CR1 + TAP2 + CD3D | 0,809 | 0,672 | 0,946 |
| 7 jours | C3AR1 + IL1R2 + IFNG + CD177 + S100A9 + CX3CR1 + TAP2 + CD3D + CTLA4 | 0,814 | 0,679 | 0,95 |
| 7 jours | C3AR1 + IL1R2 + IFNG + CD177 + S100A9 + CX3CR1 + TAP2 + CTLA4 | 0,814 | 0,679 | 0,95 |
| 7 jours | C3AR1 + IL1R2 + IFNG + CD177 + S100A9 + TAP2 | 0,801 | 0,66 | 0,941 |
| 7 jours | C3AR1 + IL1R2 + IFNG + CD177 + S100A9 + TAP2 + CD3D | 0,801 | 0,659 | 0,942 |
| 7 jours | C3AR1 + IL1R2 + IFNG + CD177 + S100A9 + TAP2 + CD3D + CTLA4 | 0,803 | 0,662 | 0,945 |
| 7 jours | C3AR1 + IL1R2 + IFNG + CD177 + S100A9 + TAP2 + CTLA4 | 0,798 | 0,656 | 0,94 |
| 7 jours | C3AR1 + IL1R2 + IFNG + CD177 + TAP2 | 0,817 | 0,68 | 0,954 |
| 7 jours | C3AR1 + IL1R2 + IFNG + CD177 + TAP2 + CD3D | 0,812 | 0,673 | 0,95 |
| 7 jours | C3AR1 + IL1R2 + IFNG + CD177 + TAP2 + CD3D + CTLA4 | 0,803 | 0,662 | 0,945 |
| 7 jours | C3AR1 + IL1R2 + IFNG + CD177 + TAP2 + CTLA4 | 0,809 | 0,669 | 0,948 |
| 7 jours | C3AR1 + IL1R2 + IFNG + CD3D | 0,734 | 0,573 | 0,895 |
| 7 jours | C3AR1 + IL1R2 + IFNG + CD3D + CTLA4 | 0,726 | 0,563 | 0,888 |
| 7 jours | C3AR1 + IL1R2 + IFNG + CIITA | 0,853 | 0,734 | 0,972 |
| 7 jours | C3AR1 + IL1R2 + IFNG + CIITA + CD3D | 0,853 | 0,734 | 0,973 |
| 7 jours | C3AR1 + IL1R2 + IFNG + CIITA + CD3D + CTLA4 | 0,867 | 0,753 | 0,981 |
| 7 jours | C3AR1 + IL1R2 + IFNG + CIITA + CTLA4 | 0,859 | 0,741 | 0,977 |
| 7 jours | C3AR1 + IL1R2 + IFNG + CIITA + TAP2 | 0,856 | 0,739 | 0,973 |
| 7 jours | C3AR1 + IL1R2 + IFNG + CIITA + TAP2 + CD3D | 0,853 | 0,734 | 0,973 |
| 7 jours | C3AR1 + IL1R2 + IFNG + CIITA + TAP2 + CD3D + CTLA4 | 0,87 | 0,757 | 0,983 |
| 7 jours | C3AR1 + IL1R2 + IFNG + CIITA + TAP2 + CTLA4 | 0,853 | 0,732 | 0,974 |
| 7 jours | C3AR1 + IL1R2 + IFNG + CTLA4 | 0,72 | 0,557 | 0,884 |
| 7 jours | C3AR1 + IL1R2 + IFNG + CX3CR1 | 0,756 | 0,6 | 0,913 |
| 7 jours | C3AR1 + IL1R2 + IFNG + CX3CR1 + CD3D | 0,745 | 0,585 | 0,906 |
| 7 jours | C3AR1 + IL1R2 + IFNG + CX3CR1 + CD3D + CTLA4 | 0,77 | 0,616 | 0,924 |
| 7 jours | C3AR1 + IL1R2 + IFNG + CX3CR1 + CIITA | 0,864 | 0,749 | 0,98 |
| 7 jours | C3AR1 + IL1R2 + IFNG + CX3CR1 + CIITA + CD3D | 0,881 | 0,772 | 0,99 |
| 7 jours | C3AR1 + IL1R2 + IFNG + CX3CR1 + CIITA + CD3D + CTLA4 | 0,884 | 0,777 | 0,99 |
| 7 jours | C3AR1 + IL1R2 + IFNG + CX3CR1 + CIITA + CTLA4 | 0,87 | 0,759 | 0,981 |
| 7 jours | C3AR1 + IL1R2 + IFNG + CX3CR1 + CIITA + TAP2 | 0,873 | 0,763 | 0,982 |
| 7 jours | C3AR1 + IL1R2 + IFNG + CX3CR1 + CIITA + TAP2 + CD3D | 0,873 | 0,763 | 0,982 |
| 7 jours | C3AR1 + IL1R2 + IFNG + CX3CR1 + CIITA + TAP2 + CD3D + CTLA4 | 0,886 | 0,783 | 0,99 |
| 7 jours | C3AR1 + IL1R2 + IFNG + CX3CR1 + CIITA + TAP2 + CTLA4 | 0,875 | 0,765 | 0,985 |
| 7 jours | C3AR1 + IL1R2 + IFNG + CX3CR1 + CTLA4 | 0,77 | 0,616 | 0,924 |
| 7 jours | C3AR1 + IL1R2 + IFNG + CX3CR1 + TAP2 | 0,77 | 0,614 | 0,926 |
| 7 jours | C3AR1 + IL1R2 + IFNG + CX3CR1 + TAP2 + CD3D | 0,778 | 0,626 | 0,931 |
| 7 jours | C3AR1 + IL1R2 + IFNG + CX3CR1 + TAP2 + CD3D + CTLA4 | 0,778 | 0,629 | 0,927 |
| 7 jours | C3AR1 + IL1R2 + IFNG + CX3CR1 + TAP2 + CTLA4 | 0,784 | 0,637 | 0,931 |
| 7 jours | C3AR1 + IL1R2 + IFNG + S100A9 | 0,751 | 0,595 | 0,906 |
| 7 jours | C3AR1 + IL1R2 + IFNG + S100A9 + CD3D | 0,759 | 0,606 | 0,912 |
| 7 jours | C3AR1 + IL1R2 + IFNG + S100A9 + CD3D + CTLA4 | 0,753 | 0,599 | 0,908 |
| 7 jours | C3AR1 + IL1R2 + IFNG + S100A9 + CIITA | 0,856 | 0,74 | 0,972 |
| 7 jours | C3AR1 + IL1R2 + IFNG + S100A9 + CIITA + CD3D | 0,87 | 0,76 | 0,979 |
| 7 jours | C3AR1 + IL1R2 + IFNG + S100A9 + CIITA + CD3D + CTLA4 | 0,87 | 0,759 | 0,98 |
| 7 jours | C3AR1 + IL1R2 + IFNG + S100A9 + CIITA + CTLA4 | 0,864 | 0,752 | 0,977 |
| 7 jours | C3AR1 + IL1R2 + IFNG + S100A9 + CIITA + TAP2 | 0,859 | 0,744 | 0,974 |
| 7 jours | C3AR1 + IL1R2 + IFNG + S100A9 + CIITA + TAP2 + CD3D | 0,87 | 0,76 | 0,979 |
| 7 jours | C3AR1 + IL1R2 + IFNG + S100A9 + CIITA + TAP2 + CD3D + CTLA4 | 0,881 | 0,777 | 0,985 |
| 7 jours | C3AR1 + IL1R2 + IFNG + S100A9 + CIITA + TAP2 + CTLA4 | 0,864 | 0,752 | 0,976 |
| 7 jours | C3AR1 + IL1R2 + IFNG + S100A9 + CTLA4 | 0,74 | 0,582 | 0,898 |
| 7 jours | C3AR1 + IL1R2 + IFNG + S100A9 + CX3CR1 | 0,778 | 0,63 | 0,927 |
| 7 jours | C3AR1 + IL1R2 + IFNG + S100A9 + CX3CR1 + CD3D | 0,787 | 0,639 | 0,934 |
| 7 jours | C3AR1 + IL1R2 + IFNG + S100A9 + CX3CR1 + CD3D + CTLA4 | 0,787 | 0,639 | 0,934 |
| 7 jours | C3AR1 + IL1R2 + IFNG + S100A9 + CX3CR1 + CIITA | 0,873 | 0,764 | 0,981 |
| 7 jours | C3AR1 + IL1R2 + IFNG + S100A9 + CX3CR1 + CIITA + CD3D | 0,881 | 0,777 | 0,985 |
| 7 jours | C3AR1 + IL1R2 + IFNG + S100A9 + CX3CR1 + CIITA + CD3D + CTLA4 | 0,9 | 0,807 | 0,993 |
| 7 jours | C3AR1 + IL1R2 + IFNG + S100A9 + CX3CR1 + CIITA + CTLA4 | 0,875 | 0,766 | 0,985 |
| 7 jours | C3AR1 + IL1R2 + IFNG + S100A9 + CX3CR1 + CIITA + TAP2 | 0,875 | 0,768 | 0,983 |
| 7 jours | C3AR1 + IL1R2 + IFNG + S100A9 + CX3CR1 + CIITA + TAP2 + CD3D | 0,886 | 0,786 | 0,987 |
| 7 jours | C3AR1 + IL1R2 + IFNG + S100A9 + CX3CR1 + CIITA + TAP2 + CD3D + CTLA4 | 0,911 | 0,824 | 0,999 |
| 7 jours | C3AR1 + IL1R2 + IFNG + S100A9 + CX3CR1 + CIITA + TAP2 + CTLA4 | 0,881 | 0,776 | 0,986 |
| 7 jours | C3AR1 + IL1R2 + IFNG + S100A9 + CX3CR1 + CTLA4 | 0,789 | 0,643 | 0,936 |
| 7 jours | C3AR1 + IL1R2 + IFNG + S100A9 + CX3CR1 + TAP2 | 0,787 | 0,642 | 0,932 |
| 7 jours | C3AR1 + IL1R2 + IFNG + S100A9 + CX3CR1 + TAP2 + CD3D | 0,784 | 0,638 | 0,93 |
| 7 jours | C3AR1 + IL1R2 + IFNG + S100A9 + CX3CR1 + TAP2 + CD3D + CTLA4 | 0,787 | 0,641 | 0,932 |
| 7 jours | C3AR1 + IL1R2 + IFNG + S100A9 + CX3CR1 + TAP2 + CTLA4 | 0,784 | 0,637 | 0,931 |
| 7 jours | C3AR1 + IL1R2 + IFNG + S100A9 + TAP2 | 0,759 | 0,605 | 0,913 |
| 7 jours | C3AR1 + IL1R2 + IFNG + S100A9 + TAP2 + CD3D | 0,765 | 0,613 | 0,916 |
| 7 jours | C3AR1 + IL1R2 + IFNG + S100A9 + TAP2 + CD3D + CTLA4 | 0,765 | 0,613 | 0,916 |
| 7 jours | C3AR1 + IL1R2 + IFNG + S100A9 + TAP2 + CTLA4 | 0,759 | 0,605 | 0,913 |
| 7 jours | C3AR1 + IL1R2 + IFNG + TAP2 | 0,753 | 0,596 | 0,91 |
| 7 jours | C3AR1 + IL1R2 + IFNG + TAP2 + CD3D | 0,753 | 0,596 | 0,91 |
| 7 jours | C3AR1 + IL1R2 + IFNG + TAP2 + CD3D + CTLA4 | 0,734 | 0,572 | 0,896 |
| 7 jours | C3AR1 + IL1R2 + IFNG + TAP2 + CTLA4 | 0,731 | 0,569 | 0,893 |
| 7 jours | C3AR1 + IL1R2 + S100A9 | 0,748 | 0,582 | 0,914 |
| 7 jours | C3AR1 + IL1R2 + S100A9 + CD3D | 0,753 | 0,594 | 0,913 |
| 7 jours | C3AR1 + IL1R2 + S100A9 + CD3D + CTLA4 | 0,751 | 0,594 | 0,907 |
| 7 jours | C3AR1 + IL1R2 + S100A9 + CIITA | 0,878 | 0,768 | 0,988 |
| 7 jours | C3AR1 + IL1R2 + S100A9 + CIITA + CD3D | 0,87 | 0,756 | 0,983 |
| 7 jours | C3AR1 + IL1R2 + S100A9 + CIITA + CD3D + CTLA4 | 0,892 | 0,792 | 0,992 |
| 7 jours | C3AR1 + IL1R2 + S100A9 + CIITA + CTLA4 | 0,87 | 0,757 | 0,983 |
| 7 jours | C3AR1 + IL1R2 + S100A9 + CIITA + TAP2 | 0,87 | 0,757 | 0,983 |
| 7 jours | C3AR1 + IL1R2 + S100A9 + CIITA + TAP2 + CD3D | 0,861 | 0,743 | 0,98 |
| 7 jours | C3AR1 + IL1R2 + S100A9 + CIITA + TAP2 + CD3D + CTLA4 | 0,875 | 0,768 | 0,983 |
| 7 jours | C3AR1 + IL1R2 + S100A9 + CIITA + TAP2 + CTLA4 | 0,87 | 0,755 | 0,985 |
| 7 jours | C3AR1 + IL1R2 + S100A9 + CTLA4 | 0,748 | 0,584 | 0,912 |
| 7 jours | C3AR1 + IL1R2 + S100A9 + CX3CR1 | 0,745 | 0,578 | 0,912 |
| 7 jours | C3AR1 + IL1R2 + S100A9 + CX3CR1 + CD3D | 0,759 | 0,603 | 0,915 |
| 7 jours | C3AR1 + IL1R2 + S100A9 + CX3CR1 + CD3D + CTLA4 | 0,762 | 0,609 | 0,915 |
| 7 jours | C3AR1 + IL1R2 + S100A9 + CX3CR1 + CIITA | 0,881 | 0,772 | 0,99 |
| 7 jours | C3AR1 + IL1R2 + S100A9 + CX3CR1 + CIITA + CD3D | 0,864 | 0,748 | 0,981 |
| 7 jours | C3AR1 + IL1R2 + S100A9 + CX3CR1 + CIITA + CD3D + CTLA4 | 0,886 | 0,784 | 0,989 |
| 7 jours | C3AR1 + IL1R2 + S100A9 + CX3CR1 + CIITA + CTLA4 | 0,873 | 0,76 | 0,985 |
| 7 jours | C3AR1 + IL1R2 + S100A9 + CX3CR1 + CIITA + TAP2 | 0,864 | 0,747 | 0,982 |
| 7 jours | C3AR1 + IL1R2 + S100A9 + CX3CR1 + CIITA + TAP2 + CD3D | 0,864 | 0,747 | 0,981 |
| 7 jours | C3AR1 + IL1R2 + S100A9 + CX3CR1 + CIITA + TAP2 + CD3D + CTLA4 | 0,875 | 0,768 | 0,982 |
| 7 jours | C3AR1 + IL1R2 + S100A9 + CX3CR1 + CIITA + TAP2 + CTLA4 | 0,873 | 0,759 | 0,986 |
| 7 jours | C3AR1 + IL1R2 + S100A9 + CX3CR1 + CTLA4 | 0,742 | 0,575 | 0,91 |
| 7 jours | C3AR1 + IL1R2 + S100A9 + CX3CR1 + TAP2 | 0,781 | 0,627 | 0,935 |
| 7 jours | C3AR1 + IL1R2 + S100A9 + CX3CR1 + TAP2 + CD3D | 0,765 | 0,607 | 0,922 |
| 7 jours | C3AR1 + IL1R2 + S100A9 + CX3CR1 + TAP2 + CD3D + CTLA4 | 0,77 | 0,618 | 0,923 |
| 7 jours | C3AR1 + IL1R2 + S100A9 + CX3CR1 + TAP2 + CTLA4 | 0,784 | 0,631 | 0,937 |
| 7 jours | C3AR1 + IL1R2 + S100A9 + TAP2 | 0,776 | 0,622 | 0,929 |
| 7 jours | C3AR1 + IL1R2 + S100A9 + TAP2 + CD3D | 0,756 | 0,598 | 0,914 |
| 7 jours | C3AR1 + IL1R2 + S100A9 + TAP2 + CD3D + CTLA4 | 0,77 | 0,618 | 0,922 |
| 7 jours | C3AR1 + IL1R2 + S100A9 + TAP2 + CTLA4 | 0,773 | 0,619 | 0,927 |
| 7 jours | C3AR1 + IL1R2 + TAP2 | 0,676 | 0,497 | 0,854 |
| 7 jours | C3AR1 + IL1R2 + TAP2 + CD3D | 0,679 | 0,501 | 0,856 |
| 7 jours | C3AR1 + IL1R2 + TAP2 + CD3D + CTLA4 | 0,698 | 0,525 | 0,871 |
| 7 jours | C3AR1 + IL1R2 + TAP2 + CTLA4 | 0,704 | 0,531 | 0,876 |
| 7 jours | C3AR1 + S100A9 | 0,742 | 0,577 | 0,908 |
| 7 jours | C3AR1 + S100A9 + CD3D | 0,74 | 0,58 | 0,9 |
| 7 jours | C3AR1 + S100A9 + CD3D + CTLA4 | 0,753 | 0,597 | 0,91 |
| 7 jours | C3AR1 + S100A9 + CIITA | 0,85 | 0,729 | 0,972 |
| 7 jours | C3AR1 + S100A9 + CIITA + CD3D | 0,856 | 0,736 | 0,976 |
| 7 jours | C3AR1 + S100A9 + CIITA + CD3D + CTLA4 | 0,859 | 0,743 | 0,974 |
| 7 jours | C3AR1 + S100A9 + CIITA + CTLA4 | 0,85 | 0,729 | 0,972 |
| 7 jours | C3AR1 + S100A9 + CIITA + TAP2 | 0,856 | 0,737 | 0,975 |
| 7 jours | C3AR1 + S100A9 + CIITA + TAP2 + CD3D | 0,85 | 0,729 | 0,972 |
| 7 jours | C3AR1 + S100A9 + CIITA + TAP2 + CD3D + CTLA4 | 0,864 | 0,751 | 0,977 |
| 7 jours | C3AR1 + S100A9 + CIITA + TAP2 + CTLA4 | 0,845 | 0,723 | 0,967 |
| 7 jours | C3AR1 + S100A9 + CTLA4 | 0,742 | 0,579 | 0,905 |
| 7 jours | C3AR1 + S100A9 + CX3CR1 | 0,737 | 0,57 | 0,904 |
| 7 jours | C3AR1 + S100A9 + CX3CR1 + CD3D | 0,756 | 0,594 | 0,919 |
| 7 jours | C3AR1 + S100A9 + CX3CR1 + CD3D + CTLA4 | 0,762 | 0,609 | 0,915 |
| 7 jours | C3AR1 + S100A9 + CX3CR1 + CIITA | 0,856 | 0,737 | 0,974 |
| 7 jours | C3AR1 + S100A9 + CX3CR1 + CIITA + CD3D | 0,875 | 0,765 | 0,985 |
| 7 jours | C3AR1 + S100A9 + CX3CR1 + CIITA + CD3D + CTLA4 | 0,87 | 0,759 | 0,981 |
| 7 jours | C3AR1 + S100A9 + CX3CR1 + CIITA + CTLA4 | 0,853 | 0,732 | 0,974 |
| 7 jours | C3AR1 + S100A9 + CX3CR1 + CIITA + TAP2 | 0,864 | 0,747 | 0,982 |
| 7 jours | C3AR1 + S100A9 + CX3CR1 + CIITA + TAP2 + CD3D | 0,867 | 0,751 | 0,983 |
| 7 jours | C3AR1 + S100A9 + CX3CR1 + CIITA + TAP2 + CD3D + CTLA4 | 0,867 | 0,756 | 0,978 |
| 7 jours | C3AR1 + S100A9 + CX3CR1 + CIITA + TAP2 + CTLA4 | 0,861 | 0,742 | 0,981 |
| 7 jours | C3AR1 + S100A9 + CX3CR1 + CTLA4 | 0,737 | 0,569 | 0,905 |
| 7 jours | C3AR1 + S100A9 + CX3CR1 + TAP2 | 0,789 | 0,638 | 0,941 |
| 7 jours | C3AR1 + S100A9 + CX3CR1 + TAP2 + CD3D | 0,778 | 0,625 | 0,932 |
| 7 jours | C3AR1 + S100A9 + CX3CR1 + TAP2 + CD3D + CTLA4 | 0,762 | 0,605 | 0,919 |
| 7 jours | C3AR1 + S100A9 + CX3CR1 + TAP2 + CTLA4 | 0,789 | 0,637 | 0,942 |
| 7 jours | C3AR1 + S100A9 + TAP2 | 0,776 | 0,623 | 0,929 |
| 7 jours | C3AR1 + S100A9 + TAP2 + CD3D | 0,759 | 0,601 | 0,917 |
| 7 jours | C3AR1 + S100A9 + TAP2 + CD3D + CTLA4 | 0,767 | 0,611 | 0,924 |
| 7 jours | C3AR1 + S100A9 + TAP2 + CTLA4 | 0,778 | 0,625 | 0,932 |
| 7 jours | C3AR1 + TAP2 | 0,676 | 0,497 | 0,855 |
| 7 jours | C3AR1 + TAP2 + CD3D | 0,69 | 0,512 | 0,867 |
| 7 jours | C3AR1 + TAP2 + CD3D + CTLA4 | 0,704 | 0,53 | 0,877 |
| 7 jours | C3AR1 + TAP2 + CTLA4 | 0,701 | 0,527 | 0,875 |

## Revendications

1. Procédé *in vitro* ou *ex vivo* pour déterminer un risque de complication choisi parmi en particulier un risque de décès ou un risque de survenue d'une infection associée aux soins, de préférence une infection nosocomiale, chez un patient, de préférence un patient au sein d'un établissement de santé, comprenant une étape de mesure de l'expression de C3AR1, dans un échantillon biologique dudit patient.

2. Procédé selon la revendication 1, **caractérisé en ce qu'**il comprend en outre une étape de mesure, dans l'échantillon biologique du patient, de l'expression :
- d'au moins un gène sélectionné parmi la famille des gènes codant pour des molécules impliquées dans le système immunitaire inné ; et/ou
- d'au moins un gène sélectionné parmi la famille des gènes codant pour des molécules du cycle cellulaire ; et/ou
- d'au moins un gène sélectionné parmi la famille des gènes codant pour des cytokines ; et/ou
- d'au moins un gène sélectionné parmi la famille des gènes codant pour des cytokines anti-inflammatoires ; et/ou
- d'au moins un gène sélectionné parmi la famille des gènes codant pour des récepteurs à cytokines pro-inflammatoires, localisés dans le chromosome 2, au niveau de la région 2q11-2q12 ; et/ou
- d'au moins un gène sélectionné parmi la famille des gènes codant pour des cytokines pro-inflammatoires ; et/ou
- d'au moins un gène sélectionné parmi la famille des gènes codant pour des molécules impliquées dans la formation du cytosquelette ; et/ou
- d'au moins un gène sélectionné parmi la famille des gènes codant pour des molécules impliquées dans l'expression et/ou la transcription de gènes ; et/ou
- d'au moins un gène sélectionné parmi la famille des gènes codant pour des facteurs de croissance ; et/ou
- d'au moins un gène sélectionné parmi la famille des gènes codant pour des molécules du métabolisme ; et/ou
- d'au moins un gène sélectionné parmi la famille des gènes codant pour des molécules impliquées dans le système immunitaire adaptatif ; et/ou
- d'au moins un gène sélectionné parmi la famille des gènes codant pour des molécules impliquées dans la transduction du signal ; et/ou
- d'au moins un gène sélectionné parmi la famille des gènes codant pour des molécules impliquées dans la modulation de la phase aigüe ; et/ou
- d'au moins un Rétrovirus Endogène Humain (HERV).

3. Procédé selon la revendication 1 ou 2, **caractérisé en ce qu'**il comprend une étape de mesure, dans l'échantillon biologique du patient, de l'expression :
- d'au moins un gène sélectionné parmi les gènes suivants : GNLY, S100A9, ADGRE3, CD177, CX3CR1, IFIH1, OAS2 et OAS3 ; et/ou
- d'au moins un gène sélectionné parmi les gènes suivants : CCNB1IP1, CDKN1A, CDKN1B, CDKN1C, CDKN2B, CDKN2C et CDKN2D ; et/ou
- d'au moins un gène sélectionné parmi les gènes suivants : IL15, IL2, MCP1(CCL2) et CXCL10 ; et/ou
- d'au moins un gène sélectionné parmi les gènes suivants : IL10 et IL1RN ; et/ou
- d'au moins un gène sélectionné parmi les gènes suivants : IL18R1, IL1R2, IL1R1 et IL18RAP ; et/ou
- d'au moins un gène sélectionné parmi les gènes suivants : IFNG, IL1B, IL17A, IL18, IL6 et TNF ; et/ou
- d'au moins un gène sélectionné parmi les gènes suivants : ARL14EP et GSN ; et/ou
- d'au moins un gène sélectionné parmi les gènes suivants : CIITA, DYRK2, GATA3, MDC1, NFKB1, RORC, STAT4, TBX21 et TDRD9 ; et/ou
- du gène CSF2 ; et/ou
- d'au moins un gène sélectionné parmi les gènes suivants : ALOX5, BPGM et TRAP1 ; et/ou
- d'au moins un gène sélectionné parmi les gènes suivants : CD40LG, CD3D, BTLA, CD274, CTLA4, ICOS, PDCD1, TNFSF4, CD74, FCGR1A, LILRB2 et TAP2 ; et/ou
- d'au moins un gène sélectionné parmi les gènes suivants : FLT1, HAVCR2, IL7R et ZAP70 ; et/ou
- du gène HP ; et/ou
- d'au moins un HERV sélectionné parmi les HERV suivants : 190665001-HERV0376, 220247002-HERV0797, 050286701-HERV0513, 130360601-HERV0808, 021456001-MALR1017, 170369402HE41, 121601801-HERV0492, 011052202-HERV1033 et 011052702-MALR1044.

4. Procédé selon la revendication 1, **caractérisé en ce qu'**il comprend une étape de mesure, dans l'échantillon biologique du patient, de l'expression d'au moins un gène sélectionné parmi les gènes suivants : GNLY, S100A9, ADGRE3, CD177, CX3CR1, OAS2, CCNB1IP1, IL10, IL1RN, IL1R2, IFNG, TNF, ARL14EP, CIITA, GATA3, MDC1, TDRD9, BPGM, CD3D, CD274, CTLA4, CD74, TAP2, IL7R, ZAP70 et HP.

5. Procédé selon la revendication 4, **caractérisé en ce qu'**il comprend une étape de mesure, dans l'échantillon biologique du patient, de l'expression d'au moins un gène sélectionné parmi les gènes suivants : S100A9, CD177, CX3CR1, IL1R2, IFNG, CIITA, CD3D, CTLA4, CD74, TAP2 et HP.

6. Procédé selon l'une des revendications 1 à 5, **caractérisé en ce que** l'échantillon biologique est un échantillon de sang, de préférence un échantillon de sang total.

7. Procédé selon l'une des revendications 1 à 6, **caractérisé en ce que** l'expression est mesurée au niveau ARN messager (ARNm).

8. Procédé selon l'une des revendications 1 à 7, **caractérisé en ce que** l'expression est mesurée par RT-PCR, de préférence par RT-qPCR.

9. Procédé selon l'une des revendications 1 à 7, **caractérisé en ce que** l'expression est mesurée par séquençage.

10. Procédé selon l'une des revendications 1 à 7, **caractérisé en ce que** l'expression est mesurée par hybridation.

11. Procédé selon l'une des revendications 7 à 10, **caractérisé en ce que** l'expression est normalisée par rapport à l'expression d'un ou plusieurs gènes de ménage.

12. Utilisation *in vitro* ou *ex vivo* :
- de moyens d'amplification et/ou de moyens de détection de l'expression de C3AR1, ou
- d'un kit comprenant de tels moyens d'amplification et/ou de détection,
pour déterminer un risque de survenue de complication chez un patient choisi parmi un risque de décès ou un risque de survenue d'une infection associée aux soins.

13. Utilisation *in vitro* ou *ex vivo* selon la revendication 12 pour déterminer le risque de survenue de complication chez un patient choisi parmi un risque de décès ou un risque de survenue d'une infection associée aux soins, comprenant également des moyens d'amplification et/ou de moyens de détection de l'expression d'au moins un autre gène sélectionné parmi ceux de la revendication 3.

## Patentansprüche

1. *In-vitro-* oder Ex-vivo-Verfahren zur Bestimmung des Risikos von Komplikationen, insbesondere des Risikos des Todes oder des Risikos des Auftretens einer mit dem Gesundheitswesen assoziierten Infektion, vorzugsweise einer nosokomialen Infektion, bei einem Patienten, vorzugsweise einem Patienten in einer Gesundheitseinrichtung, umfassend einen Schritt der Messung der Expression von C3AR1 in einer biologischen Probe des Patienten.

2. Verfahren nach Anspruch 1, **dadurch gekennzeichnet, dass** es ferner einen Schritt der Messung der Expression:
- mindestens eines Gens, ausgewählt aus der Familie von Genen, die Moleküle codieren, die am angeborenen Immunsystem beteiligt sind; und/oder
- mindestens eines Gens, ausgewählt aus der Familie von Genen, die Zellzyklus-Moleküle codieren; und/oder
- mindestens eines Gens, ausgewählt aus der Familie von Genen, die Zytokine codieren; und/oder
- mindestens eines Gens, ausgewählt aus der Familie von Genen, die antiinflammatorische Zytokine codieren; und/oder
- mindestens eines Gens, ausgewählt aus der Familie von Genen, die proinflammatorische Zytokin-Rezeptoren codieren, die sich auf Chromosom 2 in der Region 2q11-2q12 befinden; und/oder
- mindestens eines Gens, ausgewählt aus der Familie von Genen, die proinflammatorische Zytokine codieren; und/oder
- mindestens eines Gens, ausgewählt aus der Familie von Genen, die Moleküle codieren, die an der Bildung des Zytoskeletts beteiligt sind; und/oder
- mindestens eines Gens, ausgewählt aus der Familie von Genen, die Moleküle codieren, die an der Expression und/oder Transkription von Genen beteiligt sind; und/oder
- mindestens eines Gens, ausgewählt aus der Familie von Genen, die Wachstumsfaktoren codieren; und/oder
- mindestens eines Gens, ausgewählt aus der Familie von Genen, die Metabolismoleküle codieren; und/oder
- mindestens eines Gens, ausgewählt aus der Familie von Genen, die Moleküle codieren, die am adaptiven Immunsystem beteiligt sind; und/oder
- mindestens eines Gens, ausgewählt aus der Familie von Genen, die Moleküle codieren, die an der Signaltransduktion beteiligt sind; und/oder
- mindestens eines Gens, ausgewählt aus der Familie von Genen, die Moleküle codieren, die an der Modulation der akuten Phase beteiligt sind; und/oder
- mindestens eines humanen endogenen Retrovirus (HERV) in der biologischen Probe des Patienten umfasst.

3. Verfahren nach Anspruch 1 oder 2, **dadurch gekennzeichnet, dass** es einen Schritt der Messung der Expression:
- mindestens eines Gens, ausgewählt aus folgenden Genen: GNLY, S100A9, ADGRE3, CD177, CX3CR1, IFIH1, OAS2 und OAS3; und/oder
- mindestens eines Gens, ausgewählt aus folgenden Genen: CCNB1IP1, CDKN1A, CDKN1B, CDKN1C, CDKN2B, CDKN2C und CDKN2D; und/oder
- mindestens eines Gens, ausgewählt aus folgenden Genen: IL15, IL2, MCP1(CCL2) und CXCL10; und/oder
- mindestens eines Gens, ausgewählt aus folgenden Genen: IL10 und IL1RN; und/oder
- mindestens eines Gens, ausgewählt aus folgenden Genen: IL18R1, IL1R2, IL1R1 und IL18RAP; und/oder
- mindestens eines Gens, ausgewählt aus folgenden Genen: IFNG, IL1B, IL17A, IL18, IL6 und TNF; und/oder
- mindestens eines Gens, ausgewählt aus folgenden Genen: ARL14EP und GSN; und/oder
- mindestens eines Gens, ausgewählt aus folgenden Genen: CIITA, DYRK2, GATA3, MDC1, NFKB1, RORC, STAT4, TBX21 und TDRD9; und/oder
- des Gens CSF2; und/oder
- mindestens eines Gens, ausgewählt aus folgenden Genen: ALOX5, BPGM und TRAP1; und/oder
- mindestens eines Gens, ausgewählt aus folgenden Genen: CD40LG, CD3D, BTLA, CD274, CTLA4, ICOS, PDCD1, TNFSF4, CD74, FCGR1A, LILRB2 und TAP2; und/oder
- mindestens eines Gens, ausgewählt aus folgenden Genen: FLT1, HAVCR2, IL7R und ZAP70; und/oder
- des Gens HP; und/oder
- mindestens eines Gens, ausgewählt aus folgenden HERV: 190665001-HERV0376, 220247002-HERV0797, 050286701-HERV0513, 130360601-HERV0808, 021456001-MALR1017, 170369402HE41, 121601801-HERV0492, 011052202-HERV1033 und 011052702-MALR1044, in der biologischen Probe des Patienten umfasst.

4. Verfahren nach Anspruch 1, **dadurch gekennzeichnet, dass** es einen Schritt der Messung der Expression mindestens eines Gens in der biologischen Probe des Patienten umfasst, das aus folgenden Genen ausgewählt ist: GNLY, S100A9, ADGRE3, CD177, CX3CR1, OAS2, CCNB1IP1, IL10, IL1RN, IL1R2, IFNG, TNF, ARL14EP, CIITA, GATA3, MDC1, TDRD9, BPGM, CD3D, CD274, CTLA4, CD74, TAP2, IL7R, ZAP70 und HP.

5. Verfahren nach Anspruch 4, **dadurch gekennzeichnet, dass** es einen Schritt der Messung der Expression mindestens eines Gens in der biologischen Probe des Patienten umfasst, das aus folgenden Genen ausgewählt ist: S100A9, CD177, CX3CR1, IL1R2, IFNG, CIITA, CD3D, CTLA4, CD74, TAP2 und HP.

6. Verfahren nach einem der Ansprüche 1 bis 5, **dadurch gekennzeichnet, dass** die biologische Probe eine Blutprobe, bevorzugt eine Vollblutprobe, ist.

7. Verfahren nach einem der Ansprüche 1 bis 6, **dadurch gekennzeichnet, dass** die Expression auf mRNA-Ebene gemessen wird.

8. Verfahren nach einem der Ansprüche 1 bis 7, **dadurch gekennzeichnet, dass** die Expression mittels RT-PCR, bevorzugt mittels RT-qPCR, gemessen wird.

9. Verfahren nach einem der Ansprüche 1 bis 7, **dadurch gekennzeichnet, dass** die Expression mittels Sequenzierung gemessen wird.

10. Verfahren nach einem der Ansprüche 1 bis 7, **dadurch gekennzeichnet, dass** die Expression mittels Hybridisierung gemessen wird.

11. Verfahren nach einem der Ansprüche 7 bis 10, **dadurch gekennzeichnet, dass** die Expression auf die Expression eines oder mehrerer Haushaltsgene normalisiert wird.

12. *In-vitro-* oder *Ex-vivo*-Verwendung:
- von Mitteln zur Amplifikation und/oder Mitteln zum Nachweisen der Expression von C3AR1, oder
- eines Kits, das solche Mittel zur Amplifikation und/oder zum Nachweisen enthält, zur Bestimmung des Risikos des Auftretens von Komplikationen bei einem Patienten, das aus dem Risiko des Todes oder dem Risiko des Auftretens einer mit dem Gesundheitswesen assoziierten Infektion ausgewählt ist.

13. *In-vitro-* oder *Ex-vivo-*Verwendung nach Anspruch 12 zur Bestimmung des Risikos von Komplikationen bei einem Patienten, das aus dem Risiko des Todes oder dem Risiko des Auftretens einer mit dem Gesundheitswesen assoziierten Infektion ausgewählt ist, ferner umfassend Mittel zur Amplifikation und/oder Mittel zum Nachweisen der Expression mindestens eines weiteren Gens, das aus denen des Anspruchs 3 ausgewählt ist.

## Claims

1. An *in vitro* or *ex vivo* method for determining a risk of complication chosen from in particular a risk of death or a risk of incidence of a healthcare-associated infection, preferably a nosocomial infection, in a patient, preferably a patient within a healthcare facility, comprising a step of measuring the expression of C3AR1 in a biological sample from said patient.

2. The method as claimed in claim 1, **characterized in that** it additionally comprises a step of measuring, in the biological sample from the patient, the expression:
- of at least one gene selected from the family of genes encoding molecules involved in the innate immune system, and/or
- of at least one gene selected from the family of genes encoding molecules of the cell cycle, and/or
- of at least one gene selected from the family of genes encoding cytokines, and/or
- of at least one gene selected from the family of genes encoding antiinflammatory cytokines, and/or
- of at least one gene selected from the family of genes encoding pro-inflammatory cytokine receptors, located on chromosome 2 in the the region 2q11-2q12, and/or
- of at least one gene selected from the family of genes encoding pro-inflammatory cytokines, and/or
- of at least one gene selected from the family of genes encoding molecules involved in cytoskeleton formation, and/or
- of at least one gene selected from the family of genes encoding molecules involved in gene expression and/or transcription, and/or
- of at least one gene selected from the family of genes encoding growth factors, and/or
- of at least one gene selected from the family of genes encoding molecules of the metabolism, and/or
- of at least one gene selected from the family of genes encoding molecules involved in the adaptive immune system, and/or
- of at least one gene selected from the family of genes encoding molecules involved in signal transduction, and/or
- of at least one gene selected from the family of genes encoding molecules involved in modulation of the acute phase, and/or
- of at least one human endogenous retrovirus (HERV).

3. The method as claimed in claim 1 or 2, **characterized in that** it comprises a step of measuring, in the biological sample from the patient, the expression:
- of at least one gene selected from the following genes: GNLY, S100A9, ADGRE3, CD177, CX3CR1, IFIH1, OAS2, OAS3, and/or
- of at least one gene selected from the following genes: CCNB1IP1, CDKN1A, CDKN1B, CDKN1C, CDKN2B, CDKN2C, CDKN2D; and/or
- of at least one gene selected from the following genes: IL15, IL2, MCP1(CCL2), CXCL10, and/or
- of at least one gene selected from the following genes: IL10, IL1RN, and/or
- of at least one gene selected from the following genes: IL18R1, IL1R2, IL1R1 and IL18RAP, and/or
- of at least one gene selected from the following genes: IFNG, IL1B, IL17A, IL18, IL6, TNF, and/or
- of at least one gene selected from the following genes: ARL14EP, GSN, and/or
- of at least one gene selected from the following genes: CIITA, DYRK2, GATA3, MDC1, NFKB1, RORC, STAT4, TBX21, TDRD9, and/or
- of the CSF2 gene, and/or
- of at least one gene selected from the following genes: ALOX5, BPGM, TRAP1, and/or
- of at least one gene selected from the following genes: CD40LG, CD3D, BTLA, CD274, CTLA4, ICOS, PDCD1, TNFSF4, CD74, FCGR1A, LILRB2, TAP2, and/or
- of at least one gene selected from the following genes: FLT1, HAVCR2, IL7R, ZAP70, and/or
- of the HP gene, and/or
- of at least one HERV selected from the following HERVs: 190665001-HERV0376, 220247002-HERV0797, 050286701-HERV0513, 130360601-HERV0808, 021456001-MALR1017, 170369402HE41, 121601801-HERV0492, 011052202-HERV1033, 011052702-MALR1044.

4. The method as claimed in claim 1, **characterized in that** it comprises a step of measuring, in the biological sample from the patient, the expression of at least one gene selected from the following genes: GNLY, S100A9, ADGRE3, CD177, CX3CR1, OAS2, CCNB1IP1, IL10, IL1RN, IL1R2, IFNG, TNF, ARL14EP, CIITA, GATA3, MDC1, TDRD9, BPGM, CD3D, CD274, CTLA4, CD74, TAP2, IL7R, ZAP70, HP.

5. The method as claimed in claim 4, **characterized in that** it comprises a step of measuring, in the biological sample from the patient, the expression of at least one gene selected from the following genes: S100A9, CD177, CX3CR1, IL1R2, IFNG, CIITA, CD3D, CTLA4, CD74, TAP2, HP.

6. The method as claimed in one of claims 1 to 5, **characterized in that** the biological sample is a blood sample, preferably a total blood sample.

7. The method as claimed in one of claims 1 to 6, **characterized in that** the expression is measured at the messenger RNA (mRNA) level.

8. The method as claimed in one of claims 1 to 7, **characterized in that** the expression is measured by RT-PCR, preferably by RT-qPCR.

9. The method as claimed in one of claims 1 to 7, **characterized in that** the expression is measured by sequencing.

10. The method as claimed in one of claims 1 to 7, **characterized in that** the expression is measured by hybridization.

11. The method as claimed in one of claims 7 to 10, **characterized in that** the expression is normalized in relation to the expression of one or more housekeeping genes.

12. The *in vitro* or *ex vivo* use:
- of means for amplifying and/or means for detecting the expression of C3AR1, or
- of a kit comprising such amplification and/or detection means,
for determining a risk of incidence of complication in a patient chosen from a risk of death or a risk of incidence of a healthcare-associated infection,.

13. The in vitro or ex vivo use as claimed in claim 12 for determining a risk of complication chosen from a risk of death or a risk of incidence of a healthcare-associated infection, also comprising means for amplifying and/or detecting the expression of at least one other gene selected from those of claim 3.
